(19) Europäisches Patentamt
European Patent Office
Office européen des brevets

(11) EP 0 927 192 B1

(12) EUROPÄISCHE PATENTSCHRIFT

(45) Veröffentlichungstag und Bekanntmachung des Hinweises auf die Patenterteilung:
**12.05.2004 Patentblatt 2004/20**

(21) Anmeldenummer: **97938928.5**

(22) Anmeldetag: **08.09.1997**

(51) Int Cl.[7]: **C07K 5/06**, C07D 401/12, C07D 409/12, C07D 417/12, C07D 419/12

(86) Internationale Anmeldenummer:
**PCT/EP1997/004862**

(87) Internationale Veröffentlichungsnummer:
**WO 1998/011128 (19.03.1998 Gazette 1998/11)**

(54) **ABGEWANDELTE AMINOSÄUREN, DIESE VERBINDUNGEN ENTHALTENDE ARZNEIMITTEL UND VERFAHREN ZU IHRER HERSTELLUNG**

MODIFIED AMINOACIDS, PHARMACEUTICALS CONTAINING THESE COMPOUNDS AND METHOD FOR THEIR PRODUCTION

AMINOACIDES MODIFIES, MEDICAMENTS CONTENANT CES COMPOSES ET LEUR PROCEDE DE PRODUCTION

(84) Benannte Vertragsstaaten:
**AT BE CH DE DK ES FI FR GB GR IE IT LI LU MC NL PT SE**
Benannte Erstreckungsstaaten:
**LT LV RO SI**

(30) Priorität: **10.09.1996 DE 19636623**
**14.05.1997 DE 19720011**

(43) Veröffentlichungstag der Anmeldung:
**07.07.1999 Patentblatt 1999/27**

(60) Teilanmeldung:
**04003959.6**

(73) Patentinhaber: **Boehringer Ingelheim Pharma GmbH & Co.KG**
**55216 Ingelheim am Rhein (DE)**

(72) Erfinder:
- **RUDOLF, Klaus**
  **D-88400 Biberach (DE)**
- **EBERLEIN, Wolfgang**
  **D-88400 Biberach (DE)**
- **ENGEL, Wolfhard**
  **D-88400 Biberach (DE)**
- **PIEPER, Helmut**
  **D-88400 Biberach (DE)**
- **DOODS, Henri**
  **D-88447 Warthausen (DE)**
- **HALLERMAYER, Gerhard**
  **D-88437 Maselheim (DE)**
- **ENTZEROTH, Michael**
  **D-88447 Warthausen (DE)**
- **WIENEN, Wolfgang**
  **D-88400 Biberach (DE)**

(74) Vertreter: **Laudien, Dieter, Dr.**
**Boehringer Ingelheim Zentrale GmbH**
**ZA Patente**
**Postfach 200**
**55216 Ingelheim am Rhein (DE)**

(56) Entgegenhaltungen:
**EP-A- 0 284 632** **EP-A- 0 298 135**
**EP-A- 0 415 413** **EP-A- 0 706 999**
**EP-A- 0 723 774** **WO-A-96/04928**
**WO-A-96/15148** **US-A- 4 826 870**
**US-A- 4 873 342** **US-A- 5 310 743**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Gegenstand der vorliegenden Erfindung sind folgende abgewandelte Aminosäuren

(1) 1-[$N^2$-[3,5-Dibrom-N-[[4-(3,4-dihydro-2(1H)-oxochinazolin-3-yl)-1-piperidinyl]carbonyl]-D-tyrosyl]-L-lysyl]-4-(4-pyridinyl)-piperazin (lfd. Nr. 154) und

(2) 1-[4-Amino-3,5-dibrom-N-[[4-(2,3,4,5-tetrahydro-2(1H)-oxo-1,3-benzodiazepin-3-yl)-1-piperidinyl]carbonyl]-D-phenylalanyl]-4-(1-piperidinyl)-piperidin (lfd. Nr. 231),

deren Tautomere, deren Diastereomere, deren Enantiomere, deren Gemische und deren Salze, insbesondere deren physiologisch verträgliche Salze mit anorganischen oder organischen Säuren oder Basen, diese Verbindungen enthaltende Arzneimittel, deren Verwendung und Verfahren zu ihrer Herstellung.

Die Verbindungen weisen wertvolle pharmakologische Eigenschaften auf, die auf ihre selektiven CGRP-antagonistischen Eigenschaften zurückgehen.

Das humane CGRP Rezeptor-Polypeptid und die dafür kodierende DNA sind in der WO 96/04928 beschrieben. Ferner wird erwähnt, daß CGRP Rezeptor-Antagonisten nützlich für die Behandlung unter anderem von Krebs, Arthritis, Schmerz, Diabetes, Migräne und entzündlicher Erkrankungen sein können.

In der EP-A-0 723 774 wird die Verwendung von CGRP-Antagonisten für die Behandlung von empfindlicher Haut, insbesondere von nicht-allergischen Hautirritationen und Erythemen, beschrieben.

In der WO 96/15148 werden bereits Wachstumshormon freisetzende Peptide und Peptidomimetika beschrieben. Die Verbindungen sind geeignet zur Wachstumsförderung und, in Kombination mit IGF-1 (insulin like growth factor-1), zur Behandlung des Diabetes Typ II.

In der EP 706 999 werden Dipiperidin-Derivate beschrieben, die als Plättchenaggregations-Inhibitoren, Krebsmetastasen-Inhibitoren, Wundheilmittel oder Knochenresorptions-Inhibitoren verwendbar sind.

In der US-A-4,826,870 und US-A-4,873,342 werden Pyrrolidinamid-Derivate von Acylaminosäuren beziehungsweise Dipeptid-Derivate beschrieben, die aufgrund ihrer anti-Prolyl-Endopeptidase Aktivität zur Behandlung der Amnesie eingesetzt werden können.

In der US-A-5,310,743 werden 1-Acylpiperidin-Derivate mit Substanz-P-antagonistischen Eigenschaften beschrieben. Die Verbindungen können zur Behandlung von Schmerz, Migräne und einigen Störungen des Zentralnervensystems, wie Angstzuständen, Schizophrenie und Depressionen, sowie zur Behandlung der Parkinsonschen Krankheit und inflammatorischer Störungen eingesetzt werden.

In der EP-A-0 415 413 werden Aminosäureamid-Derivate als Inhibitoren von Acylcoenzym A: Cholesterol Acyltransferase beschrieben. Die Verbindungen sind verwendbar zur Behandlung der Hypercholesterolämie and Atherosklerose.

In der EP-A-0 284 632 und EP-A-0 298 135 werden Phenylalanin-Derivate als Proteinase-Inhibitoren beschrieben.

Die erfindungsgemäßen Verbindungen werden nach prinzipiell bekannten Methoden hergestellt, wobei besonders aus der Peptidchemie (siehe z. B. Houben-Weyl, Methoden der Organischen Chemie, Bd. 15/2) abgeleitete Verfahren angewandt werden. Als Aminoschutzgruppen können die in Houben-Weyl, Methoden der Organischen Chemie, Bd. 15/1, beschriebenen verwendet werden, wobei Urethanschutzgruppen, wie z. B. die Fluorenylmethoxycarbonyl-, Phenylmethoxycarbonyl- oder tert.-Butyloxycarbonylgruppe bevorzugt werden. Eventuell in den Resten der erfindungsgemäßen Verbindungen oder in deren Vorstufen vorhandene funktionelle Gruppen werden zur Verhinderung von Nebenreaktionen durch geeignete Schutzgruppen (siehe z. B.: G.B. Fields et al., Int. J. Peptide Protein Res. 35, 161 (1990); T.W. Greene, Protective Groups in Organic Synthesis) zusätzlich geschützt. Als derartige seitenkettengeschützte Aminosäuren seien Lys(Boc), Lys(Z) und Lys(Cl-Z) erwähnt, die, eventuell in Form von Derivaten, in der Regel käuflich sind. Dabei ist besonders darauf zu achten,daß für den Schutz der α-Amino- und der Seitenketten-Aminogruppe sogenannte orthogonale Kombinationen von Schutzgruppen verwendet werden, z. B.:

| Schutz des N (Seitenkette) | $N^{\alpha}$-Schutz |
|---|---|
| p-Toluolsulfonyl | Phenylmethoxycarbonyl<br>tert.Butyloxycarbonyl |

(fortgesetzt)

| Schutz des N (Seitenkette) | $N^{\alpha}$-Schutz |
|---|---|
| Phenylmethoxycarbonyl | (4-Methoxyphenyl)methoxycarbonyl<br>tert. Butoxycarbonyl<br>Adamantyloxycarbonyl<br>Biphenylylisopropyloxycarbonyl<br>Isonicotinoyloxycarbonyl<br>o-Nitrophenylsulfenyl<br>Formyl |
| tert. Butoxycarbonyl | Phenylmethoxycarbonyl<br>p-Toluolsulfonyl<br>o-Nitrophenylsulfenyl<br>Biphenylylisopropyloxycarbonyl<br>9-Fluorenylmethoxycarbonyl |
| Acetyl, Trifluoracetyl, Formyl, (2-Chlorphenyl)methoxycarbonyl, (4-Chlorphenyl)methoxycarbonyl, 4-(Nitrophenyl)methoxycarbonyl, Phthaloyl | tert.Butyloxycarbonyl |

Statt seitenkettenständige Aminogruppen zu schützen, können auch Präcursor-Funktionen tragende, in der Seitenkette insbesondere durch Nitro oder Cyan substituierte Aminosäuren bzw. deren Derivate eingesetzt werden, beispielsweise 5-Cyannorvalin.

Zur eigentlichen Kupplung werden die aus der Peptidchemie bekannten Methoden (siehe z. B. Houben-Weyl, Methoden der Organischen Chemie, Bd. 15/2) angewandt. Bevorzugt verwendet werden Carbodiimide, wie z. B. Dicyclohexylcarbodiimid (DCC), Diisopropylcarbodiimid (DIC) oder Ethyl-(3-dimethylaminopropyl)-carbodiimid, O-(1H-Benzotriazol-1-yl)-N,N-N',N'-tetramethyluroniumhexafluorophosphat (HBTU) oder -tetrafluoroborat (TBTU) oder 1H-Benzotriazol-1-yl-oxy-tris-(dimethylamino)-phosphoniumhexafluorophosphat (BOP). Durch Zugabe von 1-Hydroxybenzotriazol (HOBt) oder von 3-Hydroxy-4-oxo-3,4-dihydro-1,2,3-benzotriazin (HOObt) kann die Racemisierung gewünschtenfalls zusätzlich unterdrückt bzw. die Reaktionsgeschwindigkeit gesteigert werden. Die Kupplungen werden normalerweise mit äquimolaren Anteilen der Kupplungskomponenten sowie des Kupplungsreagenz in Lösemitteln wie Dichlormethan, Tetrahydrofuran, Acetonitril, Dimethylformamid (DMF), Dimethylacetamid (DMA), N-Methylpyrrolidon (NMP) oder Gemischen aus diesen und bei Temperaturen zwischen -30 und +30°C, bevorzugt -20 und +20°C, durchgeführt.

Sofern erforderlich, wird als zusätzliche Hilfsbase N-Ethyl-diisopropylamin (DIEA) (Hünig-Base) bevorzugt.

Als weiteres Kupplungsverfahren zur Synthese der erfindungsgemäßen Verbindungen wurde das sogenannte "Anhydridverfahren" (siehe auch: M. Bodanszky, "Peptide Chemistry", Springer-Verlag 1988, S. 58-59; M. Bodanszky, "Principles of Peptide Synthesis", Springer-Verlag 1984, S. 21-27) eingesetzt. Bevorzugt wird das "gemischte Anhydridverfahren" in der Variante nach Vaughan (J.R. Vaughan Jr., J. Amer. Chem.Soc. 73, 3547 (1951)), bei der unter Verwendung von Chlorkohlensäureisobutylester in Gegenwart von Basen, wie 4-Methylmorpholin oder 4-Ethylmorpholin, das gemischte Anhydrid aus der zu kuppelnden, gegebenenfalls $N^2$-geschützten α-Aminosäure und dem Kohlensäuremonoisobutylester erhalten wird. Die Herstellung dieses gemischten Anhydrids und die Kupplung mit Aminen erfolgt im Eintopfvefahren, unter Verwendung der vorstehend genannten Lösemittel und bei Temperaturen zwischen -20 und +20°C, bevorzugt 0 und +20°C.

Eventuelle in den Seitenketten von α-Aminosäurepartialstrukturen vorhandene Schutzgruppen werden nach Aufbau des N- und C-terminal substituierten Aminosäurederivats abschließend mit geeigneten, im Prinzip gleichfalls literaturbekannten Reagenzien abgespalten, und zwar Arylsulfonyl- und Hetarylsulfonyl-Schutzgruppen bevorzugt acidolytisch, d. h. durch Einwirkung von starken Säuren, bevorzugt Trifluoressigsäure, Nitro- und Arylmethoxycarbonylschutzgruppen hydrogenolytisch, beispielsweise mit Wasserstoff in Gegenwart von Palladiummohr und unter Verwendung von Eisessig als Lösemittel. Enthält das Substrat gegen Hydrogenolyse empfindliche Funktionen, z. B. Halogenatome, wie Chlor, Brom oder Iod, eine Phenylmethanol- oder Hetarylmethanol-Funktion oder eine andere Benzylheteroatom-Bindung, insbesondere eine Benzyl-Sauerstoff-Bindung, so gelingt die Abspaltung der Nitrogruppe auch nichthydrogenolytisch, z. B. mit Zink/2N Trifluoressigsäure (siehe auch: A. Turan, A. Patthy und S. Bajusz, Acta Chim.

Acad. Sci. Hung, Tom. 85 (3), 327-332 [1975]; C.A. 83, 206526y [1975]), mit Zinn(II)-chlorid in 60%iger wässeriger Ameisensäure (siehe auch: SUNSTAR KK, JA-A-3271-299), mit Zink in Gegenwart von Essigsäure (siehe auch: A. Malabarba, P. Ferrari, G. Cietto, R. Pallanza und M. Berti, J. Antibiot. 42 (12), 1800-1816 (1989)) oder überschüssigem wässerigem 20%igem Titan(III)-chlorid in wässerigem Methanol und in Gegenwart von wässerigem Ammoniumacetat-Puffer bei 24°C (siehe auch: R.M. Freidinger, R. Hirschmann und D.F. Veber, J. Org. Chem. 43 (25), 4800-4803 [1978]).

In der Seitenkette der α-Aminosäure gegebenenfalls vorhandene Präcursor-Funktionen können gleichfalls abschließend durch Hydrogenolyse in die gewünschten Aminofunktionen übergeführt werden; Nitroalkylgruppen ergeben dabei unter dem Chemiker geläufigen Bedingungen Aminoalkylgruppen, die Cyangruppe geht in'die Aminomethyl-Gruppe über.

Die erfindungsgemäßen abgewandelten Aminosäuren enthalten wenigstens ein Chiralitätszentrum. Die Trennung von Diastereomeren gelingt auf Grund ihrer unterschiedlichen physikochemischen Eigenschaften, z.B. durch fraktionierte Kristallisation aus geeigneten Lösemitteln, durch Hochdruckflüssigkeits- oder Säulenchromatographie unter Verwendung chiraler oder bevorzugt achiraler stationärer Phasen.

Die Trennung von Racematen gelingt beispielsweise durch HPLC an geeigneten chiralen stationären Phasen (z. B. Chiral AGP, Chiralpak AD). Racemate, die eine basische Funktion enthalten, lassen sich auch über die diastereomeren, optisch aktiven Salze trennen, die bei Umsetzung mit einer optisch aktiven Säure, beispielsweise (+)-oder (-)-Weinsäure, (+)- oder (-)-Diacetylweinsäure, (+)- oder (-)-Monomethyltartrat oder (+)-Camphersulfonsäure, entstehen.

Nach einem üblichen Verfahren zur Isomerentrennung wird das Racemat einer erfindungsgemäßen Verbindung mit einer der vorstehend angegebenen optisch aktiven Säuren in äquimolarer Menge in einem Lösungsmittel umgesetzt und die erhaltenen kristallinen, diastereomeren, optisch aktiven Salze unter Ausnutzung ihrer verschiedenen Löslichkeit getrennt. Diese Umsetzung kann in jeder Art von Lösungsmitteln durchgeführt werden, solange sie einen ausreichenden Unterschied hinsichtlich der Löslichkeit der Salze aufweisen. Vorzugsweise werden Methanol, Ethanol oder deren Gemische, beispielsweise im Volumenverhältnis 50:50, verwendet. Sodann wird jedes der optisch aktiven Salze in Wasser gelöst, mit einer Base, wie Natriumcarbonat oder Kaliumcarbonat, Natronlauge oder Kalilauge neutralisiert und dadurch die entsprechende freie Verbindung in der (+)- oder (-)-Form erhalten.

Die erfindungsgemäßen Verbindungen können, insbesondere für pharmazeutische Anwendungen, in ihre physiologisch verträglichen Salze mit anorganischen oder organischen Säuren, übergeführt werden. Als Säuren kommen hierfür beispielsweise Salzsäure, Bromwasserstoffsäure, Phosphorsäure, Salpetersäure, Schwefelsäure, Methansulfonsäure, p-Toluolsulfonsäure, Essigsäure, Fumarsäure, Bernsteinsäure, Milchsäure, Mandelsäure, Äpfelsäure, Zitronensäure, Weinsäure oder Maleinsäure in Betracht.

Die erfindungsgemäßen Verbindungen und deren physiologisch verträglichen Salze besitzen CGRP-antagonistische Eigenschaften und zeigen gute Affinitäten in CGRP-Rezeptorbindungsstudien. Die Verbindungen weisen in den nachstehend beschriebenen pharmakologischen Testsystemen CGRP-antagonistische Eigenschaften auf.

Zum Nachweis der Affinität der erfindungsgemäßen Verbindungen zu humanen CGRP-Rezeptoren und ihrer antagonistischen Eigenschaften wurden die folgenden Versuche durchgeführt:

A. Bindungsstudien mit (den humanen CGRP-Rezeptor exprimierenden SK-N-MC-Zellen

SK-N-MC-Zellen werden in "Dulbecco's modified Eagle Medium" kultiviert. Das Medium konfluenter Kulturen wird entfernt. Die Zellen werden zweimal mit PBS-Puffer (Gibco 041-04190 M) gewaschen, durch Zugabe von PBS-Puffer, versetzt mit 0.02% EDTA, abgelöst und durch Zentrifugation isoliert. Nach Resuspension in 20 ml "Balanced Salts Solution" [BSS (in mM): NaCl 120, KCl 5.4, $NaHCO_3$ 16.2, $MgSO_4$ 0.8, $NaHPO_4$ 1.0, $CaCl_2$ 1.8, D-Glucose 5.5, HEPES 30, pH7.40] werden die Zellen zweimal bei 100 x g zentrifugiert und in BSS resuspendiert. Nach Bestimmung der Zellzahl werden die Zellen mit Hilfe eines Ultra-Turrax homogenisiert und für 10 Minuten bei 3000 x g zentrifugiert. Der Überstand wird verworfen und das Pellet in Tris-Puffer (10 mM Tris, 50 mM NaCl, 5 mM $MgCl_2$, 1 mM EDTA, pH 7.40), angereichert mit 1% Rinderserüm-Albumin und 0.1% Bacitracin, rezentrifugiert und resuspendiert (1 ml / 1000000 Zellen). Das Homogenat wird bei -80°C eingefroren. Die Membranpräparationen sind bei diesen Bedingungen für mehr als 6 Wochen stabil.

Nach Auftauen wird das Homogenat 1:10 mit Assay-Puffer (50 mM Tris, 150 mM NaCl, 5 mM $MgCl_2$, 1 mM EDTA, pH 7.40) verdünnt und 30 Sekunden lang mit einem Ultra-Turrax homogenisiert. 230 µl des Homogenats werden für 180 Minuten bei Raumtemperatur mit 50 pM $^{125}$I-Iodotyrosyl-Calcitonin-Gene-Related Peptide (Amersham) und ansteigenden Konzentrationen der Testsubstanzen in einem Gesamtvolumen von 250 µl inkubiert. Die Inkubation wird durch rasche Filtration durch mit Polyethylenimin (0.1%) behandelte GF/B-Glasfaserfilter mittels eines Zellharvesters beendet. Die an Protein gebundene Radioaktivität wird mit Hilfe eines Gammacounters bestimmt. Als nichtspezifische Bindung wird die gebundene Radioaktivität nach Gegenwart von 1 µM humanem CGRP-alpha während der Inkubation definiert.

Die Analyse der Konzentrations-Bindungskurven erfolgt mit Hilfe einer computergestützten nichtlinearen Kurvenanpassung.

Die erfindungsgemäßen Verbindungen zeigen in dem beschriebenen Test $IC_{50}$-Werte ≤ 10000 nM.

B. CGRP-Antagonismus in SK-N-MC-Zellen

SK-N-MC-Zellen (1 Mio. Zellen) werden zweimal mit 250 µl Inkubationspuffer (Hanks' HEPES, 1 mM 3-Isobutyl-1-methylxanthin, 1% BSA, pH 7.4) gewaschen und bei 37°C für 15 Minuten vorinkubiert. Nach Zugabe von CGRP (10 µl) als Agonist in steigenden Konzentrationen ($10^{-11}$ bis $10^{-6}$ M) bzw. zusätzlich von Substanz in 3 bis 4 verschiedenen Konzentrationen wird.nochmals 15 Minuten inkubiert.

Intrazelluläres cAMP wird anschließend durch Zugabe von 20 µl 1M HCl und Zentrifugation (2000 x g, 4°C für 15 Minuten) extrahiert. Die Überstände werden in flüssigem Stickstoff eingefroren und bei -20°C gelagert.

Die cAMP-Gehalte der Proben werden mittels Radioimmunassay (Fa. Amersham) bestimmt und die $pA_2$-Werte antagonistisch wirkender Substanzen graphisch ermittelt.

Die erfindungsgemäßen Verbindungen zeigen in dem beschriebenen in-vitro-Testmodell CGRP-antagonistische Eigenschaften in einem Dosisbereich zwischen $10^{-11}$ bis $10^{-5}$ M.

Auf Grund ihrer pharmakologischen Eigenschaften eignen sich die erfindungsgemäßen Verbindungen und deren Salze mit physiologisch verträglichen Säuren somit zur akuten und prophylaktischen Behandlung von Kopfschmerzen, insbesondere Migräne- bzw. Cluster-Kopfschmerz. Weiterhin beeinflussen die erfindungsgemäßen Verbindungen auch die folgenden Erkrankungen positiv: Nicht-insulinabhängigen Diabetes mellitus ("NIDDM"), cardiovaskuläre Erkrankungen, Erkrankungen der Haut, insbesondere thermische und strahlenbedingte Hautschäden inclusive Sonnenbrand, entzündliche Erkrankungen, z.B. entzündliche Gelenkerkrankungen (Arthritis), entzündliche Lungenerkrankungen, allergische Rhinitis, Asthma, Erkrankungen, die mit einer überschießenden Gefäßerweiterung und dadurch bedingter verringerter Gewebedurchblutung einhergehen, z.B. Schock und Sepsis, sowie Morphintoleranz. Darüberhinaus zeigen die erfindungsgemäßen Verbindungen eine lindernde Wirkung auf Schmerzzustände im allgemeinen.

Die zur Erzielung einer entsprechenden Wirkung erforderliche Dosierung beträgt zweckmäßigerweise bei intravenöser oder subcutaner Gabe 0,0001 bis 3 mg/kg Körpergewicht, vorzugsweise 0,01 bis 1 mg/kg Körpergewicht, und bei oraler, nasaler oder inhalativer Gabe 0,01 bis 10 mg/kg Körpergewicht, vorzugsweise 0,1 bis 10 mg/kg Körpergewicht, jeweils 1 bis 3 x täglich.

Hierzu lassen sich die erfindungsgemäßen Verbindungen, gegebenenfalls in Kombination mit anderen Wirksubstanzen, wie z.B. Antiemetica, Prokinetica, Neuroleptica, Antidepressiva, Neurokinin-Antagonisten, Anticonvulsiva, Histamin-H1-Rezeptorantagonisten, Antimuscarinika, β-Blockern, α-Agonisten und α-Antagonisten, Ergotalkaloiden, schwachen Analgetica, nichtsteroidalen Antiphlogistica, Corticosteroiden, Calcium-Antagonisten, $5\text{-}HT_{1D}$-Agonisten oder anderen Antimigränemitteln, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen, Lösungen, Dosieraerosole oder Zäpfchen einarbeiten.

Für die oben erwähnten Kombinationen kommen somit als weitere Wirksubstanzen beispielsweise Meloxicam, Ergotamin, Dihydroergotamin, Metoclopramid, Domperidon, Diphenhydramin, Cyclizin, Promethazin, Chlorpromazin, Dexamethason, Flunarizin, Dextropropoxyphen, Meperidin, Propranolol, Nadolol, Atenolol, Clonidin, Indoramin, Carbamazepin, Phenytoin, Valproat, Amitryptilin, Lidocain, Diltiazem oder Sumatriptan und andere $5\text{-}HT_{1D}$-Agonisten wie z.B. Naratriptan, Zolmitriptan, Avitriptan, Rizatriptan und Eletriptan in Betracht. Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 20 bis 100 mg Sumatriptan.

Ein weiterer Gegenstand der Erfindung ist die Verwendung der erfindungsgemäßen Verbindungen als wertvolle Hilfsmittel zur Erzeugung und Reinigung (Affinitätschromatographie) von Antikörpern sowie, nach geeigneter radioaktiver Markierung, beispielsweise durch direkte Markierung mit $^{125}I$ oder $^{131}I$ oder durch Tritiierung geeigneter Vorstufen, beispielsweise durch Ersatz von Halogenatomen durch Tritium, in RIA- und ELISA-Assays und als diagnostische bzw. analytische Hilfsmittel in der Neurotransmitter-Forschung.

Die nachfolgenden Beispiele sollen die Erfindung näher erläutern:

Für die oben erwähnten Kombinationen kommen somit als weitere Wirksubstanzen beispielsweise Meloxicam, Ergotamin, Dihydroergotamin, Metoclopramid, Domperidon, Diphenhydramin, Cyclizin, Promethazin, Chlorpromazin, Dexamethason, Flunarizin, Dextropropoxyphen, Meperidin, Propranolol, Nadolol, Atenolol, Clonidin, Indoramin, Carbamazepin, Phenytoin, Valproat, Amitryptilin, Lidocain, Diltiazem oder Sumatriptan und andere $5\text{-}HT_{1D}$-Agonisten wie z.B. Naratriptan, Zolmitriptan, Avitriptan, Rizatriptan und Eletriptan in Betracht. Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 20 bis 100 mg Sumatriptan.

Ein weiterer Gegenstand der Erfindung ist die Verwendung der Verbindungen der allgemeinen Formel I als wertvolle Hilfsmittel zur Erzeugung und Reinigung (Affinitätschromatographie) von Antikörpern sowie, nach geeigneter radioaktiver Markierung, beispielsweise durch direkte Markierung mit $^{125}I$ oder $^{131}I$ oder durch Tritiierung geeigneter Vorstufen, beispielsweise durch Ersatz von Halogenatomen durch Tritium, in RIA- und ELISA-Assays und als diagnostische bzw. analytische Hilfsmittel in der Neurotransmitter-Forschung.

Die nachfolgenden Beispiele sollen die Erfindung näher erläutern:

Vorbemerkungen:

Für alle Verbindungen liegen befriedigende Elementaranalysen, IR-, UV-, $^{1}H$-NMR und in der Regel auch Massenspekten vor. Wenn nicht anders angegeben, wurden $R_f$-Werte unter Verwendung von DC-Fertigplatten Kieselgel 60 $F_{254}$ (E. Merck, Darmstadt, Artikel-Nr. 5729) ohne Kammersättigung bestimmt. Falls nähere Angaben zur Konfiguration fehlen, bleibt offen, ob es sich um reine Enantiomere handelt oder ob partielle oder gar völlige Racemisierung eingetreten ist. Zur Chromatographie wurden die folgenden Fließmittel bzw. Fließmittelgemische verwendet:

FM1 = Dichlormethan/Cyclohexan/Methanol/Ammoniak 7/1.5/1.5/0.2 (v/v/v/v)
FM2 = Dichlormethan/Methanol/Ammoniak 7.5/2.5/0.5 (v/v/v)
FM3 = Dichlormethan/Methanol 8/2 (v/v)
FM4 = Dichlormethan/Essigester/Methanol/Cyclohexan/konz. wässeriges Ammoniak = 59/25/7,5/7,5/1 (v/v/v/v/v)
FM5 = Essigester/Dichlormethan = 7/3 (v/v)
FM6 = Essigester/Petrolether = 1/1 (v/v)
FM7 = Dichlormethan/Methanol/konz. wässeriges Ammoniak = 80/20/1 (v/v/v)

In der Versuchsbeschreibung werden die folgenden Abkürzungen verwendet:

Fp. : Schmelzpunkt
(Z) : Zersetzung)
DIEA: N,N-Diisopropyl-ethylamin
Boc: 1,1-Dimethylethoxy)carbonyl
TBTU: 2-(1H-Benzotriazol-1-yl)-1,1,3,3-tetramethyluroniumtetrafluoroborat

HOBt: 1-Hydroxybenzotriazol-hydrat
CDT: 1,1'-Carbonyldi(1,2,4-triazol)
THF: Tetrahydrofuran
DMF: Dimethylformamid
Fmoc: (9-Fluorenylmethoxy)carbonyl
EE: Essigsäureethylester
PE: Petrolether
LM: Lösemittel
Lfd. Nr.: Laufende Nummer

Die Bedeutung der in den Beispielen verwendeten aus Buchstaben und Zahlen zusammengesetzten Symbole ergibt sich aus der folgenden Übersicht:

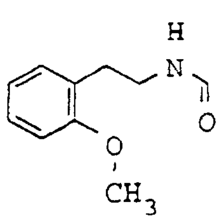
N1

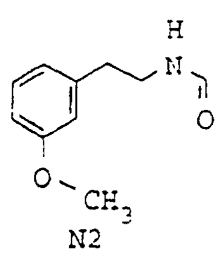
N2

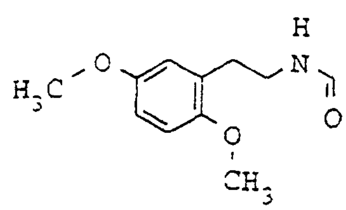
N3

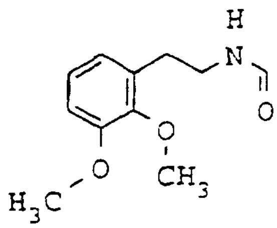

N4

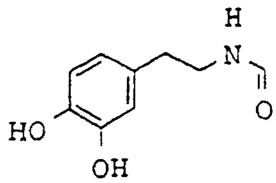

N5

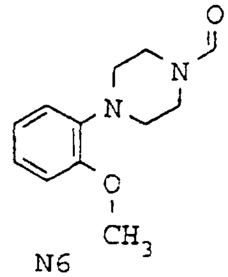

N6

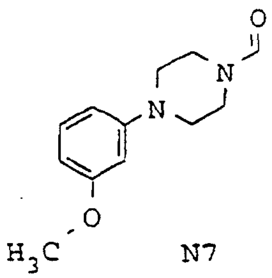

N7

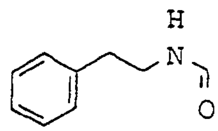

N8

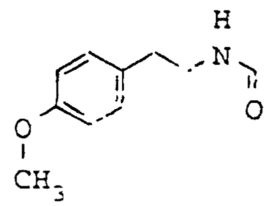

N9

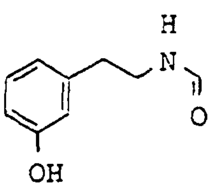

N10

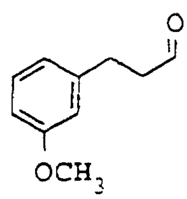

N11

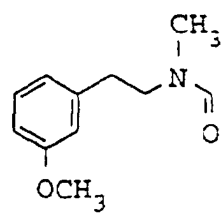

N12

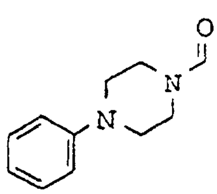

N13

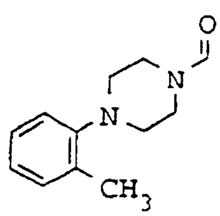

N14

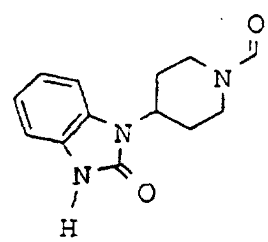

N15

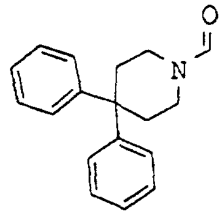

N16

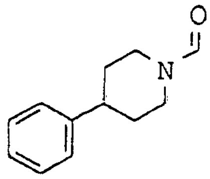

N17

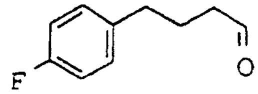

N18

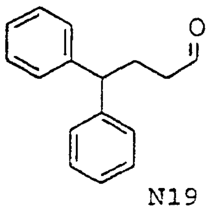

N19

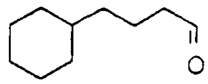

N20

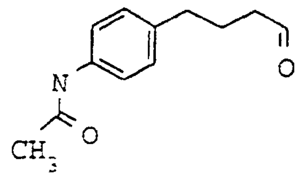

N21

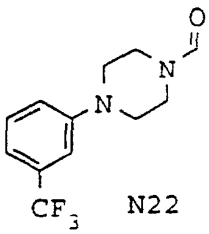

N22

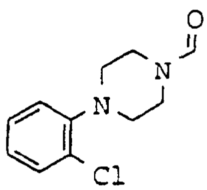

N23

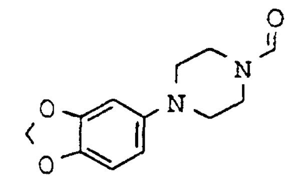

N24

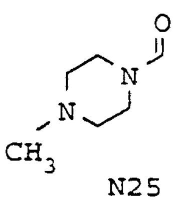

N25

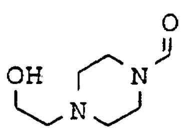

N26

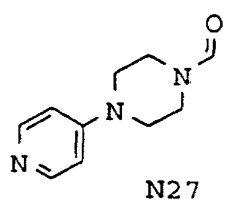

N27

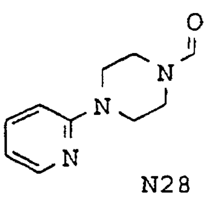

N28

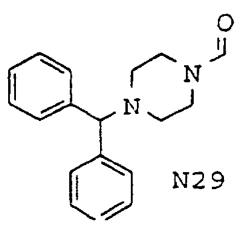

N29

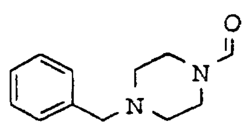

N30

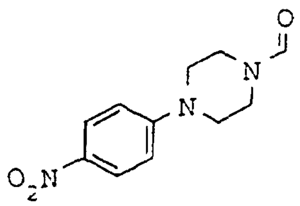
N31

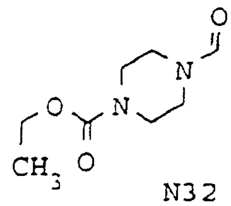
N32

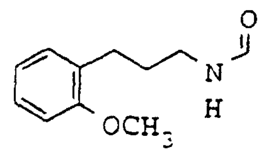
N33

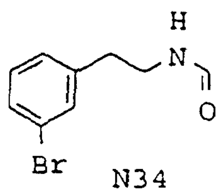
N34

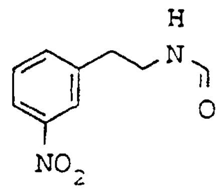
N35

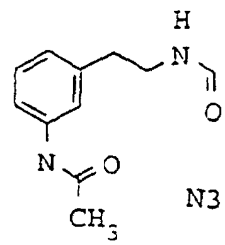
N36

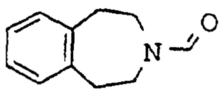
N37

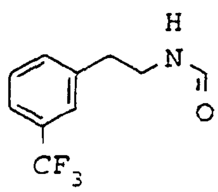
N38

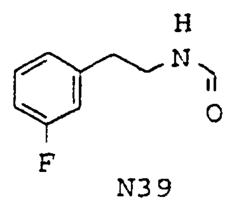
N39

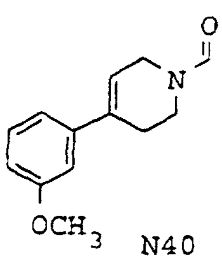
N40

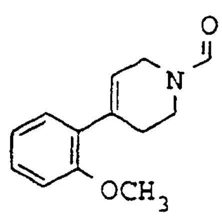
N41

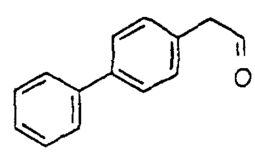
N42

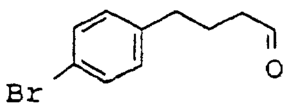

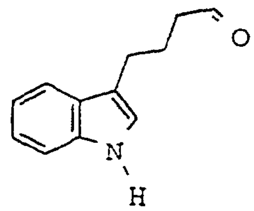

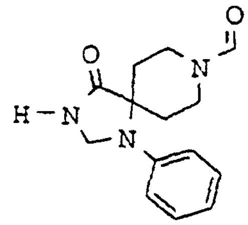

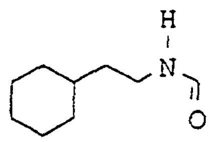

N46

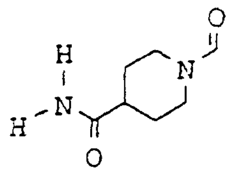

N47

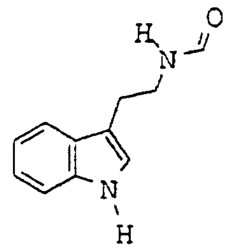

N48

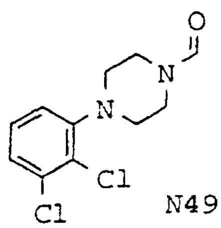

N49

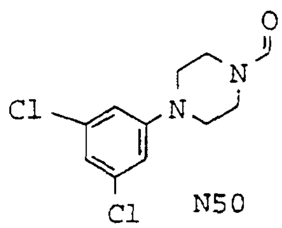

N50

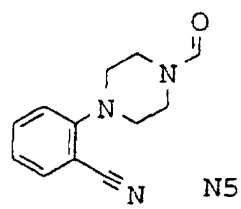

N51

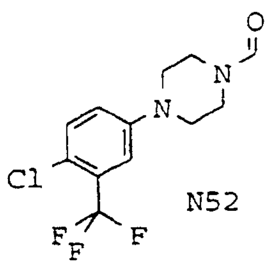

N52

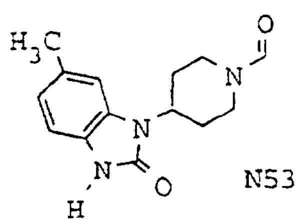

N53

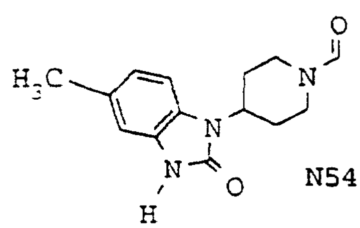

N54

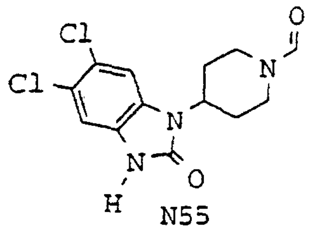
N55

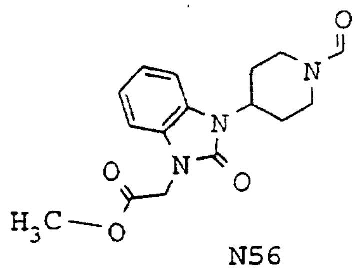
N56

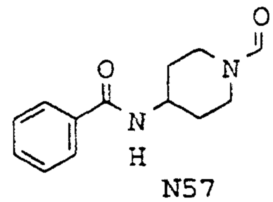
N57

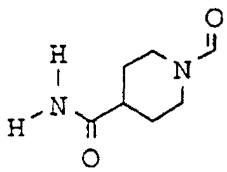
N58

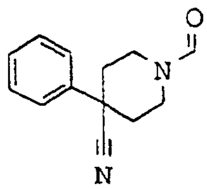
N59

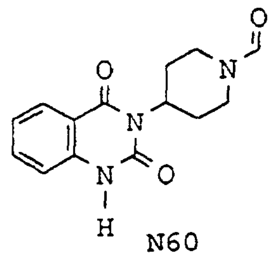
N60

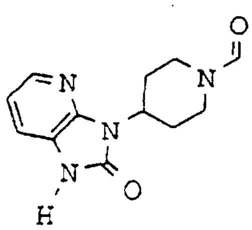
N61

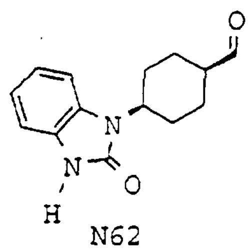
N62

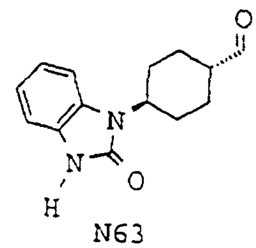
N63

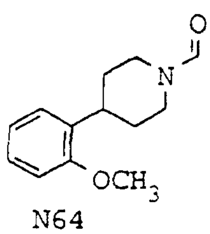
N64

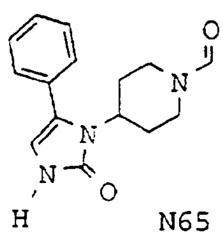
N65

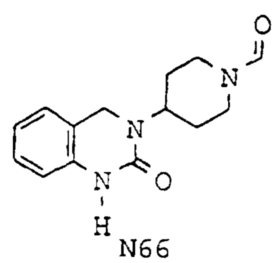
N66

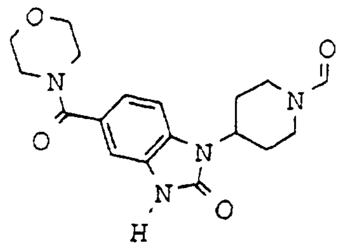
N67

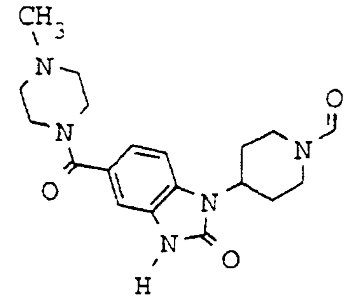
N68

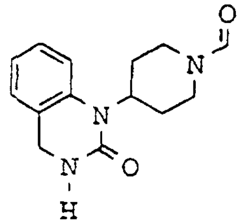
N69

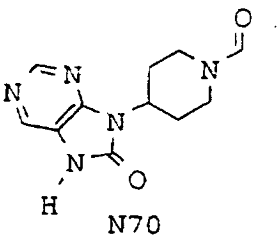
N70

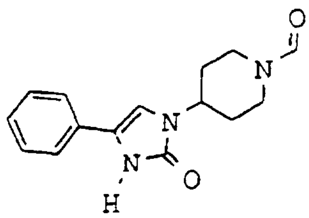
N71

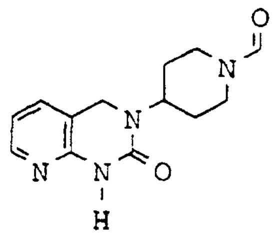
N72

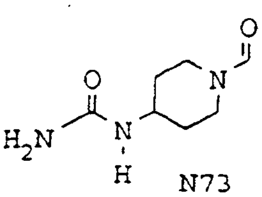
N73

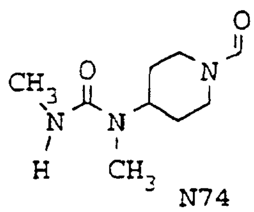
N74

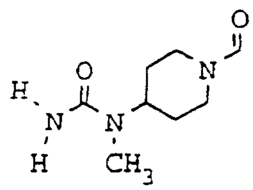
N75

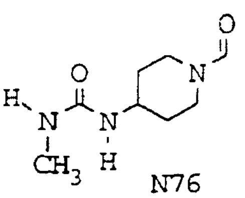
N76

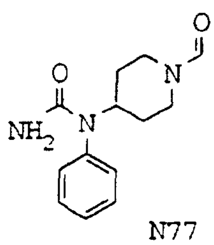
N77

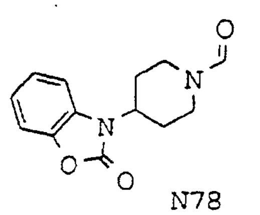
N78

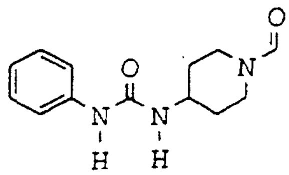
N79

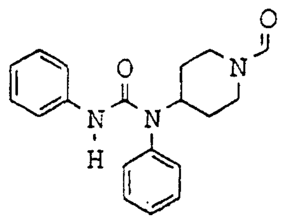
N80

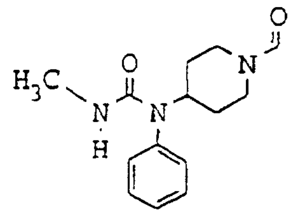
N81

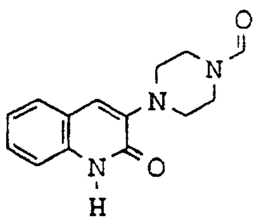
N82

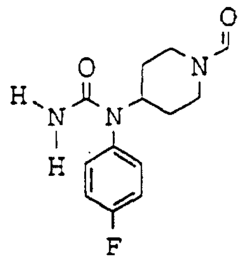
N83

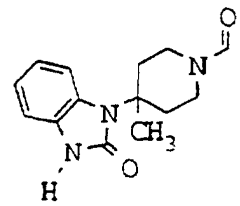
N84

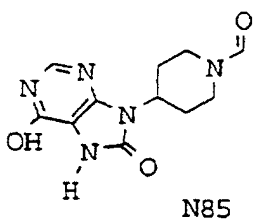
N85

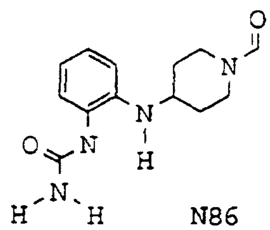
N86

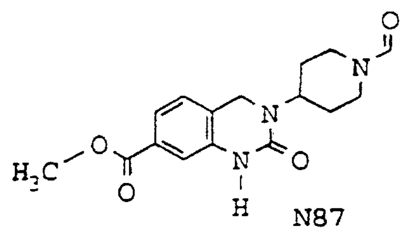
N87

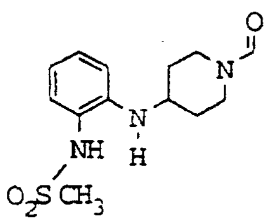
N88

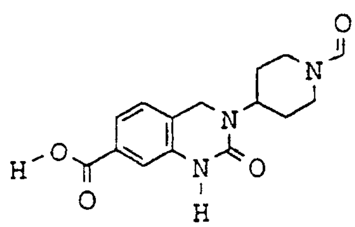
N89

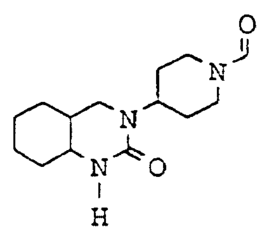
N90

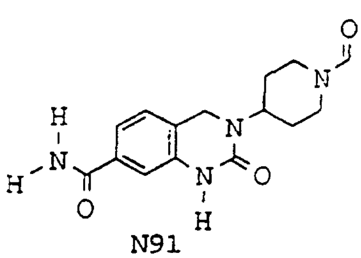
N91

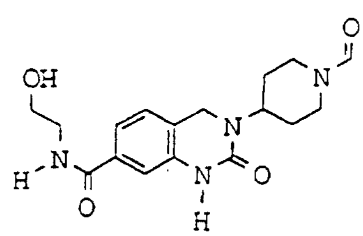
N92

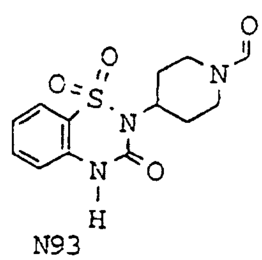
N93

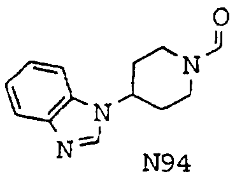
N94

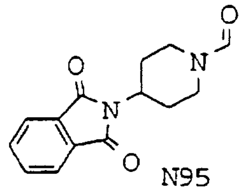
N95

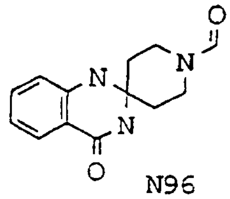
N96

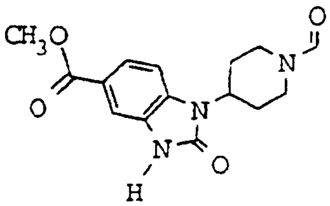
N97

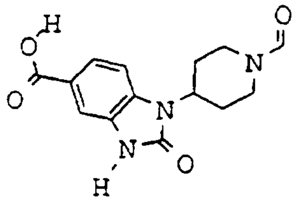
N98

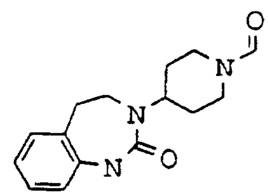
N99

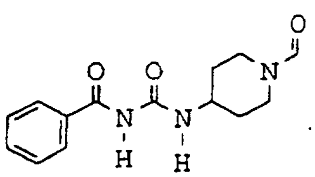
N100

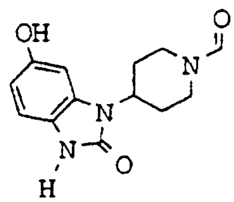
N101

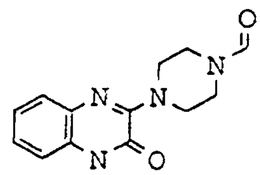
N102

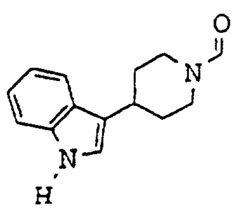
N103

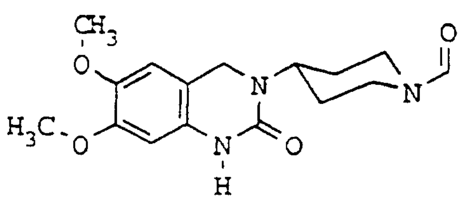
N104

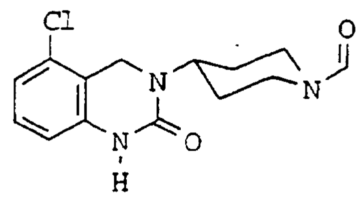
N105

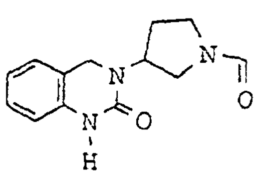
N106

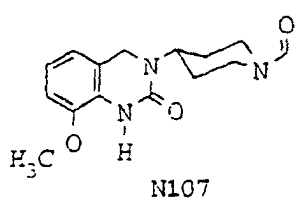
N107

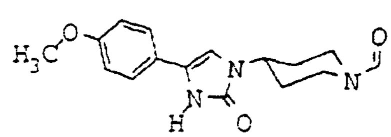
N108

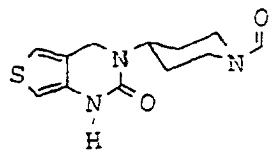

N109

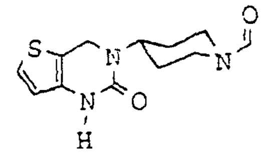

N110

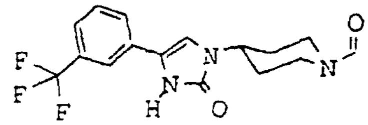

N111

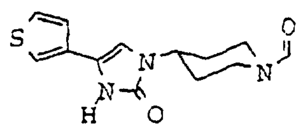

N112

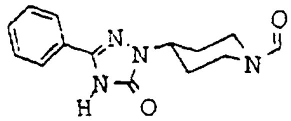

N113

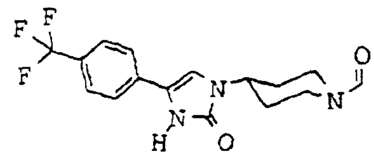

N114

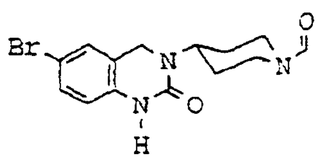

N115

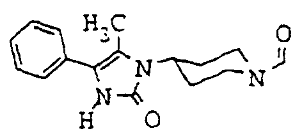

N116

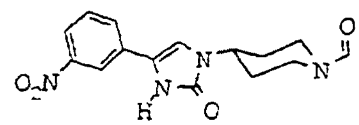

N117

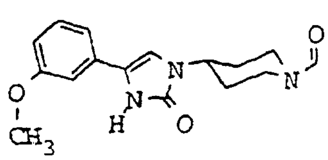

N118

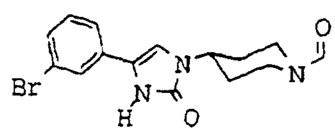

N119

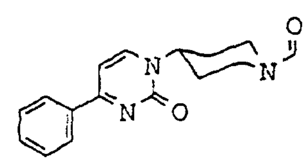

N120

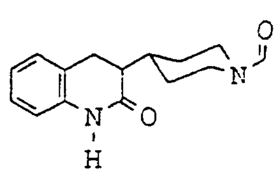

N121

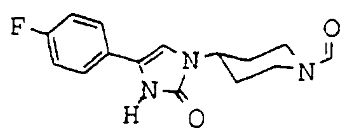

N122

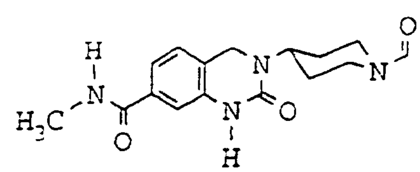

N123

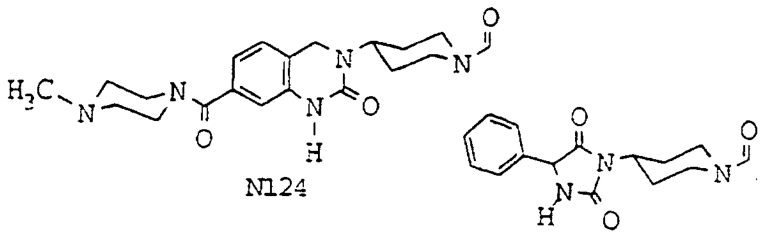

N124

N125

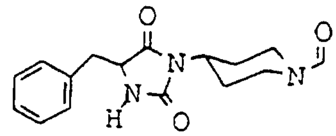

N126

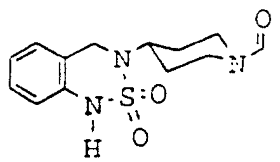

N127

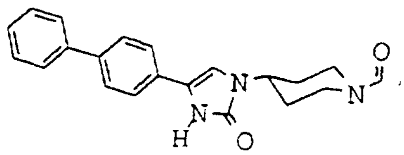

N128

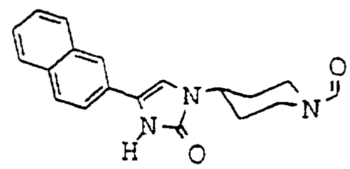

N129

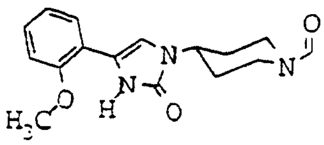

N130

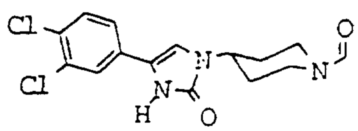

N131

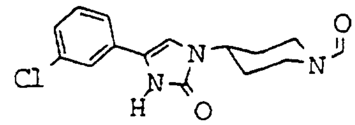

N132

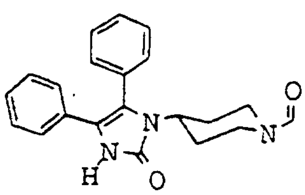

N133

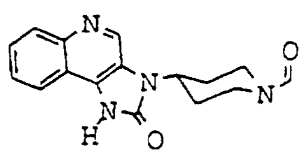

N134

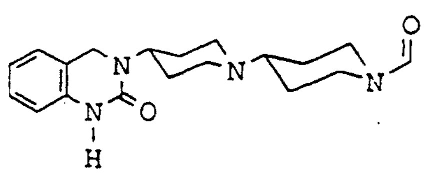

N135

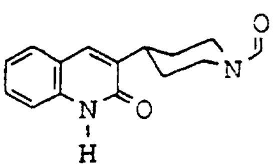

N136

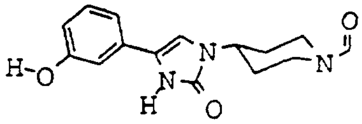

N137

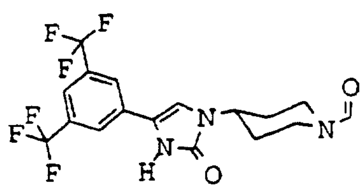

N138

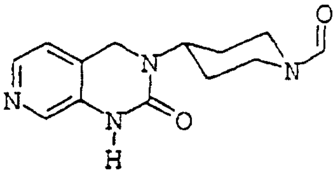
N139

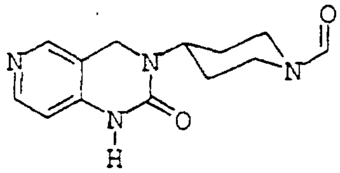
N140

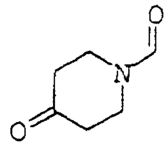
N141

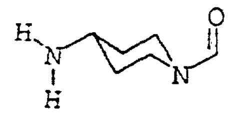
N142

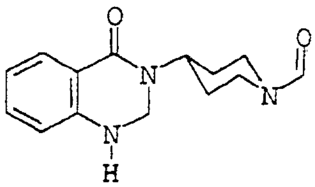
N143

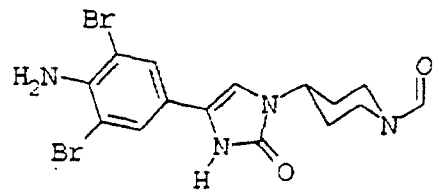
N144

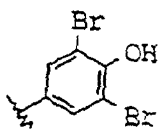
AS1

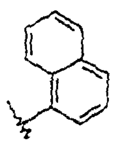
AS2

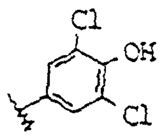
AS3

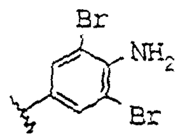
AS4

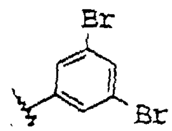
AS5

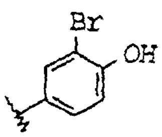
AS6

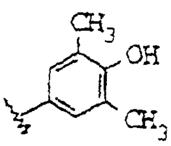
AS7

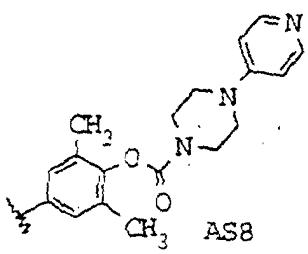
AS8

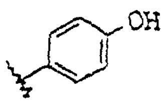
AS9

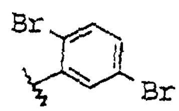
AS10

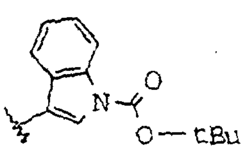
AS11

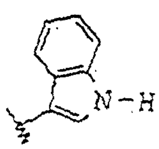
AS12

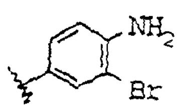
AS13

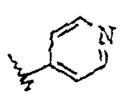
AS14

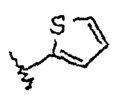
AS15

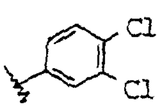
AS16

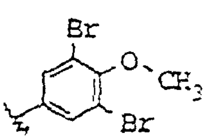
AS17

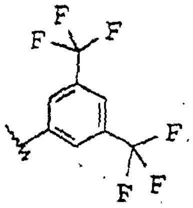
AS18

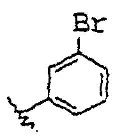
AS19

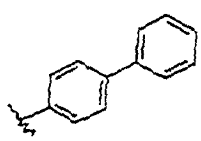
AS20

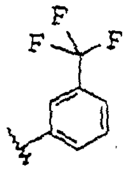
AS21

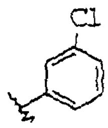
AS22

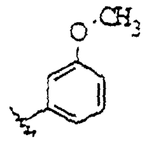
AS23

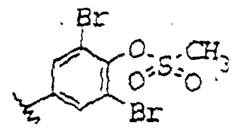
AS24

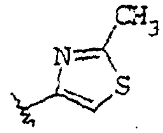
AS25

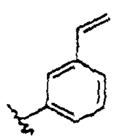
AS26

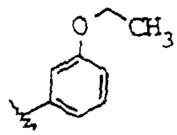
AS27

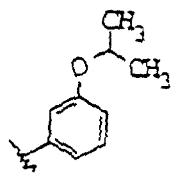
AS28

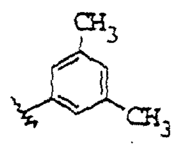
AS29

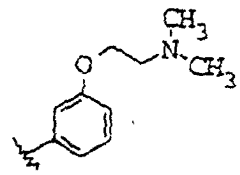
AS30

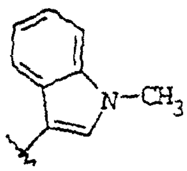
AS31

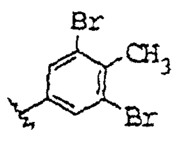
AS32

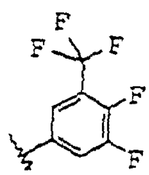
AS33

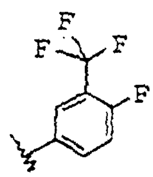
AS34

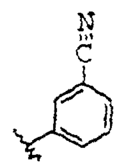
AS35

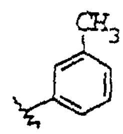
AS36

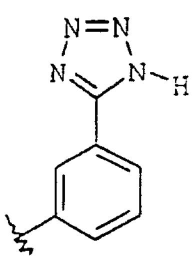
AS37

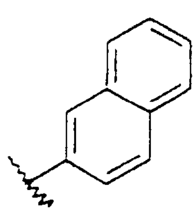
AS38

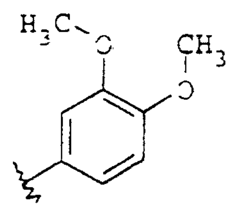
AS39

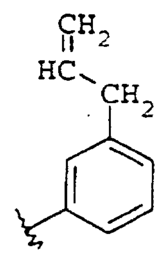
AS40

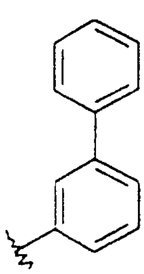
AS41

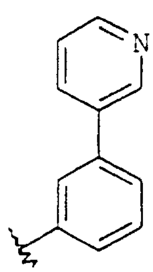
AS42

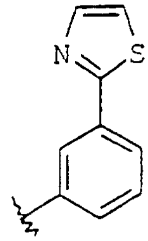
AS43

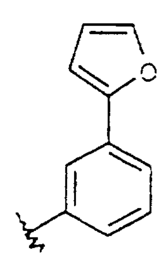
AS44

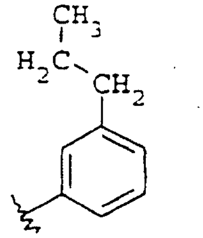
AS45

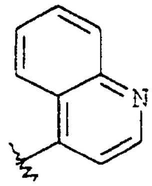
AS46

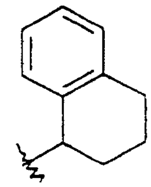
AS47

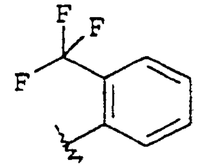
AS48

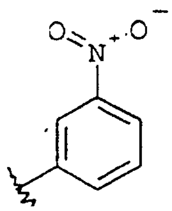
AS49

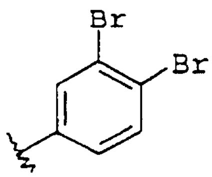
AS50

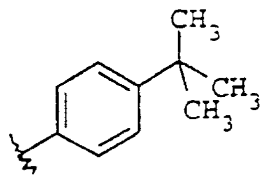
AS51

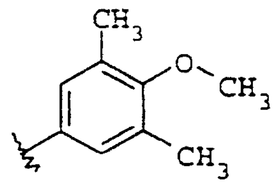
AS52

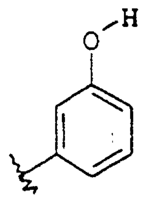
AS53

Bindung

A0

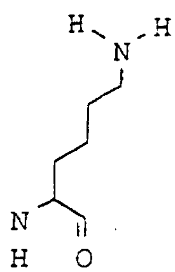
A1

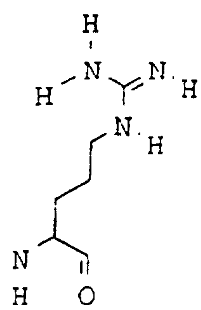
A2

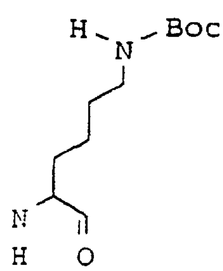
A3

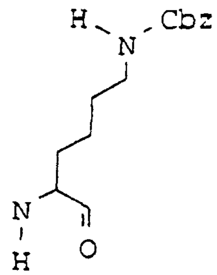
A4

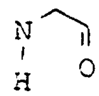
A5

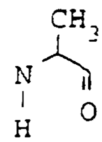
A6

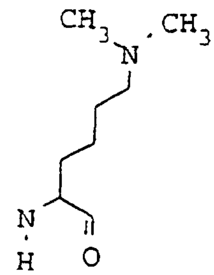
A7

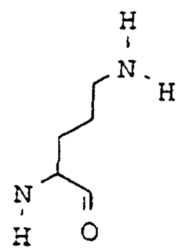
A8

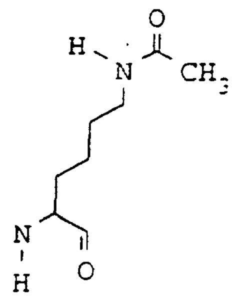
A9

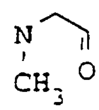
A10

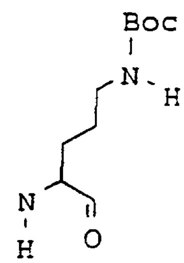
A11

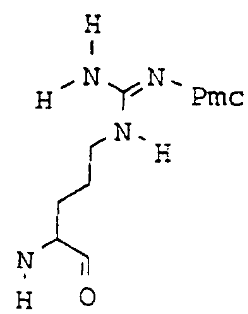
A12

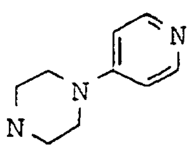
C1

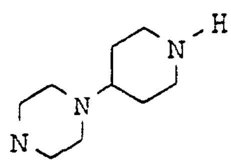
C2

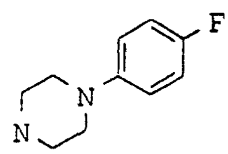
C3

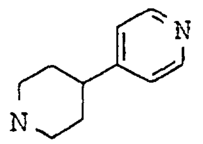
C4

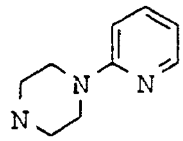
C5

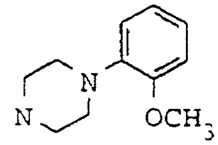
C6

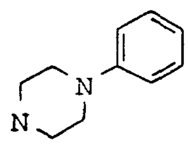
C7

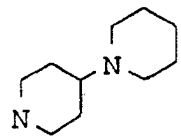
C8

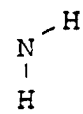
C9

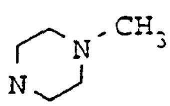
C10

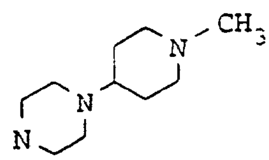
C11

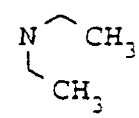
C12

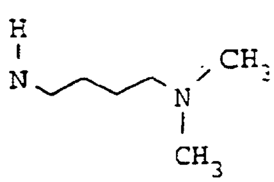
C13

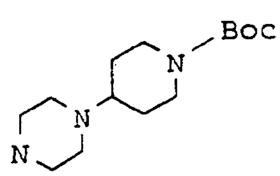
C14

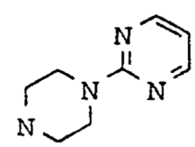
C15

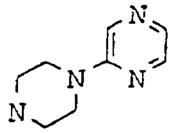
C16

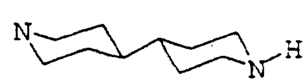
C17

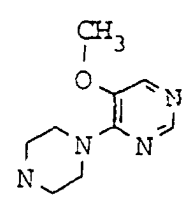
C18

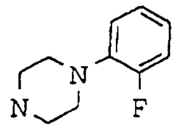
C19

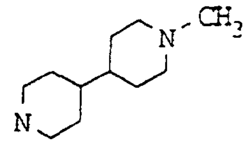
C20

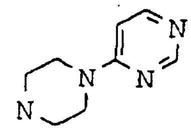
C21

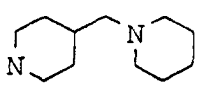
C22

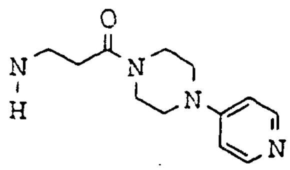
C23

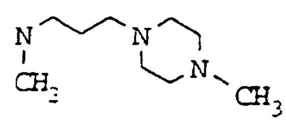
C24

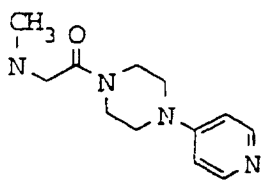
C25

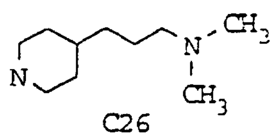
C26

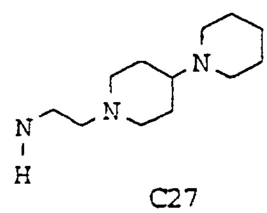
C27

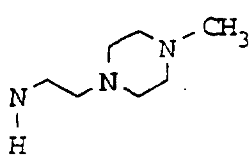
C28

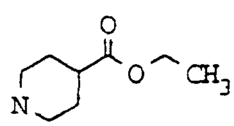
C29

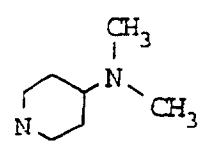
C30

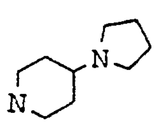
C31

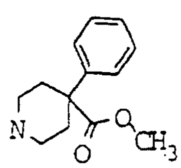
C32

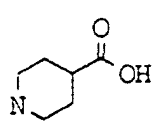
C33

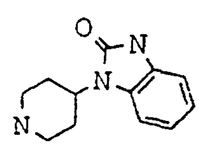
C34

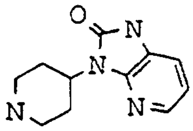
C35

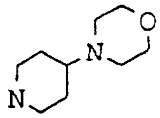
C36

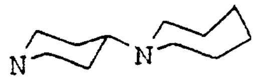
C37

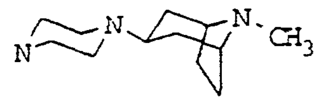
C38

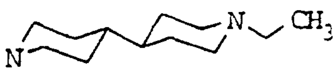
C39

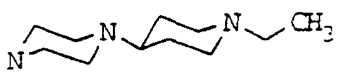
C40

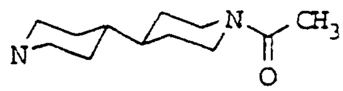
C41

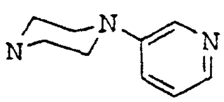
C42

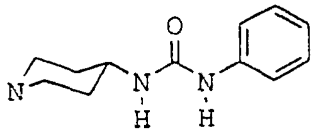
C43

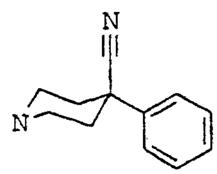
C44

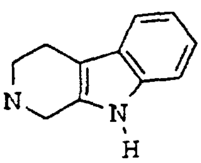
C45

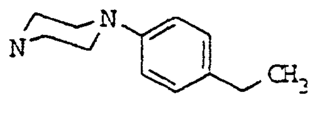
C46

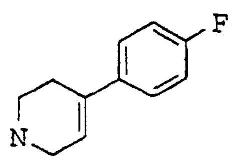
C47

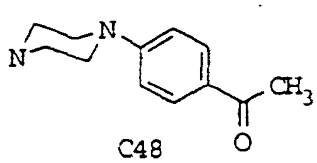
C48

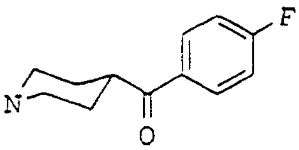
C49

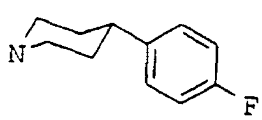
C50

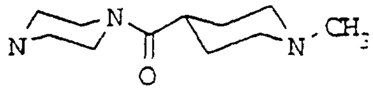
C51

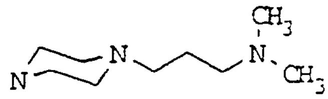
C52

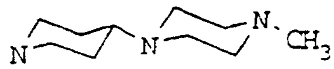
C53

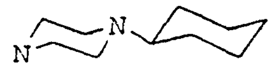
C54

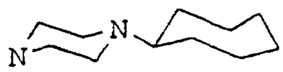
C55

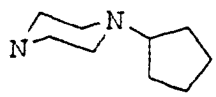
C56

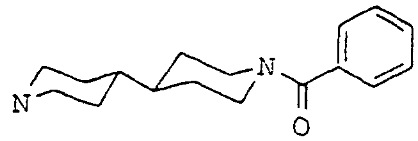
C57

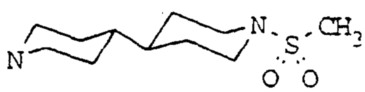
C58

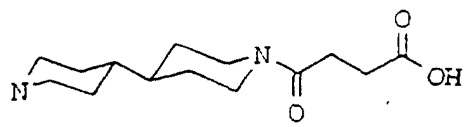
C59

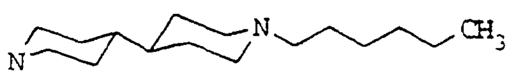
C60

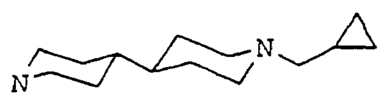
C61

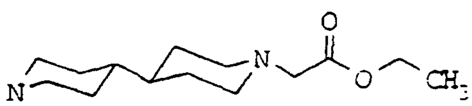
C62

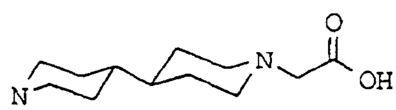
C63

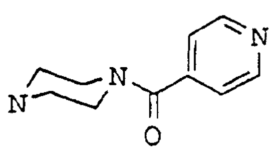
C64

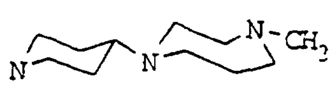
C65

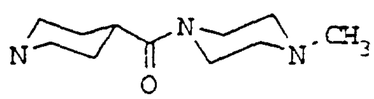
C66

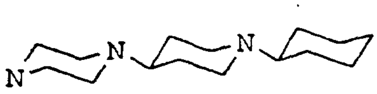
C67

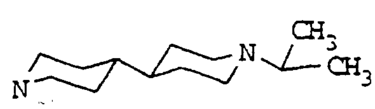
C68

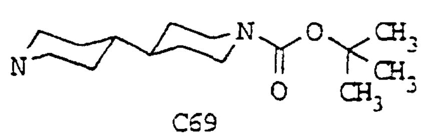
C69

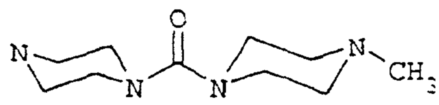

C70

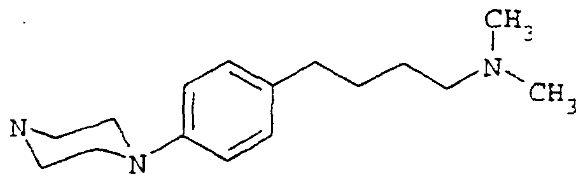

C71

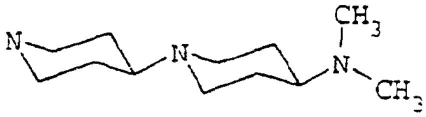

C72

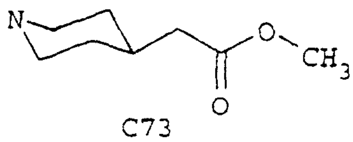

C73

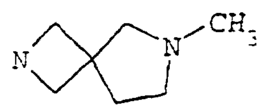

C74

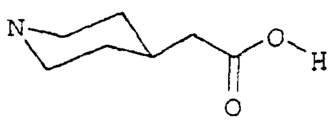

C75

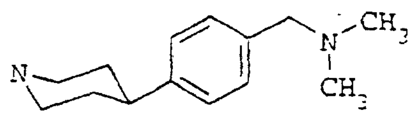

C76

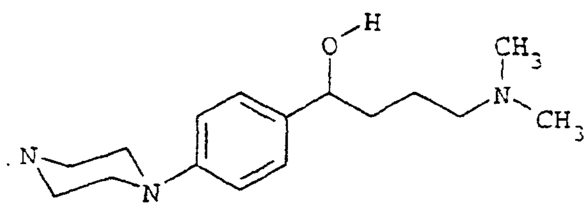

C77

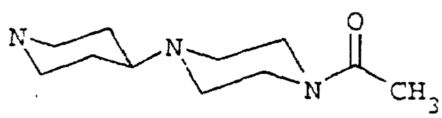

C78

**A. Herstellung von Zwischenverbindungen**

Beispiel A1

Herstellung von Verbindungen der allgemeinen Struktur:

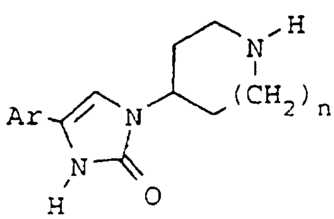

1,3-Dihydro-4-(3-methoxyphenyl)-1-(4-piperidinyl)-2H-imidazol-2-on

a) 4-[1,3-Dihydro-4-(3-methoxyphenyl)-2(2H)-oxoimidazol-1-yl]-1-(1,1-dimethylethoxycarbonyl)-piperidin

Zu der Mischung aus 20.0 g (0.10 mol) 4-Amino-1-(1,1-dimethylethoxycarbonyl)piperidin, 8.2 g (0.1 mol) wasserfreiem Natriumacetat und 150 ml Dichlormethan wurde unter Rühren und Einhaltung einer Reaktionstemperatur von 0°C bis +10°C die Lösung von 25.0 g (0.109 mol) 3-Methoxyphenacylbromid in 50 ml Dichlormethan zugetropft. Man ließ 5 Stunden bei Zimmertemperatur rühren, gab dann 19.5 g (0.296 mol) Natriumcyanat, 18 ml Eisessig und 10 ml Wasser zu und rührte weitere 12 Stunden bei Zimmertemperatur. Das Gemisch wurde in 1 l Eiswasser eingerührt, die Dichlormethanphase abgetrennt, zweimal mit je 200 ml Wasser, 5proz. wässeriger Natriumhydrogencarbonat-Lösung, 20proz. wässeriger Zitronensäure-Lösung und abermals mit Wasser gewaschen, über Magnesiumsulfat getrocknet und im Vakuum eingedampft. Der Rückstand wurde in Methanol aufgenommen. Man ließ über Nacht stehen, nutschte den auskristallisierten Niederschlag ab, wusch ihn gründlich mit tert. Butyl-methylether und erhielt nach dem Trocknen im Vakuum 11.5 g (30.8 % der Theorie) an farblosen Kristallen.
MS: $M^+$ = 373

Entsprechend wurden erhalten:

(1) 4-[1,3-Dihydro-4-phenyl-2(2H)-oxoimidazol-1-yl]-1-(1,1-dimethylethoxycarbonyl)-piperidin
$R_f$: 0.51 (FM4)

(2) 4-[1,3-Dihydro-4-(4-methoxyphenyl)-2(2H)-oxoimidazol-1-yl]-1-(1,1-dimethylethoxycarbonyl)-piperidin
Ausbeute: 23.8 % der Theorie

(3) 4-[1,3-Dihydro-4-[3-(trifluormethyl)phenyl]-2(2H)-oxoimidazol-1-yl]-1-(1,1-dimethylethoxycarbonyl)-piperidin
IR(KBr): 1685.7 $cm^{-1}$ (C=O)

(4) 4-[1,3-Dihydro-5-methyl-4-phenyl-2(2H)-oxoimidazol-1-yl]-1-(1,1-dimethylethoxycarbonyl)-piperidin
$R_f$: 0.23 (Dichlormethan/Methanol 9/1 v/v)
IR(KBr): 1687.6 $cm^{-1}$ (C=O)
MS: $M^+$ = 357

(5) 4-[1,3-Dihydro-4-(3-nitrophenyl)-2(2H)-oxoimidazol-1-yl]-1-(1,1-dimethylethoxycarbonyl)-piperidin
Ausbeute: 29.1 % der Theorie
MS: $M^+$ = 388

(6) 4-[4-(3-Bromphenyl)-1,3-dihydro-2(2H)-oxoimidazol-1-yl]-1-(1,1-dimethylethoxycarbonyl)-piperidin
Ausbeute: 13.1 % der Theorie
IR(KBr): 1685 $cm^{-1}$ (C=O)
MS: $M^+$ = 421/423 (Br)

(7) 4-[1,3-Dihydro-4,5-diphenyl-2(2H)-oxoimidazol-1-yl]-1-(1,1-dimethylethoxycarbonyl)-piperidin
IR(KBr): 1680, 1699 $cm^{-1}$ (C=O)
MS: $M^+$ = 419 .

(8) 4-[1,3-Dihydro-4-(4-fluorphenyl)-2(2H)-oxoimidazol-1-yl]-1-(1,1-dimethylethoxycarbonyl)-piperidin
IR(KBr): 1682 $cm^{-1}$ (C=O)
MS: $M^+$ = 388

(9) 4-[4-(4-Biphenylyl)-1,3-dihydro-2(2H)-oxoimidazol-1-yl]-1-(1,1-dimethylethoxycarbonyl)-piperidin
Ausbeute: 21.6 % der Theorie, farblose Kristalle
$R_f$: 0.6 (Essigsäureethylester)
IR(KBr): 1681.8 $cm^{-1}$ (C=O)

(10) 4-[1,3-Dihydro-4-(2-naphthyl)-2(2H)-oxoimidazol-1-yl]-1-(1,1-dimethylethoxycarbonyl)-piperidin
Ausbeute: 30 % der Theorie, Kristalle
IR(KBr) : 1679.9 $cm^{-1}$ (C=O)

(11) 4-[1,3-Dihydro-4-(2-methoxyphenyl)-2(2H)-oxoimidazol-1-yl]-1-(1,1-dimethylethoxycarbonyl)-piperidin

$R_f$: 0.86 (FM1)

(12) 4-[4-(3,4-Dichlorphenyl)-1,3-dihydro-2(2H)-oxoimidazol-1-yl]-1-(1,1-dimethylethoxycarbonyl)-piperidin
Ausbeute: 62 % der Theorie, farblose Kristalle
$R_f$: 0.34 (Essigsäureethylester)
IR(KBr): 1687 $cm^{-1}$ (C=O)

(13) 4-[4-(3-Chlorphenyl)-1,3-dihydro-2(2H)-oxoimidazol-1-yl]-1-(1,1-dimethylethoxycarbonyl)-piperidin
Ausbeute: 21 % der Theorie
$R_f$: 0.6 (Essigsäureethylester/Methanol 9/1 v/v)

(14) 4-[1,3-Dihydro-4-(3-hydroxyphenyl)-2(2H)-oxoimidazol-1-yl]-1-(1,1-dimethylethoxycarbonyl)-piperidin
Ausbeute: 60 % der Theorie
IR(KBr): 1682 $cm^{-1}$ (C=O)
MS: $M^+$ = 359

(15) 4-[4-[3,5-Bis-(trifluormethyl)phenyl]-1,3-dihydro-2(2H)-oxoimidazol-1-yl]-1-(1,1-dimethylethoxycarbonyl)-piperidin
Ausbeute: 3.2 % der Theorie
IR(KBr): 1687.6 $cm^{-1}$ (C=O)
$R_f$: 0.95 (Dichlormethan/Methanol 9/1 v/v)

(16) 4-[4-(4-Amino-3,5-dibromphenyl)-1,3-dihydro-2(2H)-oxoimidazol-1-yl]-1-(1,1-dimethylethoxycarbonyl)-piperidin
Ausbeute: 4.6 % der Theorie
IR(KBr): 1684 $cm^{-1}$ (C=O)
$R_f$: 0.48 (FM4; Macherey-Nagel POLYGRAM® SIL G/UV254 Fertigfolien für die DC)

b) 1,3-Dihydro-4-(3-methoxyphenyl)-1-(4-piperidinyl)-2H-imidazol-2-on

Die Lösung von 11.5 g (0.0308 mol) 4-[1,3-Dihydro-4-(3-methoxyphenyl)-2(2H)-oxoimidazol-1-yl]-1-(1,1-dimethylethoxycarbonyl)-piperidin in 150 ml Dichlormethan wurde mit 15 ml Trifluoressigsäure versetzt und anschließend über Nacht bei Zimmertemperatur gerührt. Das Reaktionsgemisch wurde im Vakuum eingeengt, der Rückstand in 10 ml Wasser aufgenommen und deutlich ammoniakalisch gestellt. Der entstandene Niederschlag wurde abgenutscht, gründlich mit Wasser gewaschen und über Nacht bei 50°C im Vakuum getrocknet. Man erhielt 7.0 g (83.1 % der Theorie) an farblosen Kristallen vom $R_f$-Wert 0.2 (Dichlormethan/Methanol 9/1 v/v).

Entsprechend wurden erhalten:

(1) 1,3-Dihydro-4-phenyl)-1-(4-piperidinyl)-2H-imidazol-2-on
$R_f$: 0.22 (FM1; Macherey-Nagel POLYGRAM® SIL G/UV254 Fertigfolien für die DC)
IR(KBr): 1672 $cm^{-1}$ (C=O)

(2) 1,3-Dihydro-4-(4-methoxyphenyl)-1-(4-piperidinyl)-2H-imidazol-2-on
IR(KBr): 1670 $cm^{-1}$ (C=O)
MS: $M^+$ = 273

(3) 1,3-Dihydro-4-[3-(trifluormethyl)phenyl]-1-(4-piperidinyl)-2H-imidazol-2-on
IR(KBr): 1687.6 $cm^{-1}$ (C=O)

(4) 1,3-Dihydro-5-methyl-4-phenyl-1-(4-piperidinyl)-2H-imidazol-2-on
Ausbeute: 76.2 % der Theorie
IR(KBr): 1679.9 $cm^{-1}$ (C=O)
MS: $M^+$ = 257

(5) 1,3-Dihydro-4-(3-nitrophenyl)-1-(4-piperidinyl)-2H-imidazol-2-on
Ausbeute: 94 % der Theorie
IR(KBr): 1677.8 (C=O); 1137.8, 1197.6, 1349.9 ($NO_2$) $cm^{-1}$

(6) 4-(3-Bromphenyl)-1,3-dihydro-1-(4-piperidinyl)-2H-imidazol-2-on
Ausbeute: quantitativ
IR(KBr): 1676 $cm^{-1}$ (C=O)

(7) 1,3-Dihydro-4,5-diphenyl-1-(4-piperidinyl)-2H-imidazol-2-on
IR(KBr): 1670 $cm^{-1}$ (C=O)
MS: $M^+$ = 319

(8) 1,3-Dihydro-4-(4-fluorphenyl)-1-(4-piperidinyl)-2H-imidazol-2-on
Ausbeute: 30 % der Theorie
$R_f$: 0.2 (Fließmittel: Essigsäureethylester/Methanol/konz. Ammoniak 9/1/0.3 v/v/v)
IR(KBr): 1682 $cm^{-1}$ (C=O)

(9) 4-(4-Biphenylyl)-1,3-dihydro-1-(4-piperidinyl)-2H-imidazol-2-on
Ausbeute: quantitativ
IR(KBr) des Trifluoracetats: 1679.9 $cm^{-1}$ (C=O)

(10) 1,3-Dihydro-4-(2-naphthyl)-1-(4-piperidinyl)-2H-imidazol-2-on
Ausbeute: 28.2 % der Theorie
$R_f$: 0.03 (FM1)
IR(KBr) des Trifluoracetats: 1678 $cm^{-1}$ (C=O)

(11) 7-(2-methoxyphenyl)-1-(4-piperidinyl)-2H-imidazol-2-on
Ausbeute: 18.8 % der Theorie
$R_f$: 0.22 (FM1)
IR(KBr) des Trifluoracetats: 1681.6 $cm^{-1}$ (C=O)

(12) 4-(3,4-Dichlorphenyl)-1,3-dihydro-1-(4-piperidinyl)-2H-imidazol-2-on
Ausbeute: quantitativ
IR(KBr) des Trifluoracetats: 3197 (N-H); 1685 (C=O) $cm^{-1}$

(13) 4-(3-Chlorphenyl)-1,3-dihydro-1-(4-piperidinyl)-2H-imidazol-2-on
Ausbeute: 98 % der Theorie
$R_f$: 0.25 (Fließmittel: Essigsäureethylester/Methanol/konz. Ammoniak 9/1/0.3 v/v/v)

(14) 1,3-Dihydro-4-(3-hydroxyphenyl)-1-(4-piperidinyl)-2H-imidazol-2-on
Ausbeute: 90 % der Theorie
$R_f$: 0.075 (FM1)
IR(KBr): 1670 (C=O) $cm^{-1}$
MS: $M^+$ = 259

(15) 4-[3,5-Bis-(trifluormethyl)phenyl]-1,3-dihydro-1-(4-piperidinyl)-2H-imidazol-2-on
Ausbeute: 71 % der Theorie
$R_f$: 0.15 (FM1)
IR(KBr): 1701 (C=O) $cm^{-1}$
MS: $M^+$ = 379

(16) 4-(4-Amino-3,5-dibromphenyl)-1,3-dihydro-1-(4-piperidinyl)-2H-imidazol-2-on
Ausbeute: 44 % der Theorie
$R_f$: 0.71 (FM1; Macherey-Nagel POLYGRAM® SIL G/UV254 Fertigfolien für die DC)
IR(KBr): 1676 (C=O) $cm^{-1}$

Beispiel A2

2,4-Dihydro-5-phenyl-2-(4-piperidinyl)-3H-1,2,4-triazol-3-on

a) 1-(9H-Fluoren-9-ylmethoxycarbonyl)-4-piperidinon-(1,1-dimethylethoxycarbonyl)hydrazon

Das Gemisch aus 16.0 g (0.05 mol) 1-(9H-Fluoren-9-ylmethoxycarbonyl)-4-piperidinon, 7.25 g (0.055 mol) Hydrazinoameisensäure-tert.-butylester und 250 ml Ethanol wurde 1 Stunde unter Rückfluß gekocht. Das Lösemittel wurde im Vakuum abdestilliert, der verbleibende ölige Rückstand mit Diethylether verrieben. Der dabei entstandene kristalline Niederschlag wurde abgenutscht und mit wenig Diethylether nachgewaschen. Nach dem Trocknen des Produkts im Vakuum erhielt man 21.7 g (99.7 % der Theorie) an farblosen Kristallen vom Fp. 156 - 158°C (Z).

b) N-(1,1-Dimethylethoxycarbonyl)-N'-[1-(9H-fluoren-9-yl-methoxycarbonyl)-4-piperidinyl]-hydrazin

Die Lösung von 21.7 g (0.05 mol) 1-(9H-Fluoren-9-ylmethoxycarbonyl)-4-piperidinon-(1,1-dimethylethoxycarbonyl)-hydrazon in 200 ml Eisessig wurde in Gegenwart von 2.0 g Plasin(IV)-oxid bei Zimmertemperatur und 3 bar Wasserstoffdruck bis zur Aufnahme des berechneten Wasserstoffvolumens hydriert. Man filtrierte vom Katalysator ab, engte das Filtrat im Vakuum ein und löste den Rückstand in wenig Diethylether. Das nach 3stündigem Stehenlassen bei Raumtemperatur ausgefallene Kristallisat wurde abgenutscht, mit wenig Diethylether gewaschen und im Vakuum bei Zimmertemperatur getrocknet. Man erhielt 21.8 g (99.6 % der Theorie) an farblosen Kristallen vom Fp. 135 - 137°C und $R_f$ = 0.235 (Fließmittel 3).
ESI-MS: $(M+H)^+$ = 438

c) [1-(9H-Fluoren-9-ylmethoxycarbonyl)-4-piperidinyl]-hydrazinhydrochlorid

21.8 g (0.0498 mol) N-(1,1-Dimethylethoxycarbonyl)-N'-[1-(9H-fluoren-9-ylmethoxycarbonyl)-4-piperidinyl]-hydrazin wurden in 100 ml Trifluoressigsäure gelöst und 1 Stunde bei Zimmertemperatur gerührt. Die überschüssige Trifluoressigsäure wurde im Vakuum entfernt, der Rückstand in 50 ml Wasser gelöst und mit 10proz. wässeriger Natriumcarbonat-Lösung alkalisch gestellt. Man extrahierte erschöpfend mit Dichlormethan, trocknete die vereinigten Extrakte über Magnesiumsulfat und dampfte sie im Vakuum ein. Der so erhaltene Rückstand wurde in Essigsäureethylester aufgenommen und durch Zugabe von etherischer Chlorwasserstofflösung ins Hydrochlorid übergeführt. Nach dem Umkristallisieren aus wasserfreiem Ethanol erhielt man 6.2 g (33.3 % der Theorie) an farblosen Kristallen vom Fp. 160-162°C.

| $C_{20}H_{23}N_3O_2$ + HCl (373.88) | | | | |
|---|---|---|---|---|
| Ber. | C 64.25 | H 6.47 | N 11.24 | Cl 9.48 |
| Gef. | 64.14 | 6.46 | 10.99 | 9.46 |

d) 2,4-Dihydro-5-phenyl-2-[1-(9H-fluoren-9-ylmethoxycarbonyl)-4-piperidinyl]-3H-1,2,4-triazol-3-on

Die Lösungen von 5.56 g (0.0165 mol) [1-(9H-Fluoren-9-yl-methoxycarbonyl)-4-piperidinyl]-hydrazin in 60 ml Tetrahydrofuran und von 3.7 g (0.0177 mol) N-(Ethoxycarbonyl)-benzthionamid in 30 ml Tetrahydrofuran wurden vereinigt und 1 Stunde lang unter Rückfluß gekocht, wobei Schwefelwasserstoff freigesetzt wurde. Das Lösemittel wurde im Vakuum abdestilliert, der verbliebene ölige Rückstand mit wenig Acetonitril aufgekocht. Man ließ erkalten, kühlte zusätzlich von außen mit Eiswasser und nutschte den entstandenen Niederschlag ab. Man erhielt 4.0 g (52 % der Theorie) an farblosen Kristallen vom Fp. 142°C und $R_f$ = 0.38 (Fließmittel 4).
IR (KBr): 1685.7 $cm^{-1}$ (C=O)

e) 2,4-Dihydro-5-phenyl-2-(4-piperidinyl)-3H-1,2,4-triazol-3-on

Die Mischung aus 9.0 g (0.0193 mol) 2,4-Dihydro-5-phenyl-2-[1-(9H-fluoren-9-ylmethoxycarbonyl)-4-piperidinyl)-3H-1,2,4-triazol-3-on, 50 ml Tetrahydrofuran und 70 ml Diethylamin wurde bis zur Beendigung der dünnschichtchromatographisch verfolgten Reaktion bei Zimmertemperatur gerührt. Das Lösemittel wurde im Vakuum entfernt, der verbliebene Rückstand mit 300 ml Wasser versetzt und 30 Minuten lang ultraschallbehandelt. Man nutschte vom Ungelösten ab und dampfte das wässerige Filtrat im Vakuum ein. Der so erhaltene Rückstand wurde mit wenig Methanol aufgekocht und nach dem Erkalten abgenutscht. Nach dem Trocknen erhielt man 0.58 g (12.3 % der Theorie) an farblosen Kristallen vom Fp. 294°C (Z) und $R_f$ = 0.1 (Fließmittel 1).

IR (KBr): 1681.8 $cm^{-1}$ (C=O)

Beispiel A3

Herstellung von Verbindungen der allgemeinen Struktur:

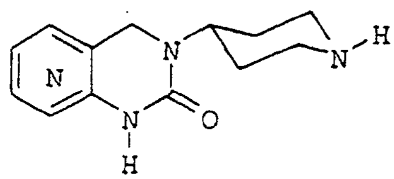

3,4-Dihydro-3-(4-piperidinyl)-2(1H)-pyrido[2,3-d]-pyrimidinon

a) N-(2-Pyridinyl)-2,2-dimethylpropanamid

Zur Lösung von 94.1 g (1.0 mol) 2-Aminopyridin und 173 ml (1.25 mol) Triethylamin in 400 ml Dichlormethan tropfte man unter äußerer Kühlung mit Eiswasser 132.5 g (1.099 mol) Pivaloylchlorid in 150 ml Dichlormethan. Man rührte 2 Stunden bei Zimmertemperatur und filtrierte vom entstandenen Triethylamin-hydrochlorid. Das Filtrat wurde mit Wasser und zweimal mit 5proz. wässeriger Natriumhydrogencarbonatlösung gewaschen, danach über Natriumsulfat getrocknet. Nach üblicher Aufarbeitung erhielt man 157.5 g (88.4 % der Theorie) an farblosen Kristallen vom Fp. 74-76°C.

Entsprechend erhielt man:

N-(4-Pyridinyl)-2,2-dimethylpropanamid
Ausbeute: 74 % der Theorie
Fp. 137 - 140 °C (Diisopropylether)
IR (KBr): 1687 $cm^{-1}$ (C=O)

b) N-(3-Formyl-2-pyridinyl)-2,2-dimethylpropanamid

Unter Einhaltung einer Reaktionstemperatur von -78°C wurden zu einer Lösung von 89.1 g (0.5 mol) N-(2-Pyridinyl)-2,2-dimethylpropanamid in 300 ml wasserfreiem Tetrahydrofuran 781 ml (1.25 mol) einer 1.6-molaren Lösung von n-Butyllithium in n-Hexan zugetropft. Man ließ die Mischung langsam auf 0°C erwärmen und rührte 3 Stunden bei dieser Temperatur. Dann ließ man erneut auf -78°C abkühlen und tropfte unter Einhaltung dieser Temperatur die Lösung von 109.6 g (1.5 mol) Dimethylformamid in 150 ml wasserfreiem Tetrahydrofuran zu. Man ließ auf 0°C kommen und rührte die Mischung anschließend in 1 l Eiswasser ein. Man säuerte zunächst mit 12proz. wässeriger Salzsäure an, stellte dann durch Zugabe von festem Kaliumcarbonat alkalisch und extrahierte erschöpfend mit Diethylether. Die vereinigten Etherauszüge wurden über Natriumsulfat getrocknet und eingedampft. Der kristalline Rückstand zeigte nach dem Umkristallisieren aus Diisopropylether den Fp. 83°C. Ausbeute: 94.0 g (91.2 % der Theorie).

Entsprechend erhielt man:

(1) N-(4-Formyl-3-pyridinyl)-2,2-dimethylpropanamid
Ausbeute: 52 % der Theorie
Rf: 0.5 (Dichlormethan/Methanol/konz. Ammoniak 90/10/0.1 v/v/v) IR (KBr) des Hydrochlorids : 1695 $cm^{-1}$ (C=O)
MS: $M^+$ = 206
(2) N-(3-Formyl-4-pyridinyl)-2,2-dimethylpropanamid
Das in quantitativer Ausbeute erhaltene rötliche Öl wurde ohne weitere Reinigung weiterverarbeitet

c) N-[3-[[[1-(Phenylmethyl)-4-piperidinyl]amino]methyl]-2-pyridinyl]-2,2-dimethylpropanamid

Die Lösung von 8.2 g (0.0398 mol) N-(3-Formyl-2-pyridinyl)-2,2-dimethylpropanamid und von 7.6 g (0.04 mol) 4-Amino-1-(phenylmethyl)piperidin in 80 ml Methanol wurde portionsweise mit insgesamt 1.7 g (0.045 mol) Natriumborhydrid versetzt und insgesamt 24 Stunden unter Rückfluß gekocht. Das Lösemittel wurde im Vakuum entfernt, der Rückstand zwischen Wasser und Essigsäureethylester verteilt. Die organische Phase wurde über Natriumsulfat getrocknet und vom Lösemittel befreit. Der Rückstand wurde mit Diisopropylether verrieben und abgenutscht. Man erhielt

6.0g (39.6 % der Theorie) an farblosen Kristallen vom Fp. 138°C.

Entsprechend erhielt man:

(1) N-[4-[[[1-(Phenylmethyl)-4-piperidinyl]amino]methyl]-3-pyridinyl]-2,2-dimethylpropanamid
Ausbeute: 94 % der Theorie
Rf: 0.4 (Dichlormethan/Methanol/konz. Ammoniak 90/10/0.1 v/v/v)
Das gelbliche Öl wurde ohne weitere Reinigung in der folgenden Stufe verwendet

(2) N-[3-[[[1-(Phenylmethyl)-4-piperidinyl]amino]methyl]-4-pyridinyl]-2,2-dimethylpropanamid
Ausbeute: 11.6 % der Theorie
IR(KBr): 1689 (C=O) $cm^{-1}$

d) 2-Amino-3-[[[1-(phenylmethyl)-4-piperidinyl]amino]methyl]-pyridin

Das Gemisch aus 6.0 g (0.0158 mol) N-[3-[[[1-(Phenylmethyl)-4-piperidinyl]amino]methyl]-2-pyridinyl]-2,2-dimethylpropanamid und 100 ml konz. Salzsäure wurde 3 Stunden unter Rückfluß gekocht. Das Gemisch wurde im Vakuum eingedampft, der verbliebene Rückstand in wenig Wasser gelöst und durch Zugabe von festem Kaliumcarbonat alkalisch gestellt. Man extrahierte erschöpfend mit Essigsäureethylester, trocknete die vereinigten Extrakte über Natriumsulfat und dampfte sie im Vakuum ein. Der Rückstand wurde mit Diisopropylether gründlich verrieben und ergab 4.2 g (89.7 % der Theorie) an farblosen Kristallen vom Fp. 114°C.

Entsprechend erhielt man:

(1) 3-Amino-4-[[[1-(phenylmethyl)-4-piperidinyl]amino]methyl]-pyridin
Ausbeute: 96 % der Theorie
$R_f$: 0.42 (Dichlormethan/Methanol/konz. Ammoniak 90/10/0.1 v/v/v)
Das gelbliche Öl wurde ohne weitere Reinigung in der folgenden Stufe verwendet

(2) 4-Amino-3-[[[1-(phenylmethyl)-4-piperidinyl]amino]methyl]-pyridin
Ausbeute: quantitativ
Das gelbliche Öl wurde ohne weitere Reinigung in der folgenden Stufe verwendet

e) 3,4-Dihydro-3-[1-(phenylmethyl)-4-piperidinyl]-2(1H)-pyrido[2,3-d]-pyrimidinon

Die Mischung aus 4.2 g (0.0142 mol) 2-Amino-3-[[[1-(Phenylmethyl)-4-piperidinyl]amino]methyl]-pyridin, 2.4 g (0.0148 mol) N,N'-Carbonyldiimidazol und 50 ml Dimethylformamid wurde 30 Minuten lang auf 100°C erhitzt. Das noch warme Gemisch wurde in 300 ml Eiswasser eingerührt, der ausfallende Niederschlag abgenutscht und aus Acetonitril umkristallisiert. Nach dem Trocknen im Vakuum erhielt man 4.5 g (98.3 % der Theorie) an farblosen Kristallen vom Fp. 187°C.

Entsprechend erhielt man:

(1) 3,4-Dihydro-3-[1-(phenylmethyl)-4-piperidinyl]-2(1H)-pyrido[3,4-d]-pyrimidinon
Farblose Kristalle
Ausbeute: 33 % der Theorie
IR (KBr): 1676 $cm^{-1}$ (C=O)
MS: $M^+$ = 322

(2) 3,4-Dihydro-3-[1-(phenylmethyl)-4-piperidinyl]-2(1H)-pyrido[4,3-d]-pyrimidinon
Fp. 155 °C (Z)
Ausbeute: 99 % der Theorie
IR (KBr): 1680 $cm^{-1}$ (C=O)

f) 3,4-Dihydro-3-(4-piperidinyl)-2(1H)-pyrido[2,3-d]-pyrimidinon

Die Lösung von 4.7 g (0.0146 mol) 3,4-Dihydro-3-[1-(phenylmethyl)-4-piperidinyl]-2(1H)-pyrido[2,3-d]-pyrimidinon in 50 ml Methanol wurde bei einer Temperatur von 50°C und in Gegenwart von 2.0 g 20proz. Palladiumkohle bis zur Beendigung der Wasserstoffaufnahme hydriert. Nach Entfernung des Katalysators und des Lösemittels erhielt man 3.3 g (97.3 % der Theorie) eines farblosen Öls vom $R_f$ = 0.35 (FM1).
IR (KBr): 1660.6 $cm^{-1}$ (C=O)

Entsprechend erhielt man:

(1) 3,4-Dihydro-3-(4-piperidinyl]-2(1H)-pyrido[3,4-d]-pyrimidinon
Farblose Kristalle
Ausbeute: 95 % der Theorie
IR (KBr): 1662 $cm^{-1}$ (C=O)
MS: $M^+$ = 232

(2) 3,4-Dihydro-3-(4-piperidinyl]-2(1H)-pyrido[4,3-d]-pyrimidinon
Gelbliches Harz
Ausbeute: 97 % der Theorie
IR (KBr) : 1672 $cm^{-1}$ (C=O)
$R_f$: 0.12 (FM1)

Beispiel A4

3,4-Dihydro-3-(4-piperidinyl)-2(1H)-oxochinazolin-7-carbonsäuremethylester

a) (E)-1-(Dimethylamino)-2-[4-(methoxycarbonyl)-2-nitrophenyl]-ethen

Die Mischung aus 98.3 g (0.504 mol) 4-Methyl-3-nitrobenzoesäuremethylester, 78.0 g (0.655 mol) N,N-Dimethylformamiddimethylacetal und 1 l Dimethylformamid wurde 3 Stunden lang auf 140°C erhitzt. Das Lösemittel wurde im Vakuum abdestilliert, der Rückstand mit 1 l Methanol gründlich verrieben. Nach dem Trocknen im Vakuum erhielt man 119.5 g (94.7 % der Theorie) einer roten, amorphen Substanz, die ohne weitere Reinigung weiterverarbeitet wurde.

b) 4-(Methoxycarbonyl)-2-nitrobenzaldehyd

Zu der Mischung aus 119.5 g (0.478 mol) (E)-1-(Dimethylamino)-2-[4-(methoxycarbonyl)-2-nitrophenyl]-ethen und 1.3 l Wasser-Tetrahydrofuran-Gemisch (1/1 v/v) gab man portionsweise 308.0 g (1.44 mol) Natriummetaperiodat, wobei man durch äußere Kühlung mit Eiswasser die Reaktionstemperatur auf unter +30°C regulierte. Man rührte die Mischung noch 2.5 Stunden bei Zimmertemperatur und filtrierte sie anschließend. Der Niederschlag wurde gründlich mit Essigsäureethylester gewaschen. Die organische Phase wurde abgetrennt, die wässerige erschöpfend mit Essigsäureethylester extrahiert. Die vereinigten Essigesterphasen wurden über Natriumsulfat getrocknet und im Vakuum eingedampft. Das nach einem Tage kristallisierende Öl wurde ohne weitere Reinigung weiterverarbeitet. Ausbeute: 87 g (87 % der Theorie).

c) 4-[[[1-(Phenylmethyl)-4-piperidinyl]amino]methyl]-3-nitrobenzoesäuremethylester

Zu der Lösung von 41.0 g (0.215 mol) 4-Amino-1-(phenylmethyl)-piperidin und 45.0 g (0.215 mol) 4-(Methoxycarbonyl)-2-nitrobenzaldehyd in 1 l Methanol gab man bei Zimmertemperatur portionsweise 8.3 g (0.22 mol) Natriumborhydrid und rührte anschließend 30 Minuten lang bei der gleichen Temperatur. Das Gemisch wurde in 1 l Eiswasser eingerührt und mit tert.-Butylmethylether erschöpfend extrahiert. Die vereinigten Extrakte wurden über Natriumsulfat getrocknet und im Vakuum eingeengt, der Rückstand in möglichst wenig Methanol gelöst und durch Behandlung mit methanolischer Chlorwasserstoff-Lösung ins Hydrochlorid übergeführt. Das kristalline Salz wurde abgenutscht, mit Methanol und Diethylether gewaschen, danach in Wasser aufgenommen und mit gesättigter wässeriger Kaliumcarbonat-Lösung alkalisch gestellt. Die erhaltene Mischung wurde mit Essigsäureethylester erschöpfend extrahiert, die vereinigten Essigesterauszüge über Natriumsulfat getrocknet und eingedampft. Man erhielt 58.2 g (70.6 % der Theorie) eines gelbroten Öls, das ohne weitere Reinigung weiterverarbeitet wurde.

d) 3-Amino-4-[[[1-(phenylmethyl)-4-piperidinyl]amino]methyl]-benzoesäuremethylester

Die Lösung von 58.0 g (0.151 mol) 4-[[[1-(Phenylmethyl)-4-piperidinyl]amino]methyl]-3-nitrobenzoesäuremethylester in 800 ml Methanol wurde in Gegenwart von 10 g 5proz. Rhodiumkohle 7 Stunden lang bei Zimmertemperatur hydriert. Der Katalysator wurde abfiltriert, das Filtrat im Vakuum eingedampft. Man erhielt 50.0 g (93.7 % der Theorie) an farblosen Kristallen, die ohne weitere Reinigung weiterverarbeitet wurden.

e) 3,4-Dihydro-3-[1-(phenylmethyl)-4-piperidinyl]-2(1H)-oxochinazolin-7-carbonsäuremethylester

Hergestellt analog Beispiel A3e) aus 3-Amino-4-[[[1-(phenylmethyl)-4-piperidinyl]amino]methyl] -benzoesäuremethylester und N,N'-Carbonyl-diimidazol in einer Ausbeute von 66.3 % der Theorie. Schwach gelbliche Kristalle.
IR (KBr): 1714.6; 1664.5 $cm^{-1}$ (C=O)

f) 3,4-Dihydro-3-(4-piperidinyl)-2(1H)-oxochinazolin-7-carbonsäuremethylester

Die Lösung von 35.5 g (0.0936 mol) 3,4-Dihydro-3-[1-(phenylmethyl)-4-piperidinyl]-2(1H)-oxochinazolin-7-carbonsäuremethylester in 400 ml Methanol wurde in Gegenwart von 5 g 10proz. Palladiumkohle 5 Stunden lang bei 50°C hydriert. Der Katalysator wurde abfiltriert, das Filtrat im Vakuum eingedampft. Der Rückstand wurde mit 150 ml Essigsäureethylester verrieben und anschließend abgenutscht. Nach dem Trocknen im Vakuum erhielt man 20.4 g (75.3 % der Theorie) an farblosen Kristallen, die ohne weitere Reinigung weiterverarbeitet wurden.
IR (KBr): 1718.5; 1672.2 $cm^{-1}$ (C=O)

Analog wurden hergestellt:

(1) 3,4-Dihydro-3-(4-piperidinyl)-2(1H)-chinazolinon
$R_f$: 0.3 (FM1)
IR (KBr): 1662.5 $cm^{-1}$ (C=O)

(2) 3,4-Dihydro-8-methoxy-3-(4-piperidinyl)-2(1H)-chinazolinon
$R_f$: 0.35 (FM1)

(3) 3,4-Dihydro-6,7-dimethoxy-3-(4-piperidinyl)-2(1H)-chinazolinon
$R_f$: 0.40 (FM1)

Beispiel A5

3,4-Dihydro-3-(4-piperidinyl)-1H-thieno[3,4-d]pyrimidin-2-on-trifluoracetat

a) 4-(Ethoxycarbonylamino)-thiophen-2-carbonsäuremethylester

Die Mischung aus 50.0 g (0.258 mol) 4-Aminothiophen-3-carbonsäuremethylester-hydrochlorid, 700 ml Toluol, 26 g (0.257 mol) Triethylamin und 27 ml (0.283 mol) Chlorkohlensäureethylester wurde 5 Stunden unter Rückfluß gekocht. Man filtrierte vom Unlöslichen, dampfte das Filtrat im Vakuum ein und kristallisierte den Rückstand aus Petrolether um. Man erhielt 59.0 g (99.8 % der Theorie) an farblosen Kristallen vom Fp. 52°C.

Entsprechend erhielt man aus 3-Aminothiophen-2-carbonsäuremethylester und Chlorkohlensäureethylester den kristallinen 3-(Ethoxycarbonylamino)-thiophen-2-carbonsäuremethylester in einer Ausbeute von 98.7 % der Theorie.
IR (KBr): 1739.7; 1622 $cm^{-1}$ (C=O, C=C)

b) 4-(Ethoxycarbonylamino)-thiophen-3-carboxaldehyd

In die eiskalte Suspension von 12.9 g (0.34 mol) Lithiumaluminiumhydrid in 800 ml tert.-Butyl-methylether wurde bei einer Reaktionstemperatur von ca. 0°C die Lösung von 59.1 g (0.258 mol) 4-(Ethoxycarbonylamino)-thiophen-3-carbonsäuremethylester in 200 ml tert.-Butyl-methylether eingetropft, die Mischung anschließend noch 2 Stunden bei 10°C gerührt. Dann tropfte man nacheinander 13 ml Wasser, 13 ml 2N wässerige Natronlauge und 39 ml Wasser zu und rührte 1 Stunde bei Zimmertemperatur. Man filtrierte, gab zum Filtrat portionsweise und unter Rühren 500 g aktiviertes Mangan(IV)-oxid. Nach Beendigung der dünnschichtchromatographisch verfolgbaren Reaktion wurde abermal filtriert, das Filtrat anschließend im Vakuum eingedampft. Der kristallin erstarrende Rückstand wurde ohne weitere Reinigung weiterverarbeitet. Ausbeute: 28.2 g (54.9 % der Theorie).

Entsprechend erhielt man aus 3-(Ethoxycarbonylamino)-thiophen-2-carbonsäuremethylester den 3-(Ethoxycarbonylamino)-thiophen-2-carboxaldehyd in einer Ausbeute von 71.9 % der Theorie.

c) 4-[[[1-(1,1-Dimethylethoxycarbonyl)-4-piperidinyl]amino]-methyl]-3-(ethoxycarbonylamino)-thiophen

Die Mischung aus 28.2 g (0.142 mol) 4-(Ethoxycarbonylamino)-thiophen-3-carboxaldehyd, 28.2 g (0.141 mol) 4-Amino-1-(1,1-dimethylethoxycarbonyl)piperidin und 300 ml Toluol wurde unter Verwendung eines Wasserabscheiders bis zur Beendigung der Wasserbildung unter Rückfluß gekocht. Das Lösemittel wurde im Vakuum entfernt, der Rückstand in 300 ml Methanol gelöst und bei Zimmertemperatur portionsweise mit 5.5 g (0.145 mol) Natriumborhydrid versetzt. Man rührte noch 1 Stunde bei Zimmertemperatur, dampfte dann im Vakuum ein und verteilte den Rückstand zwischen Wasser und tert.-Butyl-methylether. Die organische Phase wurde über Natriumsulfat getrocknet und im Vakuum vom Lösemittel befreit. Der ölige Rückstand wurde ohne Reinigung weiterverarbeitet. Ausbeute: 54.0 g (99.9 % der Theorie).

Entsprechend erhielt man aus 3-(Ethoxycarbonylamino)-thiophen-2-carboxaldehyd, 4-Amino-1-(1,1-dimethylethoxycarbonyl)piperidin und Natriumborhydrid das 2-[[[1-(1,1-Dimethylethoxycarbonyl)-4-piperidinyl]amino]methyl]-3-(ethoxycarbonylamino)-thiophen in einer Ausbeute von 100 % der Theorie.
IR (KBr): 1728.1; 1693.4 $cm^{-1}$ (C=O)

d) 3,4-Dihydro-3-[1-(1,1-dimethylethoxycarbonyl)-4-piperidinyl]-1H-thieno[3,4-d]pyrimidin-2-on

Die Lösung von 54.0 g (0.141 mol) 4-[[[1-(1,1-Dimethylethoxycarbonyl)-4-piperidinyl]amino]methyl]-3-(ethoxycarbonylamino)-thiophen in 300 ml Dimethylformamid wurde 4 Stunden lang unter Rückfluß gekocht. Nach Beendigung der dünnschichtchromatographisch verfolgbaren Reaktion wurde der noch warme Ansatz in 1 l Eiswasser eingerührt. Der kristalline Niederschlag wurde abgenutscht und im Umlufttrockenschrank bei 30°C getrocknet.
Ausbeute: 47.5 g (99.8 % der Theorie).

Entsprechend erhielt man aus 2-[[[1-(1,1-Dimethylethoxycarbonyl)-4-piperidinyl]amino]methyl]-3-(ethoxycarbonylamino)-thiophen das 3,4-Dihydro-3-[1-(1,1-dimethylethoxycarbonyl)-4-piperidinyl]-1H-thieno[3,2-d]pyrimidin-2-on in einer Ausbeute von 71 % der Theorie. Farblose Kristalle vom Fp. 200°C (Acetonitril).
IR (KBr): 1683.8; 1654.8 $cm^{-1}$ (C=O)

e) 3,4-Dihydro-3-(4-piperidinyl)-1H-thieno[3,4-d]pyrimidin-2-on-trifluoracetat

Die Mischung von 10.0 g (0.0296 mol) 3,4-Dihydro-3-[1-(1,1-dimethylethoxycarbonyl)-4-piperidinyl]-1H-thieno[3,4-d]pyrimidin-2-on und 50 ml Trifluoressigsäure wurde 30 Minuten bei Zimmertemperatur gerührt. Der nach Entfernung der überschüssigen Trifluoressigsäure verbleibende Rückstand wurde mit Diethylether verrieben und abgenutscht. Man erhielt 5.8 g (55.8 % der Theorie) an farblosen Kristallen, die ohne weitere Reinigung verwendet wurden.
IR (KBr): 1664.5 $cm^{-1}$ (C=O)

Entsprechend erhielt man aus 3,4-Dihydro-3-[1-(1,1-dimethylethoxycarbonyl)-4-piperidinyl]-1H-thieno[3,2-d] pyrimidin-2-on und Trifluoressigsäure das kristalline 3,4-Dihydro-3-(4-piperidinyl)-1H-thieno[3,2-d]pyrimidin-2-on-trifluoracetat in einer Ausbeute von 100 % der Theorie.
IR (KBr): 1685.7; 1656.8 $cm^{-1}$ (C=O)

Beispiel A6

3,4-Dihydro-3-(4-piperidinyl)-2(1H)chinolon-hydrochlorid

Die Mischung aus 1.1 g (4.949 mmol) 3-(4-Pyridinyl)-2(1H)-chinolon (D. R. Bragg und D. G. Wibberley, J. Chem. Soc. 1961, 5074 - 5077), 100 ml Ethanol, 5 ml (5 mmol) 1 N Salzsäure und 0.2 g Platin(IV)-oxid wurde 4 Stunden lang bei Zimmertemperatur hydriert. Der Katalysator wurde abfiltriert, das Filtrat im Vakuum eingedampft, der Rückstand mit Isopropanol verrieben. Die ausgefallenen Kristalle wurden abgerutscht, mit Isopropanol und Diethylether gewaschen und im Vakuum getrocknet. Ausbeute: 0.64 g (56.2 % der Theorie).
IR (KBr): 1666.4 $cm^{-1}$ (C=O)
MS: $M^+$ = 230
m/e = 146, 84

Beispiel A7

3-(4-Piperidinyl)-2(1H)-chinolon

Die Mischung aus 8.6 g (0.0387 mol) 3-(4-Pyridinyl)-2(1H)-chinolon, 1.2 l Ethanol, 39 ml (0.039 mol) 1 N Salzsäure und 8.0 g 10proz. Palladiumkohle wurde bis zur Aufnahme von ca. 0.08 mol Wasserstoff bei einer Temperatur von 40°C hydriert. Die Mischung wurde vom Katalysator befreit, das Filtrat im Vakuum eingedampft, der Rückstand in 200 ml Wasser aufgenommen und ammoniakalisch gestellt. Man gab Kochsalz bis zur Sättigung zu und extrahierte kontinuierlich mit Dichlormethan unter Verwendung eines Perforators. Die Dichlormethanphase wurde eingedampft, der verbleibende Rückstand an Kieselgel unter Verwendung von FM1 zum Eluieren chromatographisch von Nebenprodukten getrennt. Die geeigneten Fraktionen wurden vereinigt, vom Lösemittel befreit, in wenig Isopropanol gelöst und mit ethanolischer Chlorwasserstoff-Lösung ins Hydrochlorid überge. Farblose Kristalle. Ausbeute: 2.68 g (26.2 % der Theorie).
MS: $M^+$ = 228
IR (KBr): 1651 $cm^{-1}$ (C=O)

Beispiel A8

5-Chlor-3,4-dihydro-3-(4-piperidinyl)-2(1H)-chinazolinon

Die eiskalte Lösung von 6.3 g (0.0177 mol) 5-Chlor-3,4-dihydro-3-[1-(phenylmethyl)-4-piperidinyl]-2(1H)-chinazolinon (hergestellt analog Beispiel A4e)) in 50 ml Dichlormethan wurde unter Einhaltung einer Reaktionstemperatur von 0°C tropfenweise mit 3.34 g (0.0234 mol) Chlorkohlensäure-α-chlorethylester versetzt, wonach man langsam auf Zimmertemperatur kommen ließ. Das Reaktionsgemisch wurde im Vakuum eingeengt, der Rückstand in 50 ml Methanol aufgenommen und 4 Stunden unter Rückfluß gekocht. Nach dem Erkalten nutschte man den entstandenen farblosen Niederschlag ab. Ausbeute: 2.0 g (42.5 % der Theorie).
IR (KBr): 1666.4 $cm^{-1}$ (C=O)

Beispiel A9

6-Brom-3,4-dihydro-3-(4-piperidinyl)-2(1H)-chinazolinonhydrobromid

Zur Lösung von 6.16 g (0.075 mol) Natriumacetat und 11.565 g (0.05 mol) 3,4-Dihydro-3-(4-piperidinyl)-2(1H)-chinazolinon in einem Gemisch aus 150 ml Eisessig und 35 ml Wasser tropfte man unter Rühren und Einhaltung einer Reaktionstemperatur von 13 bis 15°C die Lösung von 8.8 g (0.055 mol) wasserfreiem Brom in 20 ml Eisessig. Man filtrierte und dampfte das Filtrat im Vakuum ein. Der Rückstand wurde zwecks Entfernung anorganischer Bestandteile fünfmal in jeweils 50 ml Dichlormethan aufgenommen, filtriert und eingedampft, dann mit wenig Acetonitril verrieben, wobei Kristallisation eintrat. Man nutschte ab, wusch mit Acetonitril/Diethylether (1/1 v/v) und erhielt nach dem Trocknen im Vakuum 5.5 g an farblosen Kristallen vom Fp. 288°C (Z). Durch Aufarbeiten der Mutterlaugen erhielt man weitere 4.5 g an Material gleicher Qualität. Gesamtausbeute: 10.0 g (51 % der Theorie).

| $C_{13}H_{17}Br_2N_3O$ (391.10) | | | | |
|---|---|---|---|---|
| Ber. | C 39.92 | H 4.38 | Br 40.86 | N 10.74 |
| Gef. | 39.72 | 4.36 | 41.56 | 10.24 |

IR (KBr) : 1670.3 cm-1 (C=O)

Beispiel A10

3-(4-Piperidinyl)-2,4(1H,3H)-chinazolindion

a) 2-Amino-N-[1-(phenylmethyl)-4-piperidinyl]-benzamid

Zur eiskalten Lösung von 28 ml (134 mmol) 4-Amino-1-(phenylmethyl)piperidin in 200 ml Tetrahydrofuran gab man portionsweise 21.9 g ( 134 mmol) Isatosäureanhydrid. Die entstandene Suspension wurde 2½ Stunden bei Zimmertemperatur und 2½ Stunden bei Rückflußtemperatur gerührt, danach vom Lösemittel befreit. Der Rückstand wurde in 100 ml heißem Ethanol gelöst, die entstandene Lösung nach Zugabe von 5 g Aktivkohle heiß filtriert. Der nach dem Erkalten ausgefallene Kristallbrei wurde abgenutscht, mit Diisopropylether gewaschen und im Vakuum bei 50°C getrocknet. Man erhielt 28.3 g an farblosen Kristallen. Aus den vereinigten Mutterlaugen ließen sich weitere 5.1 g eines Produktes gleicher Qualität isolieren. Gesamtausbeute: 33.4 g (80.6 % der Theorie).
IR (KBr) : 1620 $cm^{-1}$ (C=O)
MS: $M^+$ = 309

b) 3-[1-(Phenylmethyl)-4-piperidinyl]-2,4(1H,3H)-chinazolindion

Hergestellt analog Beispiel A3e) aus 2-Amino-N-[1-(phenylmethyl)-4-piperidinyl]-benzamid und N,N'-Carbonyldiimidazol in einer Ausbeute von 97.8 % der Theorie. Farblose Kristalle vom Fp. 223°C.

IR (KBr): 1720; 1647 $cm^{-1}$ (C=O)
MS: $M^+$ = 335

c) 3-(4-Piperidinyl)-2,4(1H,3H)-chinazolindion

Hergestellt analog Beispiel A3f) aus 3-[1-(Phenylmethyl)-4-piperidinyl]-2,4(1H,3H)-chinazolindion durch Hydrogenolyse in Gegenwart von Palladiumkohle in einer Ausbeute von 70 % der Theorie.
$R_f$: 0.075 (FM1)
IR (KBr): 1703; 1657 $cm^{-1}$ (C=O)

Beispiel A11

3,4-Dihydro-3-[1-(4-piperidinyl)-4-piperidinyl]-2(1H)-chinazolinon

a) 3,4-Dihydro-3-[1-[1-(phenylmethyl)-4-piperidinyl]-4-piperidinyl]-2(1H)-chinazolinon

Die Mischung aus 5.75 g (0.0249 mol) 3,4-Dihydro-3-(4-piperidinyl)-2(1H)-chinazolinon, 4.75 g (0.0251 mol) 1-(Phenylmethyl)-4-piperidinon und 100 ml Ethanol wurde 30 Minuten lang im Ultraschallbad behandelt, dann mit 9.5 ml (0.031 mol) Titan(IV)-isopropylat versetzt, wobei nach 10 Minuten ein Kristallbrei entstand. Anschließend erwärmte man unter weiterer Verwendung des Ultraschallbads 2½ Stunden auf maximal 35°C, ließ dann auf Zimmertemperatur abkühlen und setzte portionsweise 1.05 g (0.0167 mol) Natriumcyanoborhydrid zu, wobei man mittels verdünnter methanolischer Chlorwasserstofflösung den pH auf 5 - 6 hielt, und hielt 24 Stunden bei Zimmertemperatur. Nach dieser Zeit gab man abermals 1.05 g (0.0167 mol) Natriumcyanoborhydrid zu und verfuhr wie oben. Nach insgesamt 48 Stunden Reaktionszeit zersetzte man durch Zugabe von Wasser und arbeitete wie üblich auf. Das anfallende Rohprodukt wurde an Kieselgel unter Verwendung von FM4 zum Eluieren säulenchromatographisch gereinigt. Man erhielt 7.05 g (70 % der Theorie) einer farblosen kristallinen Substanz.
Entsprechend erhielt man aus Tropinon und 1-(Phenylmethyl)piperazin das exo-4-(8-Methyl-8-azabicyclo [3,2,1]oct-3-yl)-1-(phenylmethyl)piperazin in einer Ausbeute von 48.9 % der Theorie. Farblose, amorphe Substanz vom $R_f$ = 0.36 (FM1).

b) 3,4-Dihydro-3-[1-(4-piperidinyl)-4-piperidinyl]-2(1H)-chinazolinon

Hergestellt analog Beispiel A3f) aus 3,4-Dihydro-3-[1-(1-(phenylmethyl)-4-piperidinyl]-4-piperidinyl]-2(1H)-chinazolinon durch Hydrogenolyse, jedoch unter Verwendung von Pearlmans Katalysator, in einer Ausbeute von 92 % der Theorie. Farblose Kristalle vom $R_f$ = 0.48 (Macherey-Nagel, POLYGRAM® SIL G/$UV_{254}$ Fertigfolien für die DC; Fließmittel: Dichlormethan/Methanol/Cyclohexan/konz. Amoniak 68/20/10/5 v/v/v/v).
IR (KBr): 1660.6 $cm^{-1}$ (C=O)
MS: $M^+$ = 314

Beispiel A12

3-(4-Piperidinyl)-3,4,4a,5,6,7,8,8a-octahydro-2(1H)-chinazolinon-acetat

Die Lösung von 5.0 g (17.17 mmol) 3,4-Dihydro-3-(4-piperidinyl)-2(1H)-chinazolinon-acetat in 70 ml Methanol wurde bei Zimmertemperatur und in Gegenwart von 1.0 g Rhodium(III)-oxid-Platin(IV)-oxidhydrat-Katalysatcr (46.45 % Rhodium, 20.15% Platin) bis zur Beendigung der Wasserstoffaufnahme hydriert. Man befreite vom Katalysator und vom Lösemittel, verrieb den Rückstand mit 10 ml Diisopropylether und einigen Tropfen Isopropanol und nutschte das entstandene Kristallisat ab. Nach dem Trocknen im Vakuum erhielt man 4.4 g (86.2 % der Theorie) an farblosen Kristallen vom $R_f$ = 0.3 (Fließmittel: Dichlormethan/Methanol/konz. Ammoniak 7.5/2.5/0.5 v/v/v).
IR (KBr): 1641 $cm^{-1}$ (C=O)
MS: $M^+$ = 237

Beispiel A13

1,1-Dioxido-2-(4-piperidinyl)-3(4H)-1,2,4-benzothiadiazinon

a) 2-Nitro-N-[1-(phenylmethyl)-4-piperidinyl]-benzensulfonsäureamid

Unter äußerer Kühlung mit Eiswasser tropfte man zur Lösung von 38.0 g (0.2 mol) 4-Amino-1-(phenylmethyl) piperidin und 22.0 g (0.22 mol) Triethylamin in 250 ml Chloroform die Lösung von 44.3 g (0.2 mol) 2-Nitrobenzensulfcnylchlorid in 250 ml Chloroform. Nach Entfernung der Kühlung wurde noch weitere 30 Minuten bei Zimmertemperatur gerührt, die Reaktionsmischung dann zweimal mit je 1 l Wasser ausgeschüttelt. Die wässerigen Auszüge wurden noch einmal mit 100 ml Dichlormethan extrahiert, die vereinigten organischen Phasen anschließend über Natriumsulfat getrocknet und im Vakumm eingedampft. Die in einer Ausbeute von 75.0 g (99.9 % der Theorie) erhaltene hochviskose, hellbraune Substanz wurde ohne weitere Reinigung weiterverarbeitet.
IR (KBr): 3363.7 (NH); 1541.0 ($NO_2$); 1365.5 ($NO_2$ oder $SO_2$); 1346.2 ($NO_2$ oder $SO_2$); 1168.8 ($SO_2$) $cm^{-1}$

b) 2-Amino-N-[1-(phenylmethyl)-4-piperidinyl]-benzensulfonsäureamid

Zu der Lösung von 75.0 g (0.2 mol) 2-Nitro-N-[1-(phenylmethyl)-4-piperidinyl]-benzensulfonsäureamid in 2.0 l Ethanol tropfte man bei Zimmertemperatur die Lösung von 174.0 g ( 0.828 mol) Natriumdithionit-dihydrat in 700 ml Wasser. Nach Abklingen der exothermen Reaktion erhitzte man 4.5 Stunden unter Rückfluß, destillierte dann das Ethanol ab und extrahierte die verbliebene wässerige Phase erschöpfend mit Dichlormethan.
Die vereinigten Dichlormethanauszüge wurden über Natriumsulfat getrocknet und eingedampft, der verbliebene Rückstand an Kieselgel unter Verwendung von Dichlormethan/Methanol/konz. Ammoniak 80/20/0.25 (v/v/v) zum Eluieren säulenchromato-graphisch gereinigt. Man erhielt 6.5 g (8.6 % der Theorie) eines hochviskosen Öls.
IR(KBr): 1319.2, 1153.4 $cm^{-1}$ ($SO_2$)

c) 1,1-Dioxido-2-[1-(phenylmethyl)-4-piperidinyl]-3(4H)-1,2,4-benzothiadiazinon

Hergestellt analog Beispiel A3e) aus 2-Amino-N-[1-(phenylmethyl)-4-piperidinyl]-benzensulfonsäureamid und N,N'-Carbonyldiimidazol in einer Ausbeute von 78 % der Theorie. Farblose Kristalle vom Fp. 169-171°C.
IR(KBr): 1693.4 (C=O); 1359.7, 1340.4, 1188.1 ($SO_2$) $cm^{-1}$

d) 1,1-Dioxido-2-(4-piperidinyl)-3(4H)-1,2,4-benzothiadiazinon

Hergestellt analog Beispiel A3f), jedoch unter Verwendung von Pearlmans Katalysator an Stelle von Palladiumkohle, in einer Ausbeute von 90 % der Theorie. Farblose, amorphe Substanz.
IR(KBr): 1705.0 (C=O) $cm^{-1}$

Beispiel A14

3,4-Dihydro-2,2-dioxido-3-(4-piperidinyl)-2,1,3-benzothiadiazin

a) 3,4-Dihydro-2,2-dioxido-3-[1-(phenylmethyl)-4-piperidinyl]-2,1,3-benzothiadiazin

Bei Rückflußtemperatur wurde zu einer Lösung von 3.4 g (0.0354 mol) Sulfamid in 200 ml Pyridin die Lösung von 11.0 g (0.0372 mol) 2-Amino-N-[1-(phenylmethyl)-4-piperidinyl]-benzenmethanamin in 200 ml Pyridin innerhalb von 1.5 Stunden zugetropft und die Mischung anschließend 6 Stunden unter Rückfluß gekocht. Die Mischung wurde vom Lösemittel befreit, der Rückstand unter Verwendung von Essigsäureethylester/Methanol 9/1 (v/v) zum Eluieren säulenchromatographisch gereinigt. Man erhielt 5.5 g (43.5 % der Theorie) einer farblosen, amorphen Substanz.
IR(KBr): 1344.3, 1166.9 $cm^{-1}$ ($SO_2$)

b) 3,4-Dihydro-2,2-dioxido-3-(4-piperidinyl)-2,1,3-benzothiadiazin

Hergestellt analog Beispiel A3f) aus 3,4-Dihydro-2,2-dioxido-3-[1-(phenylmethyl)-4-piperidinyl]-2,1,3-benzothiadiazin durch katalytische Hydrierung in Gegenwart von Palladiumkohle in quantitativer Ausbeute. Farblose, amorphe Substanz.
IR(KBr): 1263.3, 1105.1 $cm^{-1}$ ($SO_2$)

Beispiel A15

D,L-4-Phenyl-1-(4-piperidinyl)-imidazolidin-2,5-dion

a) $N^2$-(1,1-Dimethylethoxycarbonyl)-N-[1-(phenylmethyl)-4-piperidinyl]-D,L-phenylglycinamid

Die Mischung aus 10.0 g (0.0398 mol) $N^2$-(1,1-Dimethylethoxycarbonyl)-D,L-phenylglycin, 7.57 g (0.0398 mol) 4-Amino-1-(phenylmethyl)piperidin, 10 ml Triethylamin, 12.8 g (0.0399 mol) TBTU und 5.4 g (0.0353 mol) N-Hydroxybenzotriazol-hydrat in 200 ml THF-DMF-Gemisch (1/1 v/v) wurde über Nacht bei Zimmertemperatur gerührt. Der nach Entfernung des Lösemittels verbliebene Rückstand wurde in Essigsäureethylester aufgenommen, mit gesättigter Natriumhydrogencarbonat-Lösung gewaschen, über Natriumsulfat getrocknet und im Vakuum eingedampft. Man erhielt 14.8 g (87.8 % der Theorie) einer farblosen, amorphen Substanz.
IR(KBr): 1701.1, 1676.0, 1652.9 $cm^{-1}$ (C=O)
Analog erhielt man aus $N^2$-(1,1-Dimethylethoxycarbonyl)-D,L-phenylalanin und 4-Amino-1-(phenylmethyl) piperidin in einer Ausbeute von 85 % der Theorie $N^2$-(1,1-Dimethylethoxycarbonyl)-N-[1-(phenylmethyl)-4-piperidinyl] -D,L-phenylalaninamid. Farblose, amorphe Substanz vom $R_f$ = 0.83 (Fließmittel: Dichlormethan/Cyclohexan/Methanol/ konz. Ammoniak = 70/15/15/2 v/v/v/v).
IR(KBr): 1683.8, 1651.0 $cm^{-1}$ (C=O)

b) N-[1-(Phenylmethyl)-4-piperidinyl]-D,L-phenylglycinamid-bis-trifluoracetat

Hergestellt analog Beispiel A5e) aus $N^2$-(1,1-Dimethylethoxycarbonyl)-N-[1-(phenylmethyl)-4-piperidinyl]-D, L-phenylglycinamid und Trifluoressigsäure in quantitativer Ausbeute. Farblose, amorphe Substanz vom $R_f$ = 0.56 (FM1).
Analog erhielt man aus $N^2$-(1,1-Dimethylethoxycarbonyl)-N-[1-(phenylmethyl)-4-piperidinyl]-D,L-phenylalaninamid in einer Ausbeute von 92 % der Theorie das N-[1-(Phenylmethyl)-4-piperidinyl]-D,L-phenylalaninamid-bis-trifluoracetat.
IR(KBr) : 1670.3 $cm^{-1}$ (C=O)

c) D,L-4-Phenyl-1-[1-(phenylmethyl)-4-piperidinyl]-imidazolidin-2,5-dion

Hergestellt analog Beispiel A3e) aus N-[1-(Phenylmethyl)-4-piperidinyl)-D,L-phenylglycinamid und N,N'-Carbonyldiimidazol in einer Ausbeute von 57.3 % der Theorie. Farblose Kristalle vom $R_f$ = 0.68.
IR(KBr): 1774.4, 1712.7 $cm^{-1}$ (C=O)
Analog erhielt man aus N-[1-(Phenylmethyl)-4-piperidinyl)-D,L-phenylalaninamid in einer Ausbeute von 93 % der Theorie das D,L-4-(Phenylmethyl)-1-[1-(phenylmethyl)-4-piperidinyl]-imidazolidin-2,5-dion. Farblose, feine Kristalle vom $R_f$ = 0.6 (Fließmittel: Dichlormethan/Methanol/Cyclohexan/konz. Ammoniak = 7/1.5/1.5/0.2 v/v/v/v).
IR(KBr): 1764.8, 1708.8 $cm^{-1}$ (C=O)
MS: $M^+$ = 363

d) D,L-4-Phenyl-1-(4-piperidinyl)-imidazolidin-2,5-dion

Hergestellt analog Beispiel A3f) aus D,L-4-Phenyl-1-[1-(phenylmethyl)-4-piperidinyl]-imidazolidin-2,5-dion durch Hydrogenolyse in Gegenwart von Palladiumkohle in einer Ausbeute von 84.3 % der Theorie. Farblose, amorphe Substanz vom $R_f$ = 0.5.
IR(KBr): 1766.7, 1706.9 $cm^{-1}$ (C=O)
Analog erhielt man aus D,L-4-(Phenylmethyl)-1-(1-(phenylmethyl)-4-piperidinyl]-imidazolidin-2,5-dion das D, L-4-(Phenylmethyl)-1-(4-piperidinyl)-1-imidazolidin-2,5-dion. Farblose Kristalle vom $R_f$ = 0.24 (Fließmittel: Dichlormethan/Methanol/Cyclohexan/konz. Ammoniak = 7/1.5/1.5/0.2 v/v/v/v).
IR(KBr): 1766.7, 1705.0 $cm^{-1}$ (C=O)

Beispiel A16

1,3-Dihydro-3-(4-piperidinyl)-2(2H)-imidazo[4,5-c]chinolon

a) 1-[2-(Acetylamino)phenyl]-2-bromethanon

Zur siedenden Lösung von 50.0 g (0.282 mol) 1-[2-(Acetylamino)phenyl]ethanon in 400 ml Chloroform tropfte

man bei Zimmertemperatur 45.0 g (0.282 mol) trockenes Brom. Das Lösemittel wurde abdestilliert, der Rückstand zwischen Dichlormethan und gesättigter, eiskalter Natriumhydrogencarbonat-Lösung verteilt. Die organische Phase wurde über Natriumsulfat getrocknet, im Vakuum eingedampft, der Rückstand mit Diethylether verrieben und abgenutscht. Nach dem Trocknen im Vakuum erhielt man 35.4 g (49 % der Theorie) an farblosen Kristallen vom $R_f$ = 0.48 (Fließmittel: Petrolether/Essigsäureethylester 2/1 v/v).
IR(KBr): 1685.69, 1664.47 $cm^{-1}$ (C=O)
MS: $M^+$ = 255/257 (Br)

b) 4- [2- (Acetylamino)phenyl]-1,3-dihydro-1-[1-(phenylmethyl)-4-piperidinyl]-2H-imidazol-2-on

Zur Lösung von 26.3 g (0.138 mol) 4-Amino-1-(phenylmethyl)-piperidin und 17.8 g (0.138 mol) DIEA in 300 ml Dichlormethan tropfte man die Lösung von 35.4 g (0.138 mol) 1-(2-(Acetylamino)phenyl]-2-bromethanon in 150 ml Dichlormethan und hielt die Mischung anschließend noch 2 Stunden lang bei Zimmertemperatur. Unter äußerer Kühlung mit Eis gab man dann 13.5 g (0.20 mol) Natriumcyanat und 12 ml Eisessig zu und rührte die Mischung über Nacht im auftauenden Eisbad. Man wusch mit Wasser und gesättigter Natriumhydrogencarbonat-Lösung, trocknete über Natriumsulfat und befreite vom Lösemittel. Der Rückstand wurde mit 50 ml Essigsäureethylester-Methanol-Gemisch (9/1 v/v) verrieben, die entstandenen Kristalle abgenutscht, mit Essigester gewaschen und im Vakuum getrocknet. Man erhielt 37.0 g (68.7 % der Theorie) an farblosen Kristallen vom $R_f$ = 0.41 (Fließmittel: Dichlormethan/Methanol 9/1 v/v).
IR(KBr): 1678 $cm^{-1}$ (C=O)
MS: $M^+$ = 390 (Br)

c) 4-(2-Aminophenyl)-1,3-dihydro-1-[1-(phenylmethyl)-4-piperidinyl]-2H-imidazol-2-on

Die Mischung von 3.0 g (7.68 mmol) 4-[2-(Acetylamino)phenyl]-1,3-dihydro-1-[1-(phenylmethyl)-4-piperidinyl]-2H-imidazol-2-on, 50 ml 5 N Natronlauge und 25 ml Ethanol wurde 3 Stunden unter Rückfluß gekocht. Nach dem Erkalten wurde die organische Phase abgetrennt, über Natriumsulfat getrocknet und im Vakuum eingedampft. Man erhielt in quantitativer Ausbeute eine farblose, amorphe Substanz vom $R_f$ = 0.53 (Fließmittel: Dichlormethan/Methanol 9/1 v/v).

d) 1,3-Dihydro-3-[1-(phenylmethyl)-4-piperidinyl]-2(2H)-imidazo[4,5-c]chinolon

Die Lösung von 2.67 g (7.66 mmol) 4-(2-Aminophenyl)-1,3-dihydro-1-[1-(phenylmethyl)-4-piperidinyl]-2H-imidazol-2-on in 50 ml Chloroform wurde mit 3.0 g Paraformaldehyd versetzt und 3.5 Stunden unter Rückfluß gekocht. Der nach dem Abdampfen des Lösemittels verbliebene Rückstand wurde in 100 ml Methanol aufgenommen und mit methanolischer Chlorwasserstoff-Lösung sauer gestellt. Nach einstündigem Rühren bei Zimmertemperatur wurde in 300 ml gesättigter Natriumhydrogencarbonat-Lösung eingegossen. Die entstandene Mischung wurde mit Essigsäureethylester erschöpfend extrahiert, die vereinigten Extrakte über Natriumsulfat getrocknet und im Vakuum eingedampft. Der Rückstand wurde an Kieselgel unter Verwendung von FM4 zum Eluieren säulenchromatographisch gereinigt. Aus den geeigneten Fraktionen isolierte man 0.5 g (18.2 % der Theorie) einer farblosen, amorphen Substanz vom $R_f$ = 0.24 (FM4).
IR(KBr): 1689 $cm^{-1}$ (C=O)
MS: $M^+$ = 358 (Br)

e) 1,3-Dihydro-3-(4-piperidinyl)-2(2H)-imidazo[4,5-c]chinolon

Hergestellt analog Beispiel A3f) aus 1,3-Dihydro-3-[1-(phenylmethyl)-4-piperidinyl]-2(2H)-imidazo[4,5-c]chinolon durch Hydrogenolyse in Gegenwart von Palladiumkohle in einer Ausbeute von 98.5 % der Theorie. Farblose Kristalle vom $R_f$ = 0.63 (FM1).

Beispiel A17

Herstellung von β-(Methoxycarbonyl)-arenbutansäuren

3,5-Dibrom-4-hydroxy-β-(methoxycarbonyl)-benzenbutansäure

a) 4-(Phenylmethoxy)-benzaldehyd

Zu der Lösung von 36.6 g (0.3 mol) 4-Hydroxybenzaldehyd in 100 ml Ethanol tropfte man nacheinander die Lösung von 12.0 g (0.3 mol) Natriumhydroxid in 100 ml Wasser und die Lösung von 36.5 ml (0.307 mol) Benzylbromid in 100 ml Ethanol und hielt die Mischung anschließend 1 Stunde lang bei 50°C. Das Ethanol wurde, zuletzt im Vakuum, weitestgehend abdestilliert, die verbleibende wässerige Emulsion zwischen Wasser und Essigsäureethylester verteilt. Die Essigesterphase wurde über Natriumsulfat getrocknet und im Vakuum eingedampft. Der verbliebene Rückstand kristallisierte beim Verreiben mit Petrolether und wurde aus Diisopropylether umkristallisiert. Man erhielt 48.0 g ( 75.4 % der Theorie) an farblosen Kristallen vom Fp. 118 - 122°C.

b) 3-(Methoxycarbonyl)-4-[(4-phenylmethoxy)phenyl]-3-butensäure

Zu einer frisch bereiteten Lösung von 2.3 g (0.1 mol) Natrium in 300 ml wasserfreiem Methanol gab man 14.6 g (0.1 mol) Bernsteinsäuredimethylester und tropfte nach halbstündigem Rühren die Lösung von 21.2 g (0.1 mol) 4-(Phenylmethoxy)-benzaldehyd in 100 ml wasserfreiem Methanol zu. Danach kochte man 6 Stunden unter Rückfluß, destillierte das Methanol unter Normaldruck ab und hielt den verbleibenden Sumpf 30 Minuten bei einer Reaktionstemperatur von 80°C. Der erhaltene zähe Brei wurde in 1 l eines Eisessig-Wasser-Gemischs (1/1 v/v) eingerührt, das anfallende Gemisch mit Essigsäureethylester erschöpfend extrahiert. Die vereinigten Essigesterextrakte wurden ihrerseits mit gesättigter Kaliumcarbonat-Lösung ausgezogen. Die Kaliumcarbonat-Extrakte wurden vorsichtig mit Essigsäure angesäuert und anschließend mit Essigester erschöpfend extrahiert. Diese Extrakte wurden mit Wasser gewaschen, über Natriumsulfat getrocknet und vom Lösemittel im Vakuum befreit. Der Rückstand wurde an Kieselgel unter Verwendung von Dichlormethan/Petrolether/Eisessig 25/74/1 (v/v/v) säulenchromatographisch gereinigt. Man erhielt das farblose, teilweise kristallisierende Diastereomerengemisch in einer Ausbeute von 16.0 g (49 % der Theorie).
Rf = 0.68 (Fließmittel: Essigsäureethylester/Petrolether 1:2 v/v).
IR(KBr): 1699.2 $cm^{-1}$ (C=O)
Analog erhielt man:

(1) Aus 3-(Trifluormethyl)benzaldehyd und Bernsteinsäuredimethylester die 3-(Methoxycarbonyl)-4-[3-(trifluormethyl)-phenyl]-3-butensäure in einer Ausbeute von 21 % der Theorie.
IR(KBr): 1738, 1726 $cm^{-1}$ (C=O)
ESI-MS: $(M-H)^-$ = 287
$(M+H)^-$ = 289
$(M+Na)^+$ = 311

(2) Aus 1-Naphthaldehyd und Bernsteinsäuredimethylester die 3-(Methoxycarbonyl)-4-(1-naphthyl)-3-butensäure in einer Ausbeute von 60 % der Theorie.
Farbloses Öl
IR(KBr): 1712 $cm^{-1}$ (C=O)
MS: $M^+$ = 270

(3) Aus 3,5-Dimethyl-4-phenylmethoxybenzaldehyd und Bernsteinsäuredimethylester die 3-(Methoxycarbonyl)-4-[3,5-dimethyl-4-phenylmethoxyphenyl]-3-butensäure in einer Ausbeute von 66 % der Theorie.
Farbloses Öl, das ohne Reinigung weiterverarbeitet wurde.

(4) Aus 4-Amino-3,5-dibrombenzaldehyd und Bernsteinsäuredimethylester die 4-(4-Amino-3,5-dibromphenyl)-3-(methoxycarbonyl)-3-butensäure in einer Ausbeute von 21 % der Theorie.

(5) Aus 3-Phenylmethoxybenzaldehyd und Bernsteinsäuredimethylester die 3-(Methoxycarbonyl)-4-(3-phenylmethoxyphenyl)-3-butensäure in einer Ausbeute von 37 % der Theorie.

c) 4-Hydroxy-β-(methoxycarbonyl)-benzenbutansäure

Hergestellt analog Beispiel A3f) aus 3-(Methoxycarbonyl)-4-[(4-phenylmethoxy)phenyl]-3-butensäure durch Hydrogenolyse in Gegenwart von Palladiumkohle in einer Ausbeute von 96 % der Theorie. Farbloses Öl vom $R_f$ = 0.5 (Fließmittel: Essigsäureethylester/Petrolether/Eisessig 66.3/33.3/0.4 v/v/v).

Analog erhielt man:

(1) Aus 3- (Methoxycarbonyl)-4- [3-(trifluormethyl)phenyl]-3-butensäure die β-(Methoxycarbonyl)-3-(trifluormethyl)-benzenbutansäure in einer Ausbeute von 80 % der Theorie.
$R_f$ = 0.59 (Fließmittel: Essigsäureethylester/Petrolether 1/1/v/v).
ESI-MS: $(M-H)^-$ = 289

(2) Aus 3-(Methoxycarbonyl)-4-(1-naphthyl)-3-butensäure, jedoch unter Verwendung von Platin(IV)-oxid als Katalysator, die β-(Methoxycarbonyl)-1-naphthalinbutansäure in einer Ausbeute von 31 % der Theorie.
IR (KBr): 1734, 1711 (C=O) $cm^{-1}$
MS: $M^+$ = 272

Als Nebenprodukt isolierte man in einer Ausbeute von 8.4 % der Theorie β-(Methoxycarbonyl)-1,2,3,4-tetrahydro-1-naphthalinbutansäure.
IR (KBr): 1736, 1712 (C=O) $cm^{-1}$
MS: $M^+$ = 276

(3) Aus 3-(Methoxycarbonyl)-4-[3,5-dimethyl-4-phenylmethoxyphenyl]-3-butensäure die 3,5-Dimethyl-4-hydroxy-β-(methoxycarbonyl)-benzenbutansäure in einer Ausbeute von 48 % der Theorie.
$R_f$ = 0.11 (FM1)
IR (KBr): 1716 (C=O) $cm^{-1}$
MS: $M^+$ = 266

(4) Aus 3-(Methoxycarbonyl)-4-(3-phenylmethoxyphenyl)-3-butensäure die 3-Hydroxy-β-(methoxycarbonyl)-benzenbutansäure in einer Ausbeute von 59 % der Theorie.
$R_f$ = 0.24 (Petrolether/Essigsäureethylester/Eisessig 6/4/0.2 v/v/v)
IR (KBr): 1714 (C=O) $cm^{-1}$
MS: $M^+$ = 238

(5) Aus 3-(Methoxycarbonyl)-4-(4-amino-3,5-dibromphenyl)-3-butensäure und in Gegenwart von Triethylamin die 4-Amino-β-(methoxycarbonyl)-benzenbutansäure in quantitativer Ausbeute.
$R_f$ = 0.53 (Fließmittel: Dichlormethan/Methanol/Eisessig 90/10/1.5 v/v/v))
IR (KBr): 1728 (C=O) $cm^{-1}$
MS: $M^+$ = 237

d) 3,5-Dibrom-4-hydroxy-β-(methoxycarbonyl)-benzenbutansäure

Zur Lösung von 12.0 g (0.05 mol) 4-Hydroxy-β-(methoxycarbonyl)-benzenbutansäure in 200 ml Eisessig gab man 150 ml Wasser und 8.0 g Natriumacetat und tropfte dann die Lösung von 15.58 g (0.0975 mol) Brom in 60 ml Eisessig zu. Man ließ noch 1 Stunde bei Zimmertemperatur rühren, dampfte dann den Ansatz im Vakuum zu 2 Dritteln ein und verteilte den Rest zwischen Wasser und

Essigsäureethylester. Die Essigesterextrakte wurden mit Wasser gewaschen, über Natriumsulfat getrocknet und im Vakuum eingedampft. Nach dem Verrühren mit Diisopropylether erhielt man ein farbloses Kristallisat. Ausbeute: 12.0 g (62.2 % der Theorie).
$R_f$ = 0.4 (Fließmittel: Essigsäureethylester/Petrolether/Eisessig 49.8/49.8/0.4 v/v/v).
IR(KBr): 1724 $cm^{-1}$ (C=O)
MS: $M^+$ = 394/396/398 ($Br_2$)

Beispiel A18

1-(3-Pyridinyl)piperazin

a) 1-(Phenylmethyl)-3-(3-Pyridinyl)piperazin

Zur Lösung von 5.0 g (0.0515 mol) 3-Fluorpyridin und 43.5 ml 1-(Phenylmethyl)piperazin in 300 ml wasserfreiem Diethylether tropfte man bei Siedetemperatur und innerhalb von 2.5 Stunden 56 ml (0.112 mol) einer 2molaren Lösung von Phenyllithium in Cyclohexan-Diethylether-Gemisch (7/3 v/v) und hielt anschließend noch 4 Stunden bei Rückfluß-Temperatur. Das nach üblicher Aufarbeitung als Öl anfallende rohe Reaktionsprodukt wurde an Kieselgel (30 - 60 µm) unter Verwendung von FM1/Cyclohexan (7/3 v/v) zum Eluieren säulenchromatographisch gereinigt. Man erhielt 12.0 g (92 % der Theorie) eines farblosen Öls vom $R_f$ 0.52 (FM4; Macherey-Nagel, POLYGRAM® SIL G/UV254 Pre-coated plastic sheets for TLC).
MS: $M^+$ = 253

b) 1-(3-Pyridinyl)piperazin

Hergestellt analog Beispiel A3f) aus 1-(Phenylmethyl)-3-(3-Pyridinyl)piperazin durch Hydrogenolyse in Gegenwart von Palladiumkohle in einer Ausbeute von 55 % der Theorie. Farbloses Öl vom $R_f$ 0.35 (FM1).
IR(KBr): 1652.9 $cm^{-1}$ (C=N)

Beispiel A19

1-(1-Cyclohexyl-4-piperidinyl)piperazin-tris-trifluoracetat

a) 1-(1,1-Dimethylethoxycarbonyl)-4-[1-(phenylmethyl)-4-piperidinyl]piperazin

Die Lösung von 15.0 g ( 0.8054 mol) 1-(1,1-Dimethylethoxycarbonyl)piperazin und 14.26 ml (0.08053 mol) 1-(Phenylmethyl)-4-piperidinon in 250 ml Methanol wurde durch Zutropfen von Essigsäure auf einen pH zwischen 5 und 6 gebracht und portionsweise mit insgesamt 4.13 g ( 0.0624 mol) 95proz. Natriumcyanoborhydrid versetzt, wobei durch weiteres Zutropfen von Essigsäure auf Einhaltung eines pH von 5 bis 6 geachtet wurde. Nach 18stündigem Rühren bei Zimmertemperatur wurde das Gemisch im Vakuum eingedampft, der Rückstand sodaalkalisch gestellt und zwischen Wasser und Essigsäureethylester verteilt. Nach üblicher Aufarbeitung der Essigesterphase erhielt man 21.76 g (75.2 % der Theorie) eines hochviskosen, farblosen Öls vom $R_f$ 0.66 (FM1).

b) 1-(1,1-Dimethylethoxycarbonyl)-4-(4-piperidinyl)piperazin

Hergestellt analog Beispiel A3f) aus 1-(1,1-Dimethylethoxycarbonyl)-4-[1-(phenylmethyl)-4-piperidinyl]piperazin durch Hydrogenolyse, jedoch unter Verwendung von Pearlmans Katalysator an Stelle von Palladiumkohle, in einer Ausbeute von 79.7 % der Theorie.
Farblose Kristalle vom $R_f$ = 0.3 (FM1).

c) 1-(1,1-Dimethylethoxycarbonyl)-4-(1-cyclohexyl-4-piperidinyl)piperazin

Hergestellt analog Beispiel A19a) aus 1-(1,1-Dimethylethoxycarbonyl)-4-(4-piperidinyl)piperazin und Cyclohexanon in einer Ausbeute von 99 % der Theorie. Farbloses, hochviskoses Öl.
MS: $M^+$ = 251

d) 1-(1-Cyclohexyl-4-piperidinyl)piperazin-tris-trifluoracetat

Hergestellt analog Beispiel A5e) aus 1-(1,1-Dimethylethoxycarbonyl)-4-(1-cyclohexyl-4-piperidinyl)piperazin und Trifluoressigsäure in quantitativer Ausbeute. Farblose Kristalle vom $R_f$ = 0.2 (FM1).

Beispiel A20

1-(1-Ethyl-4-piperidinyl)piperazin-trihydrochlorid

a) 1-(1-Ethyl-4-piperidinyl)-4-(phenylmethyl)piperazin

Hergestellt analog Beispiel A19a) aus 1-Ethyl-4-piperidinon und 1-(Phenylmethyl)piperazin in einer Ausbeute von 71 % der Theorie. Farblose, amorphe Substanz vom $R_f$ = 0.46 (FM4).

b) 1-(1-Ethyl-4-piperidinyl)piperazin-trihydrochlorid

Die Mischung aus 36.3 g (0.126 mol) 1-(1-Ethyl-4-piperidinyl)-4-(phenylmethyl)piperazin, 300 ml 1 N Salzsäure und 200 ml Methanol wurde bei Zimmertemperatur und in Gegenwart von 4.0 g 10proz. Palladiumkohle bis zur Beendigung der Wasserstoffaufnahme hydriert. Nach üblicher Aufarbeitung erhielt man 22.9 g (59.3 % der Theorie) einer farblosen, kristallinen Substanz.
MS: $M^+$ = 197

Entsprechend erhielt man aus exo-4-(8-Methyl-8-azabicyclo-[3,2,1]oct-3-yl)-1-(phenylmethyl)piperazin (siehe Beispiel A11a)) durch Hydrogenolyse in Gegenwart von Palladiumkohle in einer Ausbeute von 91 % der Theorie das exo-1-(8-Methyl-8-azabicyclo[3,2,1]oct-3-yl)piperazin-trihydrochlorid.
MS: $M^+$ = 209

Beispiel A21

1-Ethyl-4-(4-piperidinyl)piperidin

a) 1-(Phenylmethoxycarbonyl)-4-(4-piperidinyl)piperidin

Zu der Mischung aus 72.375 g (0.3 mol) Bipiperidin-dihydrochlorid, 1500 ml Methanol, 75 ml Wasser und 100 mg Bromphenolblau tropfte man unter Rühren und bei Zimmertemperatur gleichzeitig die Lösung von 51.18 g (0.3 mol) Chlorkohlensäurebenzylester in 75 ml Toluol und 6 N Natronlauge (ca. 80 ml) so zu, daß die Indikatorfarbe laufend wechselte. Nach beendeter Zugabe, die ca. 4 Stunden erforderte, verdünnte man mit 300 ml Wasser und destillierte die organischen Lösemittel im Vakuum ab. Die verbliebene wässerige Phase wurde unter äußerer Kühlung mit Salzsäure angesäuert, mit Diethylether erschöpfend extrahiert und dann mit 50proz. Kalilauge alkalisch gestellt. Man extrahierte erschöpfend mit Dichlormethan, trocknete die vereinigten Dichlormethanauszüge über Magnesiumsulfat und dampfte sie im Vakuum ein. Das verbleibende farblose, hochviskose, langsam kristallisierende Öl wurde ohne weitere Reinigung weiterverarbeitet. Ausbeute: 87.3 g (96.2 % der Theorie).
IR (KBr) : 1701.1 $cm^{-1}$ (C=O)

b) 1-Ethyl-4-[1-(phenylmethoxycarbonyl)-4-piperidinyl]piperidin

Zu der Lösung von 18.14 g (0.061 mol) 1-(Phenylmethoxycarbonyl)-4-(4-piperidinyl)piperidin in 450 ml eines Methanol-Wasser-Gemischs (1/1 v/v) gab man unter Rühren und Einhaltung einer Temperatur von 15 bis 20°C 10.05 g (0.152 mol) 95proz. Natriumcyanoborhydrid sowie 50 mg Bromkresolpurpur. Nun tropfte man im Wechsel die Lösung von 10.57 g (0.24 mol) Acetaldehyd in 50 ml Methanol und 1 N Salzsäure so zu, daß die Farbe der Mischung laufend von Blau nach Gelb wechselte. Nach vollständiger Zugabe und beendeter Umsetzung wurde die Mischung mit Salzsäure auf pH 2 eingestellt und zweimal mit je 200 ml Diethylether ausgezogen. Die wässerige Phase wurde anschließend alkalisch gestellt und mit Dichlormethan erschöpfend extrahiert. Die vereinigten Dichlormethanauszüge wurden über Magnesiumsulfat getrocknet und im Vakuum eingedampft. Der verbleibende farblose, kristallisierende Rückstand wurde an Kieselgel (30-60 μm) unter Verwendung von FM1 zum Eluieren säulenchromatographisch gereinigt. Ausbeute an farblosen Kristallen vom Fp. 93-96°C: 7.9 g (39.2 % der Theorie).
IR(KBr): 1699.2 $cm^{-1}$ (C=O)

c) 1-Ethyl-4-(4-piperidinyl)piperidin

Die Lösung von 7.6 g (0.023 mol) 1-Ethyl-4-[1-(phenylmethoxycarbonyl)-4-piperidinyl]piperidin in einem Gemisch aus 70 ml Methanol, 30 ml wasser und 10 ml Eisessig wurde in Gegenwart von 10proz. Palladiumkohle bei Zimmertemperatur und 3 bar Wasserstoffdruck bis zur Beendigung der Wasserstoffaufnahme hydriert. Nach üblicher Aufarbeitung erhielt man die gesuchte Verbindung als farbloses Öl in quantitativer Ausbeute.

Beispiel A22

Hexahydro-1-methyl-4-(4-piperidinyl)-1H-1,4-diazepin

a) Hexahydro-1-methyl-4-[1-(phenylmethyl)-4-piperidinyl]-1H-1,4-diazepin

Hergestellt analog Beispiel A11a) aus Hexahydro-1-methyl-1H-1,4-diazepin und 1-(Phenylmethyl)-4-piperidinon in einer Ausbeute von 35 % der Theorie. Farbloses, viskoses Öl.
MS: $M^+$ = 287

Entsprechend wurden hergestellt:

(1) 1-Methyl-4-[1-(phenylmethyl)-4-piperidinyl]piperazin
aus 1-Methylpiperazin und 1-(Phenylmethyl)-4-piperidinon Ausbeute: 39.9 % der Theorie, farbloses, viskoses Öl

(2) 1-Acetyl-4-[1-(phenylmethyl)-4-piperidinyl]piperazin aus 1-Acetylpiperazin und 1-(Phenylmethyl)-4-piperidinon
Ausbeute: 24.2 % der Theorie, farbloses, viskoses Öl
$R_f$: 0.46 (Fließmittel: Essigsäureethylester/Methanol/konz. Ammoniak 50/50/2 v/v/v)
IR(KBr): 1647 $cm^{-1}$ (C=O)
MS: $M^+$ = 301

(3) 4-(Dimethylamino)-1-[1-(phenylmethyl)-4-piperidinyl]-piperidin
aus 4-(Dimethylamino)piperidin und 1-(Phenylmethyl)-4-piperidinon
Ausbeute: 28.9 % der Theorie; farbloses, viskoses Öl
$R_f$: 0.58 (Fließmittel: Essigsäureethylester/Methanol/konz. Ammoniak 50/50/2 v/v/v)
MS: $M^+$ = 301

(4) 1-(1,1-Dimethylethoxycarbonyl)-4-[4-(phenylmethyl)-1-piperazinyl]pineridin
aus 1-(1,1-Dimethylethoxycarbonyl)-4-piperidinon und 1-(Phenylmethyl)piperazin
Ausbeute: 86.6 % der Theorie. Farblose, amorphe Substanz
$R_f$: 0.58 (Fließmittel: Dichlormethan/Methanol 9/1 v/v)

b) Hexahydro-1-methyl-4-(4-piperidinyl)-1H-1,4-diazepin
Hergestellt analog Beispiel A3f) aus Hexahydro-1-methyl-4-[1-(phenylmethyl)-4-piperidinyl]-1H-1,4-diazepin durch Hydrogenolyse, jedoch unter Verwendung von Pearlmans Katalysator an Stelle von Palladiumkohle, in quantitativer Ausbeute. Farbloses, viskoses Öl.
MS: $M^+$ = 197

Entsprechend wurden erhalten:

(1) 1-Methyl-4-(4-piperidinyl)piperazin
aus 1-Methyl-4-[1-(phenylmethyl)-4-piperidinyl]piperazin in quantitativer Ausbeute. Farbloses, viskoses Öl.
MS : $M^+$ = 183

(2) 1-Acetyl-4-(4-piperidinyl)piperazin
aus 1-Acetyl-4-[1-(phenylmethyl)-4-piperidinyl]piperazin in einer Ausbeute von 81.9 % der Theorie. Farblose Kristalle.
IR(KBr): 1631 $cm^{-1}$ (C=O)

(3) 4-(Dimethylamino)-1-(4-piperidinyl)piperidin
aus 4-(Dimethylamino)-1-[1-(phenylmethyl)-4-piperidinyl]-piperidin in einer Ausbeute von 76.8 % der Theorie. Farblose, amorphe Substanz.

(4) 1-(1,1-Dimethylethoxycarbonyl)-4-(1-piperazinyl)piperidinhydrochlorid
aus 1-(1,1-Dimethylethoxycarbonyl)-4-[4-(phenylmethyl)-1-piperazinyl]piperidin-hydrochlorid.
Ausbeute: 96 % der Theorie. Farblose Kristalle
$R_f$ : 0.23 (Fließmittel: Dichlormethan/Methanol 9/1 v/v)

Beispiel A23

4-[(4-Methyl-1-piperazinyl)carbonyl]-piperidin-bis-trifluoracetat

a) 1-(1,1-Dimethylethoxycarbonyl)-4-piperidincarbonsäure

Zur Mischung aus 25.9 g (0.2 mol) Piperidin-4-carbonsäure, 200 ml (0.2 mol) 1 N Natronlauge und 200 ml Tetrahydrofuran gab man 48.0 g (0.22 mol) Pyrokohlensäure-di-tert.-butylester und rührte über Nacht bei Zimmertemperatur. Das Tetrahydrofuran wurde, zuletzt im Vakuum, abdestilliert und die verbleibende wässerige Lösung mit Zitronensäure angesäuert. Die ausgefallenen farblosen Kristalle wurden abgenutscht und im Umlufttrockenschrank bei 40°C getrocknet. Ausbeute: 45.5 g (99.2 % der Theorie).
IR(KBr): 1733.9, 1662.5 $cm^{-1}$ (C=O)

b) 1-(1,1-Dimethylethoxycarbonyl)-4-[(4-methyl-1-piperazinyl)-carbonyl]-piperidin

Hergestellt analog Beispiel A15a) aus 1-(1,1-Dimethylethoxycarbonyl)-4-piperidincarbonsäure und 1-Methylpiperazin in Gegenwart von TBTU in einer Ausbeute von 76 % der Theorie. Farblose, amorphe Substanz vom $R_f$ = 0.64 (Fließmittel: Dichlormethan/Methanol/konz. Ammoniak 50/50/1 v/v/v).
IR(KBr): 1693, 1678 $cm^{-1}$ (C=O)

Entsprechend wurden hergestellt:

(1) 1-Methyl-4-[[4-(1,1-dimethylethoxycarbonyl)-1-piperazinyl]carbonyl]-piperidin
aus 1-Methyl-4-piperidincarbonsäure und 1-(1,1-Dimethylethoxycarbonyl)piperazin in einer Ausbeute von 97 % der Theorie. Farblose Kristalle.
IR(KBr): 1683.8, 1629.8 $cm^{-1}$ (C=O)

(2) 1-(1,1-Dimethylethoxycarbonyl)-4- (isonicotinoyl)piperazin
aus 1-(1,1-Dimethylethoxycarbonyl)- piperazin und 4-Pyridincarbonsäure in einer Ausbeute von 76.8 % der Theorie. Farblose Kristalle vom Fp. 139.2-140.2°C und $R_f$ = 0.84 (Fließmittel: Dichlormethan/Methanol/konz. Ammoniak 90/10/1 v/v/v).
IR(KBr): 1689.5, 1625.9 $cm^{-1}$ (C=O)

c) 4-[(4-Methyl-1-piperazinyl)carbonyl]-piperidin-bis-trifluoracetat

Hergestellt analog Beispiel A5e) aus 1-(1,1-Dimethylethoxycarbonyl)-4-[(4-methyl-1-piperazinyl)carbonyl]-piperidin und Trifluoressigsäure in einer Ausbeute von 89 % der Theorie. Farblose, amorphe Substanz.

Entsprechend wurden hergestellt:

(1) 1-Methyl-4-[(1-piperazinyl)carbonyl]-piperidin
aus 1-Methyl-4-[[4-(1,1-dimethylethoxycarbonyl)-1-piperazinyl]carbonyl]-piperidin und Trifluoressigsäure in einer Ausbeute von 57 % der Theorie. Farblose, amorphe Substanz.
IR(KBr): 1679.9, 1645.2 $cm^{-1}$ (C=O)
MS: $M^+$ = 211

(2) 4-(Isonicotinoyl)piperazin-trifluoracetat
aus 1-(1,1-Dimethylethoxycarbonyl)-4-(isonicotinoyl)piperazin und Trifluoressigsäure in einer Ausbeute von 98.3 % der Theorie. Farblose, amorphe Substanz.
IR(KBr): 1676.0 $cm^{-1}$ (C=O)

Beispiel A24

Herstellung von Verbindungen der allgemeinen Struktur:

$$Boc—A\text{-}NR^3R^4$$

1-[$N^2$-(1,1-Dimethylethoxycarbonyl)-$N^6$-(phenylmethoxycarbonyl)-L-lysyl]-4-(4-pyridinyl)piperazin

Zu der Mischung aus 18.8 g (0.0494 mol) Boc-Lys(Z)-OH, 6.5 g (0.05 mol) DIEA, 16 g (0.05 mol) TBTU, 6.6 g (0.049 mol) HOBt und 100 ml Dimethylformamid tropfte man unter Rühren 8.1 g (0.0494 mol) 1-(4-Pyridinyl)piperazin, gelöst in 40 ml DMF,zu und rührte über Nacht bei Raumtemperatur. Das Lösemittel wurde im Vakuum entfernt und der Rückstand in Essigsäureethylester aufgenommen. Die Essigesterphase wurde anschließend nacheinander dreimal mit 70 ml gesättigter wässeriger Natriumhydrogencarbonat-Lösung und einmal mit 70 ml gesättigter wäßriger Kochsalzlösung gewaschen, über Natriumsulfat getrocknet und im Vakuum eingeengt. Man erhielt 24.2 g (93.2% der Theorie) eines gelblichen Öls, das ohne weitere Reinigung für die nachfolgenden Umsetzungen eingesetzt wurde.
IR (KBr): 1650, 1713 $cm^{-1}$ (C=O)
$R_f$ (FM1) : 0.59

Analog wurden hergestellt:

| A | $NR^3R^4$ | Anmerkungen | % Ausbeute | $R_f$ | Fließmittel | IR [$cm^{-1}$] |
|---|---|---|---|---|---|---|
| A9 | C1 | THF als LM $KHSO_4$/NaCl Lsg | 63.2 | 0.4 | FM1 | (KBr): C=O 1705.0/1649 |
| A4 | C1 | | 93.2 | 0.59 | FM1 | (KBr): C=O 1647.7: 1712.7 |
| A5 | C1 | | 66 | 0.55 | FM1 | (KBr): C=O 1655/1709 |
| A5 | C8 | | 54 | 0.8 | FM1 | (KBr): C=O 1653/1713 |
| A6 | C8 | | 91 | 0.8 | FM1 | (KBr): C=O 1645/1710.8 |
| A10 | C1 | | 63 | 0.5 | FM1 | (KBr): C=O 1665/1695 |
| A10 | C8 | | 30 | 0.41 | FM4 | (KBr): C=O 1662/1699 |

Beispiel A25

Herstellung von Verbindungen der allgemeinen Formel:

$$Cbz\text{-}A\text{-}NR^3R^4$$

1-[$N^2$-(Phenylmethoxycarbonyl)-$N^6$-(1,1-dimethylethoxycarbonyl)-L-lysyl]-4-(4-pyridinyl)piperazin

Zu der Mischung aus 100 g (0.263 mol) Z-Lys(Boc)-OH, 86.1 g (0.268 mol) TBTU und 36.3 g (0.263 mol) HOBt in 600 ml Dimethylformamid wurden 43.0 g (0.263 mol) 1-(4-Pyridinyl)-piperazin und 47.2 ml (0.268 mol) DIEA unter Rühren zugegeben und die Mischung über Nacht bei Raumtemperatur gerührt. Das Reakticnsgemisch wurde im Vakuum eingeengt und der Rückstand zwischen Essigsäureethylester und wäßriger gesättigter Natriumhydrogencarbonat-Lösung verteilt. Die wässerige Phase wurde noch zweimal mit einer Mischung aus Essigester/Methanol (10/1 , v/v) extrahiert und die vereinigten organischen Phasen einmal mit gesättigter Natriumhydrogencarbonat-Lösung gewaschen. Die organische Phase wurde nach dem Trocknen über Natriumsulfat im Vakuum eingeengt und der Rückstand in 750 ml Essigester aufgenommen und viermal mit je 100 ml Wasser, sechsmal mit je 100 ml 1-proz. Kaliumhydrogensulfat-Lösung, einmal mit 100 ml Wasser, zweimal mit je 100 ml 3-proz. wässeriger Ammoniak-Lösung sowie zweimal mit je 100 ml Wasser gewaschen. Die organische Phase wurde nach dem Trocknen über Natriumsulfat eingeengt. Man erhielt 120 g (87% der Theorie ) des gesuchten Produktes als Öl, das ohne weitere Reinigung für die nachfolgenden Umsetzungen eingesetzt wurde.
IR (KBr) : 1709 $cm^{-1}$ (C=O)
$R_f$ (FM1) : 0.59
EI - MS: $M^+$ = 525

Analog wurden hergestellt:

| A | $NR^3R^4$ | Anmerkungen | % Ausbeute | MS | $R_f$ | Fließmittel | IR [$cm^{-1}$] |
|---|---|---|---|---|---|---|---|
| A3 | C4 | | 100 | | | | |
| A3 | C3 | Triethylamin als Base | 100 | | 0.8 | FM1 | (KBr): C=O 1643.3/1710.8 |
| A11 | C1 | | 98.8 | | 0.5 | FM1 | (KBr): C=O 1705.0/1643.3 |
| A3 | C1 | | 81 | EI: M+=525 | 0.59 | FM1 | (KBr): C=O 1708.8: |
| A3 | C5 | LC/$SiO_2$/FM4 | 95 | YED:M=525 | 0.67 | FM4 | |
| A3 | C6 | THF,LC/$SiO_2$/FM4 | 92 | | 0.82 | FM4 | (KBr): C=O 1710.8:1641.3 |
| A3 | C8 | als Rohprodukt weiter umgesetzt | 100 | | | | |

Beispiel A26

Herstellung von Verbindungen der allgemeinen Formel:

$$H—A\text{-}NR^3R^4$$

1-[$N^6$-(Phenylmethoxycarbonyl)-L-lysyl]-4-(4-pyridinyl)piperazin

Zu der Mischung aus 24.2 g (46 mmol) 1-[$N^2$-(1,1-Dimethylethoxycarbonyl)-$N^6$-(phenylmethoxycarbonyl)-L-lysyl]-4-(4-pyridinyl)piperazin und 150 ml Methylenchlorid wurden 50 ml Trifluoressigsäure gegeben und der Reaktionsansatz über Nacht bei Raumtemperatur gerührt. Das Reaktionsgemisch wurde durch Zugabe von gesättigter wäßriger Natriumhydrogencarbonat-Lösung neutralisiert, die organische Phase getrocknet und im Vakuum eingeengt. Man erhielt 12 g (62 % der Theorie) der gesuchten Verbindung als farbloses Öl.
IR (KBr): 1648 $cm^{-1}$ (C=O)
$R_f$ (FM1): 0.5

Analog wurden hergestellt:

| A | $NR^3R^4$ | Anmerkungen | % Ausbeute | $R_f$ | Fließmittel | IR [$cm^{-1}$] |
|---|---|---|---|---|---|---|
| A9 | C1 | | 100 | 0.4 | FM2 | (KBr): C=O 1676.0: 1645.2 |
| A4 | C1 | | 61.5 | 0.48 | FM1 | (KBr): C=O 1647.7: 1712.7 |
| A5 | C1 | | 55 | 0.42 | FM1 | (KBr): C=O 1651 |
| A5 | C8 | als Rohprodukt weiter umgesetzt | 100 | 0.19 | FM1 | |
| A6 | C1 | | 82 | 0.3 | FM1 | (KBr): C=O 1647: 1676 |
| A6 | C8 | als Rohprodukt weiter umgesetzt | 100 | 0.23 | FM1 | (KBr): C=O 1674 |
| A10 | C1 | | 38 | 0.55 | FM1 | (KBr): C=O 1643 |
| A10 | C8 | als Rohprodukt weiter umgesetzt | 100 | 0.15 | FM1 | |

Beispiel A27

Herstellung von Verbindungen der allgemeinen Formel:

$$H—A\text{-}NR^3R^4$$

1-[$N^6$-(1,1-Dimethylethoxycarbonyl)-L-lysyl]-4-(4-pyridinyl)-piperazin

Die Lösung von 120 g (0.228 mol) 1-[$N^2$-(Phenylmethoxycarbonyl)-$N^6$-(1,1-dimethylethoxycarbonyl)-L-lysyl]-4-(4-pyridinyl)piperazin in 1000 ml Methanol und 240 ml 1M wässeriger Kaliumhydrogensulfat-Lösung wurde in Gegenwart von 30 g Palladium auf Kohle (10proz.) bei 20°C und 3 bar Wasserstoffdruck bis zur Beendigung der Wasserstoffaufnahme hydriert. Der Katalysator wurde abfiltriert, das Filtrat im Vakuum eingedampft, der Rückstand in Isopropanol / Methanol aufgenommen und durch Zugabe einer konzentrierten wäßrigen Ammoniak-Lösung auf pH 7-8 eingestellt. Die Lösung wurde filtriert und zur Trockene eingedampft. Man erhielt 87 g (97% der Theorie) eines Öls.
IR (KBr): 1634, 1701 $cm^{-1}$ (C=O)
$R_f$: 0.79 (Essigester/Methanol/konz. wässeriges Ammoniak = 6/4/1 (v/v/v)

Analog wurden hergestellt:

| A | $NR^3R^4$ | Anmerkungen | % Ausbeute | MS | $R_f$ | Fließmittel | IR [$cm^{-1}$] |
|---|---|---|---|---|---|---|---|
| A3 | C4 | | 93 | ESI:M+H=391 (M+Na=413) | 0.6 | FM1 | (KBr): C=O 1637.5: 1706.9 |
| A3 | C3 | | 100 | | 0.3 | FM1 | (KBr): C=O 1641.3: 1705 |
| A11 | C1 | | 78.5 | | 0.2 | FM1 | (KBr): C=O 1701.1: 1641.3 |
| A7 | C1 | ohne $KHSO_4$ | 80.2 | | 0.2 | FM7 | |
| A3 | C1 | | 97 | | 0.79 | Essigester/ Methanol/ konz. wässeriges Ammoniak 6/4/1 (v/v/v) | (KBr): C=O 1633.6; 1701.1 |
| A3 | C5 | ohne $KHSO_4$ | 53 | | 0.39 | FM4 | (KBr): C=O 1733.9: 1624.0 |
| A3 | C6 | ohne $KHSO_4$ | 89 | | 0.38 | FM4 | (KBr): C=O 1706.9: 1645.2 |
| A3 | C8 | als Rohprodukt weiter umgesetzt | 100 | | 0.3 | FM1 | |

Beispiel A28

Herstellung von Verbindungen der allgemeinen Formel:

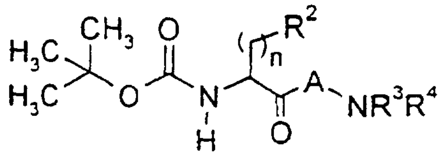

1-[$N^2$-[N-(1,1-Dimethylethoxycarbonyl)-3,5-dibrom-D-tyrosyl]-$N^6$-(phenylmethoxycarbonyl)-L-lysyl]-4-(4-pyridinyl)-piperazin

Zu der Mischung aus 2.58 g (5.88 mmol) N-[(1,1-Dimethylethoxy)-carbonyl]-3,5-dibrom-D-tyrosin, 1.03 g (8 mmol) DIEA, 1.93 g (6 mmol) TBTU, 0.79 g (5.8 mmol) HOBt und 100 ml Dimethylformamid wurden 2.5 g (5.88 mmol) 1-[$N^6$-[(Phenylmethoxy)carbonyl]-L-lysyl]-4-(4-pyridinyl)piperazin, gelöst in 50 ml Dimethylformamid, unter Rühren zugetropft. Das Reaktionsgemisch wurde über Nacht bei Raumtemperatur gerührt, anschließend im Vakuum eingeengt und der Rückstand in Essigsäureethylester aufgenommen. Die organische Phase wurde zweimal mit wäßriger gesättigter Natriumhydrogencarbonat-Lösung und einmal mit wäßriger gesättigter Kochzalzlösung gewaschen, getrocknet und im Vakuum eingeengt. Die Reinigung erfolgte säulenchromatographisch (Aluminiumoxid, Aktivitätsstufe III (6% Wassergehalt) (ICN Biomedicals), Fließmittel: Essigester/Methanol/Ammoniak = 8/2/0.5 (v/v/v), danach Methanol/Ammoniak = 7/3 (v/v)). Man erhielt 4.0 g (80 % der Theorie) einer amorphen Substanz.
IR (KBr): 1643, 1709 $cm^{-1}$ (C=O)
$R_f$: 0.52(FM1)
ESI-MS: $(M+H)^+$ = 845/847/849 ($Br_2$)
Analog wurden hergestellt (jeweils n = 1) :

| $R^2$ | A | $NR^3R^4$ | Anmerkungen | % Ausbeute | MS | $R_f$ | Fließmittel | IR [$cm^{-1}$] |
|---|---|---|---|---|---|---|---|---|
| AS7 | A0 | C4 | LM: THF; als Rohprodukt umgesetzt | 100 | | | | |
| AS1 | A0 | C11 | als Rohprodukt umgesetzt | 69 | | | | |
| AS4 | A0 | C20 | | 59 | ESI:$(M+H)^+$ = 600/2/4($Br_2$) | | | (KBr): C=O 1639: 1707 |
| AS1 | A0 | C4 | | 71 | | | | |
| AS4 | A0 | C11 | | 53 | | 0.5 | FM1 | |
| AS7 | A0 | C1 | als Rohprodukt umgesetzt | 100 | | | | (KBr): C=O 1644 |
| AS4 | A7 | C1 | $NEt_3$ als Base, als Rohprodukt umgesetzt | 100 | | 0.4 | FM8 | |
| AS1 | A4 | C1 | | 80 | ESI:$(M+H)^+$= 845/7/9 ($Br_2$) | 0.52 | FM1 | (KBr): C=O 1643.3; 1708.8 |
| AS4 | A0 | C5 | Boc-AS4 LC/$SiO_2$/FM5 | 83 | EI:$M^+$ = 581/3/5($Br_2$) | 0.69 | FM5 | (KBr): C=O 1706.9; 1641.3 |
| AS4 | A0 | C15 | LC/$SiO_2$/FM4 | 86 | EI:$M^+$ = 382/4/6($Br_2$) | 0.83 | FM4 | (KBr): C=O 1706.9; 1641.3 |
| AS1 | A0 | C5 | LC/$SiO_2$/FM5 | 81 | | 0.5 | FM5 | (KBr): C=O 1705.0; 1637.5 |
| AS4 | A0 | C16 | LC/$SiO_2$/FM4 THF | 85 | EI:$(M+H)^+$ = 582/4/6($Br_2$) | 0.42 | FM4 | (KBr): C=O 1706 9; 1643.3 |
| AS1 | A0 | C15 | THF LC/$SiO_2$/FM4 | 76 | | 0.53 | FM4 | (KBr): C=O 1701.1; 1637.5 |
| AS4 | A0 | C3 | LC/$SiO_2$/FM6 | 83 | ESI:$(M+H)^+$ = 598/600/2 ($Br_2$) | 0.71 | FM6 | (KBr): C=O 1706.9; 1641.3 |
| AS1 | A0 | C16 | LC/$SiO_2$/FM4 | 85 | | 0.35 | FM1 | (KBr): C=O 1705:; 1641.3 |
| AS1 | A0 | C6 | LC/$SiO_2$/FM6 | 84 | | 0.54 | FM6 | (KBr): C=O 1701.1; 1635.5 |
| AS4 | A0 | C18 | LC/$SiO_2$/FM4 | 95 | | 0.66 | FM4 | (KBr): C=O 1705; 1641.3 |

(fortgesetzt)

| $R^2$ | A | $NR^3R^4$ | Anmerkungen | % Ausbeute | MS | $R_f$ | Fließmittel | IR [$cm^{-1}$] |
|---|---|---|---|---|---|---|---|---|
| AS1 | A0 | C37 | | 90 | | 0.43 | FM1 | (KBr): C=O 1645; 1714.5 |
| AS4 | A0 | C37 | | 95 | | 0.51 | FM4 | (KBr): C=O 1643.3; 1705 |
| AS4 | A0 | C22 | | 75 | | | | (KBr): C=O 1635.5; 1708.8 |
| AS4 | A0 | C21 | | 92 | $M^+$ = 582/4/6($Br_2$) | 0.42 | FM4 | (KBr): C=O 1643; 1705 |
| AS4 | A5 | C1 | | 69 | ESI:$(M+H)^+$ = 939/41/43 ($Br_2$) | | | (KBr): C=O 1653; 1709 |
| AS4 | A0 | C23 | | 85 | | | | (KBr): C=O 1645; 1709 |
| AS4 | A10 | C1 | | 65 | $M^+$ :652/4/6 | | | (KBr): C=O 1649; 1707 |
| AS4 | A0 | C24 | | 79 | $M^+$: 589/91/93 | | | (KBr): C=O 1643; 1707 |
| AS4 | A5 | C8 | | 76 | | | | (KBr): C=O 1643; 1713 |
| AS4 | A6 | C1 | | 95 | | | | (KBr): C=O 1645; 1710.8 |
| AS4 | A6 | C8 | | 88 | $M^+$:657/9/61 | | | (KBr): C=O 1628; 1713 |
| AS4 | A10 | C8 | | 46 | ESI.$(M+H)^+$ = 858/60/62 ($Br_2$) | | | (KBr): C=O 1647; 1707 |
| AS4 | A0 | C26 | | 46 | | | | (KBr): C=O 1637.5; 1707 |
| AS1 | A0 | C1 | als Rohprodukt weiter umgesetzt | 100 | | | | |

(fortgesetzt)

| $R^2$ | A | $NR^3R^4$ | Anmerkungen | % Ausbeute | MS | $R_f$ | Fließmittel | IR [$cm^{-1}$] |
|---|---|---|---|---|---|---|---|---|
| AS1 | A0 | C8 | | 55 | | 0.3 | Dichlormethan/ Methanol 9/1 | (KBr): C=O 1632 |
| AS1 | A0 | C18 | | 84 | ESI:$(M+H)^+$ = 613/5/7($Br_2$) | 0.4 | FM4 | (KBr): C=O 1641; 1707 |
| AS1 | A0 | C3 | | 81 | | | | (KBr): C=O 1638; 1701 |
| AS1 | A0 | C21 | | 70 | | 0.28 | FM4 | (KBr): C=O 1643; 1707 |
| AS4 | A0 | C6 | | 47 | | | | (KBr): C=O 1639; 1707 |
| AS4 | A0 | C19 | | 90 | | | | (KBr): C=O 1639; 1707 |
| AS9 | A0 | C1 | als Rohprodukt weiter umgesetzt | 47 | | | | |
| AS1 | A7 | C1 | $NEt_3$ als Base; als Rohprodukt weiter umgesetzt | 83 | | 0.28 | FM1 | |
| AS4 | A0 | C38 | | 67 | | 0.5 | FM1 | |
| AS4 | A0 | C37 | | 84 | | | | |
| AS4 | A0 | C39 | | 100 (roh) | | 0.68 | FM1 | |
| AS4 | A0 | C40 | | 36 | | | | |
| AS1 | A0 | C42 | | 90 | | 0.43 | FM1 | (KBr): C=O 1645/1715 |
| AS4 | A0 | C42 | | 100 | | 0.51 | FM4 | (KBr): C=O 1643/1705 |
| AS1 | A0 | C43 | | 78 | | 0.9 | EE/MeOH 95/5 | (KBr): C=O 1636/ 1676/ 1659 |

(fortgesetzt)

| $R^2$ | A | $NR^3R^4$ | Anmerkungen | % Ausbeute | MS | $R_f$ | Fließmittel | IR [$cm^{-1}$] |
|---|---|---|---|---|---|---|---|---|
| AS1 | A0 | C44 | | 47 | | 0.9 | EE/MeOH 95/5 | (KBr): C=O 1638/1701 |
| AS1 | A0 | C45 | | 72 | EI:$M^+$ = 591/3/5($Br_2$) | 0.9 | EE/MeOH 9/1 | (KBr): C=O 1638/1695 |
| AS1 | A0 | C47 | | 80 | EI:$M^+$ = 596/98/600 ($Br_2$) | 0.95 | EE/MeOH 9/1 | (KBr): C=O 1636/1705 |
| AS1 | A0 | C49 | | 89 | | 0.9 | EE/MeOH 9/1 | (KBr): C=O 1636/1684 |
| AS4 | A0 | C44 | | 69 | | 0.9 | EE/MeOH 9/1 | (KBr): C=O 1643/1707; CN 2235 |
| AS1 | A0 | C50 | | 93 | EI:$M^+$ = 598/600/602 (Br2) | 0.9 | EE/MeOH 9/1 | (KBr): C=O 1636/1705 |
| AS1 | A0 | C51 | | 100 | | 0.1 | EE/MeOH/ $NH_4OH$ 5/5/0.1 | (KBr): C=O 1638/1707 |
| AS4 | A0 | C52 | | 63 | | 0.56 | FM1 | (KBr): C=O 1641/1705 |
| AS4 | A0 | C53 | | 83 | EI:$M^+$ = 601/3/5 ($Br_2$) | | | (KBr): C=O 1638/1705 |
| AS4 | A0 | C64 | | 41 | ESI:$(M+H)^+$ = 610/12/14 ($Br_2$) | | | (KBr): C=O 1639/1701 |
| AS1 | A0 | C53 | | 66 | | 0.45 | $CH_2Cl_2$/ MeOH/ $NH_4OH$ 70/30/1 | (KBr): C=O 1639/1709 |
| AS4 | A0 | C51 | | 88 | | 0.35 | $CH_2Cl_2$/ MeOH/ $NH_4OH$ 50/50/0.5 | (KBr): C=O 1641/1691 |
| AS4 | A0 | C66 | | 77 | EI:$M^+$ = 629/31/33 ($Br_2$) | 0.75 | $CH_2Cl_2$/ MeOH/ $NH_4OH$ 9/1/0.1 | (KBr): C=O 1641/1707 |
| AS16 | A0 | C8 | | 100 | | 0.8 | FM1 | |
| AS16 | A0 | C1 | | 56 | | 0.5 | EE/ MeOH/ $NH_4OH$ 9/1/1 | (KBr): C=O 1695 |

(fortgesetzt)

| $R^2$ | A | $NR^3R^4$ | Anmerkungen | % Ausbeute | MS | $R_f$ | Fließmittel | IR [$cm^{-1}$] |
|---|---|---|---|---|---|---|---|---|
| AS4 | A0 | C8 | | 100 | | | | |
| AS1 | A0 | C53 | | 70.0 | EI: $M^+$ = 502/4/6 ($Br_2$) | 0.10 | $CH_2Cl_2$/ MeOH / NH4OH 70/30/1 | (KBr): C=O 1676 |
| AS4 | A0 | C70 | | 47.0 | | | | (KBr): C=O 1645/1707 |
| AS1 | A0 | C64 | | 31.0 | | 0.50 | $CH_2Cl_2$ / MeOH / NH4OH 90/10/1 | (KBr): C=O 1639/1707 |
| AS1 | A0 | C70 | | 20.0 | | | | |
| AS4 | A0 | C72 | | 50.0 | | 0.50 | $CH_2Cl_2$/ MeOH / NH4OH 90/10/1 | |
| AS19 | A0 | C8 | | 98.0 | | | | |
| AS35 | A0 | C8 | | 92.0 | | 0.70 | FM1 | |
| AS36 | A0 | C8 | | 65.0 | | | | |

Beispiel A29

Herstellung von Verbindungen der allgemeinen Formel:

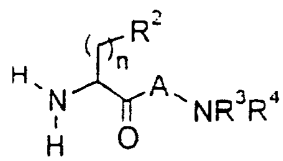

1-(4-Amino-3,5-dibrom-D-phenylalanyl)-4-(4-pyridinyl)piperazin

Eine Mischung aus 39 g (0.089 mol) 4-Amino-3,5-dibrom-N-[(1,1-dimethylethoxy)carbonyl]-D-phenylalanin, 35.7 g (0.111 mol) TBTU, 12.3 g (0.089 mol) HOBt, 14.5 g (0.089 mol) 1-(4-Pyridinyl)-piperazin und 19.6 ml (0.111 mol) DIEA in 1000 ml Tetrahydrofuran wurde über Nacht bei Raumtemperatur gerührt. Das Reaktionsgemisch wurde einmal mit gesättigter wäßriger Kochsalz-Lösung und zweimal mit gesättigter wäßriger Natriumhydrogencarbonat-Lösung ausgeschüttelt. Die vereinigten wäßrigen Phasen wurden einmal mit Tetrahydrofuran extrahiert und die vereinigten Tetrahydrofuran-Phasen einmal mit gesättigter wäßriger Kochsalz-Lösung gewaschen. Nach dem Trocknen der organischen Phase mit Natriumsulfat wurde im Vakuum eingeengt und der Rückstand in Essigsäureethylester aufgenommen. Die Essigesterphase wurde nach nochmaligem Trocknen filtriert und im Vakuum eingeengt. Man erhielt 52.5 g der Zwischenverbindung als zähes Öl, das anschließend mit 300 ml Methylenchlorid und 80 ml Trifluoressigsäure versetzt und über Nacht bei Raumtemperatur gerührt wurde. Das Reaktionsgemisch wurde im Vakuum eingedampft, der entstehende Rückstand mit Ether verrieben. Man erhielt 45.8 g (72% der Theorie) des gesuchten Produktes als weißen amorphen Feststoff.
IR (KBr): 1643, 1674 $cm^{-1}$ (C=O)
$R_f$ : 0.36 (Essigester/Methanol = 6/4 (v/v))

Analog wurden hergestellt (jeweils n = 1):

| $R^2$ | A | $NR^3R^4$ | Anmerkungen | % Ausbeute | MS | $R_f$ | Fließmittel | IR [$cm^{-1}$] |
|---|---|---|---|---|---|---|---|---|
| AS7 | A0 | C8 | Rohprodukt, Boc-Abspaltung mit reiner TFA | 84 | | | | |
| AS4 | A0 | C8 | | 63 | ESI: $(M+H)^+$ = 486/88/90 ($Br_2$) | | | (KBr): C=O 1632 |
| AS4 | A0 | C4 | | 63 | ESI: $(M+H)^+$ = 481/3/5 ($Br_2$) | | | (KBr): C=O 1620 |
| AS1 | A9 | C1 | | 55 | | 0 25 | FM2 | (KBr): C=O 1674.1 1643.3 |
| AS4 | A0 | C8 | | 81 | ESI: $(M+H)^+$ = 486/8/90 ($Br_2$) | 0.6 | FM2 | (KBr): C=O 1629.8 |
| AS4 | A0 | C1 | | 72 | | 0.38 | Essigester/ Methanol = 6/4 (v/v) | (KBr): C=O 1643.3; 1674.1 |
| AS1 | A0 | C20 | | 30 | | | | |

(fortgesetzt)

| $R^2$ | A | $NR^3R^4$ | Anmerkungen | % Ausbeute | MS | $R_f$ | Fließmittel | IR [$cm^{-1}$] |
|---|---|---|---|---|---|---|---|---|
| AS4 | A0 | C65 | | 41 | EI: $M^+$ = 515/17/19 ($Br_2$) | | | (KBr): C=O 1618 |
| AS1 | A0 | C65 | | 15 | ESI: $(M+H)^+$ = 517/19/21 ($Br_2$) | 0.08 | FM1 | (KBr): C=O 1635 |
| AS4 | A0 | C78 | | 77.0 | ESI: $(M+H)^+$ = 529/31/33 ($Br_2$) | 0.30 | $CH_2Cl_2$/ MeOH / NH4OH = 90/10/1 | (KBr): C=O 1674 |
| AS1 | A0 | C78 | | 60.0 | ESI: $(M+H)^+$ = 531/33/35 ($Br_2$) | 0.10 | $CH_2Cl_2$ / MeOH / NH4OH =80/20/1 | (KBr): C=O 1670 |
| AS4 | A0 | C71 | | 43.0 | | 0.20 | $CH_2Cl_2$/ MeOH / NH4OH =90/10/1 | (KBr): C=O 1678 |
| AS31 | A0 | C20 | | 39.0 | EI: $M^+$ = 382 | 0.30 | $CH_2Cl_2$ / MeOH / NH4OH =80/20/1 | (KBr): C=O 1678 |
| AS31 | A0 | C53 | | 83.0 | EI: $M^+$ = 383 | | | (KBr): C=O 1678 |

Beispiel A30

Herstellung von Verbindungen der allgemeinen Formel:

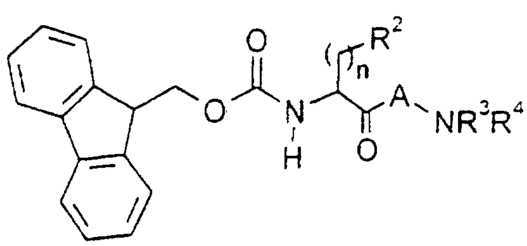

1-[$N^2$-[N-(9-Fluorenylmethoxycarbonyl)-3,5-dibrom-D-tyrosyl]-$N^6$-(1,1-dimethylethoxycarbonyl)-L-lysyl]-4-(4-pyridinyl)-piperazin

Eine Mischung aus 63 g (0.1123 mol) N-[(9-Fluorenylmethoxy)-carbonyl]-3,5-dibrom-D-tyrosin, 44 g (0.1123 mol) 1-[$N^6$-(1,1-Dimethylethoxycarbonyl)-L-lysyl]-4-(4-pyridinyl)-piperazin, 39.7 g (0.1235 mol) TBTU, 15.5 g (0.1123 mol) HOBt, 21.7 ml (0.1235 mol) DIEA und 600 ml Dimethylformamid wurde 20 Stunden bei Raumtemperatur gerührt. Das Reaktionsgemisch wurde im Vakuum eingeengt und der Rückstand zwischen Essigsäureethylester/Methanol (10/1 v/v) und gesättigter wäßriger Natriumhydrogencarbonat Lösung verteilt. Die organische Phase wurde einmal mit gesättigter wäßriger Natriumhydrogencarbonat-Lösung gewaschen und nach dem Trocknen im Vakuum eingeengt. Der Rückstand wurde zweimal aus Isopropanol umkristallisiert (22.6g; 22% der Theorie), die Mutterlaugen vereinigt,

eingedampft und säulenchromatographisch (MN-Kieselgel 60, Macherey-Nagel, 70-230 mesh ASTM; Fließmittel: Essigester/Methanol = 8/2 (v/v)) gereinigt. Man erhielt weitere 28.0 g (26.7 % der Theorie) des gesuchten Endproduktes.
Gesamtausbeute: 49% der Theorie.
IR (KBr): 1641, 1705 $cm^{-1}$ (C=O)
$R_f$: 0.46(Essigester/Methanol = 6/4 (v/v))
ESI-MS: $(M+H)^+$ = 933/935/937 ($Br_2$)

Analog wurden hergestellt:

| $R^2$ | A | $NR^3R^4$ | Anmerkungen | % Ausbeute | MS | $R_f$ | Fließmittel | IR [$cm^{-1}$] |
|---|---|---|---|---|---|---|---|---|
| AS1 | A3 | C1 | | 48 | ESI: $(M+H)^+$ = 933/5/7 ($Br_2$) | 0.46 | Essigester/ Methanol = 6/4 (v/v) | (KBr): C=O 1641.3; 1705.0 |
| AS1 | A3 | C5 | THF/SiO2/FM4 | 80 | ESI: $(M+H)^+$ = 933/5/7 ($Br_2$) | 0.72 | FM1 | (KBr): C=O 1701.1, 1635.5 |
| AS1 | A3 | C6 | THF | 60 | ESI: $M^+$ = 960/2/4 ($Br_2$) | 0.47 | FM4 | (KBr): C=O 1712.7; 1631.7 |
| AS5 | A3 | C1 | THF LC/ SiO2/FM4 Diastereomere | 61 | ESI: $(M+H)^+$ = 917/19/21 ($Br_2$) | 0.36 | FM4 | (KBr): C=O 1708.8; 1645.2 |
| AS10 | A0 | C1 | THF | 90 | | 0.52 | FM4 | |
| AS1 | A3 | C18 | | 73 | | 0.46 | FM1 | (KBr): C=O 1635.5, 1712.7 |
| AS10 | A3 | C1 | THF | 85 | | | | (KBr): C=O 1643.3; 1708.8 |
| AS10 | A3 | C4 | THF | 82 | | | | (KBr): C=O 1639.4, 1710.8 |
| AS10 | A3 | C1 | THF | 85 | | | | (KBr): C=O 1643, 1709 |
| AS4 | A3 | C18 | | 94 | ESI: $(M+H)^+$ = 963/5/7 ($Br_2$) | | | (KBr): C=O 1633.6, 1711 |
| AS15 | A0 | C8 | | 90 | | | | (KBr): C=O 1635.5, 1617.5 |
| AS12 | A0 | C8 | | 44 | ESI: $(M+H)^+$ = 577 | | | (KBr): C=O 1630; 1714.6 |
| AS10 | A0 | C4 | | 88 | | 0.49 | FM4 | (KBr): C=O 1635.5, 1716.5 |
| AS1 | A3 | C1 | | 70 | | 0.7 | FM7 | |

Beispiel A31

Herstellung von Verbindungen der allgemeinen Formel:

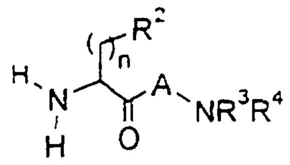

1-[$N^2$-(3,5-Dibrom-D-tyrosyl)-$N^6$-(1,1-dimethylethoxycarbonyl)-L-lysyl]-4-(4-pyridinyl)-piperazin

Zu der Mischung aus 63 g (0.1123 mol) N-[(9-Fluorenylmethoxy)carbonyl]-3,5-dibrom-D-tyrosin, 44 g (0.1123 mol) 1-[$N^6$-(1,1-Dimethylethoxycarbonyl)-L-lysyl]-4-(4-pyridinyl)piperazin, 39.7 g (0.1235 mol) TBTU, 15.5 g (0.1123 mol) HOBt und 1500 ml Tetrahydrofuran wurden 21.7 ml (0.1235 mol) DIEA langsam zugetropft und der Reaktionsansatz anschließend 2 Stunden bei Raumtemperatur gerührt. Nach Zugabe von 200 ml Diethylamin wurde abermals über Nacht bei Raumtemperatur gerührt. Das Reaktionsgemisch wurde mit 1000 ml gesättigter Kochsalz-Lösung versetzt, gut durchgerührt und die wäßrige Phase abgetrennt. Nach dem Ausschütteln der wäßrigen Phase mit dreimal je 500 ml Tetrahydrofuran und Vereinigen der organischen Phasen wurde dreimal mit je 500 ml gesättigter wäßriger Kochsalzlösung, dreimal mit je 200 ml gesättigter wäßriger Natriumhydrogencarbonat-Lösung und einmal mit 500 ml gesättigter wäßriger Kochsalzlösung gewaschen. Die organische Phase wurde getrocknet und anschließend im Vakuum eingeengt. Der Rückstand wurde säulenchromatographisch (MN-Kieselgel 60, Macherey-Nagel, 70-230 mesh ASTM, Fließmittel: Essigester/Methanol/ konz. wässeriges Ammoniak = 8/1/0.25 (v/v/v)) gereinigt. Man erhielt 40.0 g (50 % der Theorie) des gewünschten Endproduktes.
IR (KBr): 1641, 1699 $cm^{-1}$ (C=O)
$R_f$: 0.2 (Essigester/Methanol/konz. wässeriges Ammoniak = 6/4/1 (v/v/v))
ESI-MS: $(M+H)^+$ = 711/713/715 ($Br_2$)

Analog wurden hergestellt (jeweils n = 1):

| $R^2$ | A | $NR^3R^4$ | Anmerkungen | % Ausbeute | MS | $R_f$ | Fließmittel | IR [$cm^{-1}$] |
|---|---|---|---|---|---|---|---|---|
| AS4 | A3 | C8 | roh | 43 | | | | |
| AS4 | A3 | C1 | roh | 100 | | 0.4 | FM1 | |
| AS4 | A3 | C4 | | 79 | ESI: $(M+H)^+$ = 709/11/13 ($Br_2$) | 0.7 | FM7 | (KBr): C=O 1637.5; 1705 |
| AS4 | A0 | C69 | | 82 | ESI: $(M+H)^+$ = 587/9/81 ($Br_2$) | | | (KBr): C=O 1618/ 1645/ 1690 |
| AS4 | A0 | C46 | | 38 | | 0.55 | $CH_2Cl_2$/ MeOH/ $NH_4OH$ 90/10/1 | (KBr): C=O 1614/1639 |
| AS4 | A0 | C48 | | 54 | EI: $M^+$ = 522/4/6 ($Br_2$) | 0.52 | $CH_2Cl_2$/ MeOH/ $NH_4OH$ 90/10/2 | (KBr): C=O 1638 |
| AS11 | A0 | C53 | | 71.0 | EI: $M^+$ = 469 | 0.20 | $CH_2Cl_2$ / MeOH / NH4OH 90/10/1 | (KBr): C=O 1637/1732 |
| AS11 | A0 | C20 | | 45.0 | EI: $M^+$ =468 | 0.40 | $CH_2Cl_2$ / MeOH / NH4OH 90/10/1 | (KBr): C=O 1635/1732 |

(fortgesetzt)

| $R^2$ | A | $NR^3R^4$ | Anmerkungen | % Ausbeute | MS | $R_f$ | Fließmittel | IR [$cm^{-1}$] |
|---|---|---|---|---|---|---|---|---|
| AS31 | A0 | C72 | | 100.0 | EI: $M^+$ = 411 | 0.45 | FM1 | (KBr): C=O 1664 |
| AS11 | A0 | C72 | | 33.0 | EI: $M^+$ = 497 | 0.30 | FM1 | (KBr): C=O 1630/1641 |

Beispiel A32

Herstellung von Verbindungen der allgemeinen Formel:

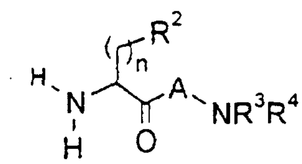

1-[$N^2$-(3,5-Dibrom-D-tyrosyl)-$N^6$-(phenylmethoxycarbonyl)-L-lysyl]-4-(4-pyridinyl)-piperazin

Zu einer Mischung aus 4 g (4.7 mmol) 1-[$N^2$-[N-(1,1-Dimethylethoxycarbonyl)-3,5-dibrom-D-tyrosyl]-$N^6$-(phenylmethoxycarbonyl)-L-lysyl]-4-(4-pyridinyl)-piperazin und 80 ml Methylenchlorid wurden 20 ml Trifluoressigsäure gegeben und der Reaktionsansatz über Nacht bei Raumtemperatur gerührt. Das Reaktionsgemisch wurde durch Zugabe von gesättigter wäßriger Natriumhydrogencarbonat Lösung neutralisiert, die organische Phase über Natriumsulfat getrocknet und im Vakuum eingeengt. Man erhielt 2.2 g (64 % der Theorie) eines amorphen Feststoffes.
IR (KBr): 1643, 1680 $cm^{-1}$ (C=O)
$R_f$: 0.5 (FM1)
ESI-MS: $(M+H)^+$ = 745/747/749 ($Br_2$)
Analog wurden hergestellt (jeweils n = 1) :

| $R^2$ | A | $NR^3R^4$ | Anmerkungen | % Ausbeute | MS | $R_f$ | Fließmittel | IR [$cm^{-1}$] | Fp. (°C) |
|---|---|---|---|---|---|---|---|---|---|
| AS7 | A0 | C4 | Rohprodukt; TFA pur | 51 | | 0.30 | FM1 | | |
| AS1 | A0 | C11 | | 95 | ESI: $(M+H)^+$ = 503/5/7 ($Br_2$) | | | (KBr): C=O 1676 | |
| AS4 | A0 | C20 | | 100 | ESI: $(M+H)^+$ = 500/2/4 ($Br_2$) | | | | |
| AS1 | A0 | C4 | | 100 | ESI: $(M+H)^+$ = 481/3/5 ($Br_2$) | | | (KBr): C=O 1678 | |
| AS4 | A0 | C11 | | 74 | | | | | |
| AS7 | A0 | C1 | Als Rohprodukt umgesetzt | 100 | | | | | |
| AS4 | A7 | C1 | Als Rohprodukt umgesetzt | 100 | | 0.40 | EE/ MeOH 7/3 v/v | | |
| AS1 | A4 | C1 | | 64 | ESI: $(M+H)^+$ =745/7/9 ($Br_2$) | 0.50 | FM1 | (KBr): C=O 1643.3; 1679.9 | |
| AS4 | A0 | C5 | | 89 | | 0.32 | FM4 | (KBr): C=O 1637.5 | |
| AS4 | A0 | C15 | | 93 | | 0.33 | FM4 | (KBr): C=O 1618.2 | |
| AS1 | A0 | C5 | | 89 | | 0.25 | FM4 | (KBr): C=O 1639.4 | 154-157 |
| AS4 | A0 | C16 | LC/ $SiO_2$/ FM4 | 90 | | 0.30 | FM4 | (KBr): C=O 1635.5 | |
| AS1 | A0 | C15 | | 89 | | 0.20 | FM4 | (KBr): C=O 1639.4 | 160-164 |
| AS4 | A0 | C3 | LC/ $SiO_2$/ FM4 | 98 | | 0.37 | FM4 | (KBr): C=O 1683.8; | |
| AS4 | A0 | C6 | | 89 | | 0.28 | FM4 | (KBr): C=O 1637.5 | |

(fortgesetzt)

| $R^2$ | A | $NR^3R^4$ | Anmerkungen | % Ausbeute | MS | $R_f$ | Fließmittel | IR [$cm^{-1}$] | Fp. (°C) |
|---|---|---|---|---|---|---|---|---|---|
| AS1 | A0 | C16 | | 95 | | 0.57 | FM1 | (KBr): C=O 1683.8 | |
| AS1 | A0 | C6 | LC/ $SiO_2$/ FM4 | 56 | EI: $M^+$= 511/3/5 ($Br_2$) | 0.24 | FM4 | (KBr): C=O 1637.5 | |
| AS4 | A0 | C18 | | 90 | EI: $M^+$ = 512/4/6 ($Br_2$) | 0.50 | FM1 | (KBr): C=O 1624.0 | |
| AS4 | A0 | C37 | | 93 | | 0.24 | FM4 | (KBr): C=O 1635.5; 1684 | |
| AS4 | A0 | C22 | | 88 | $M^+$ = 502/4/6 ($Br_2$) | | | (KBr): C=O 1618.2 | |
| AS4 | A0 | C21 | | 52 | $M^+$ = 482/4/6 ($Br_2$) | 0.55 | FM1 | (KBr): C=O 1681.8 | |
| AS1 | A0 | C37 | | 89 | | 0.32 | FM1 | (KBr): C=O 1681.8 | |
| AS4 | A5 | C1 | | roh | | | | (KBr): C=O 1645; 1676 | |
| AS4 | A0 | C23 | | 88 | | | | (KBr): C=O 1643 | |
| AS4 | A10 | C1 | | 47 | ESI: $(M+H)^+$ = 553/5/7 ($Br_2$) | | | (KBr): C=O 1653 | |
| AS4 | A5 | C8 | | 67 | $M^+$ = 543/5/7 | | | (KBr): C=O 1645 | |
| AS4 | A6 | C1 | | 59 | | | | (KBr): C=O 1643 | |
| AS4 | A0 | C24 | | 94 | $M^+$ = 489/91/93 | | | (KBr): C=O 1618; 1637.5 | |
| AS4 | A6 | C8 | | 70 | | | | (KBr): C=O 1639.4 | |

(fortgesetzt)

| $R^2$ | A | $NR^3R^4$ | Anmerkungen | % Ausbeute | MS | $R_f$ | Fließmittel | IR [$cm^{-1}$] | Fp. (°C) |
|---|---|---|---|---|---|---|---|---|---|
| AS4 | A10 | C8 | | 82 | $M^+$ = 557/9/61 | | | (KBr): C=O 1651 | |
| AS4 | A0 | C26 | | 88 | | | | (KBr): C=O 1626 | |
| AS1 | A0 | C1 | | 96 | ESI: $(M+H)^+$ = 483/5/7 ($Br_2$) | 0.18 | FM1 | (KBr): C=O 1680 | |
| AS1 | A0 | C8 | roh | 69 | | | | | |
| AS1 | A0 | C18 | | 82 | | 0.27 | FM1 | (KBr): C=O 1684 | |
| AS1 | A0 | C3 | | 100 | | 0.38 | FM1 | (KBr): C=O 1682 | |
| AS1 | A0 | C21 | | 89 | | 0.26 | FM1 | (KBr): C=O 1595; 1615 | |
| AS4 | A0 | C3 | | 99 | | 0.37 | FM4 | (KBr): C=O 1618; 1636; 1683 | |
| AS4 | A0 | C19 | | 98 | ESI: $(M+H)^+$ =498/500/50 2 ($Br_2$) | 0.47 | FM4 | (KBr): C=O 1638; 1682 | |
| AS9 | A0 | C1 | Rohprodukt | 96 | | | | | |
| AS1 | A7 | C1 | | 37 | | 0.42 | FM7 | | |
| AS4 | A0 | C38 | | 80 | | 0.25 | FM1 | | |
| AS4 | A0 | C37 | | 86 | | | | | |
| AS4 | A0 | C39 | | 73 | | | | | |
| AS4 | A0 | C40 | | 92 | EI: $M^+$ = 515/7/9 | | | (KBr): C=O 1674 | |
| AS1 | A0 | C42 | | 100 (roh) | | 0.32 | FM1 | (KBr): C=O 1682 | |
| AS4 | A0 | C42 | | 95 | | 0.24 | FM4 | (KBr): C=O 1636/1684 | |

(fortgesetzt)

| $R^2$ | A | $NR^3R^4$ | Anmerkungen | % Ausbeute | MS | $R_f$ | Fließmittel | IR [$cm^{-1}$] | Fp. (°C) |
|---|---|---|---|---|---|---|---|---|---|
| AS1 | A0 | C43 | | 66 | | 0.1 | FM7 | (KBr): C=O 1659 | |
| AS1 | A0 | C44 | | 59 | | 0.15 | $CH_2Cl_2$/ MeOH/ $NH_4OH$ 90/10/1 | (KBr): C=O 1676 | |
| AS1 | A0 | C45 | | 82 | ESI: $(M+H)^+$ = 492/4/6 ($Br_2$) | 0.10 | EE/MeOH 9/1 | (KBr): C=O 1678 | |
| AS1 | A0 | C47 | | 89 | | 0.52 | FM7 | (KBr): C=O 1634/1666 | |
| AS1 | A0 | C49 | | 84 | | 0.15 | $CH_2Cl_2$/ MeOH/ $NH_4OH$ | (KBr): C=O 1678 | |
| AS4 | A0 | C44 | | 93 | EI: $M^+$ = 504/6/8 ($Br_2$) | 0.45 | EE/MeOH 9/1 | (KBr): C=O 1653; CN 2239 | |
| AS1 | A0 | C50 | | 100 | EI: $M^+$= 498/500/502 ($Br_2$) | 0.10 | EE/MeOH 9/1 | (KBr): C=O 1636 | |
| AS1 | A0 | C51 | | 100 | EI: $M^+$ = 530/2/4($Br_2$) | 0.05 | EE/ MeOH/ NH4OH 5/5/0.1 | (KBr): C=O 1678 | |
| AS4 | A0 | C52 | | 97 | | 0.15 | FM1 | (KBr): C=O 1620/1688 | |
| AS4 | A0 | C53 | | 58 | ESI: $(M+H)^+$ = 502/4/6 ($Br_2$) | 0.05 | EE/MeOH/NH4OH 5/5/0.1 | (KBr): C=O 1678 | |
| AS4 | A0 | C64 | | 100 | | | | (KBr): C=O 1647/1678 | |
| AS1 | A0 | C53 | | 70 | EI: $M^+$ = 502/4/6 ($Br_2$) | 0.15 | $CH_2Cl_2$/ MeOH/$NH_4OH$ 70/30/1 | (KBr): C=O 1676 | |
| AS4 | A0 | C51 | | 100 | | 0.05 | $CH_2Cl_2$/ MeOH/$NH_4OH$ | (KBr): C=O 1680 | |
| AS4 | A0 | C66 | | 100 | | 0.27 | $CH_2Cl_2$/ MeOH/ $NH_4OH$ | | |
| AS16 | A0 | C8 | | 76 | | 0.40 | FM1 | | |
| AS16 | A0 | C1 | | 28 | | 0.20 | EE/ MeOH/ $NH_4OH$ 9/1/1 | | |

(fortgesetzt)

| $R^2$ | A | $NR^3R^4$ | Anmerkungen | % Ausbeute | MS | $R_f$ | Fließmittel | IR [$cm^{-1}$] | Fp. (°C) |
|---|---|---|---|---|---|---|---|---|---|
| AS4 | A0 | C70 | | 96 | | 0.20 | EE/MEOH /NH4OH 80/20/0.5 | (KBr): C=O 1676 | |
| AS1 | A0 | C64 | | 100 | EI: $M^+$ = 510/1214 | | | (KBr): C=O 1674 | |
| AS1 | A0 | C70 | | 100 | | | | (KBr): C=O 1674 | |
| AS4 | A0 | C72 | | 100 | ESI: $(M+H)^+$ = 530/2/4 ($Br_2$) | 0.10 | $CH_2Cl_2$/ MeOH/NH4OH 80/20/1 | (KBr): C=O 1678 | |
| AS19 | A0 | C8 | | 100 | | | | | |
| AS35 | A0 | C8 | | 72 | | 0.60 | FM1 | | |
| AS36 | A0 | C8 | | 80 | | 0.52 | FM1 | (KBr): C=O 1674 | |

Beispiel A33

4-(4-Pyridinyl)-1-[3-(4-pyridinyl)-D,L-alanyl]-piperazinhydrochlorid

16.4 g (0.04 mol) 1-[N-[(1,1-Dimethylethoxy)carbonyl]-3-(4-pyridinyl)-D,L-alanyl]-4-(4-pyridinyl)-piperazin, gelöst in 100 ml Methanol, wurden mit 20 ml ätherischer Salzsäure versetzt und das Reaktionsgemisch auf 40°C erwärmt. Die gesuchte Verbindung kristallisierte aus der Reaktionsmischung aus.
Ausbeute: 9.2 g (60 % der Theorie)
$R_f$: 0.1 (FM1)
Fp.: 198 - 200°C

Beispiel A34

Herstellung von Verbindungen der allgemeinen Formel:

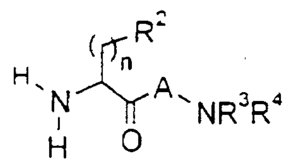

1-[$N^2$-(3,5-Dibrom-D-tyrosyl)-$N^6$-(1,1-dimethylethoxycarbonyl)-L-lysyl]-4- (4-pyridinyl)-piperazin

Die Mischung von 50 g (53.5 mmol) 1-[$N^2$-[N-(9-Fluorenylmethoxycarbonyl)-3,5-dibrom-D-tyrosyl]-$N^6$-(1,1-dimethylethoxycarbonyl)-L-lysyl]-4-(4-pyridinyl)-piperazin und 300 ml Diethylamin wurde unter Rühren auf 60°C erhitzt. Es wurden 100 ml Methanol zugegeben und weitere 5 Stunden bei 60°C gerührt. Das Reaktionsgemisch wurde im Vakuum eingedampft und der Rückstand säulenchromatographisch (MN-Kieselgel 60, Macherey-Nagel, 70-230 mesh ASTM, Fließmittel: Essigester/Methanol = 6/4 (v/v)) gereinigt. Man erhielt 26 g (68 % der Theorie) eines weißen Schaumes.
IR (KBr): 1641, 1691 $cm^{-1}$ (C=O)
$R_f$: 0.2 (Essigester/Methanol/konz. wässeriges Ammoniak = 6/4/1 (v/v/v))
ESI- MS: $(M+H)^+$ = 710/712/714 ($Br_2$)

Analog wurden hergestellt:

| $R^2$ | A | $NR^3R^4$ | n | Anmerkungen | % Ausbeute | MS | $R_f$ | Fließmittel | IR [$cm^{-1}$] |
|---|---|---|---|---|---|---|---|---|---|
| AS1 | A3 | C4 | 1 | | 85 | ESI.M+H= 710/2/4($Br_2$) | 0.2 | FM1 | (KBr): C=O 1635.5; 1695.3 |
| AS1 | A3 | C8 | 1 | | 98 | | | | (KBr): C=O 1635, 1705 |
| AS1 | A3 | C1 | 1 | | 68 | EI. M+=710/2/4 ($Br_2$) | 0.2 | Essigester/ Methanol/ $NH_4OH$ = 6/4/1 (v/v/v) | (KBr): C=O 1641.3, 1691.5 |
| AS1 | A3 | C5 | 1 | THF als Losemittel, saulenchromatographische Reinigung; Kieselgel/ FM1 | 56 | ESI: M+H= 711/3/5($Br_2$) | 0.3 | FM1 | (KBR): C=O 1695.3; 1635.5 |
| AS1 | A3 | C6 | 1 | THF als Losemittel, saulenchromatographische Reinigung; Kieselgel/ FM1 | 90 | EI M+= 739/41/43 ($Br_2$) | 0.49 | FM1 | (KBr): C=O 1695.3; 1629.8 |
| AS5 | A3 | C1 | 1 | THF als Losemittel, saulenchromatographische Reinigung; Kieselgel/ FM4. Diastereomere | 93 | | 0.25/ 0.37 | FM4 | (KBr): C=O 1705.0; 1643.3 |
| AS10 | A0 | C1 | 1 | | 71 | | 0.5 | FM1 | (KBr): C=O 1641.3 |
| AS1 | A3 | C18 | 1 | | 94 | | | | (KBr): C=O 1647; 1722.5 |
| AS10 | A3 | C1 | 1 | | 49 | M+= 694/6/8($Br_2$) | | | (KBr): C=O 1643; 1703 |
| AS10 | A3 | C4 | 1 | | 46 | ESI:M+H= 694/6/8($Br_2$) | | | (KBr): C=O 1639.4; 1705 |
| AS10 | A3 | C4 | 1 | | 46 | ESI:M+H= 694/6/8($Br_2$) | | | (KBr): C=O 1639.4; 1705 |
| AS10 | A3 | C1 | 1 | | 49 | M+= 694/68/70 ($Br_2$) | | | (KBr): C=O 1643, 1703 |
| AS4 | A3 | C18 | 1 | | 46 | ESI:M+H= 741/3/5($Br_2$) | | | (KBr): C=O 1641.3, 1705 |

(fortgesetzt)

| $R^2$ | A | $NR^3R^4$ | n | Anmerkungen | % Ausbeute | MS | $R_f$ | Fließmittel | IR [$cm^{-1}$] |
|---|---|---|---|---|---|---|---|---|---|
| AS15 | A0 | C8 | 1 | | 100 | M+ 321 | | | (KBr): C=O 1637.5 |
| AS12 | A0 | C8 | 1 | | 81 | | | | (KBr): C=O 1630 |
| AS10 | A0 | C4 | 1 | THF als Losemittel | 68 | | 0.38 | FM4 | (KBr): C=O 1635.5 |
| AS1 | A3 | C1 | 0 | Rohprodukt | 100 | | 0.3 | FM7 | |

Beispiel A35

1-[$N^2$-[N-[[[2-(2-Methoxyphenyl)ethyl]amino]carbonyl]-3,5-dibrom-D-tyrosyl]-$N^6$-(phenylmethoxycarbonyl)-L-lysyl]-4-(4-pyridinyl)-piperazin

Zu einer Lösung von 1.0 g (1.34 mmol) 1-[$N^2$-(-3,5-dibrom-D-tyrosyl)-$N^6$-(phenylmethoxycarbonyl)-L-lysyl]-4-(4-pyridinyl)-piperazin in 80 ml Tetrahydrofuran wurden 0.28 g (1.6 mmol) 2-Methoxyphenethylisocyanat zugegeben und die Mischung 3 Tage bei Raumtemperatur gerührt. Das Reaktionsgemisch wurde im Vakuum eingeengt und der Rückstand säulenchromatographisch (MN-Kieselgel 60, Macherey-Nagel, 70-230 mesh ASTM, Fließmittel: Methylenchlorid/Methanol/Cyclohexan/Ammoniak = 350/75/75/10 (v/v/v/v)) gereinigt. Man erhielt 0.5 g (40 % der Theorie) eines farblosen amorphen Feststoffes.
IR (KBr): 1639 $cm^{-1}$ (C=O)
$R_f$: 0.49 (FM1)
ESI-MS: $(M+H)^+$ = 922/924/926 ($Br_2$)

Beispiel A36

Herstellung von Verbindungen der allgemeinen Formel:

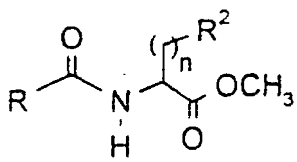

4-Amino-3, 5-dibrom-$N^2$-[[(2-phenylethyl)amino]carbonyl]-D-phenylalaninmethylester

Die Mischung aus 1.27 g (7.73 mmol) CDT in 150 ml Tetrahydrofuran wurde unter Eiskühlung mit 0.72 ml (5.15 mmol) Triethylamin und 2.0 g (5.15 mmol) 4-Amino-3,5-dibrom-D-phenylalaninmethylester-hydrochlorid versetzt, weitere 30 Minuten unter Eiskühlung und 1 Stunde bei Raumtemperatur gerührt. Anschließend wurden 0.82 ml (6.44 mmol) Benzenethanamin zugegeben und der Ansatz 5 Stunden unter Rückfluß gekocht. Das Reaktionsgemisch wurde im Vakuum eingeengt, der Rückstand in Essigsäureethylester aufgenommen und mit gesättigter wäßriger Natriumhydrogencarbonat-Lösung gewaschen. Nach dem Trocknen der organischen Phase wurde das Lösemittel im Vakuum entfernt, der Rückstand mit Ether verrührt und der Niederschlag abfiltriert. Man erhielt 1.69 g (66% der Theorie) eines amorphen Feststoffes.
IR (KBr): 1632, 1732 $cm^{-1}$ (C=O)
$R_f$: 0.63 (Essigescer)
ESI-MS: $(M+H)^+$ = 498/500/502 ($Br_2$)

Entsprechend wurden hergestellt (jeweils n = 1):

| RCO | $R^2$ | Anmerkungen | % Ausbeute | MS | $R_f$ | Fließmittel | IR [$cm^{-1}$] |
|---|---|---|---|---|---|---|---|
| N6 | AS1 | als Rohprodukt weiter umgesetzt | 100 | | 0.60 | FM1 | |
| N15 | AS6 | DMF/THF = 1/1 (v/v) als Lösemittel | 100 | ESI: $(M+H)^+$ = 517/9 (Br) | 0.65 | FM1 | (KBr): C=O 1745.5; 1676.0 |
| N2 | AS1 | | 99 | | 0.53 | FM1 | (KBr): C=O 1716.5 |

(fortgesetzt)

| RCO | $R^2$ | Anmerkungen | % Ausbeute | MS | $R_f$ | Fließmittel | IR [$cm^{-1}$] |
|---|---|---|---|---|---|---|---|
| N8 | AS4 | | 66 | ESI: $(M+H)^+$ = 498/500/502 ($Br_2$) | 0.63 | Essigester | (KBr): C=O 1631.7; 1732.0 |
| N15 | AS4 | | 92 | | 0.85 | Essigester/ Methanol = 8/2 (v/v) | (KBr): C=O 1620.1; 1737.8 |
| N23 | AS4 | | 95 | EI: $M^+$ = 572/4/6/8 ($Br_2$.Cl) | 0.86 | Essigester/ Methanol = 8/2 (v/v) | (KBr): C=O 1732.0; 1641.3 |
| N2 | AS2 | | 100 | EI: $M^+$ = 406 | 0.86 | FM1 | (KBr): C=O 1629.8; 1722.3; 1741.6 |
| N15 | AS1 | DIEA | 47 | | 0.75 | FM1 | |
| N15 | AS3 | | 38 | | 0.60 | t. -Butylmethylether/ Petrolether = 9/1 (v/v) | (KBr): C=O 1695.5 |
| N66 | AS21 | | 76 | | 0.60 | EE | (KBr): C=O 1662/ 1734 |
| N66 | AS1 | | 100 | | | | |
| N66 | AS4 | | 63 | | 0.56 | FM1 | |
| N122 | AS1 | | 95 | | | | |
| N122 | AS4 | | 88 | | | | |
| N66 | AS17 | | 22 | ESI: $(M+H)^+$ = 623/5/7 ($Br_2$) | 0.25 | FM1 | (KBr): C=O 1663/1740 |
| N66 | AS18 | | 65 | | 0.53 | EE | |
| N66 | AS19 | | 79 | | 0.50 | FM1 | (KBr): C=O 1663/1734 |
| N66 | AS5 | | 90 | ESI: $(M+H)^+$ = 607/09/11 ($Br_2$) | 0.78 | FM1 | (KBr): C=O 1637/ 1663/ 1740 |
| N66 | AS22 | | 68 | | 0.74 | FM1 | |
| N66 | AS23 | | 100 | | | | (KBr): C=O 1738/1662 |
| N66 | AS25 | | 100 | ESI: $(M+H)^+$ = 472 | 0.52 | FM1 | |
| N66 | AS49 | | 100 | | 0.80 | FM1 | |

Beispiel A37

Herstellung von Verbindungen der allgemeinen Formel:

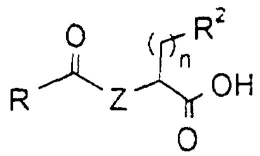

4-Amino-3,5-dibrom-$N^2$-[4-(2-chlorphenyl)-1-piperazinyl]carbonyl]-D-phenylalanin

Zu der Lösung von 2.8 g (4.9 mmol) 4-Amino-3,5-dibrom-$N^2$-[4-(2-chlorphenyl)-1-piperazinyl]carbonyl]-D-phenylalaninmethylester in einem Gemisch aus 30 ml Methanol und 20 ml Wasser wurden 0.25 g (10.0 mmol) Lithiumhydroxid gegeben und die Mischung anschließend 3 Stunden bei Raumtemperatur gerührt. Nach Zugabe von 2.0 g (50 mmol) Natriumhydroxid wurde mit 50 ml Wasser verdünnt. Das Reaktionsgemisch wurde 15 Minuten im Ultraschallbad, dann über Nacht bei Raumtemperatur gerührt und im Vakuum eingeengt. Der verbleibende Rückstand wurde mit 100 ml Wasser versetzt und die wäßrige Phase zweimal mit je 50 ml Ether ausgeschüttelt. Durch Zugabe von 2 M wäßriger Salzsäure wurde die wässerige Phase auf einen pH von 3 - 4 eingestellt und dreimal mit Essigester extrahiert. Die vereinigten Essigester-Phasen wurden einmal mit Wasser gewaschen, dann getrocknet und im Vakuum eingeengt. Man erhielt 1.6 g (58% der Theorie) eines gelbbraunen Öls.
IR (KBr): 1616, 1724 $cm^{-1}$ (C=O)
$R_f$: 0.33 (Essigester/Methanol = 8/2 (v/v))
ESI-MS: $(M+H)^+$ = 557/559/561/563 ($Br_2$, Cl)
Entsprechend wurden hergestellt (jeweils n = 1):

| RCO | Z | $R^2$ | Anmerkungen | % Ausbeute | MS | $R_f$ | Fließmittel | IR [$cm^{-1}$] |
|---|---|---|---|---|---|---|---|---|
| N8 | N-H | AS4 | | 62 | ESI: ( $M+H)^+$ = 482/4/6 ($Br_2$) | 0.61 | Essigester/ Methanol = 6/4 (v/v) | (KBr): C=O 1612.4, 1724.3: -OH, -NH- 3386.8; 3483.2 |
| N15 | N-H | AS4 | | 64 | ESI: $(M+H)^+$ = 578/80/82 ($Br_2$); $(M+H)^+$ = 580/2/4 ($Br_2$); $(M+Na)^+$ = 602/4/6 ($Br_2$) | 0.10 | Essigester/ Methanol = 8/2 (v/v) | (KBr): C=O 1703.0 |
| N23 | N-H | AS4 | | 58 | ESI: $(M+H)^+$ = 557/59/61/63 ($Br_2$,Cl) | 0.33 | Essigester/ Methanol = 8/2 (v/v) | (KBr): C=O 1616.3; 1724.3 |
| N15 | N-H | AS1 | keine Zugabe von Natriumhydroxid | 59 | ESI: $(M+H)^+$ = 579/81/83 ($Br_2$) | 0.72 | EE/ MeOH/ $NH_4OH$ = 6/4/1 (v/v/v) | (KBr): C=O 1695.3 |
| N66 | N-H | AS21 | | 95 | | 0.48 | EE/ AcOH 10/0.02 (v/v) | (KBr): C=O 1639 |
| N66 | CH2 | AS1 | | 85 | | 0.38 | $CH_2Cl_2$/ MeOH/ AcOH 9/1/0.15 (v/v/v) | |
| N71 | CH2 | AS1 | | 66.6 | ESI: $(M+H)^+$ = 606/08/10 ($Br_2$) | 0.38 | $CH_2Cl_2$/ MeOH/ AcOH 9/1/0,15 (v/v/v) | (KBr): C=O 1622/1680 |
| N66 | N-H | AS18 | | 100 | ESI: $(M+H)^+$ = 557 | 0.26 | EE/ AcOH 9/0.01 (v/v) | |
| N66 | N-H | AS19 | | 98 | | 0.22 | $CH_2Cl_2$/ MeOH/ AcOH 9/1/0,15 (v/v/v) | (KBr): C=O 1665/1740 |
| N66 | N-H | AS5 | | 73 | ESI: $(M+H)^+$ = 577/79/81 ($Br_2$) | 0.23 | FM1 | (KBr): C=O 1632/1705 |
| N66 | N-H | AS22 | | 78 | | 0.30 | FM1 | (KBr): C=O 1668/1739 |
| N66 | CH2 | AS21 | | 79 | | 0.34 | EE/ AcOH 9/0.01 (v/v) | (KBr): C=O 1643/1703 |
| N66 | CH2 | AS1 | | 90 | | 0.30 | EE/MeOH 9/1 (v/v) | |
| N15 | CH2 | AS1 | | 78 | ESI: $(M+H)^+$ = 578/80/82 ($Br_2$) | 0.30 | EE/ AcOH 9/0.01 (v/v) | (KBr): C=O 1728/1672 |

(fortgesetzt)

| RCO | Z | $R^2$ | Anmerkungen | % Ausbeute | MS | $R_f$ | Fließmittel | IR [$cm^{-1}$] |
|---|---|---|---|---|---|---|---|---|
| N66 | N-H | AS25 | | 99 | | | | |
| N66 | $CH_2$ | AS2 | | 100 | | | | (KBr): C=O 1645/1712 |
| N66 | $CH_2$ | AS23 | | 70 | | | | |
| N139 | $CH_2$ | AS2 | | 50 | | | | (KBr): C=O 1630/1662/ 1707 |
| N66 | $CH_2$ | AS27 | | 93 | | 0.20 | FM1 | |
| N66 | $CH_2$ | AS28 | LiOH | 100 | | 0.30 | FM1 | |
| N66 | $CH_2$ | AS4 | | 72 | | 0.53 | FM1 | (KBr): C=O 1639/1701 |
| N66 | $CH_2$ | AS36 | | 74 | ESI: $(M+H)^+$ = 434 | 0.36 | FM1 | (KBr): C=O 1645/1701 |
| N66 | $CH_2$ | AS38 | | 69 | | | | |
| N66 | $CH_2$ | AS48 | | 47 | EI: $M^+$ = 489 | 0.30 | FM1 | (KBr): C=O 1645 |
| N66 | N-H | AS49 | | 47 | | 0.10 | FM1 | |
| N66 | $CH_2$ | AS18 | | 60 | | 0.15 | EE | |
| N66 | $CH_2$ | AS39 | | 96 | | | | |
| N109 | $CH_2$ | AS21 | | 81 | | | | |
| N113 | $CH_2$ | AS21 | | 76 | | 0.20 | EE/AcOH 99/1 | |
| N134 | $CH_2$ | AS21 | | 89 | | 0.15 | EE/AcOH 99/1 | |
| N66 | $CH_2$ | AS47 | | 100 | ESI: $(M+H)^+$ = 476 | | | (KBr): C=O 1645/1716 |
| N66 | $CH_2$ | AS7 | | 60 | | 0.20 | FM1 | (KBr): C=O 1649/1722 |
| N66 | $CH_2$ | AS52 | | 95 | ESI: $(M+H)^+$ = 480 | 0.15 | FM1 | (KBr): C=O 1643/1722 |

Beispiel A38

Herstellung von Verbindungen der allgemeinen Formel:

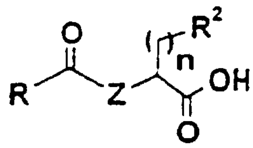

3,5-Dibrom-N-[[[2-(3-methoxyphenyl)ethyl]amino]carbonyl]-D-tyrosin

Eine Mischung aus 24 g (46.3 mmol) 3,5-Dibrom-N-[[[2-(3-methoxyphenyl) ethyl]amino]carbonyl]-D-tyrosinmethylester und 5.0 g (50 mmol) Lithiumhydroxid in 200 ml Wasser wurde 1 Stunde bei 60 °C gerührt. Das Festprodukt wurde abgesaugt und das Filtrat mit 200 ml Essigester gewaschen. Durch Zugabe von 1 M wäßriger Salzsäure wurde die wäßrige Phase auf einen pH von 3 - 4 eingestellt und dreimal mit 150 ml Essigester extrahiert. Die vereinigten Essigester-Phasen wurden einmal mit Wasser gewaschen, über Natriumsulfat getrocknet und im Vakuum eingeengt. Der Rückstand wurde mit Ether verrieben. Man erhielt 9.1 g (38 % der Theorie) eines farblosen Feststoffes.
IR (KBr): 1719 $cm^{-1}$ (C=O)
$R_f$: 0.57 (Essigester/Methanol/Eisessig = 9.5/0.5/0.2 (v/v/v))
Entsprechend wurden hergestellt (jeweils n = 1):

| RCO | Z | $R^2$ | Anmerkungen | % Ausbeute | MS | $R_f$ | Fließmittel | IR [$cm^{-1}$] |
|---|---|---|---|---|---|---|---|---|
| N6 | N-H | AS1 | | 100 | | 0.20 | FM1 | (KBr): C=O 1625.9; 1730 |
| N15 | N-H | AS6 | $H_2O$/ MeOH = 1/1 (v/v) als Lösemittel | 85 | ESI: $(M+H)^+$ = 501/3 (Br) | 0.53 | EE/ MeOH/ AcOH= 9/1/0.1 (v/v/v) | (KBr): C=O 1695.3 |
| N2 | N-H | AS1 | | 75 | | 0.57 | EE/ Methanol/ Eisessig = 9.5/0.5/0.2 (v/v/v) | (KBr): C=O 1718.5 |
| N2 | N-H | AS2 | | 71 | | 0.20 | FM1 | (KBr): C=O 1625.9, 1693.4; 1718.5; -NH- 3357.9 |
| N15 | N-H | AS3 | $H_2O$/ MeOH = 1/1 (v/v) als Lösemittel | 57 | | 0.30 | EE/ MeOH = 1/1 (v/v) | (KBr): C=O 1693.4 |
| N66 | | AS1 | | 75 | | 0.05 | EE/MeOH 8/2 | |
| N66 | | AS4 | | 85 | | | | |
| N122 | | AS1 | | 44 | | | | |
| N122 | | AS4 | | 85 | | | | |
| N66 | N-CH3 | AS1 | | 58 | ESI: $(M+H)^+$ = 607/09/11 ($Br_2$) | 0.20 | EE | (KBr): C=O 1607/1655/ 1711 |
| N66 | N-H | AS17 | | 55 | | 0.03 | FM1 | |
| N15 | CH2 | AS1 | | 78 | ESI: $(M+H)^+$ = 578/80/82 ($Br_2$) | 0.30 | EE/ MeOH 9/1 (v/v) | (KBr): C=O 1672/ 1728 |
| N66 | N-H | AS23 | | 79.0 | | 0.22 | FM1 | (KBr): C=O 1738/1664 |

Beispiel A39

$N^6$-[(1,1-Dimethylethoxy)carbonyl]-$N^2$-[N-[[[2-(3-methoxyphenyl)ethyl]amino]carbonyl]-3,5-dibrom-D-tyrosyl]-L-lysinmethylester

Zu der Mischung aus 10 g (19.4 mmol) 3,5-Dibrom-N-[[[2-(3-methoxyphenyl)ethyl] amino]carbonyl]-D-tyrosin, 2.6 g (20 mmol) DIEA, 6.4 g (20 mmol) TBTU, 2.64 g (19.5 mmol) HOBt und 200 ml Dimethylformamid tropfte man unter Rühren die Lösung von 5.04 g (19.4 mmol) H-Lys(Boc)-OMe in 50 ml Dimethylformamid und rührte den Ansatz über Nacht bei Raumtemperatur. Das Reaktionsgemisch wurde im Vakuum eingeengt und der Rückstand in 250 ml Essigester aufgenommen. Die Essigesterphase wurde anschließend zweimal mit je 100 ml gesättigter wäßriger Natriumhydrogencarbonat-Lösung, einmal mit 100 ml 20proz. wäßriger Zitronensäure-Lösung und schließlich einmal mit 100 ml gesättigter wäßriger Kochsalz-Lösung gewaschen. Die organische Phase wurde mit Natriumsulfat getrocknet, im Vakuum eingeengt und der Rückstand säulenchromatographisch (MN-Kieselgel 60, Macherey-Nagel, 70-230 mesh ASTM, Fließmittel: Essigester/ Petrolether = 2/1 (v/v)) gereinigt. Nach Entfernen des Lösemittels im Vakuum wurde der Rückstand mit Ether verrieben, der erhaltene amorphe Feststoff (9.5 g; 66 % der Theorie) abgenutscht und getrocknet.
IR (KBr): 1632; 1657, 1682, 1734 $cm^{-1}$ (C=O)
$R_f$: 0.64 (Essigsäureethylester)
ESI-MS: $(M+H)^+$ = 757/759/761 ($Br_2$)
$(M+Na)^+$ = 779/781/783 ($Br_2$)

Beispiel A40

$N^6$-[(1,1-Dimethylethoxy)carbonyl]-$N^2$-[N-[[[2-(3-methoxyphenyl)ethyl]amino]carbonyl]-3,5-dibrom-D-tyrosyl]-L-lysin

Die Mischung aus 7.75 g (10.4 mmol) $N^6$-[(1,1-Dimethylethoxy)carbonyl]-$N^2$-[N-[[[2-(3-methoxyphenyl)ethyl] amino]carbonyl]-3,5-dibrom-D-tyrosyl]-L-lysinmethylester, 3.5 g (140 mmol) Lithiumhydroxid und 150 ml Wasser wurde über Nacht bei Raumtemperatur gerührt. Die wäßrige Phase wurde einmal mit Essigester gewaschen, durch Zugabe von 1 M wäßriger Kaliumhydrogensulfat Lösung sauer gestellt und anschließend mit Essigester extrahiert. Die organische Phase wurde über Natriumsulfat getrocknet und im Vakuum eingeengt. Man erhielt 6.9 g (91% der Theorie) eines gelblichen Öls.
IR (KBr): 1653 $cm^{-1}$ (C=O)
$R_f$: 0.7 (Essigester/Methanol/Eisessig = 9/0.5/0.5 (v/v/v))
ESI-MS: $(M-H)^-$ = 741/743/745 ($Br_2$)

Beispiel A41

1-[$N^2$-[N-(Phenylmethoxycarbonyl)-3,5-dichlor-D-tyrosyl]-$N^6$-(1,1-dimethylethoxycarbonyl)-L-lysyl]-4-(4-pyridinyl)-piperazin

Die Mischung aus 5 g (13.0 mmol) 3,5-Dichlor-N-[(phenymethoxy)carbonyl]-D-tyrosin, 5.1 g (13.0 mmol) 1-[$N^6$-[(1,1-Dimethylethoxy)carbonyl]-L-lysyl]-4-(4-pyridinyl)-piperazin, 1.81 g (14 mmol) DIEA, 4.17 g (13 mmol) TBTU, 1.75 g (13.0 mmol) HOBt und 200 ml Tetrahydrofuran wurde bei Raumtemperatur über Nacht gerührt. Das Reaktionsgemisch wurde im Vakuum eingeengt, der Rückstand in Essigester/Methanol (95/5) aufgenommen und zweimal mit gesättigter wäßriger Natriumhydrogencarbonat-Lösung gewaschen. Die organische Phase wurde getrocknet, im Vakuum eingeengt und der Rückstand säulenchromatographisch (MN-Kieselgel 60, Macherey-Nagel, 70-230 mesh ASTM, Fließmittel: Essigester/Methanol = 6/4 (v/v)) gereinigt. Man erhielt 6.0 g (61 % der Theorie) eines gelblichen Öls.
$R_f$: 0.47 (FM1)
ESI-MS: $(M+H)^+$ = 757/759/761 ($Cl_2$)

Beispiel A42

Herstellung von Verbindungen der allgemeinen Formel:

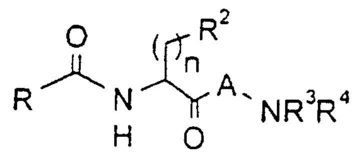

1-[$N^2$-[N-[[[2-(3-Methoxyphenyl)ethyl]amino]carbonyl]-3, 5-dibrom-D-tyrosyl]-N6-[(1,1-dimethylethoxy)carbonyl]-L-lysyl]-4-(1-methyl-4-piperidinyl)-piperazin

Zu der Mischung aus 1.1 g (1.5 mmol) $N^6$-[(Dimethylethoxy)-carbonyl]-$N^2$-[N-[[[2-(3-methoxyphenyl)ethyl] amino]carbonyl]-3,5-dibrom-D-tyrosyl]-L-lysin, 0.79 g (6.1 mmol) DIEA, 0.52 g (1.6 mmol) TBTU, 0.2 g (1.5 mmol) HOBt und 100 ml Dimethylformamid wurde die Lösung von 0.44 g (1.5 mmol) 1-(1-Methyl-4-piperidinyl)piperazin in 30 ml Dimethylformamid bei Raumtemperatur getropft, die Mischung danach über Nacht gerührt und im Vakuum eingeengt. Der Rückstand wurde in Essigester/Methanol (95/5) aufgenommen, zweimal mit je 70 ml wäßriger gesättigter Natriumhydrogencarbonat-Lösung gewaschen, über Natriumsulfat getrocknet und im Vakuum eingeengt. Man erhielt 1.1 g (81% der Theorie) eines farblosen Schaumes.
$R_f$: 0.34 (Essigester/Methanol/konz. wässeriges Ammoniak = 7/2/1 (v/v/v))
Analog wurde hergestellt (n = 1):

| RCO | $R^2$ | A | $NR^3R^4$ | Amerkungen | % Ausbeute | $R_f$ | Fließmittel | IR [$cm^{-1}$] |
|---|---|---|---|---|---|---|---|---|
| N15 | AS1 | A11 | C1 | KHSO4-Lösung | 70 | 0.40 | FM3 | (KBr): C=O1697.3; 1641.3 |

Beispiel A43

1-[$N^2$-(3,5-Dichlor-D-tyrosyl)-$N^6$-[(1,1-dimethylethoxy)carbonyl]-L-lysyl]-4-(4-pyridinyl)-piperazin

Die Lösung von 6 g (7.9 mmol) 1-[$N^2$-[N-[(Phenylmethoxy)carbonyl]-3,5-dichlor-D-tyrosyl]-$N^6$-[(1,1-dimethylethoxy)carbonyl]-L-lysyl]-4-(4-pyridinyl)-piperazin in einem Gemisch aus 200 ml Methanol und 20 ml wässeriger 1 M Kaliumhydrogensulfat-Lösung wurde in Gegenwart von 0.5 g Palladiummohr als Katalysator bei Raumtemperatur und 3 bar Wasserstoffdruck 40 Minuten lang hydriert. Der Katalysator wurde abfiltiert, das Reaktionsgemisch im Vakuum zur Trockene eingedampft und der Rückstand durch Zugabe von 2 ml konzentrierter wässeriger Ammoniaklösung auf einen pH-Wert von ca. 10 eingestellt. Das Produkt wurde mehrfach mit Isopropanol extrahiert, die vereinigten Isopropanol-Extrakte im Vakuum eingeengt und der verbleibende Rückstand säulenchromatographisch (LiChroprep, Si60 Korngröße: 20-40 µm, Fa. Merck (Darmstadt); Fließmittel: Methylenchlorid / Methanol / Ammoniak = 350/75/75/10 (v/v/v/v))gereinigt. Man erhielt 2.5 g (51 % der Theorie) einer farblosen, amorphen Festsubstanz.
IR (KBr): 1641, 1705 $cm^{-1}$ (C=O)
$R_f$: 0.27 (FM1)

Beispiel A44

Herstellung von Verbindungen der allgemeinen Formel:

$$Fmoc\text{-}A\text{-}NR^3R^4$$

1-[$N^2$-[(9-Fluorenylmethoxy)carbonyl]-$N^G$-(2,2,5,7,8-pentamethylchroman-6-sulfonyl)-L-arginyl]-4-(4-pyridinyl)-piperazin

Zu einer Mischung aus 7.0 g (10.6 mmol) Fmoc-Arg(Pmc)-OH, 1.42 g (11.0 mmol) DIEA, 3.53 g (11.0 mmol) TBTU, 1.35 g (11.0 mmol) HOBt und 50 ml DMF wurde unter Rühren tropfenweise eine Lösung von 1.74 g (10.6 mmol) 1-(4-Fyridinyl)-piperazin in 20 ml DMF zugegeben. Die Reaktionsmischung wurde weitere 3.5 Stunden bei Raumtemperatur gerührt und anschließend bei 40°C im Hochvakuum eingeengt. Der Rückstand wurde in Essigsäureethylester gelöst, die organische Phase zweimal mit gesättigter wässeriger Natriumhydrogencarbonat-Lösung gewaschen, über Natriumsulfat getrocknet und im Vakuum eingeengt. Der Rückstand wurde mit Diethylether verrieben, abgenutscht

und getrocknet. Man erhielt 7.85 g (96 % der Theorie) des gewünschten Endproduktes, das ohne weitere Reinigung weiter umgesetzt wurde.
$R_f$: 0.5 (FM1)
Analog wurde hergestellt:

| A | $NR^3R^4$ | % Ausbeute | $R_f$ | Fließmittel | IR [$cm^{-1}$] |
|---|---|---|---|---|---|
| A3 | C18 | 60 | 0.55 | FM4 | (KBr): C=O 1643. 1711 |

Beispiel A45

Herstellung von Verbindungen der allgemeinen Formel:

$$H—A—NR^3R^4$$

(1-[$N^G$-(2,2,5,7,8-Pentamethylchroman-6-sulfonyl)-L-arginyl]-4-4-pyridinyl)-piperazin

Eine Lösung von 8.5 g (11.1 mmol) 1-[$N^2$-[(9-Fluorenylmethoxy)carbonyl]-$N^G$-(2,2,5,7,8-pentamethylchroman-6-sulfonyl)-Larginyl]-4-(4-pyridinyl)-piperazin in 100 ml THF wurde mit 16 ml Diethylamin versetzt und anschließend 2.5 Stunden bei Raumtemperatur gerührt. Das Reaktionsgemisch wurde im Vakuum eingeengt und der verbleibende Rückstand säulenchromatographisch (MN-Kieselgel 60, Macherey-Nagel, 70-230 mesh ASTM, Fließmittel: FM1) gereinigt. Man erhielt 3.3 g (54 % der Theorie) eines amorphen Feststoffes.
$R_f$: = 0.19(FM1)
IR(KBr): 1637 $cm^{-1}$ (C=O)
Analog wurde hergestellt:

| A | $NR^3R^4$ | % Ausbeute | IR [$cm^{-1}$] |
|---|---|---|---|
| A3 | C18 | 80 | (KBr): C=O 1637.5; 1705 |

Beispiel A46

1-[$N^6$,$N^6$-Dimethyl-$N^2$-[(phenylmethoxy)carbonyl]-L-lysyl]-4-(4-pyridinyl)-piperazin

9.6 g (18.3 mmol) 1-[$N^6$-[(1,1-Dimethylethoxy)carbonyl]-$N^2$-[(phenylmethoxy)carbonyl]-L-lysyl]-4-(4-pyridinyl)-piperazin wurden in 200 ml einer 5proz. Lösung von Trifluoressigsäure in Dichlormethan über Nacht gerührt. Die Reaktionsmischung wurde anschließend im Vakuum eingeengt. Man erhielt 13.47 g (97% der Theorie) des gewünschten 1-[$N^2$-[(Phenylmethoxy)carbonyl]-L-lysyl]-4-(4-pyridinyl)-piperazin als Trifluoracetat-Salz. Anschließend wurden 7.0 g (9.1 mmol) des Rohproduktes in 200 ml Wasser gelöst und unter Eisbadkühlung 4.1 ml einer 40proz. Formaldehydlösung (45.6 mmol) zugegeben. Die Reaktionsmischung wurde 10 Minuten bei Raumtemperatur gerührt, unter Eisbadkühlung vorsichtig mit 1.5 g (40 mmol) Natriumborhydrid versetzt, dann unter äußerer Kühlung mit Eis mit 4.1 ml einer 40proz. Formaldehydlösung (45.6 mmol), wonach die Reaktionsmischung nochmals 10 Minuten bei Raumtemperatur gerührt und unter Eisbadkühlung abermals mit 1.5 g (40 mmol) Natriumborhydrid versetzt wurde. Der pH-Wert der Reaktionsmischung wurde während der Reaktion permanent kontrolliert und durch Zutropfen von Trifluoressigsäure immer zwischen pH 3 und pH 6 gehalten. Das Gemisch wurde dann 30 Minuten bei 5°C gerührt, durch Zugabe von Kaliumcarbonat auf pH 10 gestellt und viermal mit je 50 ml Essigsäureethylester extrahiert. Die vereinigten organischen Phasen wurden getrocknet, im Vakuum eingeengt und der verbleibende Rückstand säulenchromatographisch (MN-Kieselgel 60, Macherey-Nagel, 70-230 mesh ASTM, Fließmittel: Essigester/Methanol/konz. wässeriges Ammoniak = 6.5/3/0.3 (v/v/v)) gereinigt. Man erhielt 2.3 g (56 % der Theorie) eines farblosen Öls.
IR (KBr): 1711, 1649 $cm^{-1}$ (C=O)
$R_f$ : 0.2 (FM7)
ESI-MS: $(M+H)^+$ = 454

Beispiel A47

Herstellung von Verbindungen der allgemeinen Formel:

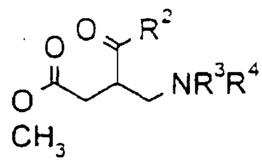

(R,S)-4-Amino-3,5-dibrom-γ-oxo-β-[[4-(4-pyridinyl)-1-piperidinyl]methyl]-benzenbutansäuremethylester

Eine Mischung aus 10 g (27 mmol) 4-Amino-3,5-dibrom-γ-oxobenzenbutansäuremethylester, 5.4 g (27 mmol) 4-(4-Pyridinyl)-piperidin und 1.5 g (45 mmol) Paraformaldehyd wurde in 20 ml Eisessig suspendiert und unter Rühren in einem Ölbad (Badtemperatur: 100°C) erwärmt. Nach 3 Stunden wurden nochmals 1.5 g (45 mmol) Paraformaldehyd zugegeben und die Mischung weitere 3 Stunden bei 100°C und dann 1 Stunde bei 125°C gerührt. Das Lösemittel wurde im Vakuum entfernt und der Rückstand in 800 ml Wasser aufgenommen. Die wässerige Phase wurde durch Zugabe von Natriumcarbonat alkalisch gestellt und zweimal mit je 500 ml Essigester extrahiert. Die vereinigten Essigester-Extrakte wurden getrocknet, im Vakuum eingeengt und der Rückstand säulenchromatographisch (MN-Kieselgel 60, Macherey Nagel, 70-230 mesh ASTM, Fließmittel: Essigester/Methanol = 9:1)) gereinigt. Man erhielt 1.0 g (6.8 % der Theorie) des gewünschten Endproduktes als Öl.
IR(KBr): 1716.5 $cm^{-1}$
$R_f$: 0.7 (FM1)
Analog wurde hergestellt:

| $R^2$ | $NR^3R^4$ | % Ausbeute | $R_f$ | Fließmittel | IR [$cm^{-1}$] |
|---|---|---|---|---|---|
| AS4 | C8 | 35 | 0.68 | FM1 | (KBr): C=O 1672.2; 1733.9 |

Beispiel A48

Herstellung von Verbindungen der allgemeinen Formel:

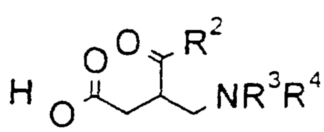

(R,S)-4-Amino-3,5-dibrom-γ-oxo-β-[[4-(4-pyridinyl)-1-piperidinyl]methyl]-benzenbutansäure

Eine Mischung aus 1.0 g (1.9 mmol) (R,S)-4-Amino-3,5-dibrom-γ - oxo-β-[[4-(4-pyridinyl)-1-piperidinyl]methyl]-phenylbutansäuremethylester, 5 ml 1 N Natronlauge und 50 ml Dioxan wurde über Nacht bei Raumtemperatur und 1 Stunde bei 60°C gerührt. Die Reaktionsmischung wurde anschließend durch Zugabe von 5 ml 1N Salzsäure neutralisiert, im Vakuum eingeengt und der Rückstand im Vakuumtrockenschrank getrocknet. Man erhielt 0.97 g (100% der Theorie) des gewünschten Produktes das ohne Reinigung weiter umgesetzt wurde.
$R_f$: 0.15 (FM1)
Analog wurde hergestellt:

| $R^2$ | $NR^3R^4$ | % Ausbeute | $R_f$ | Fließmittel | IR [$cm^{-1}$] |
|---|---|---|---|---|---|
| AS4 | C8 | 96 | 0.2 | FM1 | (KBr): C=O 1660 |

Beispiel A49

3,5-Dibrom-4-hydroxy-β-(methoxycarbonyl)-benzenbutansäure

Zu der Lösung von 12 g (0.043 mol)4-Hydroxy-β-(methoxycarbonyl)-benzenbutansäure in 200 ml Essigsäure gab man 150 ml Wasser und 8 g Natriumacetat, tropfte unter Rühren eine Lösung von 5 ml Brom in 60 ml Essigsäure zu, dampfte anschließend die Reaktionsmischung im Vakuum weitgehend ein und rührte den verbleibenden Rückstand in Wasser ein. Die wässerige Phase wurde wiederholt mit Essigester extrahiert, die vereinigten organischen Phasen mit Wasser gewaschen. Die organischen Extrakte wurden getrocknet, im Vakuum eingeengt und der verbleibende feste Rückstand aus Diisopropylether umkristallisiert. Man erhielt 12 g (70% der Theorie) des gesuchten Endprodukts.
$R_f$: 0.4 (Essigester/Petrolether/Eisessig = 5/5/0.4 (v/v/v)
ESI-MS: $(M+H)^+$ = 394/6/8 ($Br_2$)

Beispiel A50

Herstellung von Verbindungen der allgemeinen Formel:

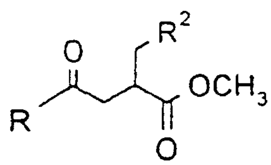

(R,S)-2-[(3,5-Dibrom-4-hydroxyphenyl)methyl]-4-[4-(1,3-dihydro-2(2H)-oxobenzimidazol-1-yl)-1-piperidinyl]-4-oxo-butansäuremethylester

Die Lösung von 2.0 g (5 mmol) 3,5-Dibrom-4-hydroxy-β-(methoxycarbonyl)-benzenbutansäure in 80 ml THF wurde unter Rühren mit 1.6 g (5 mmol) TBTU, 0.76 g (5 mmol) HOBt, 1.25 g (5 mmol) 4-(1,3-Dihydro-2(2H)-oxobenzimidazol-1-yl)piperidin und 1.03 g (8 mmol) DIEA versetzt. Die Reaktionsmischung wurde 6 Stunden bei Raumtemperatur gerührt und anschließend im Vakuum eingeengt. Der verbleibende Rückstand wurde mit gesättigter wäßriger Natriumhydrogencarbonatlösung versetzt und mehrmals mit Essigester extrahiert. Die vereinigten organischen Extrakte wurden nacheinander mit gesättigter wäßriger Natriumhydrogencarbonatlösung und Wasser gewaschen, über Natriumsulfat getrocknet und im Vakuum eingeengt. Man erhielt 3.0 g (50% der Theorie) des gesuchten Produktes, das ohne Reinigung weiter umgesetzt wurde.
IR (KBr): 1714.8 $cm^{-1}$ (C=O)
$R_f$: 0.7 (Essigester/Petrolether = 7/3 (v/v))

Analog wurden hergestellt:

| RCO | $R^2$ | % Ausbeute | MS | $R_f$ | Fließmittel | IR [$cm^{-1}$] |
|---|---|---|---|---|---|---|
| N66 | AS1 | 98 | | 0.66 | FM1 | |
| N66 | AS2 | 100 | | 0.77 | FM1 | (KBr): C=O 1664/1734 |
| N139 | AS2 | 100 | EI: $M^+$ = 486 | 0.30 | FM1 | (KBr): C=O 1643 / 1672 / 1732 |
| N66 | AS4 | 28 | EI: $M^+$ = 606/08/10 ($Br_2$) | 0.33 | FM4 | (KBr): C=O 1666/1734 |
| N66 | AS36 | 63 | | 0.56 | FM4 | |
| N66 | AS38 | 92 | | | | |
| N66 | AS48 | 100 | | 0.68 | FM1 | |
| N66 | AS18 | 22 | | | | |
| N66 | AS39 | 100 | | | | |

(fortgesetzt)

| RCO | $R^2$ | % Ausbeute | MS | $R_f$ | Fließmittel | IR [$cm^{-1}$] |
|---|---|---|---|---|---|---|
| N109 | AS21 | 39 | | 0.35 | EE | (KBr): C=O 1639/1734 |
| N113 | AS21 | 57 | | 0.15 | EE/PE 95/5 | |
| N134 | AS21 | 80 | | 0.15 | EE | |
| N66 | AS7 | 100 | | 0.75 | FM1 | |
| N66 | AS53 | 40 | | | | |

Beispiel A51

(R)-1-[2-Amino-3-(3,5-dibrom-4-hydroxyphenyl)propyl]-4-(1-piperidinyl)-piperidin

Zu der Suspension aus 3.8 g (100 mmol) Lithiumaluminiumhydrid in 400 ml THF wurden unter Rühren bei Raumtemperatur 14.4 g (20 mmol) 1-(3,5-Dibrom-D-tyrosyl)-4-(1-piperidinyl)-piperidin innerhalb von 30 Minuten portionsweise zugegeben. Die Reaktionsmischung wurde 30 Minuten bei Raumtemperatur und 2 Stunden unter Rückfluß gekocht und anschließend durch vorsichtige Zugabe von 1 ml Wasser und 5.1 ml konzentrierter wäßriger Salzsäure neutralisiert. Nach Zugabe von 100 ml Methanol wurde der feste Niederschlag abgesaugt und das Filtrat im Vakuum eingeengt. Der verbleibende Rückstand wurde säulenchromatographisch (MN-Kieselgel 60, Macherey-Nagel, 70-230 mesh ASTM, Fließmittel: Methylenchlorid/Methanol/konz. wässeriges Ammoniak = 8/2/0.2 (v/v/v)) gereinigt. Man erhielt 5.4 g (57 % der Theorie) des gesuchten Produktes als amorphe Festsubstanz.
IR (KBr) : 3420 $cm^{-1}$ ($NH_2$)
$R_f$: 0.4 (FM2)
ESI-MS: $M^+$ = 473/475/477 ($Br_2$)
Analog wurde hergestellt:

Aus 1-(4-Amino-3,5-dibrom-D-phenylalanyl)-4-(1-piperidinyl)-piperidin in einer Ausbeute von 56.5 % der Theorie das (R)-1-[2-Amino-3-(4-amino-3,5-dibromphenyl)propyl]-4-(1-piperidinyl)-piperidin vom $R_f$ 0.12 (Fließmittel: Dichlormethan/Methanol/Cyclohexan/konz.Ammoniak 7/1.5/1.5/0.2 (v/v/v/v)).

Beispiel A52

(R)-1-[3-(4-Amino-3,5-dibromphenyl)-2-[N-[(1,1-dimethylethoxy)carbonyl]amino]propyl]-4-(1-piperidinyl)-piperidin

Zu der Lösung von 10 g (0.017 mol) 1-[4-Amino-3,5-dibrom-N-[(1,1-dimethylethoxy)carbonyl]-D-phenylalanyl]-4-(1-piperidinyl)-piperidin in 350 ml Dioxan wurden 3.1 g (0.082 mol) Natriumborhydrid gegeben und das Reaktionsgemisch auf 5°C gekühlt. Unter Rühren wurde anschließend eine Lösung von 4.92 g (0.082 mcl) Essigsäure in 100 ml Dioxan zugetropft. Das Reaktionsgemisch wurde eine weitere Stunde bei Raumtemperatur und 3 Stunden bei 85°C gerührt. Anschließend wurde mit Eiswasser versetzt, das organische Lösemittel im Vakuum entfernt und der verbleibende wässerige Rückstand wiederholt mit Methylenchlorid extrahiert. Die vereinigten organischen Phasen wurden getrocknet, im Vakuum eingeengt und der verbleibende Rückstand säulenchromatographisch (MN-Kieselgel 60, Macherey-Nagel, 70-230 mesh ASTM, Fließmittel: Methylenchlorid/Methanol/Cyclohexan/konz. wäßriges Ammoniak = 3600/150/150/20 (v/v/v/v) gereinigt. Man erhielt 4.1g (42 % der Theorie) eines farblosen Schaumes.
IR (KBr): 1705 $cm^{-1}$ (C=O)

Beispiel A53

(R)-1-[2-Amino-3-(4-amino-3,5-dibromphenyl)propyl]-4-(1-piperidinyl)-piperidin

Zu einer Mischung aus 4 g (7 mmol) (R)-1-[3-(4-Amino-3,5-dibromphenyl)-2-[N-[(1,1-dimethylethoxy)carbonyl]amino]-propyl]-4-(1-piperidinyl)-piperidin und 100 ml Methylenchlorid wurden unter Rühren bei 10°C langsam 40 ml Trifluoressigsäure zugetropft. Das Reaktionsgemisch wurde 2 Stunden bei Raumtemperatur gerührt und anschließend im Vakuum eingeengt. Der verbleibende Rückstand wurde mit Eiswasser versetzt, durch Zugabe von konzentrierter wässeriger Ammoniaklösung basisch gestellt und dreimal mit je 200 ml Diethylether extrahiert. Die vereinigten

Etherextrakte wurden getrocknet und im Vakuum eingeengt. Man erhielt 3.4 g (100% der Theorie) eines amorphen Feststoffes.
IR (KBr): 1683.8, 1616.3 (C=O)
$R_f$: 0.02(FM 4)

Beispiel A54

(R,S)-4-[4-[3,4-Dihydro-2(1H)-oxochinazolin-3-yl]-1-piperidinyl]-4-oxo-2-[[3-(trifluormethyl)phenyl]methyl]-butansäuremethylester

Hergestellt analog Beispiel A15a) aus (R,S)-3-Carboxy-2-[[3-(trifluormethyl)phenyl]methyl]-propansäuremethylester und 3,4-Dihydro-3-(4-piperidinyl)-2(1H)-chinazolinon in einer Ausbeute von 27.3 % der Theorie. Farblose, amorphe Substanz vom $R_f$ = 0.25 (Fließmittel: Essigsäureethylester).
MS: $M^+$ = 503
Entsprechend wurde erhalten:

Aus (R,S)-3-Carboxy-2-[(3,5-dibrom-4-hydroxyphenyl)methyl]-propansäuremethylester und 3,4-Dihydro-3-(4-piperidinyl)-2(1H)-chinazolinon in einer Ausbeute von 98 % der Theorie der (R,S)-4-[4-[3,4-Dihydro-2(1H)-oxochinazolin-3-yl]-1-piperidinyl]-4-oxo-2-[(3,5-dibrom-4-hydroxyphenyl)methyl]-butansäuremethylester vom $R_f$ = 0.66 (Fließmittel: Dichlormethan/Cyclohexan/Methanol/konz. Ammoniak 7/1.5/1.5/0.2 (v/v/v/v)).

Beispiel A55

(R,S)-4-[4-[3,4-Dihydro-2(1H)-oxochinazolin-3-yl]-1-piperidinyl]-4-oxo-2-[(3,5-dibrom-4-hydroxyphenyl)methyl]-butansäure

Die Mischung aus 3.0 g (4.92 mmol) (R,S)-4-[4-(3,4-Dihydro-2(1H)-oxochinazolin-3-yl]-1-piperidinyl]-4-oxo-2-[(3,5-dibrom-4-hydroxyphenyl)methyl]-butansäuremethylester, 30 ml (30 mmol) 1N Natronlauge und 20 ml Methanol wurde 3 Stunden lang bei Zimmertemperatur gerührt, dann mit 100 ml Wasser verdünnt und tropfenweise mit 30 ml 1N Salzsäure versetzt. Der Niederschlag wurde abgenutscht und bei 50°C im Umlufttrockenschrank getrocknet. Farblose, amorphe Substanz vom $R_f$ = 0.38 (Fließmittel: Dichlormethan/Methanol/Eisessig 9/1/0.15 (v/v/v)). Ausbeute: 2.5 g (85.4 % der Theorie).
Entsprechend wurde erhalten:

Aus (R,S)-4-[4-[3,4-Dihydro-2(1H)-oxochinazolin-3-yl]-1-piperidinyl]-4-oxo-2-[[3-(trifluormethyl)phenyl]methyl]-butansäuremethylester in einer Ausbeute von 79 % der Theorie die (R,S)-4-[4-[3,4-Dihydro-2(1H)-oxochinazolin-3-yl]-1-piperidinyl]-4-oxo- 2-[[3-(trifluormethyl)phenyl]methyl]-butansäure vom $R_f$ = 0.34 (Fließmittel: Essigsäureethylester/Eisessig 99.8/0.2 (v/v)). IR(KBr): 1703, 1643 $cm^{-1}$ (C=O)

Beispiel A56

3,5-Dibrom-N-[[4-[3,4-dihydro-2(1H)-oxochinazolin-3-yl]-1-piperidinyl]carbonyl]-N-methyl-D-tyrosinmethylester

a) 1-(Chlorcarbonyl)-4-[3,4-dihydro-2(1H)-oxochinazolin-3-yl]piperidin

Zu der Mischung aus 7.0 ml (ca. 14 mmol) einer 20proz. Lösung von Phosgen in Toluol und 2.02 g (20 mmol) Triethylamin in 300 ml Tetrahydrofuran gab man portionsweise unter Einhaltung einer Reaktionstemperatur von ca. 0°C die Suspension von 1.5 g (5.60 mmol) 4-[3,4-Dihydro-2(1H)-oxochinazolin-3-yl]piperidinhydrochlorid in 100 ml Tetrahydrofuran. Man rührte noch 1 Stunde bei einer Temperatur zwischen 0°C und +5°C, filtrierte vom entstandenen Triethylamin-hydrochlorid ab und befreite das Filtrat vom Lösemittel. Der verbleibende Rückstand wurde mit Diisopropylether verrieben und abgenutscht. Nach dem Trocknen im Vakuum erhielt man 0.7 g (42.6 % der Theorie) an farblosen Kristallen vom $R_f$ = 0.17 (Fließmittel: Dichlormethan/Aceton 9.5/0.5 (v/v)), die ohne weitere Reinigung weiterverarbeitet wurden.

b) 3,5-Dibrom-N-[[4-[3,4-dihydro-2(1H)-oxochinazolin-3-yl]-1-piperidinyl]carbonyl]-N-methyl-D-tyrosinmethylester

Zu der Lösung von 4.9 g ( 13.3 mmol) 3,5-Dibrom-N-methyl-D-tyrosinmethylester und 4.04 g (40 mmol) Triethylamin in 500 ml Tetrahydrofuran tropfte man bei Zimmertemperatur innerhalb von 3 Stunden die Lösung von

3.92 g (13.34 mmol) 1-(Chlorcarbonyl)-4-[3,4-dihydro-2(1H)-oxochinazolin-3-yl]piperidin in 1 l Tetrahydrofuran. Anschließend erhitzte man 12 Stunden auf Rückflußtemperatur, ließ erkalten und filtrierte vom ausgefallenen Triethylamin ab. Das Filtrat wurde eingedampft, der Rückstand zwischen Essigsäureethylester und 20proz. wässeriger Zitronensäure verteilt. Die organische Phase wurde über Natriumsulfat getrocknet, abermals im Vakuum eingedampft, der Rückstand an Kieselgel unter Verwendung von Essigsäureethylester/Petrolether 9/1 (v/v) zum Eluieren säulenchromatographisch gereinigt. Aufarbeitung der geeigneten Fraktionen ergab 3.2 g (38.5 % der Theorie) einer farblosen, amorphen Substanz vom $R_f$ = 0.45 (Fließmittel: Essigsäureethylester).
IR(KBr): 1739.7, 1660.6 $cm^{-1}$ (C=O)
ESI-MS: $(M+H)^+$ = 623/625/627 ($Br_2$)
$(M+Na)^+$ = 645/647/649 ($Br_2$)
$(M+K)^+$ = 661/663/665 ($Br_2$)

Beispiel A57

3,5-Dibrom-4-methoxy-D-phenylalaninmethylester

Zu der Mischung aus 5.5 g (14.12 mmol) 3,5-Dibrom-4-methoxy-D-phenylalanin-hydrochlorid und 55 ml Methanol gab man 150 ml einer gesättigten methanolischen Chlorwasserstoff-Lösung und rührte 20 Stunden bei Zimmertemperatur. Der nach dem Vertreiben des Lösemittels verbleibende Rückstand wurde mit 50 ml Wasser verrührt und mit geättigter Natriumhydrogencarbonat-Lösung auf pH 8 gebracht. Der Niederschlag wurde abgenutscht, mit 10 ml Isopropanol verrührt und über Nacht stehen gelassen. Man filtrierte vom Unlöslichen und dampfte das Filtrat im Vakuum ein. Der Rückstand wurde als Rohprodukt weiter umgesetzt. Ausbeute: 1.0 g (28.7 % der Theorie) eines farblosen Öls vom $R_f$ = 0.55 (Fließmittel:
Dichlormethan/Essigsäureethylester/Cyclohexan/Methanol/konz. Ammoniak = 300/80/25/25/3 (v/v/v/v/v)).

Beispiel A58

1-(4-Amino-3,5-dibrom-D-phenylalanyl)-4-(4-pyrimidinyl)-piperazin

a) 1-(2-Chlor-4-pyrimidinyl)-4-(phenylmethyl)piperazin

Das Gemisch aus 9.9 g (0.0664 mol) 2,4-Dichlorpyrimidin, 200 ml Wasser und 11.7 ml (0.0673 mol) 1-(Phenylmethyl)piperazin wurde 2 Stunden lang im Ultraschallbad auf 40°C erwärmt. Nach dem Erkalten stellte man mit Kaliumcarbonat alkalisch und extrahierte erschöpfend mit Essigsäureethylester. Das nach üblicher aufarbeitung erhaltene Rohprodukt wurde an Kieselgel (30-60 μm) unter Verwendung eines Fließmittels aus FM2 und FM4 (2/1 v/v) zum Eluieren säulenchromatographisch gereinigt. Aufarbeitung der geeigneten Fraktionen ergab 7.4 g (38.6 % der Theorie) eines farblosen Öls vom $R_f$ = 0.51 (FM4; Macherey-Nagel POLYGRAM® SIL G/$UV_{254}$, Fertigfolien für die DC).
MS: $M^+$ = 288/290 (Cl)

b) 1-(4-Pyrimidinyl)piperazin

Die Lösung von 7.4 g (0.0256 mol) 1-(2-Chlor-4-pyrimidinyl)-4-(phenylmethyl)piperazin in 100 ml Ethanol wurde in Gegenwart von 2 g 10proz. Palladiumkohle 4 Stunden bei 40°C und 5 bar Wasserstoffdruck hydriert. Das nach üblicher Aufarbeitung erhaltene rohe Produkt wurde an Kieselgel (30-60 μm) unter Verwendung von FM1/Cyclohexan 9/1 (v/v) zum Eluieren säulenchromatographisch gereinigt. Farblose Kristalle vom $R_f$ = 0.3 (FM1/Cyclohexan 9/1 (v/v)); Macherey-Nagel POLYGRAM® SIL G/$UV_{254}$, Fertigfolien für die DC). Ausbeute: 1.7 g (40.7 % der Theorie).

c) 1-[4-Amino-3,5-dibrom-N-(1,1-dimethylethoxycarbonyl)-D-phenylalanyl)-4-(4-pyrimidinyl)piperazin

Hergestellt analog Beispiel A15a) aus 4-Amino-3,5-dibrom-N-(1,1-dimethylethoxycarbonyl)-D-phenylalanin und 1-(4-Pyrimidinyl)piperazin in Gegenwart von TBTU in einer Ausbeute von 92 % der Theorie. Farblose, amorphe Substanz vom $R_f$ = 0.42 (FM4; Macherey-Nagel POLYGRAM® SIL G/$UV_{254}$, Fertigfolien für die DC).
IR(KBr): 1705.0, 1643.3 $cm^{-1}$ (C=O)
MS: $M^+$ = 582/584/586 ($Br_2$)

d) 1-(4-Amino-3,5-dibrom-D-phenylalanyl)-4-(4-pyrimidinyl)-piperazin

Hergestellt analog Beispiel A1b) aus 1-[4-Amino-3,5-dibrom-N-(1,1-dimethylethoxycarbonyl)-D-phenylalanyl)-4-(4-pyrimidinyl)-piperazin in einer Ausbeute von 52 % der Theorie. Farblose, amorphe Substanz vom $R_f$ = 0.55 (FM1; Macherey-Nagel POLYGRAM® SIL G/$UV_{254}$, Fertigfolien für die DC).
IR(KBr): 1681.8 $cm^{-1}$ (C=O)
MS: $M^+$ = 482/484/486 ($Br_2$)

Beisipiel A59

Herstellung von Verbindungen der allgemeinen Formel:

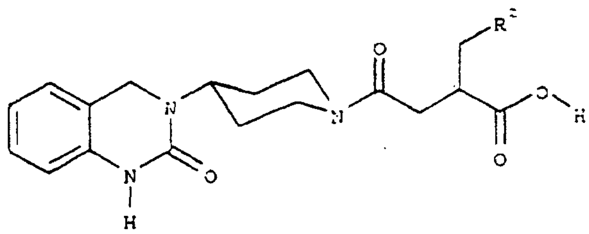

(R,S)-4-[4-(3,4-Dihydro-2(1H)-oxochinazolin-3-yl)-1-piperidinyl]-2-[[4-(1,1-dimethylethyl)phenyl]methyl]-4-oxobutan säure

Die Mischung aus 4.8 g (8.3 mMol) 4-[4-(3,4-Dihydro-2(1H)-oxochinazolin-3-yl)-1-piperidinyl]-2-[[4-(1,1-dimethylethyl)phenyl]methyl]-2-(ethoxycarbonyl)-4-oxobutansäureethylester, 200 ml Ethanol und 41.5 ml 1N Natronlauge wurde 3 Stunden unter Rückfluß gekocht.
Man entfernte das Ethanol im Vakuum, verdünnte den Rückstand mit 50 ml Wasser und stellte mit 1N wässeriger Salzsäure auf pH 3. Die ausgefallene Substanz wurde abgenutscht, mit Wasser gründlich gewaschen und im Vakuum getrocknet. Man erhielt 3.8 g (96 % der Theorie) an farblosen Kristallen vom Fp. 139 - 141 °C und $R_f$ = 0.65 (Fließmittel: EE/MeOH/Eisessig 90/10/1 v/v/v). IR(KBr): 1724, 1647 $cm^{-1}$ (C=O)
MS: kein $M^+$, m/e = 246, 231, 147

Analog wurden hergestellt:

| $R^2$ | Anmerkungen | % Ausbeute | MS | $R_f$ | Fließmittel | IR [$cm^{-1}$] |
|---|---|---|---|---|---|---|
| AS29 | | 100 | | | | |
| AS16 | | 17 | ESI: $(M+H)^+$ = 488/90/92 ($Cl_2$) | 0.30 | EE / MeOH / AcOH 80/10/1 | |
| AS5 | | 62 | | 0.60 | $CH_2Cl_2$/ MeOH / $NH_4OH$ 90/10/1 | |
| AS32 | | 100 | ESI: $(M+Na)^+$ = 614/6/8 ($Br_2$) | 0.67 | EE / MeOH / AcOH 90/10/1 | (KBr): C=O 1645/1728 |
| AS33 | | 90 | EI: $M^+$= 525 | 0.20 | EE / MeOH / AcOH 90/10/1 | (KBr): C=O 1643/1701 |
| AS31 | | 100 | | 0.20 | $CH_2Cl_2$/ MeOH / $NH_4OH$ 80/20/1 | |
| AS17 | | 100 | ESI: $(M+H)^+$= 608/10/12 ($Br_2$) | 0.50 | EE / MeOH / AcOH 90/10/1 | (KBr): C=O 1643 |
| AS34 | | 76 | ESI: $(M+H)^+$ = 506 | 0.65 | EE / MeOH / AcOH 90/10/1 | |

(fortgesetzt)

| $R^2$ | Anmerkungen | % Ausbeute | MS | $R_f$ | Fließmittel | IR [$cm^{-1}$] |
|---|---|---|---|---|---|---|
| AS19 | | 70 | | 0.46 | EE / MeOH / AcOH 9/1/0.5 | (KBr): C=O 1643/1701 |
| AS46 | | 78 | ESI: $(M+H)^+$= 471 | 0.20 | FM1 | (KBr): C=O 1647 |
| AS50 | | 97 | | 0.05 | EE | |
| AS2 | LiOH statt NaOH | 86 | ESI: $(M+H)^+$= 472 | | | (KBr): C=O 1643/1705 |
| AS29 | | 100 | ESI: $(M+H)^+$= 448 | | | (KBr): C=O 1645/1705 |
| AS31 | | 87 | | | | |

Beispiel A60

Herstellung von Verbindungen der allgemeinen Formel:

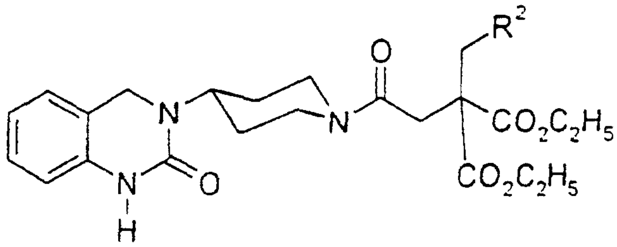

4-[4-(3,4-Dihydro-2(1H)-oxochinazolin-3-yl)-1-piperidinyl]-2-[[4-(1,1-dimethylethyl)phenyl]methyl]-2-(ethoxycarbonyl)-4-oxobutansäureethylester

Die Mischung aus 2.31 g (10 mMol) 4-(3,4-Dihydro-2(1H)-oxochinazolin-3-yl)-piperidin, 3.64 g (10 mMol) β, β-Bis-(ethoxycarbonyl)-4-(1,1-dimethylethyl)-benzenbutansäure, 5 ml Triethylamin, 3.5 g (11 mMol) TBTU, 200 ml Tetrahydrofuran und 20 ml Dimethylformamid wurde 5 Stunden lang bei Zimmertemperatur gerührt. Das Lösemittel wurde im Vakuum entfernt, der Rückstand in Dichlormethan aufgenommen, die entstandene Lösung über Natriumsulfat getrocknet und vom Lösemittel befreit. Nach säulenchromatographischer Reinigung an 400 g Kieselgel (Amicon, 35 - 70 µm, Essigsäureethylester als Eluens) erhielt man 4.8 g (83 % der Theorie) einer farblosen, amorphen Substanz vom $R_f$ = 0.63 (Fließmittel: EE).
IR(KBr): 1734, 1668, 1653 $cm^{-1}$ (C=O)
MS: $M^+$ = 577 ($Br_2$)

Analog wurden hergestellt:

| R2 | % Ausbeute | MS | $R_f$ | Fließmittel | IR [$cm^{-1}$] |
|---|---|---|---|---|---|
| AS29 | 75 | | 0.8 | FM1 | |
| AS16 | 59 | | 0.5 | EE | |
| AS5 | 65 | EI: $M^+$= 677/79/81 ($Br_2$) | 0.7 | FM4 | (KBr): C=O 1649 / 1668 / 1734 |

(fortgesetzt)

| R2 | % Ausbeute | MS | $R_f$ | Fließmittel | IR [$cm^{-1}$] |
|---|---|---|---|---|---|
| AS32 | 74 | | 0.5 | FM4 | (KBr): C=O 1647 / 1668 / 1734 |
| AS33 | 85 | | 0.5 | EE | (KBr): C=O 1649/1734 |
| AS31 | 82 | EI: $M^+$= 574 | 0.5 | $CH_2Cl_2$ / MeOH / $NH_4OH$ 90/10/1 | (KBr): C=O 1658/1741 |
| AS17 | 93 | EI: $M^+$= 707/09/11 ($Br_2$) | 0.5 | EE | (KBr): C=O 1645 / 1666 / 1736 / 1759 |
| AS34 | 75 | EI: $M^+$= 607 | 0.8 | EE | (KBr): C=O 1649 / 1668 / 1736 |
| AS19 | 67 | | 0.8 | FM1 | (KBr): C=O 1647/1668/ 1734 |
| AS46 | 80 | EI: $M^+$= 572 | 0.8 | FM1 | (KBr): C=O 1737 |
| AS50 | 78 | EI: $M^+$= 677/9/81 ($Br_2$) | 0.6 | EE | (KBr): C=O 1645/1666/ 1730 |
| AS2 | 51 | | | | |

Beispiel A61

Herstellung von Verbindungen der allgemeinen Formel:

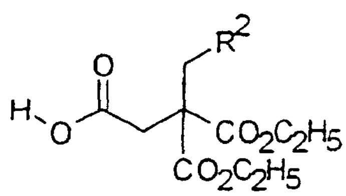

β,β-Bis-(ethoxycarbonyl)-1-methyl-1H-indol-3-butansäure

Hergestellt analog Beispiel A1b) aus β,β-Bis-(ethoxycarbonyl)-1-methyl-1H-indol-3-butansäure-tert.-butylester durch Einwirkung von Trifluoressigsäure in Dichlormethan in einer Ausbeute von 63.5 % der Theorie. Farblose Kristalle vom Fp. 127 - 130 °C (Diisopropylether).
IR(KBr): 1738, 1712 $cm^{-1}$ (C=O)

Analog wurden hergestellt:

| $R^2$ | % Ausbeute | MS | $R_f$ | Fließmittel | IR [$cm^{-1}$] |
|---|---|---|---|---|---|
| AS29 | 100 | | | | |
| AS16 | 100 | | 0.7 | EE / MeoH / AcOH 97.5/2.2/0.25 | |
| AS5 | 100 | | 0.5 | PE/EE 2/1 | |

(fortgesetzt)

| $R^2$ | % Ausbeute | MS | $R_f$ | Fließmittel | IR [$cm^{-1}$] |
|---|---|---|---|---|---|
| AS32 | 100 | | 0.58 | PE/EE 2/1 | (KBr): C=O 1759/1711 |
| AS33 | 100 | | | | (KBr): C=O 1736 |
| AS17 | 52 | | | | (KBr): C=O 1707/1726 / 1755 |
| AS34 | 90 | | 0.8 | EE / MeOH / AcOH 97.5/2.5/0.25 | (KBr): C=O 1705/1743 |
| AS19 | 100 | | 0.76 | PE / EE / AcOH 6/3/1 | (KBr): C=O 1738 |
| AS46 | 92 | | 0.35 | FM1 | (KBr): C=O 1732 |
| AS50 | 71 | | | | (KBr): C=O 1712/1734 /1759 |
| AS2 | 31 | EI: $M^+$ = 272 | 0.42 | PE / EE / AcOH 6/4/0.2 | (KBr): C=O 1711/1734 |

Beispiel A62

Herstellung von Verbindungen der allgemeinen Formel:

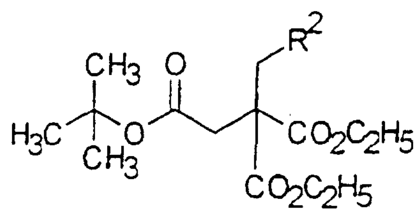

β,β-Bis-(ethoxycarbonyl)-3,5-dimethylbenzenbutansäure-tert.-butylester

Zur Lösung von 13.8 g (50.2 mMol) [(1,1-Dimethylethoxycarbonyl)methyl]-malonsäurediethylester in 400 ml wasserfreiem Tetrahydrofuran gab man unter äußerer Kühlung mit Eiswasser 2.3 g (52.7 mMol) Natriumhydrid. Nach halbstündigem Rühren tropfte man unter Einhaltung einer Reaktionstemperatur von 0 bis +5 °C die Lösung von 10.0 g (50.2 mMol) 3,5-Dimethylbenzylbromid in 80 ml Tetrahydrofuran zu und ließ den Ansatz danach innerhalb von 14 Stunden auf Zimmertemperatur erwärmen. Das Reaktionsgemisch wurde im Vakuum vom Lösemittel befreit, der Rückstand mit 200 ml 10proz. Zitronensäure versetzt und mit Essigsäureethylester erschöpfend extrahiert. Die vereinigten Extrakte ergaben nach üblicher Aufarbeitung 19.7 g (100 % der Theorie) eines farblosen Öls vom $R_f$ = 0.67 (Fließmittel: Dichlormethan), das ohne Reinigung in der folgenden Stufe eingesetzt wurde.

Analog wurden hergestellt:

| $R^2$ | Anmerkungen | % Ausbeute | MS | $R_f$ | Fließmittel | IR [$cm^{-1}$] |
|---|---|---|---|---|---|---|
| AS29 | | 100 | | | | |

(fortgesetzt)

| $R^2$ | Anmerkungen | % Ausbeute | MS | $R_f$ | Fließmittel | IR [$cm^{-1}$] |
|---|---|---|---|---|---|---|
| AS16 | | 62 | | 0.6 | $CH_2Cl_2$ | |
| AS5 | | 91 | ESI: $(M+H)^+$ = 521/3/5 ($Br_2$) | 0.8 | PE/EE 2/1 | (KBr): C=O 1734 |
| AS32 | | 96 | | 0.76 | PE/EE 2/1 | (KBr): C=O 1734 |
| AS33 | | 78 | | 0.55 | $CH_2Cl_2$ | (KBr): C=O 1736 |
| AS31 | Unter Verwendung von 3-(Dimethylaminomethyl)-1-methyl-1H-indolmethoiodid | 74 | EI: $M^+$ = 417 | 0.7 | Totuol / t-BME 4/1 | (KBr): C=O 1734 |
| AS17 | | 70 | EI: $M^+$ = 550/52/54 ($Br_2$) | 0.5 | $CH_2Cl_2$ | (KBr): C=O 1734 |
| AS34 | | 93 | EI: $M^+$ = 450 | 0.5 | $CH_2Cl_2$/PE 1/1 | (KBr): C=O 1736 |
| AS19 | | 87 | | 0.89 | $CH_2Cl_2$ | (KBr): C=O 1736 |
| AS46 | | 54 | EI: $M^+$ = 415 | 0.7 | FM4 | |
| AS50 | | 60 | EI: $M^+$ = 520/22/24 ($Br_2$) | 0.7 | $CH_2Cl_2$ | (KBr): C=O 1734 |

Beispiel A63

β,β-Bis-(ethoxycarbonyl)-4-(1,1-dimethylethyl)-benzenbutansäure-(phenylmethyl)-ester

Hergestellt analog Beispiel A62 aus [(Phenylmethoxycarbonyl)-methyl]-malonsäurediethylester und 4-(1,1-Dimethylethyl)-benzylbromid in Gegenwart von Natriumhydrid in einer Ausbeute von 53 % der Theorie.
Farbloses Öl vom $R_f$ = 0.21 (Fließmittel: Dichlormethan/Petrolether 2/1 v/v).
IR(KBr): 1738 $cm^{-1}$ (C=O)

Beispiel A64

4-[4-(3,4-Dihydro-2(1H)-oxochinazolin-3-yl)-1-piperidinyl]-2-[[3-(1-methylethoxy)phenyl]methyl]-4-oxobutansäuremethylester

Zu der Lösung von 2.0 g (4.43 mMol) 4-[4-(3,4-Dihydro-2(1H)-oxochinazolin-3-yl)-1-piperidinyl]-2-[(3-hydroxyphenyl)methyl]-4-oxobutansäuremethylester in 30 ml wasserfreiem Dimethylformamid gab man 0.2 g (4.4 mMol) einer 55proz. Suspension von Natriumhydrid in Paraffinöl. Nach 30minütigem Rühren bei Zimmertemperatur tropfte man 0.5 ml (4.8 mMol) Isopropyliodid zu und hielt je zwei Stunden bei Zimmertemperatur und bei 70 °C. Der nach Entfernen der flüchtigen Anteile verbleibende Rückstand wurde zwischen Wasser und Essigsäureethylester verteilt. Die organische Phase wurde mit gesättigter Natriumchlorid-Lösung gewaschen, über Magnesiumsulfat getrocknet und erneut eingedampft. Das rohe Produkt wurde an Kieselgel (60 µm) unter Verwendung von anfangs Dichlormethan, später Methanol/konz. Ammoniak (9/1 v/v) zum Eluieren säulenchromatographisch gereinigt. Man erhielt 0.9 g (42 % der Theorie) einer farblosen, amorphen Substanz vom $R_f$ = 0.32 (FM4).
IR(KBr): 1734, 1668 $cm^{-1}$ (C=O)
MS: $M^+$ = 493

Entsprechend erhielt man aus 4-[4-(3,4-Dihydro-2(1H)-oxochinazolin-3-yl)-1-piperidinyl]-2-[(3-hydroxyphenyl)methyl]-4-oxobutansäuremethylester und Ethyliodid den 4-[4-(3,4-Dihydro-2(1H)-oxochinazolin-3-yl)-1-piperidinyl]-2-[(3-ethoxyphenyl)-methyl]-4-oxobutansäuremethylester in einer Ausbeute von 67 % der Theorie. Farblose,

amorphe Substanz vom $R_f$ = 0.29 (FM4).
IR(KBr): 1734, 1666 $cm^{-1}$ (C=O)
MS: $M^+$ = 479

Beispiel A65

β,β-Bis-(ethoxycarbonyl)-4-(1,1-dimethylethyl)-benzenbutansäure

Hergestellt analog Beispiel A58b) aus β,β-Bis-(ethoxycarbonyl)-4-(1,1-dimethylethyl)-benzenbutansäurephenylmethylester durch katalytische Hydrierung in Gegenwart von Palladiumkohle in einer Ausbeute von 95 % der Theorie. Farbloses, hochviskoses Öl vom $R_f$ = 0.16 (Fließmittel: Dichlormethan).
IR(KBr): 1739 $cm^{-1}$ (C=O)

Beispiel A66

1-Methyl-4-[(1-piperazinyl)carbonyl]-piperazin-bis-(trifluoracetat)

a) 4-[[4-(1,1-Dimethylethoxycarbonyl)-1-piperazinyl]carbonyl]-1-methylpiperazin

Zu der Lösung von 1.1 g Triphosgen (3.7 mMol) in 20 ml Dichlormethan tropfte man bei Zimmertemperatur innerhalb von 30 Minuten das Gemisch aus 1.2 g (10 mMol) 1-Methylpiperazin, 0.38 ml (22 mMol) DIEA und 35 ml Dichlormethan und gab dann auf einmal die Lösung von 1.9 g (10 mMol) 1-(1,1-Dimethylethoxycarbonyl)-piperazin und von 0.38 ml DIEA in 20 ml Dichlormethan zu. Nach einstündigem Rühren bei Raumtemperatur wurde vom Unlöslichen abfiltriert und das Filtrat im Vakuum eingedampft. Nach der Reinigung des Rohprodukts an Kieselgel (Amicon, 35 - 70 μm) unter Verwendung von Dichlormethan/Methanol/konz. Ammoniak (80/20/1 v/v/v) zum Eluieren erhielt man 700 mg (22 % der Theorie) an farblosen Kristallen vom Fp. 130 °C.
IR(KBr): 1691, 1641 $cm^{-1}$ (C=O)

b) 1-Methyl-4-[(1-piperazinyl)carbonyl]-piperazin-bis-(trifluoracetat)

Hergestellt analog Beispiel A1b), jedoch unter Verzicht auf die Behandlung mit wässerigem Ammoniak, aus 4-[[4-(1,1-Dimethylethoxycarbonyl)-1-piperazinyl]carbonyl]-1-methylpiperazin und Trifluoressigsäure in einer Ausbeute von 99.6 % der Theorie. Farblose, amorphe Substanz vom $R_f$ = 0.17 (Fließmittel: Dichlormethan/Methanol/konz. Ammoniak 50/50/0.5).
IR(KBr): 1678 $cm^{-1}$ (C=O)
MS: $M^+$ = 212

Beispiel A67

1-[4-[4-(Dimethylamino)butyl]phenyl]-piperazin-dihydrochlorid

a) N,N-Dimethyl-4-fluor-γ-oxobenzenbutansäureamid

Zu der Lösung von 30.5 g (0.155 Mol) 4-Fluor-γ-oxobenzenbutansäure in 470 ml Tetrahydrofuran gab man unter Rühren und bei Zimmertemperatur 35.0 g (0.216 Mol) N,N'-Carbonyldiimidazol und hielt weitere 2.5 Stunden bei Raumtemperatur. Unter kräftiger äußerer Kühlung mit Eis-Ethanol-Gemisch wurden danach 13.7 g (0.304 Mol) Dimethylamin eingeleitet. Nach 12stündigem Stehenlassen bei Zimmertemperatur wurde das Lösemittel im Vakuum entfernt, der Rückstand zwischen Dichlormethan und 10proz. wässeriger Zitronensäure-Lösung verteilt, die organische Phase über Natriumsulfat getrocknet und abermals im Vakuum eingedampft. Das rohe Produkt ergab nach säulenchromatographischer Reinigung (Fließmittel: Essigsäureethylester) an Kieselgel 30.22 g (87 % der Theorie) an farblosen Kristallen vom $R_f$ = 0.31 (Fließmittel: Essigsäureethylester/Eisessig 99.99/0.01).
IR(KBr): 1680, 1647 $cm^{-1}$ (C=O)

b) N,N-Dimethyl-γ-oxo-4-[4-(phenylmethyl)-1-piperazinyl]-benzenbutansäureamid

Das Gemisch aus 33.48 g (0.15 Mol) N,N-Dimethyl-4-fluor-γ-oxobenzenbutansäureamid, 29.6 g (0.168 Mol) 1-(Phenylmethyl)piperazin und 6 ml DIEA wurde 6 Stunden unter Rückfluß gekocht. Man gab nochmals 30 g (0.17 Mol) (Phenylmethyl)piperazin zu und erhitzte weitere 7 Stunden auf Rückflußtemperatur. Die Mischung wurde in wenig

Dichlormethan aufgenommen und unter Verwendung von Dichlormethan/Methanol/konz. Ammoniak 99/1/0.5 zum Eluieren säulenchromatographisch an Kieselgel gereinigt. Der aus den geeigneten Fraktionen erhaltene Rückstand wurde mit Diisopropylether verrührt, die entstandenen Kristalle anschließend aus Ethanol umkristallisiert. Man erhielt 42.22 g (74 % der Theorie) an farblosen Kristallen vom $R_f$ = 0.69 (Fließmittel: Dichlormethan/Methanol/konz. Ammoniak 95/5/0.5 v/v/v).
IR(KBr): 1662, 1643 $cm^{-1}$ (C=O)

c) 4-[4-[4-(Dimethylamino)-1-hydroxybutyl]phenyl]-1-(phenylmethyl)piperazin

Hergestellt analog Beispiel A51 aus N,N-Dimethyl-γ-oxo-4-[4-(phenylmethyl)-1-piperazinyl]-benzenbutansäureamid durch Reduktion mit Lithiumaluminiumhydrid in einer Ausbeute von 61 % der Theorie. Farblose, amorphe Substanz vom $R_f$ = 0.62 (Fließmittel: Essigsäureethylester/Methanol 1/1 v/v).
MS: $M^+$ = 367

d) 1-[4-[4-(Dimethylamino)butyl]phenyl]-piperazin-dihydrochlorid

Hergestellt analog Beispiel A20b) aus 4-[4-[4-(Dimethylamino)-1-hydroxybutyl]phenyl]-1-(phenylmethyl)piperazin durch katalytische Hydrierung in Gegenwart von Palladiumkohle und Salzsäure in quantitativer Ausbeute. Farblose, amorphe Substanz vom $R_f$ = 0.37 (Fließmittel: Essigsäureethylester/Methanol 50/50/0.5 v/v/v).

**B. Herstellung der Endverbindungen**

Referenzbeispiel 1

Herstellung von Verbindungen der allgemeinen Formel:

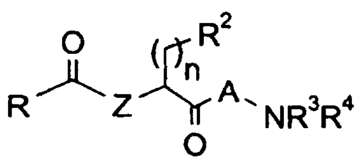

1-[N-[4-(1,3-Dihydro-2(2H)-oxobenzimidazol-1-yl)-1-piperidinyl]carbonyl]-3,5-dibrom-D-tyrosyl]-4-(4-pyridinyl)-piperazin (Lfd. Nr. 83)

Eine Mischung aus 2 g (3.44 mmol) 3.5-Dibrom-$N^2$-[4-(1,3-Dihydro-2(2H)-oxobenzimidazol-1-yl)-1-piperidinyl]carbonyl]-D-tyrosin, 0.59 g (3.6 mmol) 1-(4-Pyridinyl)-piperazin, 1.27 g (3.96 mmol) TBTU, 0.47 g (3.44 mmol) HOBt, 0.7 ml (3.96 mmol) DIEA und 100 ml Tetrahydrofuran wurde bei Raumtemperatur über Nacht gerührt. Das Reaktionsgemisch wurde einmal mit gesättigter wäßriger Kochsalz-Lösung, zweimal mit gesättigter wäßriger Natriumhydrogencarbonat-Lösung und wiederum mit gesättigter wäßriger Kochsalz-Lösung ausgeschüttelt. Die organische Phase wurde getrocknet, im Vakuum eingeengt und das Rohprodukt anschließend säulenchromatographisch (MN-Kieselgel 60, Macherey-Nagel, 70-230 mesh ASTM, Fließmittel: Essigester/Methanol = 9/1/ (v/v/)) gereinigt. Man erhielt 550 mg (22 % der Theorie) eines amorphen Festproduktes.
IR (KBr) : 1601, 1636, 1696 $cm^{-1}$ (C=O)
$R_f$: 0.67 (FM2)
ESI-MS: $(M+H)^+$ = 726/728/730 ($Br_2$)
Analog wurden hergestellt (jeweils n = 1):

| Lfd. Nr. | RCO | Z | $R^2$ | A | $NR^3R^4$ | Anmerkungen | % Ausbeute | MS | $R_f$ | Fließmittel | IR [$cm^{-1}$] |
|---|---|---|---|---|---|---|---|---|---|---|---|
| | N6 | N-H | AS1 | A3 | C4 | | 88 | ESI: $(M+H)^+$ = 928/30/32 ($Br_2$) | 0.8 | FM1 | (KBr): C=O 1629 8. 1708.8 |
| 193 | N15 | N-H | AS6 | A0 | C7 | DMF als LM | 26 | ESI: $(M+H)^+$ = 647/9 (Br) | 0.9 | EE/ MeOH/ AcOH 75/23/2 v/v/v | (KBr): C=O 1693.4, 1622.0 |
| 194 | N66 | N-H | AS1 | A0 | C67 | | 49 | ESI: $(M+H)^+$ = 828/30/32 ($Br_2$) | 0.33 | FM1 | (KBr): C=O 1622/1664 |
| 202 | N15 | N-H | AS1 | A0 | C36 | DMF als Losemittel, DIEA | 9 | ESI: $(M+H)^+$ = 733/5/7 ($Br_2$) | 0.49 | FM1 | (KBr): C=O 1695.3, 1622.0 NH 3417.7 |
| 203 | N15 | N-H | AS1 | A0 | C29 | DMF als Losemittel, DIEA | 41 | ESI: $(M+H)^+$ = 718/20/ 22 ($Br_2$) | 0.58 | EE/MeOH 9/1 v/v | (KBr): C=O 1695.3 |
| 204 | N15 | N-H | AS1 | A0 | C30 | DMF als Losemittel, DIEA | 27 | ESI: $(M+H)^+$ = 691/3/5 ($Br_2$) | 0.1 | FM1 | (KBr): C=O 1695.3; 1624.0 |
| 205 | N15 | N-H | AS6 | A0 | C8 | DMF als Losemittel, DIEA | 23 | ESI: $(M+H)^+$ = 653/5 (Br) | 0.46 | FM1 | (KBr): C=O 1695.3, 1622.0 |
| 206 | N15 | N-H | AS1 | A0 | C31 | DMF als Losemittel, DIEA | 33 | ESI: $(M+H)^+$ = 717/19/21 ($Br_2$) | 0.25 | FM1 | (KBr): C=O 1695.3, 1624.0 |
| 207 | N15 | N-H | AS1 | A0 | C32 | DMF als Losemittel, DIEA | 55 | ESI: $(M+H)^+$ = 780/2/4 ($Br_2$) | 0.46 | FM1 | (KBr): C=O 1690; 1650 |
| 212 | N15 | N-H | AS1 | A7 | C1 | DMF als Losemittel, DIEA | 37 | ESI: $(M+H)^+$ = 882/4/6 ($Br_2$) | 0.27 | FM1 | (KBr): C=O 1697.3, 1639.4 NH 3423.4 |
| 217 | N15 | N-H | AS6 | A3 | C1 | | 51 | | 0.9 | FM1 | (KBr): C=O 1693.4; 1641.3 |
| 222 | N15 | N-H | AS1 | A0 | C27 | THF/DMF 1/1 als LM $NEt_3$ als Base | 10 | ESI: $(M+H)^+$ = 774/6/8 ($Br_2$) | 0.35 | FM1 | (KBr): C=O 1695.3 |
| 286 | N15 | N-H | AS1 | A0 | C28 | THF/DMF 1/1 als LM $NEt_3$ als Base | 9 | ESI: $(M+H)^+$ = 706/8/10 ($Br_2$) | 0.4 | FM1 | (KBr): C=O 1699.2 |

(fortgesetzt)

| Lfd. Nr. | RCO | Z | $R^2$ | A | $NR^3R^4$ | Anmerkungen | % Ausbeute | MS | $R_f$ | Fließmittel | IR [$cm^{-1}$] |
|---|---|---|---|---|---|---|---|---|---|---|---|
| 81 | N15 | N-H | AS4 | A0 | C4 | | 64 | ESI: $(M+H)^+$ = 724/6/8 ($Br_2$); $(M+Na)^+$ = 746/48/50 ($Br_2$) | 0.75 | FM1 | (KBr): C=O 1618.2; 1703.0 |
| 82 | N15 | N-H | AS4 | A0 | C1 | | 53 | ESI: $(M+H)^+$ = 725/7/9 ($Br_2$) | 0.55 | FM3 | (KBr): C=O 1620.1, 1703.0 |
| 84 | N66 | N-H | AS21 | A0 | C68 | | 31 | ESI: $(M+H)^+$ = 683 | 0.52 | FM1 | (KBr): C=O 1608/ 1628/ 1666 |
| 85 | N15 | N-H | AS4 | A0 | C7 | | 42 | ESI: $(M+H)^+$ = 724/6/8 ($Br_2$), $(M+Na)^+$ = 746/48/50 ($Br_2$) | 0.8 | FM1 | (KBr): C=O 1618.2, 1697.3, -NH- $-NH_2$ 3379.1 |
| 90 | N15 | N-H | AS1 | A0 | C8 | | 40 | ESI: $(M+H)^+$ = 731/3/5 ($Br_2$) | 0.78 | FM2 | (KBr): C=O 1624.0, 1697.3 |
| | N2 | N-H | AS2 | A3 | C1 | DMF als Losemittel, DIEA | 73 | ESI: $(M+H)^+$ = 766 | 0.42 | FM1 | (KBr): C=O 1654.2, 1708.8 |
| 354 | N15 | N-H | AS1 | A0 | C4 | | 21 | ESI: $(M+H)^+$ =725/7/9 ($Br_2$), $(M+Na)^+$ = 747/49/51 ($Br_2$) | 0.76 | FM2 | (KBr): C=O 1622.0; 1695.3, -OH, NH- 3417.7 |
| 98 | N15 | N-H | AS1 | A0 | C9 | | 60 | ESI: $(M+H)^+$ = 580/2/4 ($Br_2$), $(M+H)^+$ = 578/80/82 ($Br_2$), $(M+Na)^+$= 602/4/6 ($Br_2$) | 0.41 | FM2 | (KBr): C=O 1624.0, 1685.7, -OH, -NH- 3421.5 |
| 102 | N15 | N-H | AS1 | A0 | C12 | | 43 | ESI: $(M+H)^+$ = 636/38/40 ($Br_2$), $(M+Na)^+$ = 658/60/62 ($Br_2$) | 0.76 | FM2 | (KBr): C=O 1622.0. 1695.3, |

(fortgesetzt)

| Lfd. Nr. | RCO | Z | $R^2$ | A | $NR^3R^4$ | Anmerkungen | % Ausbeute | MS | $R_f$ | Fließmittel | IR [$cm^{-1}$] |
|---|---|---|---|---|---|---|---|---|---|---|---|
| 99 | N15 | N-H | AS1 | A0 | C10 | | 54 | ESI: $(M+H)^+$ =663/5/7 ($Br_2$) | 0.61 | FM2 | (KBr): C=O 1622.9. 1700.9, -OH, NH- 3421.5 |
| 100 | N15 | N-H | AS1 | A0 | C11 | | 54 | ESI: $(M+H)^+$ = 746/48/50 ($Br_2$) | 0.5 | FM2 | (KBr): C=O 1624.0, 1695.3; -NH-, -OH 3423.4; |
| 101 | N15 | N-H | AS1 | A0 | C7 | | 62 | ESI: $(M+H)^+$ = 725/7/9 ($Br_2$), $(M+Na)^+$ = 747/49/51 ($Br_2$) | 0.82 | FM2 | (KBr): C=O 1622.0, 1695.3, -OH, NH- 3253.7 |
| 103 | N15 | N-H | AS1 | A0 | C13 | | 37 | ESI: $(M+H)^+$ = 679/81/83 ($Br_2$) | 0.72 | FM2 | (KBr): C=O 1625.9. 1693.4, 1666.4, -OH, NH- 3409.9 |
| 106 | N15 | N-H | AS1 | A0 | C14 | | 72 | ESI: $(M+H)^+$ = 832/4/6 ($Br_2$), $(M+Na)^+$ = 854/6/8 ($Br_2$) | 0.66 | FM1 | (KBr): C=O 1674.1, 1689.5 |
| 104 | N15 | N-H | AS6 | A0 | C4 | | 36 | ESI: $(M+H)^+$ = 647/9 (Br): $(M+Na)^+$ = 669/71 (Br), $(M-H)^-$ 645/7 (Br) | 0.71 | FM1 | (KBr): C=O 1695.3 |
| 105 | N15 | N-H | AS6 | A0 | C1 | | 25 | ESI: $(M+H)^+$ = 648/50 (Br) | 0.25 | FM3 | (KBr): C=O 1695.3 |
| | N2 | N-H | AS1 | A12 | C1 | DMF als Losemittel, DIEA | 72 | ESI: $(M+H)^+$ = 1082/4/6 ($Br_2$) | 0.4 | FM1 | KBr : C=O 1641 |
| 199 | N15 | N-H | AS3 | A0 | C8 | THF/DMF = 9/1 (v/v) als Losemittel | 86 | ESI: $(M+H)^+$ = 643/5/7 ($Br_2$) | 0.37 | Essigester/ Methanol/ Petrolether = 1/2/1 (v/v/v) | KBr : C=O 1697, 1624 |

(fortgesetzt)

| Lfd. Nr. | RCO | Z | $R^2$ | A | $NR^3R^4$ | Anmerkungen | % Ausbeute | MS | $R_f$ | Fließmittel | IR [$cm^{-1}$] |
|---|---|---|---|---|---|---|---|---|---|---|---|
| 200 | N15 | N-H | AS3 | A0 | C1 | | 40 | ESI: $(M+H)^+$ = 638/40/42 ($Br_2$) | 0.45 | Essigester/ Methanol/ Petrolether = 1/2/1 (v/v/v) | KBr : C=O 1695, 1636 |
| 419 | N66 | N-H | AS21 | A0 | C38 | | 28 | ESI: $(M+H)^+$ = 682 | 0.1 | FM1 | (KBr): C=O 1628/ 1662 |
| 425 | N66 | N-H | AS1 | A0 | C36 | | 42 | ESI: $(M+H)^+$ = 747/49/51 ($Br_2$) | 0.4 | FM1 | (KBr): C=O 1624/1657 |
| 426 | N66 | N-H | AS4 | A0 | C30 | | 66 | ESI: $(M+H)^+$ = 704/6/8 ($Br_2$) | 0.45 | FM1 | (KBr): C=O 1618/1663 |
| 427 | N66 | N-H | AS1 | A0 | C31 | | 38 | ESI: $(M+H)^+$ = 731/3/5 ($Br_2$) | 0.2 | FM1 | (KBr): C=O 1630/1653 |
| 428 | N66 | N-H | AS4 | A0 | C36 | | 40 | ESI: $(M+H)^+$ = 746/48/50 ($Br_2$) | 0.6 | FM1 | (KBr): C=O 1618/1662 |
| 429 | N66 | N-H | AS1 | A0 | C30 | | 47 | ESI: $(M+H)^+$ = 705/7/9 ($Br_2$) | 0.15 | FM1 | (KBr): C=O 1635/1653 |
| 435 | N66 | N-H | AS4 | A0 | C31 | | 20 | ESI: $(M+H)^+$ = 730/2/4 ($Br_2$) | 0.55 | FM1 | (KBr): C=O 1608/1631 |
| 436 | N66 | N-H | AS1 | A0 | C11 | | 15 | ESI: $(M+H)^+$ = 760/2/4 ($Br_2$) | 0.1 | FM1 | (KBr): C=O 1624/1653 |
| 437 | N66 | N-H | AS4 | A0 | C11 | | 25 | ESI: $(M+H)^+$ = 759/61/63 ($Br_2$) | 0.5 | FM1 | (KBr): C=O 1622/1661 |
| 438 | N66 | N-H | AS4 | A0 | C54 | | 13 | ESI: $(M+H)^+$ = 744/6/8 (Br2) | 0.7 | FM1 | (KBr): C=O 1620/1660 |
| 439 | N66 | N-H | AS1 | A0 | C54 | | 31 | ESI: $(M+H)^+$ = 745/7/9 ($Br_2$) | 0.5 | FM1 | (KBr): C=O 1626/1661 |
| 439 | N66 | N-H | AS1 | A0 | C54 | | 31 | ESI: $(M+H)^+$ = 745/7/9 ($Br_2$) | 0.5 | FM1 | (KBr): C=O 1626/1661 |

(fortgesetzt)

| Lfd. Nr. | RCO | Z | $R^2$ | A | $NR^3R^4$ | Anmerkungen | % Ausbeute | MS | $R_f$ | Fließmittel | IR [$cm^{-1}$] |
|---|---|---|---|---|---|---|---|---|---|---|---|
| 443 | N122 | N-H | AS1 | A0 | C11 | | 44 | ESI: $(M+H)^+$ = 790/2/4 ($Br_2$) | 0.1 | FM1 | (KBr): C=O 1624/1680 |
| 444 | N122 | N-H | AS1 | A0 | C8 | | 62 | ESI: $(M+H)^+$ = 775/7/9 ($Br_2$) | 018 | FM1 | (KBr): C=O 11624/1678 |
| 445 | N122 | N-H | AS1 | A0 | C1 | | 60 | ESI: $(M+H)^+$ = 770/2/4 ($Br_2$) | 0.2 | FM1 | (KBr): C=O 1630/1680 |
| 446 | N122 | N-H | AS1 | A0 | C20 | | 59 | ESI: $(M+H)^+$ = 789/91/93 ($Br_2$) | 0.15 | FM1 | (KBr): C=O 1622/1680 |
| 447 | N122 | N-H | AS4 | A0 | C1 | | 54 | ESI: $(M+H)^+$ = 769/71/73 ($Br_2$) | 0.6 | FM1 | (KBr): C=O 1622/1682 |
| 448 | N122 | N-H | AS4 | A0 | C20 | | 68 | ESI: $(M+H)^+$ = 788/90/92 ($Br_2$) | 0.5 | FM1 | (KBr): C=O 1620/1682 |
| 449 | N122 | N-H | AS4 | A0 | C8 | | 59 | ESI: (M+H) = 774/6/8 ($Br_2$) | 0.58 | FM1 | (KBr): C=O 1620/1682 |
| 450 | N66 | N-$CH_3$ | AS1 | A0 | C4 | | 36 | ESI: $(M+H)^+$ = 753/5/7 ($Br_2$) | 0.39 | FM1 | (KBr): C=O 1653 |
| 451 | N66 | $CH_2$ | AS1 | A0 | C1 | | 20 | ESI: $(M+H)^+$ = 739/41/43 ($Br_2$) | 0.3 | FM1 | (KBr): C=O 1638 |
| 452 | N71 | $CH_2$ | AS1 | A0 | C1 | | 16 | ESI: $(M+H)^+$ = 751/53/55 ($Br_2$) | 0.4 | FM1 | (KBr): C=O 1638/1680 |
| 453 | N66 | $CH_2$ | AS1 | A0 | C11 | | 17 | ESI: $(M+H)^+$ = 758/60/62 ($Br_2$) | 0.13 | FM1 | (KBr): C=O 1636 |
| 454 | N66 | $CH_2$ | AS1 | A0 | C20 | | 33 | ESI: $(M+H)^+$ = 757/59/61 ($Br_2$) | 0.23 | FM1 | (KBr): C=O 1632 |
| 455 | N71 | $CH_2$ | AS1 | A0 | C8 | | 35 | ESI: $M^+$ = 755/7/9 ($Br_2$) | 0.42 | FM1 | (KBr): C=O 1624/1684 |
| 457 | N71 | $CH_2$ | AS1 | A0 | C4 | | 49 | ESI: $(M+H)^+$ 750/2/4 ($Br_2$) | 0.77 | FM1 | (KBr): C=O 1626/1682 |

(fortgesetzt)

| Lfd. Nr. | RCO | Z | $R^2$ | A | $NR^3R^4$ | Anmerkungen | % Ausbeute | MS | $R_f$ | Fließmittel | IR [$cm^{-1}$] |
|---|---|---|---|---|---|---|---|---|---|---|---|
| 458 | N71 | $CH_2$ | AS1 | A0 | C37 | | 25 | ESI: $(M+H)^+$ = 769/71/73 ($Br_2$) | 0.2 | FM1 | (KBr): C=O 1638/1682 |
| 459 | N66 | $CH_2$ | AS1 | A0 | C37 | | 50 | EI. $M^+$ = 757/59/61 ($Br_2$) | 0.2 | FM1 | (KBr): C=O 1636 |
| 460 | N66 | N-H | AS1 | A0 | C55 | | 72 | ESI: $(M+H)^+$ = 759/61/63 ($Br_2$) | 0.27 | EE/ MeOH/ $NH_4OH$ = 8/1.5/0.1 v/v/v | (KBr): C=O 1626/1661 |
| 461 | N66 | N-H | AS1 | A0 | C56 | | 77 | ESI: $(M+H)^+$ = 731/3/5 ($Br_2$) | 0.77 | FM1 | (KBr): C=O 1626/1661 |
| 462 | N66 | N-H | AS17 | A0 | C8 | | 51 | ESI: $(M+H)^+$ = 759/61/63 ($Br_2$) | 0.44 | FM1 | (KBr): C=O 1628/1663 |
| 463 | N66 | N-H | AS18 | A0 | C1 | | 59 | ESI: $(M+H)^+$ = 704 | 0.7 | FM1 | (KBr): C=O 1661 |
| 464 | N66 | N-H | AS18 | A0 | C8 | | 51 | ESI: $(M+H)^+$ = 709 | 0.76 | FM1 | (KBr): C=O 1628/1663 |
| 465 | N66 | N-H | AS18 | A0 | C37 | | 73 | ESI: $(M+H)^+$ = 723 | 0.7 | FM1 | (KBr): C=O 1628/1663 |
| 469 | N66 | N-H | AS19 | A0 | C8 | | 34 | ESI: $(M+H)^+$ = 651/53 (Br) | 0.34 | FM1 | (KBr): C=O 1626/1664 |
| 471 | N66 | N-H | AS20 | A0 | C8 | | 41 | ESI: $(M+H)^+$ = 649 | 0.68 | FM1 | (KBr): C=O 1624/1684 |
| 472 | N66 | N-H | AS5 | A0 | C8 | | 26 | ESI: $(M+H)^+$ = 729/31/33 ($Br_2$) | 0.73 | FM1 | (KBr): C=O 1626/1664 |
| 475 | N66 | N-H | AS18 | A0 | C20 | | 58 | ESI: $(M+H)^+$ = 723 | 0.22 | FM1 | (KBr): C=O 1628/1664 |
| 476 | N66 | N-H | AS18 | A0 | C11 | | 44 | ESI: $(M+H)^+$ = 724 | 0.27 | MeOH | (KBr): C=O 1630/1662 |
| 478 | N66 | N-H | AS19 | A0 | C37 | | 62 | ESI: $(M+H)^+$ = 665/7 (Br) | 0.8 | FM1 | (KBr): C=O 1626/1662 |

(fortgesetzt)

| Lfd. Nr. | RCO | Z | $R^2$ | A | $NR^3R^4$ | Anmerkungen | % Ausbeute | MS | $R_f$ | Fließmittel | IR [$cm^{-1}$] |
|---|---|---|---|---|---|---|---|---|---|---|---|
| 479 | N66 | N-H | AS19 | A0 | C20 | | 55 | ESI: $(M+H)^+$ = 665/7 (Br) | 0.64 | FM1 | (KBr): C=O 1664 |
| 480 | N66 | N-H | AS19 | A0 | C4 | | 55 | ESI: $(M+H)^+$ = 645/7 (Br) | 0.77 | FM1 | (KBr): C=O 1628/1662 |
| 506 | N66 | N-H | AS21 | A0 | C20 | | 46 | ESI: $(M+H)^+$ = 655 | 0.75 | FM1 | (KBr): C=O 1626/1664 |
| 507 | N66 | N-H | AS22 | A0 | C8 | | 65 | ESI: $(M+H)^+$ = 607/9 (Cl) | 0.78 | FM1 | (KBr): C=O 1624/1664 |
| 508 | N56 | $CH_2$ | AS21 | A0 | C20 | | 15 | ESI: $(M+H)^+$ = 654 | 0.15 | MeOH | (KBr): C=O 1639/1670 |
| 246 | N15 | $CH_2$ | AS1 | A0 | C8 | | 19 | ESI: $(M+H)^+$ = 730/2/4 ($Br_2$) | 0.35 | EE/MeOH/ $NH_4OH$ 9/1/0 5 v/v/v | (KBr): C=O 1635/1707 |
| 285 | N15 | CH2 | AS1 | A0 | C4 | | 42 | ESI: $(M+H)^+$ = 724/6/8 ($Br_2$) | 0.45 | EE/ MeOH/ $NH_4OH$ 9/1/0.5 v/v/v | (KBr): C=O 1684/1711 |
| 289 | N66 | CH2 | AS1 | A0 | C8 | | 40 | ESI: $(M+H)^+$ = 744/6/8 ($Br_2$) | 0.38 | EE/ MeOH/ $NH_4OH$ 9/1/0.5 v/v/v | (KBr): C=O 1635/1668 |
| 290 | N66 | CH2 | AS1 | A0 | C4 | | 30 | ESI: $(M+H)^+$ = 738/40/42 ($Br_2$) | 0.45 | EE/ MeOH/ $NH_4OH$ 9/1/0.5 v/v/v | (KBr): C=O 1634/1664 |
| 511 | N66 | N-H | AS23 | A0 | C8 | DMF | 80 | ESI: $(M+H)^+$ = 603 | 0.57 | FM1 | (KBr): C=O 1664/1626 |
| 512 | N66 | N-H | AS23 | A0 | C11 | DMF | 60 | ESI: $(M+H)^+$ = 618 | 0.30 | FM1 | (KBr): C=O 1645 |
| 513 | N66 | N-H | AS23 | A0 | C1 | DMF | 54 | ESI: $(M+H)^+$ = 598 | 0.50 | FM1 | (KBr): C=O 1662/1712 |

(fortgesetzt)

| Lfd. Nr. | RCO | Z | $R^2$ | A | $NR^3R^4$ | Anmerkungen | % Ausbeute | MS | $R_f$ | Fließmittel | IR [$cm^{-1}$] |
|---|---|---|---|---|---|---|---|---|---|---|---|
| 514 | N66 | N-H | AS23 | A0 | C38 | DMF | 65 | ESI: $(M+H)^+$ = 644 = 644 | 0.20 | FM1 | (KBr): C=O 1664 / 1626 / 1712 |
| 515 | N66 | N-H | AS23 | A0 | C40 | DMF | 7 | ESI: $(M+H)^+$ = 632 | 0.40 | FM1 | (KBr): C=O 1630/1662 |
| 527 | N66 | N-H | AS25 | A0 | C8 | | 49 | ESI: $(M+H)^+$ = 594 | 0.48 | FM1 | (KBr): C=O 1647 |
| 528 | N66 | N-H | AS25 | A0 | C1 | | 29 | ESI: $(M+H)^+$ = 589 | 0.48 | FM1 | (KBr): C=O 1646 |
| 529 | N66 | $CH_2$ | AS2 | A0 | C8 | | 27 | ESI: $(M+H)^+$ = 622 | 0.50 | FM1 | (KBr): C=O 1635 / 1668 / 1716 |
| 530 | N66 | $CH_2$ | AS2 | A0 | C20 | | 5 | EI: $M^+$ = 635 | 0.49 | FM1 | (KBr): C=O 1637 / 1668 / 1714 |
| 531 | N66 | $CH_2$ | AS23 | A0 | C8 | | 30 | EI: $M^+$ = 601 | 0.50 | FM1 | |
| | N66 | $CH_2$ | AS23 | | | | 95 | | | | |
| 538 | N139 | $CH_2$ | AS2 | A0 | C20 | | 49 | EI: $M^+$ = 636 | 0.30 | FM1 | (KBr): C=O 1635/1674 |
| 539 | N139 | $CH_2$ | AS2 | A0 | C53 | | 52 | EI: $M^+$ = 637 | 0.30 | FM1 | (KBr): C=O 1637/1674 |
| 540 | N139 | $CH_2$ | AS2 | A0 | C8 | | 60 | | 0.37 | FM1 | (KBr): C=O 1635/1674 |
| 541 | N66 | $CH_2$ | AS27 | A0 | C53 | | 32 | EI: $M^+$ = 630 | 0.65 | FM1 | (KBr): C=O 1639/1670 |
| 542 | N66 | $CH_2$ | AS27 | A0 | C8 | | 32 | EI: $M^+$ = 615 | 0.80 | FM1 | (KBr): C=O 1639/1670 |
| 543 | N66 | $CH_2$ | AS27 | A0 | C20 | | 21 | EI: $M^+$ = 629 | 0.59 | FM1 | (KBr): C=O 1639/1672 |
| 544 | N66 | $CH_2$ | AS28 | A0 | C20 | | 35 | EI: $M^+$ = 643 | 0.50 | FM1 | (KBr): C=O 1641/1670 |

(fortgesetzt)

| Lfd. Nr. | RCO | Z | $R^2$ | A | $NR^3R^4$ | Anmerkungen | % Ausbeute | MS | $R_f$ | Fließmittel | IR [$cm^{-1}$] |
|---|---|---|---|---|---|---|---|---|---|---|---|
| 545 | N66 | $CH_2$ | AS28 | A0 | C53 | | 54 | EI: $M^+$ = 644 | 0.50 | FM1 | (KBr): C=O 1639/1670 |
| 546 | N66 | $CH_2$ | AS28 | A0 | C8 | | 53 | EI: $M^+$ = 629 | 0.60 | FM1 | (KBr): C=O 1639/1672 |
| 547 | N66 | $CH_2$ | AS29 | A0 | C8 | | 14 | EI: $M^+$ = 599 | 0.53 | FM1 | (KBr): C=O 1630 |
| 548 | N66 | $CH_2$ | AS29 | A0 | C53 | | 12 | EI: $M^+$ = 614 | 0.48 | FM1 | |
| 549 | N66 | $CH_2$ | AS29 | A0 | C20 | | 15 | EI: $M^+$ = 613 | 0.48 | FM1 | (KBr): C=O 1637/1668 |
| 550 | N66 | $CH_2$ | AS30 | A0 | C53 | | 4 | | 0.48 | FM1 | |
| 574 | N66 | CH2 | AS16 | A0 | C20 | | 55 | EI: $M^+$ = 653/5/7 ($Cl_2$) | 0.80 | $CH_2Cl_2$/ MeOH/ NH4OH 80/20/1 | (KBr): C=O 1635/1670 |
| 575 | N66 | $CH_2$ | AS16 | A0 | C53 | | 54 | EI: $M^+$ = 654/6/8 ($Cl_2$) | 0.20 | EE/MeOH/ $NH_4OH$ 70/30/3 | (KBr): C=O 1635/1668 |
| 578 | N66 | $CH_2$ | AS5 | A0 | C53 | | 32 | EI: $M^+$ = 742/4/6 ($Br_2$) | 0.30 | FM5 | (KBr): C=O 1637/1670 |
| 579 | N66 | $CH_2$ | AS5 | A0 | C20 | | 37 | EI: $M^+$ = 741/3/5 ($Br_2$) | 0.50 | FM5 | (KBr): C=O 1635/1670 |
| 589 | N66 | $CH_2$ | AS32 | A0 | C53 | | 49 | EI: $M^+$ = 756/58/60 ($Br_2$) | 0.33 | FM5 | (KBr): C=O 1639/1670 |
| 590 | N66 | $CH_2$ | AS32 | A0 | C20 | | 36 | EI: $M^+$ = 755/7/9 ($Br_2$) | 0.47 | FM5 | (KBr): C=O 1658/1672 |
| 591 | N66 | $CH_2$ | AS33 | A0 | C20 | | 43 | EI: $M^+$ = 689 | 0.40 | EE/MeOH/ $NH_4OH$ 50/50/0.5 | (KBr): C=O 1637/1670 |
| 592 | N66 | $CH_2$ | AS33 | A0 | C53 | | 52 | EI: $M^+$ = 690 | 0.20 | EE/MeOH/ $NH_4OH$ 70/30/5 | (KBr): C=O 1633/1668 |
| 593 | N66 | $CH_2$ | AS16 | A0 | C29 | | 11 | EI: $M^+$ = 628/30/32 ($Cl_2$) | 0.65 | EE/MeOH 9/1 | (KBr): C=O 1606/ 1637 / 1668 / 1728 |

(fortgesetzt)

| Lfd. Nr. | RCO | Z | $R^2$ | A | $NR^3R^4$ | Anmerkungen | % Ausbeute | MS | $R_f$ | Fließmittel | IR [$cm^{-1}$] |
|---|---|---|---|---|---|---|---|---|---|---|---|
| 594 | N66 | $CH_2$ | AS16 | A0 | C73 | | 48 | EI: $M^+$ = 628/30/32 ($Cl_2$) | 0.50 | EE/MeOH 9/1 | (KBr): C=O 1637/ 1668 / 1736 |
| 595 | N66 | $CH_2$ | AS16 | A0 | C74 | | 10 | EI: $M^+$ = 597/99/601 ($Cl_2$) | 0.30 | EE/MeOH/ $NH_4OH$ 50/50/0.5 | |
| 597 | N66 | $CH_2$ | AS31 | A0 | C53 | | 25 | EI: $M^+$ = 639 | 0.30 | $CH_2Cl_2$/ MeOH / $NH_4OH$ 90/10/1 | (KBr): C=O 1635/1668 |
| 598 | N66 | $CH_2$ | AS31 | A0 | C20 | | 31 | EI: $M^+$ = 638 | 0.10 | $CH_2Cl_2$/ MeOH / $NH_4OH$ 90/10/0.3 | (KBr): C=O 1635/1668 |
| 600 | N73 | $CH_2$ | AS31 | A0 | C20 | | 10 | ESI: $(M+H)^+$ = 551 | 0.15 | $CH_2Cl_2$/ MeOH/ $NH_4OH$ 90/10/1 | (KBr): C=O 1628 |
| 602 | N66 | $CH_2$ | AS17 | A0 | C53 | | 56 | EI: $M^+$ = 772/4/6 ($Br_2$) | 0.25 | EE / MeOH / $NH_4OH$ 50/50/0.5 | (KBr): C=O 1637/1668 |
| 603 | N66 | $CH_2$ | AS16 | A0 | C33 | | 93 | EI: $M^+$ = 600/02/04 ($Br_2$) | 0.75 | EE / MeOH / AcOH 70/30/1 | (KBr): C=O 1635/1716 |
| 604 | N66 | $CH_2$ | AS17 | A0 | C20 | | 47 | EI: $M^+$ = 771/3/5 ($Br_2$) | 0.20 | EE / MeOH / $NH_4OH$ 50/50/0.5 | (KBr): C=O 1635/1668 |
| 605 | N66 | $CH_2$ | AS34 | A0 | C53 | | 70 | EI: $M^+$ = 672 | 0.25 | EE / MeOH / $NH_4OH$ 60/40/0.5 | (KBr): C=O 1633/1666 |
| 606 | N66 | $CH_2$ | AS34 | A0 | C20 | | 45 | EI. $M^+$ = 671 | 0.15 | EE / MeOH / $NH_4OH$ 50/50/0 5 | (KBr): C=O 1635/1668 |
| 607 | N66 | N-H | AS21 | A0 | C40 | | 27 | ESI: $(M+H)^+$ = 670 | 0.65 | FM1 | (KBr): C=O 1608 / 1628 / 1664 |
| 608 | N66 | N-H | AS21 | A0 | C11 | | 34 | ESI: $(M+H)^+$ = 656 | 0.50 | FM1 | (KBr): C=O 1606 / 1628 / 1664 |
| 609 | N66 | N-H | AS21 | A0 | C8 | | 30 | ESI: $(M+H)^+$ = 641 | 0 80 | FM1 | (KBr): C=O 1626/1664 |
| 610 | N66 | N-H | AS21 | A0 | C1 | | 55 | ESI: $(M+H)^+$ = 636 | 0.80 | FM1 | (KBr): C=O 1635/1662 |

(fortgesetzt)

| Lfd. Nr. | RCO | Z | $R^2$ | A | $NR^3R^4$ | Anmerkungen | % Ausbeute | MS | $R_f$ | Fließmittel | IR [$cm^{-1}$] |
|---|---|---|---|---|---|---|---|---|---|---|---|
| 611 | N66 | N-H | AS21 | A0 | C4 | | 80 | ESI: $(M+H)^+$ = 635 | 0.70 | FM1 | (KBr): C=O 1606 / 1628 / 1664 |
| 612 | N66 | $CH_2$ | AS4 | A0 | C8 | | 43 | EI. $M^+$ = 742/4/6 ($Br_2$) | 0.85 | FM1 | (KBr): C=O 1668 / 1631 / 1606 |
| 613 | N66 | N-H | AS22 | A0 | C20 | | 62 | ESI: $(M+H)^+$ = 621/23(Cl) | 0.73 | FM1 | (KBr): C=O 1626/1664 |
| 614 | N66 | N-H | AS22 | A0 | C37 | | 55 | ESI: $(M+H)^+$ = 621/23 (Cl) | 0.68 | FM1 | (KBr): C=O 1626/1664 |
| 615 | N66 | N-H | AS22 | A0 | C56 | | 77 | ESI: $(M+H)^+$ = 593 | 0.76 | FM1 | (KBr): C=O 1628/1664 |
| 616 | N66 | $CH_2$ | AS36 | A0 | C8 | | 32 | ESI: $(M+H)^+$ = 585 | 0.76 | FM1 | (KBr): C=O 1637/1668 |
| 617 | N66 | $CH_2$ | AS21 | A0 | C37 | | 15 | EI: $M^+$ = 653 | 0.15 | MeOH | (KBr): C=O 1639/1670 |
| 618 | N66 | $CH_2$ | AS21 | A0 | C38 | | 24 | EI: $M^+$ = 680 | 0.10 | MeOH | (KBr): C=O 1639/1670 |
| 628 | N66 | $CH_2$ | AS21 | A0 | C8 | | 31 | EI: $M^+$ = 639 | 0.25 | MeOH | (KBr): C=O 1639/1670 |
| 629 | N66 | $CH_2$ | AS21 | A0 | C11 | | 43 | EI: $M^+$ = 654 | 0.10 | MeOH | (KBr): C=O 1641/1668 |
| 630 | N66 | $CH_2$ | AS21 | A0 | C1 | | 74 | EI: $M^+$ = 634 | 0.10 | MeOH | (KBr): C=O 1641/1668 |
| 631 | N66 | $CH_2$ | AS21 | A0 | C28 | | 63 | EI: $M^+$ = 614 | 0.30 | MeOH | (KBr): C=O 1666 |
| 634 | N66 | $CH_2$ | AS38 | A0 | C8 | | 35 | ESI: $(M+H)^+$ = 622 | 0.25 | MeOH | (KBr): C=O 1635/1668 |
| 635 | N66 | $CH_2$ | AS48 | A0 | C8 | | 40 | EI: $M^+$ = 639 | 0.68 | FM1 | (KBr): C=O 1643/1670 |

(fortgesetzt)

| Lfd. Nr. | RCO | Z | $R^2$ | A | $NR^3R^4$ | Anmerkungen | % Ausbeute | MS | $R_f$ | Fließmittel | IR [$cm^{-1}$] |
|---|---|---|---|---|---|---|---|---|---|---|---|
| 636 | N66 | N-H | AS49 | A0 | C20 | | 25 | ESI: $(M+H)^+$ = 632 | 0.40 | EE / MeOH / $NH_4OH$ 9/1/0.5 | (KBr): C=O 1664 |
| 637 | N66 | $CH_2$ | AS4 | A0 | C20 | | 11 | ESI: $(M+H)^+$ = 757/59/61 ($Br_2$) | 0.60 | FM1 | (KBr): C=O 1635/1668 |
| 638 | N66 | $CH_2$ | AS48 | A0 | C20 | | 11 | ESI: $(M+H)^+$ = 654 | 0.66 | FM1 | (KBr): C=O 1641/1668 |
| 639 | N66 | $CH_2$ | AS18 | A0 | C20 | | 4 | EI: $M^+$ = 721 | 0.10 | MeOH | (KBr): C=O 1637/1670 |
| 640 | N66 | $CH_2$ | AS39 | A0 | C20 | | 38 | EI: $M^+$ = 645 | 0.80 | $CH_2Cl_2$/MeOH/ $NH_4OH$ 8/2/0.3 | (KBr): C=O 1635/1670 |
| 641 | N66 | $CH_2$ | AS38 | A0 | C20 | | 49 | EI: $M^+$ = 635 | 0.80 | $CH_2Cl_2$/ MeOH / $NH_4OH$ 8/2/0.3 | (KBr): C=O 1635/1668 |
| 642 | N66 | $CH_2$ | AS39 | A0 | C8 | | 45 | EI: $M^+$ = 631 | 0.10 | EE / MeOH / $NH_4OH$ 9/1/0.3 | (KBr): C=O 1635/1670 |
| | N66 | $CH_2$ | AS21 | A0 | C69 | | 70 | EI: $M^+$ = 739 | | | (KBr): C=O 1684 |
| 644 | N109 | $CH_2$ | AS21 | A0 | C20 | | 46 | ESI: $(M+H)^+$ = 659 | 0.10 | MeOH | (KBr): C=O 1643 |
| 645 | N66 | $CH_2$ | AS19 | A0 | C20 | | 21 | EI: $M^+$ = 763/5 (Br) | 0.53 | FM1 | (KBr): C=O 1669/1634 |
| 646 | N66 | $CH_2$ | AS19 | A0 | C8 | | 45 | EI: $M^+$ = 649/651 (Br) | 0.60 | FM1 | (KBr): C=O 1637/1668 |
| 653 | N113 | $CH_2$ | AS21 | A0 | C20 | | 55 | EI: $M^+$ = 666 | 0.60 | FM1 | (KBr): C=O 1630/1701 |
| 654 | N134 | $CH_2$ | AS21 | A0 | C20 | | 22 | EI: $M^+$ = 690 | 0.60 | FM1 | (KBr): C=O 1714 |
| 655 | N66 | $CH_2$ | AS46 | A0 | C20 | | 43 | EI: $M^+$ = 636 | 0.50 | FM1 | (KBr): C=O 1630/1664 |

(fortgesetzt)

| Lfd. Nr. | RCO | Z | $R^2$ | A | $NR^3R^4$ | Anmerkungen | % Ausbeute | MS | $R_f$ | Fließmittel | IR [$cm^{-1}$] |
|---|---|---|---|---|---|---|---|---|---|---|---|
| 656 | N66 | $CH_2$ | AS46 | A0 | C8 | | 71 | EI: $M^+$ = 622 | 0.60 | FM1 | (KBr): C=O 1635 |
| 657 | N66 | $CH_2$ | AS47 | A0 | C20 | | 63 | EI: $M^+$ = 639 | 0.50 | FM1 | (KBr): C=O 1635 / 1668 / 1716 |
| 658 | N66 | $CH_2$ | AS50 | A0 | C20 | | 70 | ESI: $(M+H)^+$ = 741/3/5 ($Br_2$) | 0.20 | EE/MeOH/ $NH_4OH$ 50/50/0.5 | (KBr): C=O 1635/1668 |
| 659 | N66 | $CH_2$ | AS50 | A0 | C53 | | 60 | ESI: $(M+H)^+$ = 743/5/7 ($Br_2$) | 0.20 | EE / MeOH $NH_4OH$ 50/50/0.5 | / (KBr): C=O 1635/1668 |
| 660 | N66 | $CH_2$ | A546 | A0 | C53 | | 41 | EI: $M^+$ = 637 | 0.65 | FM1 | (KBr): C=O 1630 |
| 661 | N66 | $CH_2$ | AS7 | A0 | C8 | | 75 | EI: $M^+$ = 615 | 0.70 | FM1 | (KBr): C=O 1626/1660 |
| 662 | N66 | $CH_2$ | AS7 | A0 | C53 | | 41 | EI: $M^+$ = 630 | 0.55 | FM1 | (KBr): C=O 1628/1662 |
| 663 | N66 | $CH_2$ | AS7 | A0 | C20 | | 78 | EI: $M^+$ = 629 | 0.60 | FM1 | (KBr): C=O 1628/1662 |
| 664 | N66 | $CH_2$ | AS52 | A0 | C8 | | 66 | EI: $M^+$ = 629 | 0.75 | FM1 | (KBr): C=O 1635 |
| 665 | N66 | $CH_2$ | AS52 | A0 | C53 | | 37 | EI: $M^+$ = 644 | 0.70 | FM1 | (KBr): C=O 1633/1664 |
| 666 | N66 | $CH_2$ | AS52 | A0 | C20 | | 61 | EI: $M^+$ = 643 | 0.80 | FM1 | (KBr): C=O 1635/1664 |
| 667 | N66 | $CH_2$ | AS2 | A0 | C53 | | 47 | EI. $M^+$ = 636 | 0.60 | FM1 | (KBr): C=O 1630/1664 |
| | N66 | $CH_2$ | AS2 | A0 | C69 | | 78 | | 0.75 | FM1 | |
| 669 | N66 | $CH_2$ | AS32 | A0 | C71 | | 44 | EI: $M^+$ = 834/6/8 ($Br_2$) | 0.20 | EE / MeOH / $NH_4OH$ 50/50/0.5 | (KBr): C=O 1641/1670 |

(fortgesetzt)

| Lfd. Nr. | RCO | Z | $R^2$ | A | $NR^3R^4$ | Anmerkungen | % Ausbeute | MS | $R_f$ | Fließmittel | IR [$cm^{-1}$] |
|---|---|---|---|---|---|---|---|---|---|---|---|
| 670 | N66 | $CH_2$ | AS51 | A0 | C20 | Vorstufen-Sonderfälle | 47 | EI: $M^+$=641 | 0.15 | EE / MeOH / $NH_4OH$ 50/50/0.5 | (KBr): C=O 1635/1664 |
| 671 | N66 | $CH_2$ | AS51 | A0 | C53 | | 45 | EI: $M^+$ =642 | 0.15 | EE / MeOH / $NH_4OH$ 50/50/0.5 | (KBr): C=O 1637/1670 |
| 672 | N66 | $CH_2$ | AS16 | A0 | C76 | | 55 | EI: $M^+$ = 689/91/93 ($Cl_2$) | 0.66 | FM1 | (KBr): C=O 1635 |

Referenzbeispiel 2

Herstellung von Verbindungen der allgemeinen Formel:

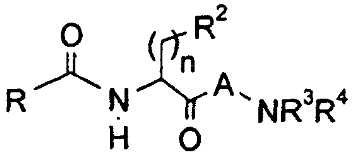

1-[$N^2$-[4-Amino-N-[[4-(2-chlorphenyl)-1-piperazinyl]carbonyl]-3,5-dibrom-D-phenylalanyl]-L-lysyl]-4-(4-pyridinyl)piperazin-bis-(trifluoracetat) (Lfd. Nr. 61)

Die Mischung aus 0.56 g (1.0 mmol) 4-Amino-$N^2$-[[4-(2-chlorphnyl)-1-piperazinyl]carbonyl]-3.5-dibrom-D-phenylalanin, 0.41 g (1.05 mmol) 1-[$N^6$-(1.1-Dimethylethoxycarbonyl)-L-lysyl]-4-(4-pyridinyl)piperazin, 0.35 g (1.10 mmol) TBTU, 0.14 g (1.0 mmol) HOBt, 0.2 ml (1.10 mmol) DIEA und 100 ml Dimethylformamid wurde bei Raumtemperatur über Nacht gerührt. Das Reaktionsgemisch wurde im Vakuum eingeengt und zwischen Methylenchlorid und gesättigter wäßriger Natriumhydrogencarbonat-Lösung verteilt. Die organische Phase wurde anschließend je einmal mit gesättigter wäßriger Natriumhydrogencarbonat-Lösung und mit Wasser ausgeschüttelt, getrocknet und im Vakuum eingeengt. Das Rohprodukt wurde säulenchromatographisch (MN-Kieselgel 60, Macherey-Nagel, 70-230 mesh ASTM, Fließmittel: Essigester/Methanol = 8/2 (v/v/)) gereinigt, in 30 ml Methylenchlorid aufgenommen und mit 3 ml Trifluoressigsäure 3 Stunden bei Raumtemperatur gerührt. Die Reaktionsmischung wurde im Vakuum eingedampft, der Rückstand mit Ether verrieben und der anfallende amorphe Feststoff (0.43 g, 37 % der Theorie) abgesaugt.
IR (KBr): 1643, 1678 $cm^{-1}$ (C=O)
$R_f$: 0.6 (FM1)
ESI-MS: $(M+H)^+$ = 832/834/836/838 ($Br_2$,Cl)
Analog wurden hergestellt (jeweils n = 1):

| Lfd. Nr. | RCO | $R^2$ | A | $NR^3R^4$ | %Ausbeute | MS | $R_f$ | Fließmittel | IR [$cm^{-1}$] |
|---|---|---|---|---|---|---|---|---|---|
| 48 | N6 | AS1 | A1 | C3 | 79 | ESI:M+H= 946/48/50 ($Br_2$) | 0.3 | FM1 | (KBr): C=O 1652.9; 1674.1 |
| 213 | N15 | AS6 | A1 | C8 | 14.7 | ESI: M+H= 781/3 (Br) | 0.45 | FM2 | (KBr): C=O 1691.5; 1629.8 |
| 49 | N8 | AS4 | A1 | C1 | 57.14 | ESI: M+H= 757/59/61 ($Br_2$) | 0.5 | FM1 | (KBr):C=O 1643.3; 1676.0 |
| 58 | N15 | AS4 | A1 | C4 | 21 | ESI: M+H= 852/4/6 ($Br_2$) | 0.57 | FM1 | (KBr): C=O 1635.5; 1695.3 |
| 59 | N15 | AS4 | A1 | C1 | 45.6 | ESI: M+H= 853/5/7 ($Br_2$) | 0.44 | FM1 | (KBr): C=O 1635.5; 1695.3 |
| 60 | N23 | AS4 | A1 | C4 | 19.2 | ESI: M+H= 831/3/5/7 ($Br_2$,Cl) | 0.65 | FM1 | (KBr): C=O 1633.6 |

(fortgesetzt)

| Lfd. Nr. | RCO | $R^2$ | A | $NR^3R^4$ | %Ausbeute | MS | $R_f$ | Fließmittel | IR [$cm^{-1}$] |
|---|---|---|---|---|---|---|---|---|---|
| 61 | N23 | AS4 | A1 | C1 | 36.6 | ESI: M+H= 832/4/6/8 ($Br_2$,Cl) | 0.6 | FM1 | (KBr): C=O 1643.3; 1678.0 |

Referenzbeispiel 3

Herstellung von Verbindungen der allgemeinen Formel:

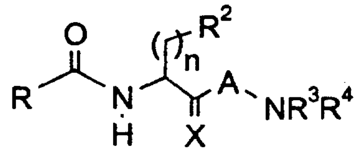

1-[$N^2$-[3,5-Dibrom-N-[[[(2-methoxyphenyl)methyl]amino]carbonyl]-D,L-tyrosyl]-$N^6$-(phenylmethoxycarbonyl)-L-lysyl]-4-(4-pyridi-nyl)-piperazin

Eine Tetrahydrofuran-Lösung (50 ml) von 1.0 g (1.34 mmol) 1-[$N^2$-(3,5-Dibrom-D-tyrosyl)-$N^6$-(phenylmethoxy-carbonyl)-Llysyl]-4-(4-pyridinyl)-piperazin wurde über einen Zeitraum von 40 Minuten zu einer auf -10 °C gekühlten und gerührten Suspension von 0.33 g (2.01 mmol) CDT in 50 ml Tetrahydrofuran tropfenweise zugegeben. Die Reaktionsmischung wurde anschließend 2 Stunden bei Raumtemperatur gerührt und mit 0.22 ml (1.675 mmol) 2-Methoxybenzenmethanamin versetzt. Anschließend wurde das Gemisch 2 Stunden unter Rückfluß gekocht und über Nacht bei Raumtemperatur gerührt. Das Reaktionsgemisch wurde im Vakuum eingeengt, der Rückstand mit Ether verrieben und der anfallende Feststoff (1.1 g; 90% der Theorie) abgenutscht und getrocknet.
IR (KBr): 1641, 1717 $cm^{-1}$ (C=O)
ESI-MS: $(M+H)^+$ = 908/910/912 ($Br_2$)
$(M+H+Na)^{++}$ = 466.7 ($Br_2$)

Analog wurden hergestellt:

| Lfd. Nr. | RCO | $R^2$ | n | X | A | $NR^3R^4$ | Anmerkungen | % Ausbeute | MS | $R_f$ | Fließmittel | IR [$cm^{-1}$] | F p. (°C) |
|---|---|---|---|---|---|---|---|---|---|---|---|---|---|
| 195 | N15 | AS1 | 1 | O | A3 | C8 | | 21 | ESI: $(M+H)^+$ = 959/61/63 ($Br_2$) | 0.8 | FM7 | (KBr): C=O 1699.2. 1635 5 | |
| 196 | N51 | AS1 | 1 | O | A3 | C8 | | 26:1 | ESI: $(M+H)^+$ = 929/31/33 ($Br_2$) | 0.85 | FM7 | (KBr): C=O 1710: CN 2219 8 | |
| 201 | N101 | AS4 | 1 | O | A0 | C8 | DIEA | 34 | ESI: $(M+H)^+$ = 746/8/50 ($Br_2$) | 0.58 | FM1 | (KBr): C=O 1693.4, 1618.2 | |
| 215 | N15 | AS1 | 1 | O | A0 | C34 | $NEt_3$ als Base | 92 | ESI: $(M+H)^+$ = 778/80/82 ($Br_2$) | 0.36 | FM1 | (KBr): C=O 1695.3 | |
| 216 | N15 | AS1 | 1 | O | A0 | C35 | $NEt_3$ als Base | 69 | ESI: (M H) = 779/81/ 82 ($Br_2$) | 0.3 | FM1 | (KBr): C=O 1701 1 | |
| 221 | N15 | AS4 | 1 | O | A7 | C1 | $NEt_3$ als Base | 39 | ESI: $(M+H)^+$ = 881/3/5 ($Br_2$) | 0.38 | FM1 | (KBr): C=O 1697 3. 1637.5 | |
| 288 | N85 | AS1 | 1 | O | A0 | C8 | THF/DMP als LM $NEt_3$ als Base | 30 | ESI: $(M+H)^+$ = 749/51/53 ($Br_2$) | 0.3 | MeOH | (KBr): C=O 1683 8: 1624 0: OH 3429.2 | |
| 293 | N66 | AS1 | 1 | O | A9 | C1 | DIEA | 11 | ESI: $(M+H)^+$ = 910/2/4 ($Br_2$) | 0.4 | FM1 | (KBr): C=O 1645.2 | |
| 295 | N66 | AS1 | 1 | O | A7 | C1 | $NEt_3$ als Base | 70 | ESI: $(M+H)^+$ = 896/8/ 900 ($Br_2$) | 0.45 | FM1 | (KBr): C=O 1716.5, 1647.1 | |
| 303 | N86 | AS4 | 1 | O | A0 | C8 | DIEA | 20 | ESI: $(M+H)^+$ = 747/9/51 ($Br_2$) | 0.7 | FM 2 | (KBr): C=O 1618.2 | |

(fortgesetzt)

| Lfd. Nr. | RCO | $R^2$ | n | X | A | $NR^3R^4$ | Anmerkungen | % Ausbeute | MS | $R_f$ | Fließmittel | IR [$cm^{-1}$] | F p. (°C) |
|---|---|---|---|---|---|---|---|---|---|---|---|---|---|
| 304 | N87 | AS4 | 1 | O | A0 | C8 | THF als Losemittel: $NEt_3$ als Base | 30 | ESI: $(M+H)^+$ = 802/4/6 ($Br_2$) | 0.75 | FM1 | (KBr): C=O 1720.4. 1668.3: 1620.1, NH,NH2 3431.2: 3379.1 | |
| 305 | N88 | AS4 | 1 | O | A0 | C8 | DIEA | 45 | ESI: $(M+H)^+$ = 782/4/6 ($Br_2$) | 0.6 | FM1 | (KBr): C=O 1616. SO2 13231.1, 1151.4 | |
| 308 | N90 | AS4 | 1 | O | A0 | C8 | DIEA | 27 | ESI: $(M+H)^+$ = 750/2/4 ($Br_2$) | 0.5 | FM1 | (KBr): C=O 1637.5 | |
| 80 | N15 | AS1 | 1 | O | A3 | C1 | | 62 | ESI: $(M+H)^+$ = 954/6/8 ($Br_2$) | 0.8 | FM2 | (KBr): C=O 1697.3: 1639.4 | |
| | N8 | AS1 | 1 | O | A3 | C1 | | 66 | ESI: $(M+H)^+$ = 858/60/62 ($Br_2$) | 0.22 | Essigester/ M ethanol = 6/4 (v/v) | (KBr): C=O 1641.3 | |
| | NS | AS1 | 1 | O | A3 | C1 | | 59 | ESI: $(M+H)^+$ = 888/90/92 ($Br_2$) | 0.22 | Essigester/ M ethanol = 6/4 (v/v) | (KBr): C=O 1652.9 | |
| | N2 | AS1 | 1 | O | A4 | C1 | | 40 | ESI: $(M+H)^+$ = 922/4/6 ($Br_2$); $(M+Na)^+$ = 944/6/8 ($Br_2$) | 0.45 | FM1 | (KBr): C=O 1641.3, 1710.8 OH,NH 3396.4 | |
| | N4 | AS1 | 1 | O | A4 | C1 | | 73 | ESI: $(M+H)^+$ = 952/4/6 ($Br_2$) | 0.13 | FM1 | (KBr): C=O 1641.3, 1714.6 | |

(fortgesetzt)

| Lfd. Nr. | RCO | $R^2$ | n | X | A | $NR^3R^4$ | Anmerkungen | % Ausbeute | MS | $R_f$ | Fließmittel | IR [$cm^{-1}$] | F p. (°C) |
|---|---|---|---|---|---|---|---|---|---|---|---|---|---|
| 62 | N15 | AS1 | 1 | O | A3 | C5 | saulenchromatographische Reinigung Kieselgel/ FM4 | 65 | ESI: $(M+H)^+$ = 1003/5/7 ($Br_2$), (M+Na) + = 1025/7/9 ($Br_2$) | 0.27 | FM1 | (KBR) C=O 1685.7. 1635.5: OH, NH 3419.6 | |
| | N15 | AS1 | 1 | O | A3 | C6 | saulenchromatographische Reinigung Kieselgel/ FM4 | 86 | ESI: $(M+H)^+$ = 983/5/7 ($Br_2$) | 0.45 | FM1 | (KBr): C=O 1695.3, 1633.6 | |
| 73 | N15 | AS5 | 1 | O | A3 | C1 | saulenchromatographische Reinigung Kieselgel/ FM4 Diastereomere | 64 | ESI: $(M+H)^+$ = 938/40/42 ($Br_2$) | 0.75 | FM1 | (KBr): C=O 1699.2; 1641.3 | |
| 78 | N45 | AS5 | 1 | O | A3 | C1 | saulenchromatographische Reinigung Kieselgel/ FM4; Diastereomere | 44 | ESI: $(M+H)^+$ = 952/4/6 ($Br_2$) | 0.73 | FM1 | (KBr): C=O 1712.7, 1637.5; -NH- 3300.0 | |
| 110 | N15 | AS4 | 1 | O | A0 | C5 | saulenchromatographische Reinigung Kieselgel/ FM4 | 82 | ESI: $(M+H)^+$ = 0.79 725/7/9 ($Br_2$) | | FM1 | (KBr): C=O 1620.1, 1514.0 | |
| 111 | N15 | AS4 | 1 | O | A0 | C15 | saulenchromatographische Reinigung Kieselgel/ FM4 | 58 | ESI: $M^+$ = 726/28/30 ($Br_2$) | 0 77 | FM1 | (KBr): C=O 1697.3, 1620.1 | |
| 114 | N45 | AS4 | 1 | O | A0 | C5 | saulenchromatographische Reinigung Kieselgel/ FM4 | 75 | ESI: $(M+H)^+$ = 739/41/43 ($Br_2$) | 0.78 | FM1 | (KBr): C=O 1710.8, 1620.1 | |
| 112 | N15 | AS1 | 1 | O | A0 | C5 | saulenchromatographische Reinigung Kieselgel/ FM4 | 45 | ESI: $(M+H)^+$ = 726/28/30 ($Br_2$) | 0.33 | FM1 | (KBr): C=O 1695.3, 1624.0 | |
| 115 | N45 | AS1 | 1 | O | A0 | C5 | saulenchromatographische Reinigung Kieselgel/ FM4 | 28 | ESI: $(M+H)^+$ = 740/2/4 ($Br_2$) | 0.35 | FM1 | (KBr): C=O 1710.8. 1622 | |

(fortgesetzt)

| Lfd. Nr. | RCO | $R^2$ | n | X | A | $NR^3R^4$ | Anmerkungen | % Ausbeute | MS | $R_f$ | Fließmittel | IR [$cm^{-1}$] | F p. (°C) |
|---|---|---|---|---|---|---|---|---|---|---|---|---|---|
| 113 | N15 | AS4 | 1 | O | A0 | C16 | saulenchromatographische Reinigung Kieselgel/ FM4 | 56 | ESI: $(M+H)^+$ = 726/8/30 ($Br_2$) | 0.68 | FM1 | (KBr): C=O 1699.2: 1618.2 | 173-176 |
| 119 | N45 | AS4 | 1 | O | A0 | C16 | saulenchromatographische Reinigung Kieselgel/ FM4 | 81 | ESI: $(M+H)^+$ = 762/4/6 ($Br_2$) | 0.69 | FM1 | (KBr): C=O 1701.8, 1618.2 | 148-152 |
| 120 | N15 | AS1 | 1 | O | A0 | C15 | saulenchromatographische Reinigung, Kieselgel/ FM4 | 27 | ESI: $(M+H)^+$ = 749/51/53 ($Br_2$) | 0.31 | FM1 | (KBr): C=O 1695.3 | 173-175 |
| 128 | N15 | AS4 | 1 | O | A0 | C3 | saulenchromatographische Reinigung Kieselgel/ FM4 | 61 | ESI: $(M+Na)^+$ = 764/6/8 ($Br_2$) | 0.72 | FM1 | (KBr): C=O 1699.2, 1618.2 | 174-177 |
| 130 | N15 | AS4 | 1 | O | A0 | C6 | saulenchromatographische Reinigung Kieselgel/ FM4 | 60 | ESI: $(M+H)^+$ = 754/6/8 ($Br_2$) | 0.7 | FM1 | (KBr): C=O 1703, 1620.1 | 150-154 |
| 129 | N15 | AS1 | 1 | O | A0 | C16 | saulenchromatographische Reinigung Kieselgel/ FM4 | 18 | ESI: $(M+H)^+$ = 725/7/9 ($Br_2$) | 0.27 | FM1 | (KBr): C=O 1693.4. 1627.8 | 173-176 |
| 131 | N45 | AS4 | 1 | O | A0 | C6 | saulenchromatographische Reinigung Kieselgel/ FM4 | 69 | ESI: $(M+H)^+$ = 768/70/72 ($Br_2$) | 0.73 | FM1 | (KBr): C=O 1712.7, 1620.1 | 159-162 |
| 132 | N45 | AS4 | 1 | O | A0 | C3 | saulenchromatographische Reinigung Kieselgel/ FM4 | 27 | ESI: $(M+Na)^+$ = 778/80/2 ($Br_2$) | 0.72 | FM1 | (KBr): C=O 1712.7 1618.2 | 142-146 |
| 133 | N15 | AS1 | 1 | O | A0 | C6 | saulenchromatographische Reinigung Kieselgel/ FM4 | 24 | ESI: $(M+H)^+$ = 755/7/9 ($Br_2$) | 0.33 | FM1 | (KBr): C=O 1697.3, 1624.0 | 161-164 |
| 134 | N15 | AS4 | 1 | O | A0 | C18 | saulenchromatographische Reinigung Kieselgel/ FM4 | 69 | ESI: $(M+H)^+$ = 756/8/60 ($Br_2$) | 0.59 | FM1 | (KBr): C=O 1699.2, | 171-174 |

(fortgesetzt)

| Lfd. Nr. | RCO | $R^2$ | n | X | A | $NR^3R^4$ | Anmerkungen | % Ausbeute | MS | $R_f$ | Fließmittel | IR [$cm^{-1}$] | F p. (°C) |
|---|---|---|---|---|---|---|---|---|---|---|---|---|---|
| 135 | N15 | AS1 | 1 | O | A0 | C18 | saulenchromatographische Reinigung Kieselgel/ FM4 | 17 | ESI: $(M+H)^+$ = 757/9/61 ($Br_2$) : | 0.25 | FM1 | (KBr): C=O 1691.5: 1625.9 | 74-77 |
| | N29 | AS1 | 1 | O | A3 | C1 | | 61 | ESI: $(M+H)^+$ = 903/5/7 ($Br_2$) | 0.62 | FM7 | (KBr): C=O 1641.3 | |
| | N36 | AS1 | 1 | O | A3 | C1 | | 33 | ESI: $(M+H)^+$ = 915/7/9 ($Br_2$) | 0.49 | FM7 | (KBr): C=O 1641.3 | |
| 44 | N34 | AS1 | 1 | O | A3 | C1 | | 35 | ESI: $(M+H)^+$ = 936/38/40/42 ($Br_3$) | 0.3 | FM1 | (KBr): C=O 1641.3 | |
| 378 | N15 | AS1 | 0 | O | A3 | C1 | | 30 | ESI: $(M+H)^+$ = 940/2/4 ($Br_2$) | 0.13 | FM1 | (KBr): C=O 1701.1, 1641.3 | |
| | N48 | AS1 | 1 | O | A3 | C1 | | 52 | | 0.58 | FM7 | (KBr): C=O 1641.3 | |
| | N77 | AS1 | 1 | O | A3 | C4 | | 32 | ESI: $(M+H)^+$ = 955/7/9 ($Br_2$) | | | (KBr): C=O 1645; 1713 | |
| 294 | N66 | AS4 | 1 | O | A7 | C1 | $NEt_3$ als Base | 31 | ESI: $(M+H)^+$ = 895/7/9 ($Br_2$) | 0.3 | FM1 | (KBr): C=O 1653. 1772.5, 1716.5 | |
| 323 | N104 | AS1 | 1 | O | A0 | C4 | DIEA | 18 | ESI: $(M+H)^+$ = 799/801/803 ($Br_2$) | 0.4 | FM1 | (KBr): C=O 1624 | |
| 324 | N105 | AS1 | 1 | O | A0 | C4 | DIEA | 24 | ESI: $(M+H)^+$ = 773/5/7/9 ($Br_2$/Cl) | 0.38 | FM1 | (KBr): C=O 1624/1667 | |

(fortgesetzt)

| Lfd. Nr. | RCO | $R^2$ | n | X | A | $NR^3R^4$ | Anmerkungen | % Ausbeute | MS | $R_f$ | Fließmittel | IR [$cm^{-1}$] | F p. (°C) |
|---|---|---|---|---|---|---|---|---|---|---|---|---|---|
| 325 | N106 | AS1 | | O | A0 | C4 | DIEA | 22 | ESI: $(M+H)^+$ = 725/7/9 ($Br_2$) | 0.35 | FM1 | (KBr): C=O 1626/1662.5 | |
| 326 | N71 | AS1 | 1 | O | A01 | C1 | DIEA | 20 | ESI: $(M+H)^+$ = 752/4/6 ($Br_2$) | 0.16 | FM1 | (KBr): C=O 1624/1680 | |
| 327 | N71 | AS4 | 1 | O | A0 | C4 | DIEA | 24 | ESI: (M+H)+ = 750/214 ($Br_2$) | 0.48 | FM1 | (KBr): C=O 1618/1682 | |
| 328 | N107 | AS1 | 1 | O | A0 | C4 | DIEA | 17 | ESI: $(M+H)^+$ = 769/71/73 ($Br_2$) | 0.38 | FM1 | (KBr): C=O 1651 | |
| 329 | N108 | AS4 | 1 | O | A0 | C4 | DIEA | 16 | ESI: $(M+H)^+$ = 781/83/85 ($Br_2$) | 0.31 | FM1 | (KBr): C=O 1620/1674 | |
| 330 | N108 | AS4 | 1 | O | A0 | C1 | DIEA/ DMF | 15 | ESI: $(M+H)^+$ = 781/83/85 ($Br_2$) | 0.45 | FM1 | (KBr): C=O 1620/1678 | |
| 331 | N108 | AS4 | 1 | O | A0 | C20 | DIEA/ DMF | 15 | ESI: $(M+H)^+$ = 800/802/804 ($Br_2$) | 0.4 | FM1 | (KB4): C=O 1616/1680 | |
| 332 | N109 | AS1 | 1 | O | A0 | C4 | DIEA/ DMF | 39 | ESI: $(M+H)^+$ = 745/7/9 ($Br_2$) | 0.32 | FM1 | (KBr): C=O 1665 | |
| 333 | N110 | AS1 | 1 | O | A0 | C4 | DIEA/ DMF | 52 | ESI: $(M+H)^+$ = 745/7/9 ($Br_2$) | 0.34 | FM1 | (KBr): C=O 1636 | |
| 334 | N111 | AS14 | 1 | O | A0 | C1 | DIEA/ DMF | 18 | ESI: $(M+H)^+$ = 649 | 0.5 | FM1 | (KBr): C=O 1626/1688 | |

(fortgesetzt)

| Lfd. Nr. | RCO | $R^2$ | n | X | A | $NR^3R^4$ | Anmerkungen | % Ausbeute | MS | $R_f$ | Fließmittel | IR [$cm^{-1}$] | F p. (°C) |
|---|---|---|---|---|---|---|---|---|---|---|---|---|---|
| 335 | N109 | AS4 | 1 | O | A0 | C4 | DIEA/ DMF | 46 | ESI: $(M+H)^+$ = 744/6/8 ($Br_2$) | 0.47 | FM1 | (KBr): C=O 1618 | |
| 336 | N110 | AS1 | 1 | O | A0 | C8 | DIEA/ DMF | 25 | ESI: $(M+H)^+$ = 751/3/5 ($Br_2$) | 0.22 | FM1 | (KBr): C=O 1645 | |
| 337 | N109 | AS1 | 1 | O | A0 | C8 | DIEA/ DMF | 32 | ESI: (M+H)+ = 751/3/5 ($Br_2$) | 0.21 | FM1 | (KBr): C=O 1645 | |
| 338 | N109 | AS4 | 1 | H 2 | A0 | C8 | DIEA/ DMF | 38 | ESI: $(M+H)^+$ = 736/8/40 ($Br_2$) | 0.44 | FM1 | (KBr): C=O 1653 | |
| 339 | N110 | AS4 | 1 | H 2 | A0 | C8 | DIEA/ DMF | 39 | ESI: $(M+H)^+$ = 736/8/40 ($Br_2$): | 0.44 | FM1 | (KBr): C=O 1653 | |
| 340 | N66 | AS1 | 1 | O | A0 | C20 | DIEA/ DMF | 33 | ESI: $(M+H)^+$ = 759/61/63 ($Br_2$) | 0.12 | FM1 | (KBr): C=O 1618/1657 | |
| 341 | N71 | AS1 | 1 | O | A0 | C20 | DIEA/ DMF | 31 | ESI: $(M+H)^+$ = 771/3/5 ($Br_2$) | 0 1 | FM1 | (KBr): C=O 1620/1680 | |
| 342 | N112 | AS4 | 1 | O | A0 | C20 | DIEA/ DMF | 27 | ESI: $(M+H)^+$ = 776/8/80 ($Br_2$) | 0.47 | FM1 | (KBr): C=O 1618/1682 | |
| 343 | N112 | AS1 | 1 | O | A0 | C20 | DIEA/DMF | 26 | ESI: $(M+H)^+$ = 777/9/81 ($Br_2$) | 0.11 | FM1 | (KBr): C=O 1624/1678 | |
| 344 : | N71 | AS1 | 1 | O | A0 | C37 | DIEA/ DMF | 52 | ESI: $(M+H)^+$ = 771/3/5 ($Br_2$) | 0.65 | FM1 | (KBr): C=O 1622/1680 | |

(fortgesetzt)

| Lfd. Nr. | RCO | $R^2$ | n | X | A | $NR^3R^4$ | Anmerkungen | % Ausbeute | MS | $R_f$ | Fließmittel | IR [$cm^{-1}$] | F p. (°C) |
|---|---|---|---|---|---|---|---|---|---|---|---|---|---|
| 345 | N66 | AS1 | 1 | O | A0 | C37 | DIEA/ DMF | 50 | ESI: $(M+H)^+$ = 759/61/63 ($Br_2$) | 0.7 | FM1 | (KBr): C=O 1626/1659 | |
| 346 | N71 | AS4 | 1 | O | A0 | C37 | DIEA/ DMF | 37 | ESI: $(M+H)^+$ = 770/72/74 ($Br_2$) | 0.75 | FM1 | (KBr): C=O 1620/1682 | |
| 347 | N6 | AS4 | 1 | O | A0 | C37 | DIEA/ DMF | 45 | ESI: $(M+H)^+$ = 758/60/62 ($Br_2$); | 0 8 | FM1 | (KBr): C=O 1620/1663 | |
| 348 | N113 | AS4 | 1 | O | A0 | C20 | DIEA/ DMF | 24 | ESI: $(M+H)^+$ = 771/3/5 (BR2) | 0.8 | FM1 | (KBr): C=O 1616/1701 | |
| 349 | N113 | AS4 | 1 | O | A0 | C8 | DIEA/ DMF | 40 | ESI: $(M+H)^+$ = 757/59/61 ($Br_2$) | 0.8 | FM1 | (KBr): C=O 1616/1699 | |
| 350 | N111 | AS4 | 1 | O | A0 | C20 | | 33 | ESI: $(M+H)^+$ = 838/40/42 ($Br_2$) | 0.63 | FM1 | (KBr): C=O 1620/1688 | |
| 351 | N111 | AS4 | 1 | O | A0 | C8 | | 39 | ESI: $(M+H)^+$ = 824/6/8 ($Br_2$) | 0.64 | FM1 | (KBr): C=O 1620/1688 | |
| 352 | N111 | AS1 | 1 | O | A0 | C8 | | 36 | ESI: $(M+H)^+$ = 825/7/9 ($Br_2$) | 0.37 | FM1 | (KBr): C=O 1644/1688 | |
| 353 | N112 1 | AS1 | 1 | O | A0 | C8 | | 24 | ESI: $(M+H)^+$ = 763/5/7 ($Br_2$) | 0.28 | FM1 | (KBr): C=O 1624/1684 | |
| 355 | N113 | AS4 | 1 | O | A0 | C11 | DIEA/ DMF | 6 | ESI: $(M+H)^+$ = 772/4/6 ($Br_2$) | 0.5 | FM1 | (KBr): C=O 1620/1697 | |

(fortgesetzt)

| Lfd. Nr. | RCO | $R^2$ | n | X | A | $NR^3R^4$ | Anmerkungen | % Ausbeute | MS | $R_f$ | Fließmittel | IR [$cm^{-1}$] | F p. (°C) |
|---|---|---|---|---|---|---|---|---|---|---|---|---|---|
| 356 | N66 | AS4 | 1 | O | A0 | C38 | DIEA/ DMF | 31 | ESI: $(M+H)^+$ = 785/7/9 ($Br_2$) | 0.23 | FM1 | (KBr): C=O 1624/1664 | |
| 357 | N112 | AS4 | 1 | O | A0 | C11 | DIEA/ DMF | 5 | ESI: $(M+H)^+$ = 777/79/81 ($Br_2$) | 0.37 | FM1 | | |
| 358 | N111 | AS1 | 1 | O | A0 | C11 | DIEA/ DMF | 13 | ESI: $(M+H)^+$ = 840/42/44 ($Br_2$) | 0.09 | FM1 | (KBr): C=O 1624/1686 | |
| 359 | N111 | AS4 | 1 | O | A0 | C11 | DIEA/ DMF | 24 | | 0.39 | FM1 | (KBr): C=O 1622/1686 | |
| 360 | N109 | AS4 | 1 | O | A0 | C8 | DIEA/ DMF | 25 | ESI: $(M+H)^+$ = 750/52/54 ($Br_2$) | 0.7 | FM1 | (KBr): C=O 1618/1657 | |
| 361 | N110 | AS4 | 1 | O | A0 | C11 | DIEA/ DMF | 35 | ESI: $(M+H)^+$ = 750/52/54 ($Br_2$) | 0.5 | FM1 | (KBr): C=O 1622/1649 | |
| 362 | N110 | AS4 | 1 | O | A0 | C8 | DIEA/ DMF | 24 | ESI: $(M+H)^+$ = 750/52/54 ($Br_2$) | 0.5 | FM1 | (KBr): C=O 1649 | |
| 363 | N111 | AS4 | 1 | O | A0 | C37 | DIEA/ DMF | 25 | | 0.53 | FM1 | (KBr): C=O 1622/1688 | |
| 364 | N111 | AS1 | 1 | O | A0 | C37 | DIEA/ DMF | 24 | ESI: $(M+H)^+$ = 839/41/43 ($Br_2$) | 0.31 | FM1 | (KBr): C=O 1624/1686 | |
| 366 | N66 | AS4 | 1 | O | A0 | C39 | | 67 | ESI: $(M+H)^+$ = 772/4/6 ($Br_2$) | 0.53 | FM1 | (KBr): C=O 1618/1665 | |

(fortgesetzt)

| Lfd. Nr. | RCO | $R^2$ | n | X | A | $NR^3R^4$ | Anmerkungen | % Ausbeute | MS | $R_f$ | Fließmittel | IR [$cm^{-1}$] | F p. (°C) |
|---|---|---|---|---|---|---|---|---|---|---|---|---|---|
| 367 | N71 | AS4 | 1 | O | A0 | C39 | DIEA/ DMF | 12 | ESI: $(M+H)^+$ = 784/6/8 ($Br_2$) | 0.52 | FM1 | (KBr): C=O 1618/1684 | |
| 368 | N111 | AS4 | 1 | O | A0 | C39 | DIEA/ DMF | 37 | ESI: $(M+H)^+$ = 852/4/6 ($Br_2$) | 0.8 | FM1 | (KBr): C=O 1618/1686 | |
| 369 | N114 | AS4 | 1 | O | A0 | C8 | | 15 | ESI: $(M+H)^+$ = 824/6/8 ($Br_2$) | 0.58 | FM1 | (KBr): C=O 1618/1686 | |
| 370 | N66 | AS4 | 1 | O | A0 | C40 | DIEA/ DMF | 35 | ESI: $(M+H)^+$ = 773/5/7 ($Br_2$) | 0.44 | FM1 | (KBr): C=O 1622/1660 | |
| 371 | N111 | AS4 | 1 | O | A0 | C40 | DIEA/ DMF | 58 | ESI: $(M+H)^+$ = 853/5/7 ($Br_2$) | 0.44 | FM1 | (KBr): C=O 1622/1687 | |
| 373 | N115 | AS4 | 1 | O | A0 | C8 | DIEA/ DMF | 43 | ESI: $(M+H)^+$ = 822/4/6/8 ($Br_3$) | 0.7 | FM1 | (KBr): C=O 1620/1670 | |
| 374 | N116 | AS4 | 1 | O | A0 | C20 | | 27 | ESI: $(M+H)^+$ = 784/6/8 (Br) | 0.53 | FM1 | (KBr): C=O 1618/1680 | |
| 375 | N117 | AS4 | 1 | O | A0 | C20 | | 23 | ESI: $(M+H)^+$ = 815/7/9 (Br) | 0.52 | FM1 | (KBr): C=O 1620/1687 | |
| 376 | N118 | AS4 | 1 | O | A0 | C20 | | 30 | | 0.56 | FM1 | (KBr): C=O 1620/1684 | |
| 377 | N119 | AS4 | 1 | O | A0 | C20 | | 74 | ESI: $(M+H)^+$ = 848/50/52/54 ($Br_3$) | 0.61 | FM1 | (KBr): C=O 1616/1685 | |
| 418 | N111 | AS4 | 1 | O | A0 | C38 | DIEA/ DMF | 23 | ESI: $(M+H)^+$ = 865/7/9 (Br) | 0.27 | FM1 | (KBr): C=O 1622/1687 | |

(fortgesetzt)

| Lfd. Nr. | RCO | $R^2$ | n | X | A | $NR^3R^4$ | Anmerkungen | % Ausbeute | MS | $R_f$ | Fließmittel | IR [$cm^{-1}$] | F p. (°C) |
|---|---|---|---|---|---|---|---|---|---|---|---|---|---|
| 490 | N113 | AS1 | 1 | O | A0 | C20 | DIEA/ DMF | 37 | ESI: $(M+H)^+$ = 772/4/6 ($Br_2$) | 0.1 | FM1 | (KBr): C=O 1622/1699 | |
| 491 | N113 | AS1 | 1 | O | A0 | C8 | DIEA/ DMF | 94 | ESI: $(M+H)^+$ = 758/60/62 ($Br_2$) | 0.37 | FM1 | (KBr): C=O 1624/1691 | |
| 492 | N113 | AS1 | 1 | O | A0 | C11 | DIEA/ DMF | 42 | ESI: $(M+H)^+$ = 773/5/7 ($Br_2$) | 0.1 | FM1 | (KBr): C=O 1678 | |
| 495 | N133 | AS4 | 1 | O | A0 | C20 | | 45 | ESI: $(M+H)^+$ = 846/48/50 ($Br_2$) | 0.5 | FM1 | (KBr): C=O 1620/1682 | |
| 379 | N71 | AS1 | 1 | O | A0 | C3 | | 39 | ESI: $(M+H)^+$ = 769/71/73 ($Br_2$) | 0.41 | FM4 | (KBr): C=O 1680 | |
| 380 | N71 | AS4 | 1 | O | A0 | C3 | | 40 | ESI: $(M+H)^+$ = 768/70/72 ($Br_2$) | 0.47 | FM4 | (KBr): C=O 1618/1682 | |
| 381 | N71 | AS1 | 1 | O | A0 | C42 | | 11 | ESI: $(M+H)^+$ = 752/54/56 ($Br_2$) | 0.53 | FM1 | (KBr): C=O 1624/1682 | |
| 382 | N66 | AS1 | 1 | O | A0 | C42 | | 18 | ESI: $(M+H)^+$ = 740/42/44 ($Br_2$) | 0.16 | FM4 | (KBr): C=O 1630/1653 | |
| 393 | N66 | AS4 | 1 | O | A0 | C42 | | 47 | ESI: $(M+H)^+$ = 739/41/43 ($Br_2$) | 0.25 | FM4 | (KBr): C=O 1626/1659 | |
| 384 | N93 | AS1 | 1 | O | A0 | C1 | | 11 | ESI: $(M+H)^+$ = 790/92/94 ($Br_2$) | 0.2 | FM7 | (KBr): C=O 1636/1705 | |

(fortgesetzt)

| Lfd. Nr. | RCO | $R^2$ | n | X | A | $NR^3R^4$ | Anmerkungen | % Ausbeute | MS | $R_f$ | Fließmittel | IR [$cm^{-1}$] | F p. (°C) |
|---|---|---|---|---|---|---|---|---|---|---|---|---|---|
| 385 | N71 | AS4 | 1 | O | A0 | C42 | | 37 | ESI: $(M+H)^+$ = 751/53/55 ($Br_2$) | 0.3 | FM4 | (KBr): C=O 1620/1680 | |
| 386 | N71 | AS4 | 1 | O | A0 | C18 | | 26 | ESI: $(M+H)^+$ = 782/4/6 ($Br_2$) | 0.27 | FM4 | (KBr): C=O 1620/1682 | |
| 387 | NS6 | AS4 | 1 | O | A0 | C5 | | 62 | ESI: $(M+H)^+$ = 739/41/43 ($Br_2$) | 0.38 | FM4 | (KBr): C=O 1626/1663 | |
| 388 | N71 | AS4 | 1 | O | A0 | C5 | | 55 | ESI: $(M+H)^+$ = 751/3/5 ($Br_2$) | 0.39 | FM4 | (KBr): C=O 1618/1684 | |
| 389 | N66 | AS1 | 1 | O | A0 | C43 | | 59 | ESI: $(M+H)^+$ = 796/98/800 ($Br_2$) | 0.32 | $CH_2Cl_2$/ MeOH/ $NH_4OH$ 9/1/0 1 | (KBr): C=O 1653 | |
| 390 | N135 | AS4 | 1 | O | A0 | C18 | | 5 | ESI: $(M+H)^+$ = 853/5/7 ($Br_2$) | 0.71 | FM1 | (KBr): C=O 1622/1653 | |
| 391 | N135 | AS1 | 1 | O | A0 | C18 | | 6 | ESI: $(M+H)^+$ = 854/6/8 ($Br_2$) | 0.54 | FM1 | (KBr): C=O 1659 | |
| 392 | N120 | AS1 | 1 | O | A0 | C4 | | 12 | ESI: $(M+H)^+$ = 763/5/7 ($Br_2$) | 0.41 | $CH_2Cl_2$/ MeOH/ $NH_4OH$ 9/1/0.1 | (KBr): C=O 1618/1639 | |
| 393 | N66 | AS1 | 1 | O | A0 | C44 | | 28 | ESI: $(M+H)^+$ = 763/5/7 ($Br_2$) | 0.5 | $CH_2Cl_2$/ MeOH/ $NH_4OH$ 9/1/0.1 | (KBr): C=O 1659 | |

(fortgesetzt)

| Lfd. Nr. | RCO | $R^2$ | n | X | A | $NR^3R^4$ | Anmerkungen | % Ausbeute | MS | $R_f$ | Fließmittel | IR [$cm^{-1}$] | F p. (°C) |
|---|---|---|---|---|---|---|---|---|---|---|---|---|---|
| 394 | N66 | AS4 | 1 | O | A0 | C21 | | 37 | ESI: $(M+H)^+$ = 740/42/44 ($Br_2$) | 0.35 | EE/ MeOH/ AcOH 75/25/0.5 | (KBr): C=O 1659 | |
| 396 | N71 | AS1 | 1 | O | A0 | C21 | | 49 | ESI: (M+H)+ = 753/5/7 ($Br_2$) | 0.3 | EE/ MeOH/ AcOH 75/25/0.5 | (KBr): C=O 1622/1678 | |
| 397 | N66 | AS1 | 1 | O | A0 | C21 | | 62 | ESI: $(M+H)^+$ = 741/3/5 ($Br_2$) | 0.35 | EE/ MeOH/ AcOH 75/25/0.5 | (KBr): C=O 1649 | |
| 398 | N121 | AS4 | 1 | O | A0 | C8 | | 80 | ESI: $(M+H)^+$ = 743/5/7 ($Br_2$) | 0.55 | $CH_2Cl_2$/ MeOH/ $NH_4OH$ 9/1/0.1 | (KBr): C=O 1618/1668 | |
| 399 | N122 | AS4 | 1 | O | A0 | C18 | | 40 | ESI: (M+H)+ = 800/2/4 ($Br_2$) | 0.62 | FM1 | (KBr): C=O 1622/1682 | |
| 400 | N136 | AS4 | 1 | O | A0 | C8 | | 11 | ESI: (M+H)+ = 741/3/5 ($Br_2$) | 0.65 | FM1 | (KBr): C=O 1622/1653 | |
| 401 | N66 | AS1 | 1 | O | A0 | C45 | | 19 | ESI: $(M+H)^+$ = 743/51/53 ($Br_2$) | 0.65 | $CH_2Cl_2$/ MeOH/ $NH_4OH$ 9/1/0.1 | (KBr): C=O 1659 | |
| 402 | N136 | AS4 | 1 | O | A0 | C1 | | 10 | ESI: $(M+H)^+$ = 736/8/40 ($Br_2$) | 0.42 | $CH_2Cl_2$/ MeOH/ $NH_4OH$ 9/1/0.1 | (KBr): C=O 1649 | |
| 403 | N121 | AS4 | 1 | O | A0 | C1 | | 25 | ESI: $(M+H)^+$ = 738/40/42 ($Br_2$) | 0.43 | $CH_2Cl_2$/ MeOH/ $NH_4OH$ 9/1/0.1 | (KBr): C=O 1626/1676 | |

(fortgesetzt)

| Lfd. Nr. | RCO | $R^2$ | n | X | A | $NR^3R^4$ | Anmerkungen | % Ausbeute | MS | $R_f$ | Fließmittel | IR [$cm^{-1}$] | F p. (°C) |
|---|---|---|---|---|---|---|---|---|---|---|---|---|---|
| 404 | N66 | AS4 | 1 | O | A0 | C46 | | 58 | ESI: $(M+H)^+$ = 766/8/70 ($Br_2$) | 0.29 | $CH_2Cl_2$/ MeOH/ $NH_4OH$ 9/1/0.1 | (KBr): C=O 1624/1663 | |
| 405 | N66 | AS1 | 1 | O | A0 | C47 | | 40 | ESI: $(M+H)^+$ = 752/4/6 ($Br_2$) | 0.3 | EE/ MeOH 9/1 | (KBr): C=O 1659 | |
| 406 | N136 | AS1 | 1 | O | A0 | C1 | | 16 | ESI: $(M+H)^+$ = 737/39/41 ($Br_2$) | 0.34 | FM7 | (KBr): C=O 1645 | |
| 407 | N121 | AS1 | 1 | O | A0 | C1 | | 15 | ESI: $(M+H)^+$ = 739/41/43 ($Br_2$) | 0.36 | FM7 | (KBr): C=O 1638 | |
| 408 | N71 | AS4 | 1 | O | A0 | C48 | | 47 | ESI: $(M+H)^+$ = 792/4/6 ($Br_2$) | 0.17 | $CH_2Cl_2$/ MeOH/ $NH_4OH$ 9/1/0.1 | (KBr): C=O 1620/1680 | |
| 409 | N66 | AS4 | 1 | O | A0 | C48 | | 31 | ESI: $(M+H)^+$ = 780/2/4 ($Br_2$) | 0.43 | $CH_2Cl_2$/ MeOH/ $NH_4OH$ 9/1/0.1 | (KBr): C=O 1665/1736 | |
| 410 | N66 | AS1 | 1 | O | A0 | C49 | | 51 | | 0.24 | EE/ MeOH 9/1 | (KBr): C=O 1661 | |
| 411 | N71 | AS4 | 1 | O | A0 | C44 | | 45 | ESI: $(M+Na)^+$ = 796/98/800 ($Br_2$) | 0.35 | EE/ MeOH 9/1 | (KBr): C=O 1728 | |
| 412 | N66 | AS1 | 1 | O | A0 | C50 | | 58 | ESI: $(M+H)^+$ = 756/58/60 ($Br_2$) | 0.47 | EE/ MeOH 9/1 | (KBr): C=O 1642/1661 | |

(fortgesetzt)

| Lfd. Nr. | RCO | $R^2$ | n | X | A | $NR^3R^4$ | Anmerkungen | % Ausbeute | MS | $R_f$ | Fließmittel | IR [$cm^{-1}$] | F p. (°C) |
|---|---|---|---|---|---|---|---|---|---|---|---|---|---|
| 413 | N66 | AS1 | 1 | O | A0 | C51 | | 16 | ESI: $(M+H)^+$ = 758/90/92 ($Br_2$) | 0.47 | EE/ MeOH/ 5/5/0.1 | (KBr): C=O 1631 | |
| 414 | N66 | AS4 | 1 | O | A0 | C52 | | 34 | ESI: $(M+H)^+$ = 747/49/51 ($Br_2$) | 0.54 | FM1 | (KBr): C=O 1622/1662 | |
| 415 | N71 | AS4 | 1 | O | A0 | C52 | | 35 | ESI: $(M+H)^+$ = 759/61/63 ($Br_2$) | 0.52 | FM1 | (KBr): C=O 1620/1682 | |
| 416 | N66 | AS4 | 1 | O | A0 | C53 | | 53 | ESI: $(M+H)^+$ = 759/61/63 ($Br_2$) | 0.45 | $CH_2Cl_2$/ MeOH/ $NH_4OH$ 9/1/0.1 | (KBr): C=O 1620/1664 | |
| 417 | N71 | AS4 | 1 | O | A0 | C53 | | 39 | ESI: $(M+H)^+$ = 771/3/5 ($Br_2$) | 0 43 | | (KBr): C=O 1620/1684 | |
| 496 | N66 | AS4 | 1 | O | A0 | C64 | | 57 | | 0.5 | $CH_2Cl_2$/ MeOH/ $NH_4OH$ 9/1/0 | (KBr): C=O 1635 | |
| 497 | N66 | AS1 | 1 | O | A0 | C53 | | 60 | ESI: $(M+H)^+$ = 760/2/4 ($Br_2$) | 0.31 | $CH_2Cl_2$/ MeOH/ $NH_4OH$ 50/50/0.5 | (KBr): C=O 1676 | |
| 498 | N66 | AS4 | 1 | O | A0 | C65 i | | 60 | ESI: $(M+H)^+$ = 760/2/4 ($Br_2$) | 0.39 | FM1 | (KBr): C=O 1676 | |
| 499 | N71 | AS4 | 1 | O | A0 | C65 | | 53 | ESI: $(M+H)^+$ = 785/7/9 ($Br_2$) | 0.39 | FM1 | (KBr): C=O 1618/1684 | |

(fortgesetzt)

| Lfd. Nr. | RCO | $R^2$ | n | X | A | $NR^3R^4$ | Anmerkungen | % Ausbeute | MS | $R_f$ | Fließmittel | IR [$cm^{-1}$] | F p. (°C) |
|---|---|---|---|---|---|---|---|---|---|---|---|---|---|
| 500 | N66 | AS4 | 1 | O | A0 | C51 | | 71 | ESI: $(M+H)^+$ = 787/89/91 ($Br_2$) | 0.15 | $CH_2Cl_2$/ MeOH/ $NH_4OH$ 9/1/0.1 | (KBr): C=O 1628 | |
| 501 | N71 | AS1 | 1 | O | A0 | C53 | | 71 | ESI: $(M+H)^+$ = 772/4/6 ($Br_2$) | 0.25 | $CH_2Cl_2$/ MeOH/ NH, OH 50/50/0.5 | (KBr): C=O 1676 | |
| 502 | N66 | AS1 | 1 | O | A0 | C65 | | 42 | ESI: $(M+H)^+$ = 774/6/8 ($Br_2$) | 0.15 | FM1 | (KBr): C=O 1626/1657 | |
| 503 | N71 | AS1 | 1 | O | A0 | C65 | | 48 | ESI: $M+H)^+$ = 786/88/90 ($Br_2$): | 0.12 | FM1 | (KBr): C=O 1620/1682 | |
| 504 | N66 | AS4 | 1 | O | A0 | C66 | | 67 | ESI: $(M+H)^+$ = 787/89/91 ($Br_2$) | 0.65 | $CH_2Cl_2$/ MeOH/ $NH_4OH$ 50/50/0 5 | (KBr): C=O 1624 | |
| 297 | N71 | AS4 | 1 | H 2 | A0 | C8 | | 9 | ESI: $(M+H)^+$ = 742/4/6 ($Br_2$) | 0.2 | FM1 Fm1 | (KBr): C=O 1684 | |
| 298 | N71 | AS1 3 | 1 | H 2 | A0 | C8 | | 6 | ESI: $(M+H)^+$ = 664/6 (Br) | 0.16 | FM1 | (KBr): C=O 1622/1682 | |
| 299 | N66 | A54 | 1 | H 2 | A0 | C8 | | 21 | ESI: $(M+H)^+$ = 730/2/4 ($Br_2$) | 0.25 | FM1 | (KBr): C=O 1666 | |
| 300 | N66 | AS1 3 | 1 | H 2 | A0 | C8 | | 14 | ESI: $(M+H)^+$ = 652/4 (Br) | 0.19 | FM1 | (KBr): C=O 1666 | |
| 301 | N71 | AS1 | | H 2 | A0 | C8 | | 26 | ESI: $(M+H)^+$ = 743/5/7 ($Br_2$) | 0.2 | FM1 | (KBr): C=O 1682 | |

(fortgesetzt)

| Lfd. Nr. | RCO | $R^2$ | n | X | A | $NR^3R^4$ | Anmerkungen | % Ausbeute | MS | $R_f$ | Fließmittel | IR [$cm^{-1}$] | F p. (°C) |
|---|---|---|---|---|---|---|---|---|---|---|---|---|---|
| 420 | N87 | AS1 | 1 | O | A0 | C4 | | 45 | ESI: $(M+H)^+$ = 797/99/801 ($Br_2$) | 0.5 | FM1 | (KBr): C=O 1624/1665/ 1719 | |
| 422 | N87 | AS1 | 1 | O | A0 | C8 | | 35 | ESI: (M+H)+ = 805/7/9 ($Br_2$) | 0.54 | FM1 | (KBr): C=O 1628/1668/ 1720 | |
| 431 | N125 | AS4 | 1 | O | A0 | C8 | | 89 | ESI: $(M+H)^+$ = 772/4/6 ($Br_2$) | 0.75 | FM1 | (KBr): C=O 1713/1773 | |
| 432 | N125 | AS1 | 1 | O | A0 | C4 | | 59 | ESI: $(M+H)^+$ = 767/69/71 ($Br_2$). | 0.65 | FM1 | (KBr): C=O 1622/1711/ 1773 | |
| 433 | N126 | AS4 | 1 | O | A0 | C4 | | 33 | ESI: $(M+H)^+$ = 780/2/4 ($Br_2$) | 0.65 | FM1 | (KBr): C=O 1709/1769 | |
| 434 | N126 | AS1 | 1 | O | A0 | C8 | | 53 | ESI: $(M+H)^+$ = 787/89/91 ($Br_2$) | 0.53 | FM1 | (KBr): C=O 1626/1707 | |
| 440 | N127 | AS4 | 1 | O | A0 | C8 | | 67 | ESI: $(M+H)^+$ = 780/2/4 ($Br_2$) | 0.67 | FM1 | (KBr): C=O 1618 | |
| 441 | N127 | AS4 | 1 | O | A0 | C20 | | 89 | ESI: $(M+H)^+$ = 794/6/8 ($Br_2$) | 0.24 | EE/MeOH/ $NH_4OH$ 8/1.5/0.3 | (KBr): C=O 1618 | |
| 442 | N127 | AS4 | 1 | O | A0 | C4 | | 83 | ESI: $(M+H)^+$ = 774/6/8 ($Br_2$) | 0.37 | EE/MeOH/ $NH_4OH$ 8/1.5/0 3 | (KBr): C=O 1616/1732 | |
| 456 | N66 | AS1 6 | 1 | O | A0 | C8 | | 83 | ESI: $(M+H)^+$ = 641/3/5 ($Cl_2$) | 0.32 | EE/MeOH/ $NH_4OH$ 8/1.5/0.1 | (KBr): C=O 1624/1665 | |

(fortgesetzt)

| Lfd. Nr. | RCO | $R^2$ | n | X | A | $NR^3R^4$ | Anmerkungen | % Ausbeute | MS | $R_f$ | Fließmittel | IR [$cm^{-1}$] | F p. (°C) |
|---|---|---|---|---|---|---|---|---|---|---|---|---|---|
| 466 | N128 | AS4 | 1 | O | A0 | C8 | | 13 | ESI: $(M+H)^+$ = 832/4/6 ($Br_2$) | 0.63 | FM1 | (KBr): C=O 1684 | |
| 467 | N129 | AS4 | 1 | O | A0 | C8 | | 16 | ESI: $(M+H)^+$ = 806/08/10 ($Br_2$) | 0.63 | FM1 | (KBr): C=O 1618/1682 | |
| 468 | N129 | AS1 | 1 | O | A0 | C8 | | 28 | ESI: $(M+H)^+$ = 807/09/11 ($Br_2$) | 0.29. | FM1 | (KBr): C=O 1630/1680 | |
| 470 | N128 | AS1 | 1 | O | A0 | C8 | | 81 | ESI: $(M+H)^+$ = 835/7/9 ($Br_2$) | 0.63 | FM1 | (KBr): C=O 1684 | |
| 473 | N130 | AS1 | 1 | O | A0 | C8 | | 40 | ESI: $(M+H)^+$ = 787/89/91 ($Br_2$) | 0,51 | FM1 | (KBr): C=O 1624/1678 | |
| 474 | N130 | AS4 | 1 | O | A0 | C8 | | 17 | ESI: $(M+H)^+$ = 786/88/90 ($Br_2$) | 0.71 | FM1 | (KBr): C=O 1618/1684 | |
| 477 | N66 | AS16 | 1 | O | A0 | C1 | | 33 | ESI: $(M+H)^+$ = 636/38/40 ($Cl_2$) | 0.53 | EE/MeOH/ $NH_4OH$ 9/1/1 (v/v/v) | (KBr): C=O 1661 | |
| 481 | N131 | AS4 | 1 | O | A0 | C37 | | 30 | ESI: $(M+H)^+$ = 838/40/42/44 ($Br_2$, $Cl_2$) | 0.15 | $CH_2Cl_2$/ MeOH 7/3 (v/v) | (KBr): C=O 1618/1685 | |
| 482 | N131 | AS4 | 1 | O | A0 | C20 | | 24 | ESI: $(M+H)^+$ = 838/40/42/44 ($Br_2$; $Cl_2$) | 0.15 | $CH_2Cl_2$/ MeOH 7/3 (v/v) | (KBr): C=O 1618/1685 | |

(fortgesetzt)

| Lfd. Nr. | RCO | $R^2$ | n | X | A | $NR^3R^4$ | Anmerkungen | % Ausbeute | MS | $R_f$ | Fließmittel | IR [$cm^{-1}$] | F p. (°C) |
|---|---|---|---|---|---|---|---|---|---|---|---|---|---|
| 483 | N132 | AS4 | 1 | O | A0 | C20 | | 62 | ESI: $(M+H)^+$= 804/6/8/10 ($Br_2$; Cl) | 0.55 | FM1 | (KBr): C=O 1684 | |
| 484 | N132 | AS4 | 1 | O | A0 | C37 | | 85 | ESI: $(M+H)^+$ = 804/6/8/10 ($Br_2$; Cl) | 0.60 | FM1 | (KBr): C=O 1616/1686 | |
| 505 | N134 | AS4 | 1 | O | A0 | C8 | | 81 | ESI: $(M+H)^+$ = 781/3/5 (Br2) | 0,74 | FM1 | (KBr): C=O 1616/1714 | |
| 292 | N66 | AS1 | 1 | H 2 | A0 | C8 | | 6 | ESI: $(M+H)^+$ = 731/3/5 (Br2) | 0.25 | FM1 | (KBr): C=O 1607/1664 | |
| 245 | N72 | AS4 | 1 | H 2 | A0 | C8 | | 19 | ESI: $(M+H)^+$ = 731/3/5 ($Br_2$) | 0.30 | FM1 | (KBr): C=O 1668 | |
| 220 | N15 | AS1 | 1 | H 2 | A0 | C8 | | 6 | ESI: $(M+H)^+$ = 717/19/21 ($Br_2$) | 0.30 | FM1 | (KBr): C=O 1697.3 | |
| 307 | N87 | AS4 | 1 | O | A0 | C4 | | 27 | ESI: $(M+H)^+$ =796/98/800 ($Br_2$) | 0.50 | FM1 | (KBr): C=O 1618/ 1670/ 1718 | |
| 178 | N74 | AS1 | 1 | O | A0 | C4 | | 33 | ESI: $(M+H)^+$ = 679/81/83 ($Br_2$) | 0,60 | EE/ MeOH/ AcOH 50/50/1 (v/ v/v) | (KBr): C=O 1624 | |
| 395 | N71 | AS4 | 1 | O | A0 | C21 | | 22 | ESI: $(M+H)^+$ = 752/4/6 ($Br_2$) | 0.25 | EE/ MeOH/ AcOH 50/25/0.5 (v/v/v) | | |

(fortgesetzt)

| Lfd. Nr. | RCO | $R^2$ | n | X | A | $NR^3R^4$ | Anmerkungen | % Ausbeute | MS | $R_f$ | Fließmittel | IR [$cm^{-1}$] | F p. (°C) |
|---|---|---|---|---|---|---|---|---|---|---|---|---|---|
| 509 | N119 | AS4 | 1 | O | A0 | C38 | DMF | 45 | ESI: $(M+H)^+$ = 875/7/9/81 = ($Br_3$) | 0.2 | FM1 | (KBr): C=O 1687/1618 | |
| 510 | N118 | AS4 | 1 | O | A0 | C38 | DMF | 34 | ESI: $(M+H)^+$ = 827/29/31 ($Br_2$) | 0.4 | FM1 | (KBr): C=O 1682/1620 | |
| 519 | N137 | AS4 | 1 | O | A0 | C20 | THF/DMF | 62 | ESI: $(M+H)^+$ = 786/88/90 ($Br_2$) | 0.1 | FM1 | (KBr): C=O 1618/1678 | |
| 520 | N138 | AS4 | 1 | O | A0 | C20 | THF/DMF | 31 | ESI: $(M+H)^+$ = 906/08/10 ($Br_2$) | 0.45 | FM1 | (KBr): C=O 1693 | |
| | N66 | AS4 | 1 | O | A0 | C69 | DMF | 100 | | | | | |
| 533 | N139 | AS1 | 1 | O | A0 | C8 | | 44 | ESI: $(M+H)^+$ = 746/48/50 ($Br_2$) | 0.1 | FM5 | (KBr): C=O 1628 | |
| 534 | N139 | AS4 | 1 | O | A0 | C20 | | 60 | ESI: $(M+H)^+$ = 759/61/63 ($Br_2$) | 0.2 | FM5 | (KBr): C=O 1618/1672 | |
| 535 | N139 | AS1 | 1 | O | A0 | C53 | | 34 | ESI: $(M+H)^+$= 761/63/65 ($Br_2$) | 0.1 | FM5 | (KBr): C=O 1624/1670 | |
| 536 | N140 | AS4 | 1 | O | A0 | C8 | | 40 | ESI: $(M+H)^+$= 745/7/9 ($Br_2$) | 0.37 | FM1 | (KBr): C=O 1616/1676 | |
| 537 | N140 | AS1 | 1 | O | A0 | C8 | | 334 | ESI: $(M+H)^+$ = 746/48/50 ($Br_2$) | 0.3 | FM1 | (KBr): C=O 1614/1672 | |
| 551 | N66 | AS1 | 1 | O | A0 | C66 | | 41 | ESI: $(M+H)^+$ = 788/90/92 ($Br_2$) | 0.3 | EE / MeOH /$NH_4OH$ 50/50/0.5 | (KBr): C=O 1628 | |

(fortgesetzt)

| Lfd. Nr. | RCO | $R^2$ | n | X | A | $NR^3R^4$ | Anmerkungen | % Ausbeute | MS | $R_f$ | Fließmittel | IR [$cm^{-1}$] | F p. (°C) |
|---|---|---|---|---|---|---|---|---|---|---|---|---|---|
| 552 | N66 | AS4 | 1 | O | A0 | C78 | | 83 | ESI: $(M+H)^+$ = 787/89/91 ($Br_2$) | 0.6 | $CH_2Cl_2$/ MeOH/ $NH_4OH$ 80/20/1 | (KBr): C=O 1620 | |
| 553 | N66 | AS1 | 1 | O | A0 | C78 | | 30 | ESI: $(M+H)^+$ = 788/90/92 ($Br_2$) | 0.5 | $CH_2Cl_2$/ MeOH/ $NH_4OH$ 80/20/1 | (KBr): C=O 1626 | |
| 554 | N71 | AS4 | 1 | O | A0 | C78 | | 67 | ESI: $(M+H)^+$ = 799/801/803 ($Br_2$) | 0.5 | $CH_2Cl_2$/ MeOH/ $NH_4OH$ 80/20/1 | (KBr): C=O 1622/1684 | |
| 555 | N71 | AS1 | 1 | O | A0 | C78 | | 26 | ESI: $(M+H)^+$ = 800/02/04 ($Br_2$) | 0.5 | $CH_2Cl_2$/ MeOH/ $NH_4OH$ 80/20/1 | (KBr): C=O 1624/1684 | |
| 556 | N66 | AS4 | 1 | O | A0 | C70 | | 71 | ESI: $(M+H)^+$ = 788/90/92 ($Br_2$) | 0.6 | $CH_2Cl_2$/ MeOH/ $NH_4OH$ 50/50/0.5 | (KBr): C=O 1653 | |
| 557 | N71 | AS4 | 1 | O | A0 | C70 | | 27 | ESI: $(M+H)^+$ = 800/02/04 ($Br_2$) | 0.8 | $CH_2Cl_2$/ MeOH/ $NH_4OH$ 50/50/0.5 | | |
| 558 | N66 | AS1 | 1 | O | A0 | C64 | | 61 | ESI: $(M+H)^+$ = 790/92/94 ($Br_2$) | 0.3 | $CH_2Cl_2$/ MeOH / $NH_4OH$ MeOH/ 90/10/1 | (KBr): C=O 1635 | |

(fortgesetzt)

| Lfd. Nr. | RCO | $R^2$ | n | X | A | $NR^3R^4$ | Anmerkungen | % Ausbeute | MS | $R_f$ | Fließmittel | IR [$cm^{-1}$] | F p. (°C) |
|---|---|---|---|---|---|---|---|---|---|---|---|---|---|
| 559 | N141 | AS4 | 1 | O | A0 | C20 | | 80 | ESI: $(M+H)^+$ = 626/28/30 ($Br_2$) | 0.3 | $CH_2Cl_2$/ MeOH/ $NH_4OH$ 90/10/1 | (KBr): C=O 1618/1714 | |
| 560 | N66 | AS4 | 1 | O | A0 | C71 | | 59 | ESI: $(M+H)^+$ = 837/39/41 837/39/41 ($Br_2$) | 0.3 | $CH_2Cl_2$/ MeOH / $NH_4OH$ 90/10/1 | (KBr): C=O 1628/62 | |
| 561 | N71 | AS4 | 1 | O | A0 | C71 | | 56 | ESI: $(M+H)^+$ = 849/51/53 ($Br_2$) | 0.2 | $CH_2Cl_2$/ MeOH / $NH_4OH$ 90/10/1 | (KBr): C=O 1618/1684 | |
| 562 | N66 | AS1 | 1 | O | A0 | C70 | | 15 | ESI: $(M+H)^+$ = 789/91/93 ($Br_2$) | 0.6 | $CH_2Cl_2$/ MeOH / $NH_4OH$ 50/50/0.5 | (KBr): C=O 1676 | |
| 563 | N71 | AS1 | 1 | O | A0 | C70 | | 27 | ESI: $(M+H)^+$ = 801/3/5 ($Br_2$) | 0.6 | $CH_2Cl_2$/ MeOH / $NH_4OH$ 70/30/1 | (KBr): C=O 1678 | |
| 565 | N66 | AS4 | 1 | O | A0 | C72 | | 51 | ESI: $(M+H)^+$ = 787/89/91 ($Br_2$) | 0.2 | $CH_2Cl_2$/ MeOH / $NH_4OH$ 80/20/1 | (KBr): C=O 1622/1658 | |
| 566 | N71 | AS4 | 1 | O | A0 | C72 | | 65 | ESI: $(M+H)^+$ = 799/801/803 ($Br_2$) | 0.2 | $CH_2Cl_2$/ MeOH / $NH_4OH$ 80/20/1 | (KBr): C=O 1620/1682 | |
| 567 | N136 | AS4 | 1 | O | A0 | C53 | | 65 | ESI: $(M+H)^+$ = 756/58/60 ($Br_2$) | 0.5 | $CH_2Cl_2$/ MeOH / $NH_4OH$ 80/20/1 | (KBr): C=O 1641 | |

(fortgesetzt)

| Lfd. Nr. | RCO | $R^2$ | n | X | A | $NR^3R^4$ | Anmerkungen | % Ausbeute | MS | $R_f$ | Fließmittel | IR [$cm^{-1}$] | F p. (°C) |
|---|---|---|---|---|---|---|---|---|---|---|---|---|---|
| 568 | N136 | AS4 | 1 | O | A0 | C72 | | 76 | ESI: $(M+H)^+$ = 784/6/8 ($Br_2$) | 0.2 | $CH_2Cl_2$/ MeOH / $NH_4OH$ 80/20/1 | (KBr): C=O 1637 | |
| 569 | N136 | AS1 | 1 | O | A0 | C53 | | 63 | ESI: $(M+H)^+$ = 757/59/61 ($Br_2$) | 0.4 | $CH_2Cl_2$/ MeOH / $NH_4OH$ 80/20/1 | (KBr): C=O 1643 | |
| 570 | N66 | AS31 | 1 | O | A0 | C20 | | 88 | ESI: $(M+H)^+$ = 640 | 0.6 | $CH_2Cl_2$/ MeOH / $NH_4OH$ 80/20/1 | (KBr): C=O 1622/1664 | |
| 571 | N66 | AS31 | 1 | O | A0 | C53 | | 82 | ESI: $(M+H)^+$ = 641 | 0.8 | $CH_2Cl_2$/ MeOH / $NH_4OH$ 80/20/1 | (KBr): C=O 1664 | |
| 572 | N71 | AS31 | 1 | O | A0 | C20 | | 100 | ESI: $(M+H)^+$ = 652 | 0.4 | $CH_2Cl_2$/ MeOH / $NH_4OH$ 80/20/1 | (KBr): C=O 1620/1684 | |
| 573 | N71 | AS31 | 1 | O | A0 | C53 | | 48 | ESI: $(M+H)^+$ = 653 | 0. 4 | $CH_2Cl_2$/ MeOH / $NH_4OH$ 80/20/1 | (KBr): C=O 1622/1684 | |
| 576 | N66 | AS11 | 1 | O | A0 | C53 | | 35 | ESI: $(M+H)^+$ = 727 | 0.65 | $CH_2Cl_2$/ MeOH / $NH_4OH$ 80/20/1 | (KBr): C=O 1664/1732 | |
| 577 | N71 | AS11 | 1 | O | A0 | C53 | | 73 | ESI: $(M+H)^+$ = 739 | 0.18 | EE / MeOH / $NH_4OH$ 50/50/0.5 | (KBr): C=O 1684/1734 | |

(fortgesetzt)

| Lfd. Nr. | RCO | $R^2$ | n | X | A | $NR^3R^4$ | Anmerkungen | % Ausbeute | MS | $R_f$ | Fließmittel | IR [$cm^{-1}$] | F p. (°C) |
|---|---|---|---|---|---|---|---|---|---|---|---|---|---|
| 580 | N66 | AS11 | 1 | O | A0 | C20 | | 65 | ESI: $(M+H)^+$ = 706 | 0.5 | $CH_2Cl_2$/ MeOH / $NH_4OH$ 80/20/1 | (KBr): C=O 1664/1732 | |
| 581 | N71 | AS11 | 1 | O | A0 | C20 | | 38 | ESI: $(M+H)^+$ = 738 | 0.2 | $CH_2Cl_2$/ MeOH / $NH_4OH$ 90/10/1 | (KBr): C=O 1684/1734 | |
| 582 | N143 | AS4 | 1 | O | A0 | C20 | | 61 | ESI: $(M+H)^+$ = 758/60/62 | 0.5 | $CH_2Cl_2$/ MeOH / $NH_4OH$ 90/10/1 | (KBr): C=O 1615 | |
| 583 | N66 | AS31 | 1 | O | A0 | C72 | | 50 | ESI: $(M+H)^+$ = 669 | 0.35 | FM1 | (KBr): C=O 1664 | |
| 584 | N71 | AS31 | 1 | O | A0 | C72 | | 68 | ESI: $(M+H)^+$ = 681 | 0.35 | FM1 | (KBr): C=O 1622/1684 | |
| 585 | N144 | AS4 | 1 | O | A0 | C8 | | 50 | ESI: $(M+H)^+$ = 927/29/31/33 (Br4) | 0.43 | FM5 | (KBr): C=O 1616/1684 | |
| 586 | N144 | AS4 | 1 | O | A0 | C53 | | 85 | ESI: $(M+H)^+$ = 942/4/6/8 ($Br_4$) | 0.67 | FM1 | (KBr): C=O 1680 | |
| 587 | N66 | AS11 | 1 | O | A0 | C72 | | 37 | ESI: $(M+H)^+$ = 755 | 0.35 | FM1 | (KBr): C=O 1622/1658/ 1732 | |
| 588 | N71 | AS11 | 1 | O | A0 | C72 | | 81 | ESI: $(M+H)^+$ = 767 | 0.5 | FM1 | (KBr): C=O 1684/1732 | |
| 619 | N71 | AS19 | 1 | O | A0 | C8 | | 27.9 | | 0.3 | EE / MeOH / $NH_4OH$ 9/1/0.3 | (KBr): C=O 1622/1684 | |

(fortgesetzt)

| Lfd. Nr. | RCO | $R^2$ | n | X | A | $NR^3R^4$ | Anmerkungen | % Ausbeute | MS | $R_f$ | Fließmittel | IR [$cm^{-1}$] | F p. (°C) |
|---|---|---|---|---|---|---|---|---|---|---|---|---|---|
| 620 | N66 | AS35 | 1 | O | A0 | C8 | | 36 | ESI: $(M+H)^+$ = 598 | 0.25 | MeOH | (KBr): C=O 1628/1664 | |
| 621 | N66 | AS36 | 1 | O | A0 | C8 | | 32 | ESI: $(M+H)^+$ = 587 | 0.56 | FM1 | (KBr): C=O 1626/1666 | |
| 622 | N71 | AS36 | 1 | O | A0 | C8 | | 32 | ESI: $(M+H)^+$ = 599 | 0.44 | FM1 | (KBr): C=O 1622/1684 | |
| 623 | N109 | AS36 | 1 | O | A0 | C8 | | 37 | ESI: $(M+H)^+$ = 593 | 0.6 | FM1 | (KBr): C=O 1626/1649 | |
| 624 | N118 | AS36 | 1 | O | A0 | C8 | | 55 | ESI: $(M+H)^+$ = 629 | 0.6 | FM1 | (KBr): C=O 1622/1684 | |
| 625 | N111 | AS36 | 1 | O | A0 | C8 | | 47 | ESI: $(M+H)^+$ = 667 | 0.61 | FM1 | (KBr): C=O 1624/1687 | |
| 626 | N111 | AS19 | 1 | O | A0 | C8 | | 20 | ESI: $(M+H)^+$ = 731/3(Br) | 0.28 | EE/ MeOH / $NH_4OH$ 9/1/0 3 | (KBr): C=O 1624/1687 | |
| 627 | N109 | AS19 | 1 | O | A0 | C8 | | 16 | ESI: $(M+H)^+$ = 657/9(Br) | 0.28 | EE / MeOH / $NH_4OH$ 9/1/0.3 | (KBr): C=O 1653 | |
| 633 | N118 | AS19 | 1 | O | A0 | C8 | | 20 | ESI: $(M+H)^+$ = 693/5 (Br) | 0.18 | EE/ MeOH / $NH_4OH$ 9/1/0.3 | (KBr): C=O 1622/1684 | |
| | N66 | AS1 | 1 | O | A0 | C69 | | 100 | | 0.3 | FM4 | (KBr): C=O 1668 | |

Beispiel 4

Herstellung von Verbindungen der allgemeinen Formel:

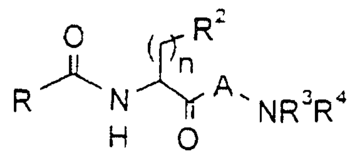

1-[4-Amino-3,5-dibrom-N-[4-(1,3,3a,4,5,6,7,7a-octahydro-2(2H)-oxobenzimidazol-1-yl)-1-piperidinyl]carbonyl]-D-phenylalanyl]-4-(4-pyridinyl)-piperazin (Lfd. Nr. 91)

Die Mischung von 0.35 g (2.1 mmol) CDT, 1.0 g (1.4 mmol) 4-(1,3,3a,4,5,6,7,7a-Octahydro-2(2H)-oxobenzimidazol-1-yl)-piperidin, 0.5 ml (2.8 mmol) DIEA und 100 ml Tetrahydrofuran wurde 1 Stunde unter Eiskühlung und anschließend 30 Minuten bei Raumtemperatur gerührt. Unter Rühren wurden 0.46 g (1.75 mmol) 1-(4-Amino-3,5-dibrom-D-phenylalanyl)-4-(4-pyridinyl)piperazin und 0.32 ml (1.8 mmol) DIEA zugegeben und 3 Stunden unter Rückfluß gekocht. Das Reaktionsgemisch wurde mit 100 ml Essigester verdünnt und die organische Phase zweimal mit wäßriger gesättigter Natriumhydrogencarbonat-Lösung gewaschen. Die vereinigten wäßrigen Phasen wurden anschließend einmal mit Essigester/Tetrahydrofuran = 1/1 (v/v) extrahiert und die vereinigten organischen Phasen einmal mit gesättigter wäßriger Kochsalz-Lösung gewaschen. Nach dem Trocknen der organischen Phase und Entfernen des Lösemittels im Vakuum wurde der Rückstand säulenchromatographisch (MN-Kieselgel 60, Macherey-Nagel, 70-230 mesh ASTM, Fließmittel: Essigester/Methanol = 9/1 (v/v)) gereinigt. Man erhielt 120 mg (12 % der Theorie) eines farblosen Schaumes.
IR (KBr): 1626. 1686 $cm^{-1}$ (C=O)
$R_f$: 0.62 (FM3)
ESI-MS: $(M+H)^+$ = 731/733/735 ($Br_2$)
$(M+H+Na)^{++}$ = 377/378/379 ($Br_2$)

Analog wurden hergestellt (jeweils n = 1):

| Lfd. Nr. | RCO | $R^2$ | A | $NR^3R^4$ | n | Anmerkunaen | % Ausbeute | MS | $R_f$ | Fließmittel | IR [$cm^{-1}$] | Fp. (°C) |
|---|---|---|---|---|---|---|---|---|---|---|---|---|
| | N16 | AS1 | A3 | C1 | 1 | Triethylamin als Base | 31 | ESI: $(M+H)^+$ = 974/6/8 ($Br_2$) | 0.2 | FM1 | (KBr): C=O 1641.3; 1695.3 | |
| 86 | N53 | AS4 | A0 | C1 | 1 | DMF als Losemittel | 23 | ESI: $(M+H)^+$ = 739/41/43 ($Br_2$) | 0.5 | FM1 | (KBr): C=O 1641.3, 1697.3 | |
| 87 | N54 | AS4 | A0 | C1 | 1 | DMF als Losemittel | 81 | ESI: $(M+H)^+$ = 739/41/43 ($Br_2$) | 0.5 | FM1 | (KBr): C=O 1620.1; 1697.3 | |
| 92 | N57 | AS4 | A0 | C1 | 1 | DMF als Losemittel | 60 | ESI: $(M+H)^+$ = 712/4/6 ($Br_2$) | 0.6 | FM1 | (KBr): C=O 1597; 1635.5 | |
| 93 | N47 | AS4 | A0 | C1 | 1 | | 23 | ESI: $(M+H)^+$ = 636/8/40 ($Br_2$) | 0.4 | FM1 | (KBr): C=O 1622; 1675 | |
| 94 | N45 | AS4 | A0 | C1 | 1 | | 45 | ESI: $(M+H)^+$ = 739/41/43 ($Br_2$) | 0.6 | FM1 | (KBr): C=O 1598.9; 1620.1 | |
| 95 | N57 | AS1 | A0 | C1 | 1 | DMF als Losemittel | 13 | ESI: $(M+H)^+$ = 713/5/7 ($Br_2$) | 0.2 | FM 1 | (KBr): C=O 1637.5 | |
| 96 | N53 | AS1 | A0 | C1 | 1 | DMF als Losemittel | 15 | ESI: $(M+H)^+$ = 740/2/4 ($Br_2$) | 0.2 | FM 1 | (KBr): C=O 1633.6, 1687.6 | |
| 97 | N54 | AS1 | A0 | C1 | 1 | DMF als Losemittel | 31 | ESI: $(M+H)^+$ = 740/2/4 ($Br_2$) | 0.2 | FM 1 | (KBr): C=O 1635.5, 1695.3 | |
| 107 | N59 | AS4 | A0 | C1 | 1 | | 76 | ESI: $(M+H)^+$ = 694/6/8 ($Br_2$) | 0.7 | FM 1 | (KBr): C=O 1597; 1635.5 | |

(fortgesetzt)

| Lfd. Nr. | RCO | $R^2$ | A | $NR^3R^4$ | n | Anmerkunaen | % Ausbeute | MS | $R_f$ | Fließmittel | IR [$cm^{-1}$] | Fp. (°C) |
|---|---|---|---|---|---|---|---|---|---|---|---|---|
| 108 | N45 | AS1 | A0 | C1 | 1 | | 37 | ESI: $(M+H)^+$ = 740/2/4 ($Br_2$) | 0.3 | FM 1 | (KBr): C=O 1633.6, 1708.8 | |
| 109 | N59 | AS1 | A0 | C1 | 1 | | 30 | ESI: $(M+H)^+$ = 695/7/9 ($Br_2$) | 0.3 | FM 1 | (KBr): C=O 1647.4 | |
| 116 | N60 | AS4 | A0 | C1 | 1 | DMF als Losemittel | 80 | ESI: $(M+H)^+$ = 753/5/7 ($Br_2$) | 0.6 | FM 1 | (KBr): C=O 1623.7, 1676.1; 1712.7 | |
| 117 | N60 | AS1 | A0 | C1 | 1 | DMF als Losemittel | 50 | ESI: $(M+H)^+$ = 754/6/8 ($Br_2$) | 0.4 | FM 1 | (KBr): C=O 1617, 1650, 1670, 1712.7 | |
| 118 | N47 | AS1 | A0 | C1 | 1 | | 29 | ESI: $(M+H)^+$ = 637/9/41 ($Br_2$) | 0.1 | FM 1 | (KBr): C=O 1639.4 | |
| 121 | N61 | AS1 | A0 | C1 | 1 | | 12.4 | ESI: $(M+H)^+$ = 727/9/31 ($Br_2$) | 0.2 | FM 1 | (KBr): C=O 1635.5, 1705 | |
| 122 | N61 | AS4 | A0 | C1 | 1 | | 42 | ESI: $(M+H)^+$ = 726/8/30 ($Br_2$) | 0.6 | FM 1 | (KBr): C=O 1620.1; 1706.9 | |
| 125 | N15 | AS7 | A0 | C1 | 1 | $NEt_3$ als Base | 4 4 | ESI: $(M+H)^+$ = 598 | 0.6 | FM 1 | (KBr): C=O 1708.8 | |
| 126 | N15 | AS7 | A0 | C4 | 1 | $NEt_3$ als Base | 57 | ESI: $(M+H)^+$ = 597 | 0.6 | FM 1 | (KBr): C=O 1622, 1708.8 | 188.0 |
| 127 | N15 | AS7 | A0 | C8 | 1 | $NEt_3$ als Base | 16 | ESI: $(M+H)^+$ = 603 | 0.6 | FM 1 | (KBr): C=O 1622: 1697.3 | 168-170 |
| 137 | N94 | AS4 | A0 | C4 | 1 | | 42 | ESI: $(M+H)^+$ = 708/10/12 ($Br_2$) | 0.8 | FM1 | (KBr): C=O 1618 | |

(fortgesetzt)

| Lfd. Nr. | RCO | $R^2$ | A | $NR^3R^4$ | n | Anmerkunaen | % Ausbeute | MS | $R_f$ | Fließmittel | IR [$cm^{-1}$] | Fp. (°C) |
|---|---|---|---|---|---|---|---|---|---|---|---|---|
| 138 | N95 | AS4 | A0 | C8 | 1 | $NEt_3$ als Base | 29 | ESI: $(M+H)^+$ = 743/5/7 ($Br_2$) | 0.8 | FM1 | (KBr): C=O 1713 | |
| 139 | N61 | AS1 | A3 | C1 | 1 | | 41 | ESI: $(M+H)^+$ = 955/7/9 ($Br_2$) | 0.4 | FM1 | (KBr): C=O 1640, 1709 | |
| 140 | N60 | AS1 | A3 | C1 | 1 | | 66 | ESI: $(M+H)^+$ = 982/4/6 ($Br_2$) | 0.5 | FM1 | (KBr): C=O 1645; 1712 | |
| 143 | N66 | AS1 | A0 | C4 | 1 | DMF als Losemittel | 69 | ESI: $(M+H)^+$ = 739/41/43 ($Br_2$) | 0.4 | FM1 | (KBr): C=O 1624, 1659 | |
| 144 | N96 | AS1 | A0 | C4 | 1 | | 54 | ESI: $(M+H)^+$ = 725/7/9 ($Br_2$) | 0.54 | FM1 | (KBr): C=O 1616 | |
| 145 | N61 | AS1 | A0 | C4 | 1 | | 48 | ESI: $(M+H)^+$ = 724/6/8 ($Br_2$) | 0.6 | FM1 | (KBr): C=O 1624; 1709 | |
| 146 | N15 | AS14 | A0 | C1 | 1 | DMF als Losemittel | 53 | ESI: $(M+H)^+$ = 555 | 0.15 | FM1 | (KBr): C=O 1636; 1701 | |
| 147 | N61 | AS4 | A0 | C11 | 1 | | 30 | ESI: $(M+H)^+$ = 746/48/50 ($Br_2$) | 0.7 | FM1 | (KBr): C=O 1620; 1713 | |
| 148 | N66 | AS1 | A0 | C8 | 1 | THF/DMF als Losemittel | 58 | ESI: $(M+H)^+$ = 745/7/9 ($Br_2$) | 0.68 | FM1 | (KBr): C=O 1628, 1663 | |
| 149 | N69 | AS1 | A0 | C4 | 1 | THF/DMF als Losemittel | 61 | ESI: $(M+H)^+$ = 739/41/43 ($Br_2$) | 0.68 | FM1 | (KBr): C=O 1624.1675 | |

(fortgesetzt)

| Lfd. Nr. | RCO | $R^2$ | A | $NR^3R^4$ | n | Anmerkunaen | % Ausbeute | MS | $R_f$ | Fließmittel | IR [$cm^{-1}$] | Fp. (°C) |
|---|---|---|---|---|---|---|---|---|---|---|---|---|
| 150 | N97 | AS1 | A0 | C4 | 1 | THF/DMSO als Losemittel | 32 | ESI: $(M+H)^+$ = 783/85/87 ($Br_2$) | 0.4 | FM1 | (KBr): C=O 1709 | |
| 152 | N71 | AS1 | A0 | C4 | 1 | | 40 | ESI: $(M+H)^+$ = 751/53/55 ($Br_2$) | 0.68 | FM1 | (KBr): C=O 1622; 1684 | |
| 153 | N65 | AS1 | A0 | C4 | 1 | | 51 | ESI: $(M+H)^+$ = 751/53/55 ($Br_2$) | 0.68 | FM1 | (KBr): C=O 1626: 1678 | |
| | N66 | AS1 | A3 | C1 | 1 | | 37 | | | | | |
| 225 | N66 | AS1 | A0 | C1 | 1 | THF/DMF als Losemittel | 48 | ESI: $(M+H)^+$ = 740/42/44 ($Br_2$) | 0.35 | FM1 | (KBr): C=O 1650; 1670 | |
| 226 | N66 | AS4 | A0 | C8 | 1 | THF/DMF als Losemittel | 88 | ESI: $(M+H)^+$ = 744/6/8 ($Br_2$) | 0.6 | FM1 | (KBr): C=O 1618; 1661 | |
| 227 | N69 | AS4 | A0 | C8 | 1 | THF/DMF als Losemittel | 72 | ESI: $(M+H)^+$ = 744/6/8 ($Br_2$) | 0.6 | FM1 | (KBr): C=O 1618, 1680 | |
| 228 | N69 | AS1 | A0 | C8 | 1 | THF/DMF als Losemrttel | 69 | ESI: $(M+H)^+$ = 745/7/9 ($Br_2$) | 0.45 | FM1 | (KBr): C=O 1628 | |
| 229 | N70 | AS1 | A0 | C4 | 1 | THF/DMF als Losemittel | 39 | ESI: $(M+H)^+$ = 727/29/31 ($Br_2$) | 0.3 | FM1 | (KBr): C=O 1730 | |
| 230 | N66 | AS4 | A0 | C20 | 1 | | 49 | ESI: $(M+H)^+$ = 758/60/62 ($Br_2$) | 0.55 | FM1 | (KBr): C=O 1614 | |

(fortgesetzt)

| Lfd. Nr. | RCO | $R^2$ | A | $NR^3R^4$ | n | Anmerkunaen | % Ausbeute | MS | $R_f$ | Fließmittel | IR [$cm^{-1}$] | Fp. (°C) |
|---|---|---|---|---|---|---|---|---|---|---|---|---|
| 231 | N99 | AS4 | A0 | C8 | 1 | THF/DMF als Losemittel | 68 | ESI: M+H)$^+$ = 758/60/62 ($Br_2$) | 0.6 | FM1 | (KBr): C=O 1624 | |
| 239 | N71 | AS1 | A0 | C8 | 1 | THF/DMF als Losemittel | 59 | ESI: (M+H)$^+$ = 757/59/61 ($Br_2$) | 0.45 | FM1 | (KBr): C=O 1626; 1680 | |
| 240 | N71 | AS4 | A0 | C11 | 1 | | 35 | ESI: (M+H)$^+$ = 771/3/5 ($Br_2$) | 0.68 | FM1 | (KBr): C=O 1615; 1684 | |
| 241 | N71 | AS4 | A0 | C8 | 1 | | 88 | ESI: (M+H)$^+$ = 756/58/60 ($Br_2$) | 0.68 | FM1 | (KBr): C=O 1620; 1682 | |
| 242 | N71 | AS4 | A0 | C1 | 1 | | 40 | ESI: (M+H)$^+$ = 751/3/5 ($Br_2$) | 0.64 | FM1 | (KBr): C=O 1615; 1684 | |
| 243 | N71 | AS4 | A0 | C20 | 1 | | 38 | ESI: (M+H)$^+$ = 770/2/4 ($Br_2$) | 0.65 | FM1 | (KBr): C=O 1618; 1684 | |
| 244 | N71 | AS1 | A0 | C11 | 1 | | 36 | ESI: (M+H)$^+$ = 772/4/6 ($Br_2$) | 0.35 | FM1 | (KBr): C=O 1622: 1684 | |
| | N5 | AS1 | A3 | C1 | 1 | $NEt_3$ als Base | 24 | ESI: (M+H)$^+$ = 890/2/4 ($Br_2$) | 0.06 | FM1 | (KBr): C=O 1641.3 | |
| | N10 | AS1 | A3 | C1 | 1 | $NEt_3$ als Base | 55 | ESI: (M+H)$^+$ = 874/6/8 ($Br_2$) | 0.38 | FM1 | (KBr): C=O 1641.3 | |
| | N12 | AS1 | A3 | C1 | 1 | $NEt_3$ als Base | 35480 | ESI: (M+H)$^+$ = 902/4/6 ($Br_2$) | 0.43 | FM1 | (KBr): C=O 1639.4 | |

(fortgesetzt)

| Lfd. Nr. | RCO | $R^2$ | A | $NR^3R^4$ | n | Anmerkunaen | % Ausbeute | MS | $R_f$ | Fließmittel | IR [$cm^{-1}$] | Fp. (°C) |
|---|---|---|---|---|---|---|---|---|---|---|---|---|
| | N22 | AS1 | A3 | C1 | 1 | $NEt_3$ als Base | 35.5 | | 0.5 | FM1 | | |
| | N23 | AS1 | A3 | C1 | 1 | $NEt_3$ als Base | 31 | | 0.5 | FM1 | | |
| | N24 | AS1 | A3 | C1 | 1 | $NEt_3$ als Base | 35607 | | 0.5 | FM1 | | |
| | N46 | AS1 | A3 | C1 | 1 | $NEt_3$ als Base | 36.2 | | 0.5 | FM1 | (KBr): C=O 1641.3 | |
| 83 | N15 | AS1 | A0 | C1 | 1 | | 36.7 | ESI: $(M+H)^+$ = 726/28/30 ($Br_2$) | 0.62 | FM2 | (KBr): C=O 1641.3; 1695.3 | |
| 84 | N15 | AS1 | A0 | C4 | 1 | | 36.3 | ESI: $(M+H)^+$ = 725/7/9 ($Br_2$), $(M+Na)^+$ = 747/49/51 ($Br_2$) | 0.69 | FM2 | (KBr): C=O 1624.0, 1699.2 | |
| 88 | N55 | AS4 | A0 | C1 | 1 | | 93.6 | ESI: $(M+H)^+$ = 793/5/7/9 ($Br_2$,$Cl_2$) | 0.75 | FM3 | (KBr): C=O 1641.3, 1708.8 | |
| 89 | N56 | AS4 | A0 | C1 | 1 | | 30 | ESI: $(M+H)^+$ = 797/799/801 ($Br_2$) | 0.81 | FM1 | (KBr): C=O 1641.3, 1697.3, 1749.3 | |
| 136 | N15 | AS9 | A0 | C1 | 1 | | 14.6 | ESI: $(M+H)^+$ = 570 | 0.55 | FM3 | (KBr): C=O 1635.5, 1701.1 | |
| 91 | N64 | AS4 | A0 | C1 | 1 | | 11.7 | ESI: $(M+H)^+$ = 731/733/735 | 0.62 | FM3 | (KBr)· C=O 1625.9, 1685.7 | |
| | N16 | AS1 | A3 | C5 | 1 | saulenchromatographische Reingung, Kieselgel/ FM4 | 74 | ESI: $(M+H)^+$ = 974/976/978 ($Br_2$) | 0.48 | FM4 | (KBr): C=O 1685.7; 1635.5 | |

(fortgesetzt)

| Lfd. Nr. | RCO | $R^2$ | A | $NR^3R^4$ | n | Anmerkunaen | % Ausbeute | MS | $R_f$ | Fließmittel | IR [$cm^{-1}$] | Fp. (°C) |
|---|---|---|---|---|---|---|---|---|---|---|---|---|
| 155 | N15 | AS1 | A0 | C3 | 1 | | 34 | ESI: $(M+H)^+$ = 743/745/747 ($Br_2$) | 0.45 | FM1 | (KBr): C=O 1626; 1695 | 165-9 |
| 156 | N15 | AS1 | A0 | C19 | 1 | | 40 | ESI: $(M+H)^+$ = 743/5/7 ($Br_2$) | 0.47 | FM1 | (KBr): C=O 1624; 1695 | 155-9 |
| 157 | N15 | AS4 | A0 | C19 | 1 | | 54 | ESI: $(M+H)^+$ = 742/4/6 ($Br_2$) | 0.79 | FM1 | (KBr): C=O 1616: 1697 | 151-4 |
| 158 | N79 | AS4 | A0 | C1 | 1 | | 15 | ESI: $(M+H)^+$ = 727/729/731 ($Br_2$) | 0.63 | FM1 | (KBr): C=O 1616. 1695; 1732 | 132-5 |
| 159 | N77 | AS4 | A0 | C8 | 1 | | 34 | ESI: $(M+H)^+$ = 732/4/6 ($Br_2$) | 0.63 | FM1 | (KBr): C=O 1632 | |
| 160 | N73 | AS1 | A0 | C4 | 1 | | 12 | ESI: $(M+H)^+$ = 649/651/653 ($Br_2$) | 0.14 | FM1 | (KBr): C=O 1626 | |
| 170 | N15 | AS4 | A0 | C37 | 1 | | 62 | ESI: $(M+H)^+$ = 725/7/9 ($Br_2$) | 0.45 | FM1 | (KBr): C=O 1620 1701 | 165-70 |
| 171 | N79 | AS1 | A0 | C1 | 1 | | 60 | ESI: $(M+H)^+$ = 728/30/32 ($Br_2$) | 0.21 | FM1 | (KBr): C=O 1637.5 | 193-7 |
| 172 | N79 | AS1 | A0 | C8 | 1 | | 27 | ESI: $(M+H)^+$ = 733/5/7 ($Br_2$) | 0.32 | FM1 | (KBr): C=O 1622 | 163-9 |

(fortgesetzt)

| Lfd. Nr. | RCO | $R^2$ | A | $NR^3R^4$ | n | Anmerkunaen | % Ausbeute | MS | $R_f$ | Fließmittel | IR [$cm^{-1}$] | Fp. (°C) |
|---|---|---|---|---|---|---|---|---|---|---|---|---|
| 185 | N77 | AS4 | A0 | C4 | 1 | | 66 | ESI: $(M+H)^+$ = 726/28/30 ($Br_2$) | 0.49 | FM1 | (KBr): C=O 1624 | |
| 186 | N77 | AS4 | A0 | C1 | 1 | | 76 | ESI: $(M+H)^+$ = 727/29/31 ($Br_2$) | 0.63 | FM1 | (KBr): C=O 1635.5 | |
| 187 | N77 | AS1 | A0 | C4 | 1 | | 67 | ESI: $(M+H)^+$ = 727/29/31 ($Br_2$) | 0.33 | FM1 | (KBr): C=O 1627.8 | |
| 188 | N78 | AS1 | A3 | C1 | 1 | | 63 | ESI: $(M+H)^+$ = 955/7/9 ($Br_2$) | 0.45 | FM1 | (KBr): C=O 1637.5; 1774.4, 1701 | |
| 189 | N103 | AS4 | A0 | C8 | 1 | | 50 | ESI: $(M+H)^+$ = 713/5/7 ($Br_2$) | 0.71 | FM1 | (KBr): C=O 1616.3 | |
| 192 | N77 | AS1 | A0 | C1 | 1 | | 47 | ESI: $(M+H)^+$ = 728/30/32 ($Br_2$) | 0.2 | FM1 | (KBr): C=O 1643.3 | |
| 247 | N15 | AS10 | A3 | C4 | 1 | | 60 | ESI: $(M+H)^+$ = 937/39/41 ($Br_2$) | 0.6 | FM1 | (KBr): C=O 1639.4, 1701 | |
| 249 | N15 | AS4 | A0 | C22 | 1 | | 52 | ESI: $(M+H)^+$ = 744/6/8 ($Br_2$) | 0.59 | FM1 | (KBr): C=O 1695.3 | |
| 161 | N15 | AS4 | A0 | C21 | 1 | | 32 | ESI: $(M+H)^+$ = 726/28/30 ($Br_2$) | 0.61 | FM1 | (KBr): C=O 1622: 1701 | 163-7 |
| 162 | N78 | AS1 | A0 | C4 | 1 | | 15 | ESI: $(M+H)^+$ = 726/28/30 ($Br_2$) | 0.48 | FM1 | (KBr): C=O 1624, 1772.5 | |

(fortgesetzt)

| Lfd. Nr. | RCO | $R^2$ | A | $NR^3R^4$ | n | Anmerkunaen | % Ausbeute | MS | $R_f$ | Fließmittel | IR [$cm^{-1}$] | Fp. (°C) |
|---|---|---|---|---|---|---|---|---|---|---|---|---|
| 163 | N73 | AS1 | A0 | C1 | 1 | | 43 | ESI: $(M+H)^+$ = 752/4/6 ($Br_2$) | 0.07 | FM1 | (KBr): C=O 1637.5 | |
| 164 | N79 | AS4 | A0 | C8 | 1 | | 48 | ESI: $(M+H)^+$ = 732/4/6 ($Br_2$) | 0.6 | FM1 | (KBr): C=O 1616.3 | 127-32 |
| 165 | N15 | AS1 | A0 | C21 | 1 | | 27 | ESI: $(M+H)^+$ = 727/29/31 ($Br_2$) | 0.26 | FM1 | (KBr): C=O 1697.3 | 184-9 |
| 166 | N76 | AS1 | A0 | C4 | 1 | | 17 | ESI: $(M+H)^+$ = 665/7/9 ($Br_2$) | 0.23 | FM1 | (KBr): C=O 1616; 1734 | |
| 167 | N75 | AS1 | A0 | C4 | 1 | | 20 | ESI: $(M+H)^+$ = 665/7/9 ($Br_2$) | 0.18 | FM1 | (KBr): C=O 1624 | |
| 168 | N73 | AS1 | A3 | C4 | 1 | | 39 | ESI: $(M+H)^+$ = 879/81/83 ($Br_2$) | 0.15 | FM1 | (KBr): C=O 1631.7 | |
| 169 | N15 | AS1 | A0 | C37 | 1 | | 17 | ESI: $(M+H)^+$ = 726/28/30 ($Br_2$) | 0.31 | FM1 | (KBr): C=O 1627.8, 1697.3 | 156-61 |
| 173 | N15 | AS10 | A0 | C4 | 1 | | 66 | ESI: $(M+H)^+$ = 709/11/13 ($Br_2$) | 0.68 | FM1 | (KBr): C=O 1627.8, 1656.8, 1695.3 | 283-4 |
| 174 | N15 | AS10 | A0 | C1 | 1 | | 42 | ESI: $(M+H)^+$ = 710/2/4 ($Br_2$) | 0.61 | FM1 | (KBr): C=O 1706.9 | 266-9 |
| 175 | N77 | AS1 | A0 | C8 | 1 | | 36 | ESI: $(M+H)^+$ = 733/5/7 ($Br_2$) | 0.24 | FM1 | (KBr): C=O 1641.3 | |

(fortgesetzt)

| Lfd. Nr. | RCO | $R^2$ | A | $NR^3R^4$ | n | Anmerkunaen | % Ausbeute | MS | $R_f$ | Fließmittel | IR [$cm^{-1}$] | Fp. (°C) |
|---|---|---|---|---|---|---|---|---|---|---|---|---|
| 176 | N76 | AS1 | A3 | C4 | 1 | | 47 | ESI: $(M+H)^+$ = 893/5/7 ($Br_2$) | 0.21 | FM1 | (KBr): C=O 1635.5 | |
| 177 | N75 | AS1 | A3 | C4 | 1 | | 53 | ESI: $(M+H)^+$ = 893/5/7 ($Br_2$) | 0.14 | FM1 | (KBr): C=O 1637.5 | |
| 180 | N74 | AS1 | A3 | C4 | 1 | | 44 | ESI: $(M+H)^+$ = 907/9/11 ($Br_2$) | 0.26 | FM1 | (KBr): C=O 1631.7; 1689.5 | |
| 190 | N15 | AS1 | A3 | C18 | 1 | | 44 | ESI: $(M+H)^+$ = 985/7/9 ($Br_2$) | 0.38 | FM1 | (KBr): C=O 1635.5; 1695.3 | |
| 191 | N15 | AS10 | A3 | C1 | 1 | | 64 | ESI: $(M+H)^+$ = 938/40/42 ($Br_2$) | 0.56 | FM1 | (KBr): C=O 1645.2; 1701 | |
| | N15 | AS10 | A3 | C4 | 1 | | 91 | ESI: $(M+H)^+$ = 937/39/41 ($Br_2$) | | | (KBr): C=O 1643.3, 1701 | |
| | N15 | AS10 | A3 | C1 | 1 | | 64 | ESI: $(M+H)^+$ = 938/40/42 ($Br_2$) | | | (KBr): C=O 1645; 1701 | |
| 254 | N77 | AS1 | A3 | C1 | 1 | | 37 | ESI: $(M+H)^+$ = 956/8/60 ($Br_2$) | 0.3 | FM1 | (KBr): C=O 1641 | |
| | N15 | AS4 | A3 | C18 | 1 | | 90 | ESI: $(M+H)^+$ = 984/6/8 ($Br_2$) | | | (KBr): C=O 1641.3; 1699 | |
| 257 | N15 | AS4 | A5 | C1 | 1 | | 17 | ESI: $(M+H)^+$ = 782/4/6 ($Br_2$) | 0.53 | FM1 | (KBr): C=O 1643; 1697 | |

(fortgesetzt)

| Lfd. Nr. | RCO | $R^2$ | A | $NR^3R^4$ | n | Anmerkunaen | % Ausbeute | MS | $R_f$ | Fließmittel | IR [$cm^{-1}$] | Fp. (°C) |
|---|---|---|---|---|---|---|---|---|---|---|---|---|
| 258 | N100 | AS4 | A0 | C1 | 1 | | 69 | ESI: $(M+H)^+$ = 755/7/9 ($Br_2$) | 0.54 | FM1 | (KBr): C=O 1627.8; 1705 | |
| 259 | N100 | AS4 | A0 | C8 | 1 | | 70 | ESI: $(M+H)^+$ = 760/2/4 ($Br_2$) | 0.54 | FM1 | (KBr): C=O 1695 | |
| 260 | N15 | AS4 | A0 | C23 | 1 | | 39 | ESI: $(M+H)^+$ = 796/798/800 ($Br_2$) | 0.36 | FM1 | (KBr): C=O 1635.5; 1699 | |
| 261 | N15 | AS4 | A10 | C1 | 1 | | 26 | ESI: $(M+H)^+$ = 796/798/800 ($Br_2$) | 0.38 | FM1 | (KBr): C=O 1631.4; 1701 | |
| 265 | N15 | AS4 | A5 | C8 | 1 | | 20 | ESI: $(M+H)^+$ = 787/9/91 ($Br_2$) | 0.41 | FM1 | (KBr): C=O 1635.5; 1697 | |
| 266 | N15 | AS4 | A6 | C1 | 1 | | 24 | ESI: $(M+H)^+$ = 796/798/800 ($Br_2$) | 0.43 | FM1 | (KBr): C=O 1647; 1689.5 | |
| 262 | N80 | AS4 | A0 | C1 | 1 | | 25 | ESI: $(M+H)^+$ = 803/5/7 ($Br_2$) | 0.54 | FM1 | (KBr): C=O 1637.5 | |
| 263 | N15 | AS15 | A0 | C8 | 1 | | 64 | ESI: $(M+H)^+$ = 565 | 0.43 | FM1 | (KBr): C=O 1627.8; 1707 | |
| 264 | N15 | AS4 | A0 | C24 | 1 | | 62 | ESI: $(M+H)^+$ = 733/5/7 ($Br_2$) | 0.4 | FM1 | (KBr): C=O 1622; 1701 | |

(fortgesetzt)

| Lfd. Nr. | RCO | $R^2$ | A | $NR^3R^4$ | n | Anmerkunaen | % Ausbeute | MS | $R_f$ | Fließmittel | IR [$cm^{-1}$] | Fp. (°C) |
|---|---|---|---|---|---|---|---|---|---|---|---|---|
| 267 | N81 | AS4 | A3 | C8 | 1 | | 46 | ESI: $(M+H)^+$ = 974/6/8 ($Br_2$) | 0.55 | FM1 | (KBr): C=O 1641; 1707 | |
| 268 | N15 | AS4 | A6 | C8 | 1 | | 27 | ESI: $(M+H)^+$ = 801/3/5 ($Br_2$) | 0.5 | FM1 | (KBr): C=O 1633.6, 1697 | |
| 269 | N82 | AS4 | A0 | C8 | 1 | | 86 | ESI: $(M+H)^+$ = 742/4/6 ($Br_2$) | 0.66 | FM1 | (KBr): C=O 1620; 1649 | |
| 270 | N82 | AS4 | A0 | C1 | 1 | | 56 | ESI: $(M+H)^+$ = 737/39/41 ($Br_2$) | 0.59 | FM1 | (KBr): C=O 1641 | |
| 272 | N15 | AS11 | A0 | C8 | 1 | | 76 | ESI: $(M+H)^+$ = 698 | 0.6 | FM1 | (KBr): C=O 1627.8, 1714.6 | |
| 273 | N15 | AS4 | A10 | C8 | 1 | | 68 | ESI: $(M+H)^+$ = 801/3/5 ($Br_2$) | 0.52 | FM1 | (KBr): C=O 1637.5; 1701 | |
| 274 | N102 | AS4 | A0 | C1 | 1 | | 76 | ESI: $(M+H)^+$ = 738/40/42 ($Br_2$) | 0.56 | FM1 | (KBr): C=O 1664.5 | |
| 275 | N102 | AS4 | A0 | C8 | 1 | | 55 | ESI: $(M+H)^+$ = 743/5/7 ($Br_2$) | 0.59 | FM1 | (KBr): C=O 1618; 1664.5 | |
| 276 | N83 | AS4 | A0 | C1 | 1 | | 30 | ESI: $(M+H)^+$ = 745/7/9 ($Br_2$) | 0.48 | FM1 | (KBr): C=O 1633.6 | |
| 277 | N84 | AS4 | A0 | C8 | 1 | | 63 | ESI: $(M+H)^+$ = 744/6/8 ($Br_2$) | 0.54 | FM1 | (KBr): C=O 1616; 1691.5 | |

(fortgesetzt)

| Lfd. Nr. | RCO | $R^2$ | A | $NR^3R^4$ | n | Anmerkunaen | % Ausbeute | MS | $R_f$ | Fließmittel | IR [$cm^{-1}$] | Fp. (°C) |
|---|---|---|---|---|---|---|---|---|---|---|---|---|
| 278 | N84 | AS4 | A3 | C4 | 1 | 88 | ESI: $(M+H)^+$ = 966/8/70 ($Br_2$) | 0.53 | FM1 | (KBr): C=O 1633.6; 1691.5 | | |
| 279 | N15 | AS4 | A0 | C26 | 1 | 75 | ESI: $(M+H)^+$ = 732/4/6 ($Br_2$) | 0.44 | FM1 | (KBr): C=O 1618; 1709 | | |
| 281 | N15 | AS12 | A0 | C8 | 1 | 21 | ESI: $(M+H)^+$ = 598 | 0.42 | FM1 | (KBr): C=O 1697 | | |
| 282 | N66 | AS1 | A0 | C18 | 1 | 19 | ESI: $(M+H)^+$ = 770/2/4 ($Br_2$) | 0.51 | FM1 | (KBr): C=O 1624; 1660.6, 1734 | | |
| 284 | N66 | AS1 | A0 | C18 | 1 | 29 | ESI: $(M+H)^+$ = 771/3/5 ($Br_2$) | 0.3 | FM1 | (KBr): C=O 1630, 1655 | | |
| 314 | N93 | AS4 | A0 | C8 | 1 | 81 | ESI: $(M+H)^+$ = 794/6/8 ($Br_2$) | 0.5 | FM1 | (KBr): C=O 1618; 1701 | | |
| 315 | N93 | AS4 | A0 | C1 | 1 | 77 | ESI: $(M+H)^+$ = 789/91/93 ($Br_2$) | 0.49 | FM1 | (KBr): C=O 1627.6; 1705 | | |
| 316 | N65 | AS1 | A0 | C18 | 1 | 15 | ESI: $(M+H)^+$ = 783/5/7 ($Br_2$) | 0.3 | FM1 | (KBr): C=O 1624, 1681.8 | | |
| 317 | N66 | AS4 | A0 | C3 | 1 | 51 | ESI: $(M+H)^+$ = 778/80/82 ($Br_2$) | 0.62 | FM1 | (KBr): C=O 1627.6, 1662.5 | | |
| 318 | N66 | AS1 | A0 | C3 | 1 | 40 | ESI: $(M+H)^+$ = 557/559/561 ($Br_2$) | 0.41 | FM1 | (KBr): C=O 1659 | | |

(fortgesetzt)

| Lfd. Nr. | RCO | $R^2$ | A | $NR^3R^4$ | n | Anmerkunaen | % Ausbeute | MS | $R_f$ | Fließmittel | IR [$cm^{-1}$] | Fp. (°C) |
|---|---|---|---|---|---|---|---|---|---|---|---|---|
| 319 | N66 | AS4 | A0 | C19 | 1 | 55 | ESI: $(M+H)^+$ = 778/780/782 ($Br_2$) | 0.68 | FM1 | (KBr): C=O 1664.5 | | |
| 320 | N65 | AS4 | A0 | C19 | 1 | 61 | ESI: $(M+H)^+$ = 768/70/72 ($Br_2$) | 0.62 | FM1 | (KBr): C=O 1618; 1682 | | |
| 321 | N93 | AS1 | A0 | C4 | 1 | 29 | ESI: $(M+H)^+$ = 789/91/93 ($Br_2$) | 0.35 | FM1 | (KBr): C=O 1622; 1705 | | |
| | N15 | AS1 | A3 | C5 | 1 | 47 | | 0.32 | FM1 | | | |
| | N19 | AS1 | A3 | C1 | 1 | 80 | ESI: $(M+H)^+$ = 933/5/7 ($Br_2$) | | | (KBr): C=O 1641.3 | | |

Referenzbeispiel 5

Herstellung von Verbindungen der allgemeinen Formel:

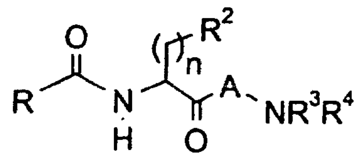

1-[$N^2$-[N-(4-Phenyl-1-piperazinyl)carbonyl]-3,5-dibrom-D-tyrosyl]-L-lysyl]-4-(4-pyridinyl)-piperazin (Lfd. Nr. 17)

Zu der Lösung von 800 mg (0.86 mmol) 1-[$N^2$-[N-(4-Phenyl-1-piperazinyl)carbonyl]-3,5-dibrom-D-tyrosyl]-$N^6$-[(1,1-dimethylethoxy)carbonyl]-L-lysyl]-4-(4-pyridinyl)-piperazin in Methanol wurden 2 ml mit Chlorwasserstoff gesättigten Methanols zugegeben und die Mischung über Nacht bei Raumtemperatur gerührt. Das Reaktionsgemisch wurde bis zur vollständigen Ausfällung des Hydrochlorids mit Essigester versetzt und der ausgefallene Niederschlag abfiltriert. Nach Waschen des Niederschlages mit Ether wurde säulenchromatographisch (MN-Kieselgel 60, Macherey-Nagel, 70-230 mesh ASTM, Fließmittel: Essigester/Methanol/konz. wässeriges Ammoniak = 5/5/0.5 (v/v/v)) gereinigt. Man erhielt 0.38 g (55 % der Theorie) eines amorphen Feststoffes.
IR (KBr): 1639 $cm^{-1}$ (C=O)
$R_f$: 0.55 (FM2)
ESI-MS: $(M+H)^+$ = 799/801/803 ($Br_2$)
$(M+2H)^{++}$ = 400/401/402 ($Br_2$)

Analog wurden hergestellt (jeweils n=1):

| Lfd. Nr. | RCO | $R^2$ | A | $NR^3R^4$ | n | % Ausbeute | MS | $R_f$ | Fließmittel | IR [$cm^{-1}$] |
|---|---|---|---|---|---|---|---|---|---|---|
| 11 | N8 | AS1 | A1 | C1 | 1 | 70 | ESI: $(M+H)^+$ = 758/60/62 ($Br_2$) | 0.43 | FM2 | (KBr): C=O 1656.8 |
| 12 | N9 | AS1 | A1 | C1 | 1 | 60 | ESI: $(M+H)^+$ = 788/90/92 ($Br_2$) | 0.46 | FM2 | (KBr): C=O 1643.3 |
| 8 | N5 | AS1 | A1 | C1 | 1 | 53.7 | ESI: $(M+H)^+$ = 790/2/4 ($Br_2$) | 0.2 | Methanol / Eisessig / Wasser = 9/1/1 (v/v/v) | (KBr): C=O 1641.3 |
| 15 | N11 | AS1 | A1 | C1 | 1 | 56 | ESI: $(M+H)^+$ = 773/5/7 ($Br_2$) | 0.4 | FM2 | |
| 6 | N2 | AS1 | A1 | C11 | 1 | 66.4 | ESI: $(M+H)^+$ = 808/10/12 ($Br_2$) | 0.39 | FM2 | (KBr): C=O 1656.8 |

(fortgesetzt)

| Lfd. Nr. | RCO | $R^2$ | A | $NR^3R^4$ | n | % Ausbeute | MS | $R_f$ | Fließmittel | IR [$cm^{-1}$] |
|---|---|---|---|---|---|---|---|---|---|---|
| 7 | N2 | AS1 | A1 | C2 | 1 | 46.2 | ESI: $(M+H)^+$ = 794/6/8 ($Br_2$) | 0.13 | FM2 | (KBr): C=O 1637.5 |
| 13 | N2 | AS2 | A1 | C1 | 1 | 84.7 | ESI: $(M+H)^+$ = 666 | 0.46 | FM2 | (KBr): C=O 1641.3 |

Beispiel 6

Herstellung von Verbindungen der allgemeinen Formel:

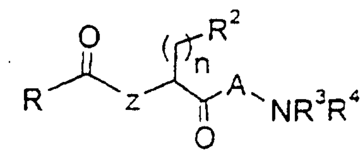

1-[4-Amino-3,5-dibrom-$N^2$-[N-[4-(2-chlorphenyl)-1-piperazinyl]-carbonyl]-D-phenylalanyl]-L-lysyl]-4-(4-pyridinyl)-piperazin-bis-(trifluoracetat)(Lfd. Nr. 61)

Zu einer Mischung aus 0.42 g (0.45 mmol) 1-[4-Amino-3,5-dibrom-$N^2$-[N-[4-(2-chlorphenyl)-1-piperazinyl]carbonyl]-D-phenylalanyl]-N6-[(1.1-dimethylethoxy)carbonyl]-L-lysyl]-4-(4-pyridinyl)-piperazin in 30 ml Methylenchlorid wurden 3 ml Trifluoressigsäure zugegeben. Das Reaktionsgemisch wurde 3 Stunden bei Raumtemperatur gerührt und anschließend im Vakuum eingeengt. Der verbleibende Rückstand wurde mit Ether verrieben und der erhaltene beigefarbene amorphe Feststoff (0.43g; 37% der Theorie) abgenutscht.
IR (KBr): 1643, 1678 $cm^{-1}$ (C=O)
$R_f$: 0.6 (FM1)
ESI-MS: $(M+H)^+$ = 832/834/836/838 ($Br_2$,Cl)

Analog wurden hergestellt:

| Lfd. Nr. | RCO | Z | $R^2$ | A | $NR^3R^4$ | n | Anmerkungen | % Ausbeute | MS | $R_f$ | Fließmittel | IR [$cm^{-1}$] |
|---|---|---|---|---|---|---|---|---|---|---|---|---|
| 21 | N6 | N-H | AS1 | A1 | C4 | 1 | | 65 | ESI: $(M+H)^+$ = 828/30/32 ($Br_2$) | 0.3 | FM1 | (KBr): C=O 1635.5 |
| 22 | N16 | N-H | AS1 | A1 | C1 | 1 | | 98 | ESI: $(M+H)^+$ = 87416/8 ($Br_2$) | 0.2 | FM1 | $(KBr)^+$ C=O 1643.3; 1676 |
| 141 | N61 | N-H | AS1 | A1 | C1 | 1 | | 46 | ESI: $(M+H)^+$ = 855/7/9 ($Br_2$) | 0.1 | FM1 | (KBr): C=O 1634; 1705 |
| 142 | N60 | N-H | AS1 | A1 | C1 | 1 | | 50 | ESI: $(M+H)^+$ = 882/4/6 ($Br_2$) | 0.1 | FM1 | (KBr): C=O 1643; 1711 |
| 154 | N66 | N-H | AS1 | A1 | C1 | 1 | | 60 | ESI: $(M+H)^+$ = 868/70/72 ($Br_2$) | 0.1 | FM1 | (KBr): C=O 1645; 1653 |
| 197 | N15 | N-H | AS1 | A1 | C8 | 1 | | 21 | ESI: $(M+H)^+$ = 859/61/63 ($Br_2$) | 0.18 | FM7 | (KBr): C=O CF3 1180.4; 1134.1 |
| 198 | N51 | N-H | AS1 | A1 | C8 | 1 | | 27 | ESI: $(M+H)^+$ = 829/31/33 ($Br_2$) | 0.22 | FM7 | (KBr): C=O 1676; CN 2221.9; CF3 1203.5; 1180.4; 1132 |
| 218 | N15 | N-H | AS6 | A1 | C1 | 1 | | 25.7 | ESI: $(M+H)^+$ = 776/8 (Br) | 0.45 | FM1 | (KBr): C=O 1695.3; 1635.5 |
| 287 | N15 | N-H | AS1 | A8 | C1 | 1 | | 36.5 | ESI: $(M+H)^+$ = 840/2/4 ($Br_2$) | 0.5 | FM2 | (KBr): C=O 1695.3, 1637.5 |
| 19 | N15 | N-H | AS1 | A1 | C1 | 1 | | 44 | ESI: $(M+H)^+$ = 854/6/8 ($Br_2$) | 0.43 | FM2 | (KBr): C=O 1695.3, 1637.5 |
| 14 | N10 | N-H | AS1 | A1 | C1 | 1 | | 25.5 | ESI: $(M+H)^+$ = 774/6/8 ($Br_2$) | 0.33 | FM2 | (KBr): C=O 1683.8 |
| 16 | N12 | N-H | AS1 | A1 | C1 | 1 | | 64.4 | ESI: $(M+H)^+$ = 802/4/6 ($Br_2$) | 0.55 | FM2 | (KBr): C=O 1639.4 |

(fortgesetzt)

| Lfd. Nr. | RCO | Z | $R^2$ | A | $NR^3R^4$ | n | Anmerkungen | % Ausbeute | MS | $R_f$ | Fließmittel | IR [$cm^{-1}$] |
|---|---|---|---|---|---|---|---|---|---|---|---|---|
| 29 | N22 | N-H | AS1 | A1 | C1 | 1 | | 91.2 | ESI: $(M+H)^+$ = 867/69/71 ($Br_2$) | 0.5 | FM2 | (KBr): -NH- 3427.3. C=O 1643.3, 1678.0 |
| 30 | N23 | N-H | AS1 | A1 | C1 | 1 | | 83.3 | ESI: $(M+H)^+$ = 833/5/7/9 ($Br_2$, Cl) | 0.5 | FM2 | (KBr): C=O 1643.3, 1676.0 |
| 31 | N24 | N-H | AS1 | A1 | C1 | 1 | | 100 | ESI: $(M+H)^+$ = 843/5/7 ($Br_2$) | 0.51 | FM2 | (KBr): C=O 1645.2, 1676.0 |
| 63 | N46 | N-H | AS1 | A1 | C1 | 1 | | 100 | ESI: $(M+H)^+$ = 764/6/8 ($Br_2$) | 0.58 | FM2 | (KBr): C=O 1643.3; 1676.0 |
| 68 | N15 | N-H | AS1 | A1 | C6 | 1 | | 80 | ESI: $(M+H)^+$ = 833/5/7 ($Br_2$) | 0.18 | FM1 | (KBr): C=O 1683.8 |
| 69 | N15 | N-H | AS1 | A1 | C5 | 1 | | 74 | ESI: $(M+H)^+$ = 854/6/8($Br_2$) | 0.18 | FM1 | (KBr): C=O 1683.8; 1639.4 |
| 70 | N45 | N-H | AS1 | A1 | C6 | 1 | | 89 | ESI: $(M+H)^+$ = 897/9/901 ($Br_2$) | 0.2 | FM1 | (KBr): C=O 1695.3; 1676.0 |
| 71 | N16 | N-H | AS1 | A1 | C5 | 1 | | 82 | ESI: $(M+H)^+$ = 874/6/8 ($Br_2$) | 0.22 | FM1 | (KBr): C=O 1678.0; 1639.4 |
| 72 | N15 | N-H | AS5 | A1 | C1 | 1 | | 97 | ESI: $(M+H)^+$ = 838/40/42 ($Br_2$) | 0.16/0.2 | FM1 | (KBr): C=O 1685.7; 1643 3 |
| 77 | N45 | N-H | AS5 | A1 | C1 | 1 | | 66 | ESI: $(M+H)^+$ = 852/4/6 ($Br_2$) | 0.33/0.4 | FM1 | (KBr): C=O 1683.8; 1645.2 NH3 3427.3 |
| 24 | N18 | N-H | AS1 | A1 | C1 | 1 | | 94 | ESI: $(M+H)^+$ = 775/7/9 ($Br_2$) | 0.11 | FM1 | (KBr): C=O 1676.0, 1643 3 |
| 25 | N19 | N-H | AS1 | A1 | C1 | 1 | | 92 | ESI: $(M+H)^+$ = 833/5/7 ($Br_2$) | 0.13 | FM1 | (KBr): C=O 1676.0, 1643.3 |
| 26 | N20 | N-H | AS1 | A1 | C1 | 1 | | 98 | El. $M^+$ = 762/4/6 ($Br_2$) | 0.11 | FM1 | (KBr): C=O 1676.0, 1643.3 |

(fortgesetzt)

| Lfd. Nr. | RCO | Z | $R^2$ | A | $NR^3R^4$ | n | Anmerkungen | % Ausbeute | MS | $R_f$ | Fließmittel | IR [$cm^{-1}$] |
|---|---|---|---|---|---|---|---|---|---|---|---|---|
| 27 | N21 | N-H | AS1 | A1 | C1 | 1 | | 98 | ESI: $(M+H)^+$ = 814/6/8 ($Br_2$) | 0.04 | FM1 | (KBr): C=O 1676.0; 1645.2 |
| 41 | N34 | N-H | AS1 | A1 | C1 | 1 | | 97 | ESI: $(M+H)^+$ = 835/38/40/42 ($Br_3$) | 0.08 | FM1 | (KBr): C=O 1676.0, 1643.3 |
| 42 | N35 | N-H | AS1 | A1 | C1 | 1 | | 83 | ESI: $(M+H)^+$ = 803/5/7($Br_2$) | 0.09 | FM1 | (KBr): C=O 1676.0, 1643.3 |
| 43 | N36 | N-H | AS1 | A1 | C1 | 1 | | 87 | ESI: $(M+H)^+$ = 815/7/9 ($Br_2$) | 0.04 | FM1 | (KBr): C=O 1676.0, 1645.2 |
| 53 | N42 | N-H | AS1 | A1 | C1 | 1 | | 89 | ESI: $(M+H)^+$ = 805/7/9 ($Br_2$) | 0.11 | FM1 | (KBr): C=O 1676.0, 1634.3 |
| 54 | N43 | N-H | AS1 | A1 | C1 | 1 | | 84 | ESI: $(M+H)^+$ = 835/7/9/41 ($Br_3$) | 0.11 | FM1 | (KBr): C=O 1678.0; 1643.3 |
| 55 | N44 | N-H | AS1 | A1 | C1 | 1 | | 95 | ESI: $(M+H)^+$ = 796/8/800 ($Br_2$) | 0.07 | FM1 | (KBr): C=O 1676.0, 1643.3 |
| 67 | N48 | N-H | AS1 | A1 | C1 | 1 | | 90 | ESI: $(M+H)^+$ = 797/99/801 ($Br_2$) | 0.06 | FM1 | (KBr): C=O 1682.9, 1643.3 |
| 184 | N77 | N-H | AS1 | A1 | C4 | 1 | | 88 | ESI: $(M+H)^+$ = 855/7/9 ($Br_2$) | 0.11 | FM1 | (KBr): C=O 1637.5; 1676 |
| 248 | N78 | N-H | AS1 | A1 | C1 | 1 | | 97 | ESI: $(M+H)^+$ = 855/7/9 ($Br_2$) | 0.14 | FM1 | (KBr): C=O 1643.3, 1676; 1772.5 |
| 181 | N75 | N-H | AS1 | A1 | C4 | 1 | | 95 | ESI: $(M+H)^+$ = 793/5/7 ($Br_2$) | 0.04 | FM1 | (KBr): C=O 1637.5; 1676 |
| 182 | N76 | N-H | AS1 | A1 | C4 | 1 | | 93 | ESI: $(M+H)^+$ = 793/5/7 ($Br_2$) | 0.04 | FM1 | (KBr): C=O 1637.5; 1678 |
| 183 | N74 | N-H | AS1 | A1 | C4 | 1 | | 91 | ESI: $(M+H)^+$ = 807/9/11 ($Br_2$) | 0.08 | FM1 | (KBr): C=O 1635.5, 1678 |

(fortgesetzt)

| Lfd. Nr. | RCO | Z | $R^2$ | A | $NR^3R^4$ | n | Anmerkungen | % Ausbeute | MS | $R_f$ | Fließmittel | IR [$cm^{-1}$] |
|---|---|---|---|---|---|---|---|---|---|---|---|---|
| 250 | N15 | N-H | AS1 | A1 | C18 | 1 | | 98 | ESI: $(M+H)^+$ = 885/7/9 ($Br_2$) | 0.14 | FM1 | (KBr): C=O 1633.6; 1680 |
| 251 | N15 | N-H | AS10 | A1 | C4 | 1 | | 84 | ESI: $(M+H)^+$ = 837/39/41 ($Br_2$) | 0.27 | FM1 | (KBr): C=O 1635.5; 1693.4 |
| 252 | N15 | N-H | AS10 | A1 | C4 | 1 | | 87 | ESI: $(M+H)^+$ = 837/39/41 ($Br_2$) | 0.31 | FM1 | (KBr): C=O 1637.5, 1685.7 |
| 253 | N15 | N-H | AS10 | A1 | C1 | 1 | | 82 | ESI: $(M+H)^+$ = 838/40/42 ($Br_2$) | 0.18 | FM1 | (KBr): C=O 1690 |
| 255 | N77 | N-H | AS1 | A1 | C1 | 1 | | 94 | ESI: $(M+H)^+$ = 856/8/60 ($Br_2$) | 0.08 | FM1 | (KBr): C=O 1645; 1676 |
| 256 | N15 | N-H | AS4 | A1 | C18 | 1 | | 74 | ESI: $(M+H)^+$ = 884/6/8($Br_2$) | 0.28 | FM1 | (KBr): C=O 1683.6, 1683.8 |
| 271 | N81 | N-H | AS4 | A1 | C8 | 1 | | 76 | ESI: $(M+H)^+$ = 874/6/8 ($Br_2$) | 0.2 | FM1 | (KBr): C=O 1674 |
| 280 | N84 | N-H | AS4 | A1 | C4 | 1 | | 66 | ESI: $(M+H)^+$ = 866/8/70 ($Br_2$) | 0.26 | FM1 | (KBr): C=O 1635.5, 1685.7 |
| | N15 | N-H | AS1 | A1 | C1 | 0 | | 98 | ESI: $(M+H)^+$ = 840/2/4 ($Br_2$) | 0.2 | ButOH/ AcOH/ H2O = 4/1/1 (v/v/ v) | (KBr): C=O 1643, 1680 |
| 179 | N73 | N-H | AS1 | A1 | C4 | 1 | | 86 | ESI: $(M+H)^+$ = 779/81/83 ($Br_2$) | 0.03 | FM1 | (KBr): C=O 1642.8; 1676 |
| | N66 | N-H | AS4 | A0 | | 1 | | 75 | ESI: $(M+H)^+$ = 744/6/8 ($Br_2$) | 0.3 | FM1 | (KBr): C=O 1620/1666 |
| 516 | N66 | N-H | AS1 | A1 | C1 | 1 | Isomeres zu Lfd. Nr (154) | 82 | | 0.1 | FM1 | |
| 517 | N66 | N-H | AS1 | A1 | C1 | 1 | Isomeres zu Lfd Nr (154) | 80 | | 0.1 | FM1 | |
| 518 | N66 | N-H | AS1 | A1 | C1 | 1 | Isomeres zu Lfd Nr (154) | 89 | | 0.1 | FM1 | |

(fortgesetzt)

| Lfd. Nr. | RCO | Z | $R^2$ | A | $NR^3R^4$ | n | Anmerkungen | % Ausbeute | MS | $R_f$ | Fließmittel | IR [$cm^{-1}$] |
|---|---|---|---|---|---|---|---|---|---|---|---|---|
| 521 | N66 | N-H | AS4 | A0 | C17 | 1 | THF/DMF | 75 | ESI: $(M+H)^+$ = 744/6/8 ($Br_2$) | 0.15 | FM1 | (KBr): C=O 1666/ 1620 |
| 522 | N66 | N-H | AS1 | A0 | C17 | 1 | THF/DMF | 100 | ESI: $(M+H)^+$ = 745/7/9 ($Br_2$) | 0.15 | FM1 | (KBr): C=O 1624/ 1655 |
| 643 | N66 | $CH_2$ | AS21 | A0 | C17 | 1 | | 53 | ESI: $(M+H)^+$ = 640 | 0.35 | FM1 | (KBr): C=O 1635/ 1668 |
| | N66 | $CH_2$ | AS2 | A0 | C17 | 1 | | 100 | EI: $M^+$ = 621 | 0.35 | FM1 | (KBr): C=O 1670 |

Referenzbeispiel 7

Herstellung von Verbindungen der allgemeinen Formel:

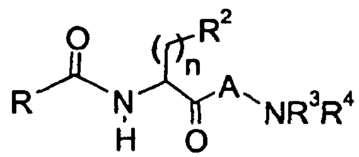

1-[$N^2$-[N-[[[(2-Methoxyphenyl)methyl]amino]carbonyl]-3,5-dibrom-D,L-tyrosyl]-L-lysyl]-4-(4-pyridinyl)-piperazin

Die Mischung aus 910 mg (1.0 mmol) 1-[$N^2$-[N-[[[2-(2-Methoxyphenyl)methyl] amino]carbonyl]-3,5-dibrom-D,L-tyrosyl]-$N^6$-[(phenylmethoxy)carbonyl]-L-lysyl]-4-(4-pyridinyl)-piperazin, 50 ml Eisessig, 25 ml einer 33proz. Lösung von Bromwasserstoff in Eisessig und 2 ml Anisol wurde bei Raumtemperatur über Nacht gerührt. Das Reaktionsgemisch wurde in Diethylether eingerührt und der entstandene klebrige Niederschlag abgenutscht. Das rohe Produkt wurde säulenchromatographisch (MN-Kieselgel 60, Macherey-Nagel, 70-230 mesh ASTM, Fließmittel: Essigester/Methanol/konz. wässeriges Ammoniak = 8/2/0.2 (v/v/v)) gereinigt. Man erhielt 0.37 g (48 % der Theorie) eines amorphen Feststoffes.
IR (KBr): 1630 $cm^{-1}$ (C=O)
ESI-MS: $(M+H)^+$ = 774/776/778 ($Br_2$)
$(M+2H)^{++}$ = 387.7/388.7/389.7 ($Br_2$)
Analog wurden hergestellt (jeweils n = 1):

| Lfd. Nr. | RCO | $R^2$ | A | $NR^3R^4$ | % Ausbeute | MS | $R_f$ | Fließmittel | IR [$cm^{-1}$] |
|---|---|---|---|---|---|---|---|---|---|
| 1 | N1 | AS1 | A1 | C1 | 46.9 | ESI: $(M+H)^+$ = 788/90/92 ($Br_2$) | 0.36 | FM1 | (KBr): C=O 1627.8 |
| 2 | N2 | AS1 | A1 | C1 | 100 | ESI: $(M+H)^+$ = 788/90/92 ($Br_2$) | 0.48 | FM2 | (KBr): C=O 1641.3; NH, $NH^+$ 3419.6 |
| 4 | N4 | AS1 | A1 | C1 | 2.8 | ESI: $(M+H)^+$ = 818/20/22 ($Br_2$) | 0.52 | FM2 | |

Referenzbeispiel 8

Herstellung von Verbindungen der allgemeinen Formel:

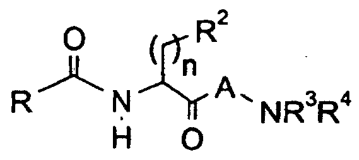

1-[$N^2$-[N-[4-(4-Fluorphenyl)-1-oxobutyl]-3,5-dibrom-D-tyrosyl]-$N^6$-[(1.1-dimethylethoxy)carbonyl]-L-lysyl]-4-(4-pyridinyl)-piperazin

Zu der Lösung von 0.18 g (0.001 mol) 4-(4-Fluorphenyl)-butansäure in einem Gemisch aus 4 ml Dimethylformamid und 10 ml Tetrahydrofuran wurde unter Rühren eine Mischung aus 0.71 g (0.001 mol) 1-[N2-(3,5-Dibrom-D-tyrosyl)-$N^6$-[(phenylmethoxy)carbonyl]-L-lysyl]-4-(4-pyridinyl)-piperazin, 0.32 g (0.001 mol) TBTU und 0.13g (0.001 mol) DIEA gegeben und die Mischung in einer Stickstoffatmosphäre 2 Tage gerührt. Das Reaktionsgemisch wurde dann im Vakuum eingeengt und der verbleibende Rückstand in Dichlormethan aufgenommen. Die organische Phase wurde mit 20proz. wässeriger Zitronensäurelösung und anschließend mit 10proz. wässeriger Natriumhydrogencarbonatlösung ausgeschüttelt, über Natriumsulfat getrocknet, filtriert und im Vakuum eingeengt. Nach Verrühren des Rückstandes mit Äther verblieben 0.68 g (77% der Theorie) des gesuchten Produktes als amorpher Rückstand.
IR (KBr): 1641, 1676 $cm^{-1}$ (C=O)
$R_f$: 0.65 (FM2)
ESI-MS: $(M+H)^+$ = 875/877/879 ($Br_2$)
$(M+H+Na)^{++}$ = 449/450/451 ($Br_2$)

Analog wurden hergestellt (jeweils n = 1):

| Lfd. Nr. | RCO | $R^2$ | A | $NR^3R^4$ | Anmerkungen | % Ausbeute | MS | $R_f$ | Fließmittel | IR [$cm^{-1}$] |
|---|---|---|---|---|---|---|---|---|---|---|
| 123 | N62 | AS1 | A0 | C1 | DMF/THF als Lösemittel | 20 | ESI: $(M+H)^+$ = 725/7/9 ($Br_2$) | 0.3 | FM 1 | (KBr): C=O 1641.3; 1691.5 |
| 124 | N63 | AS1 | A0 | C1 | DMF/THF als Lösemittel | 53 | ESI: $(M+H)^+$ = 725/7/9 ($Br_2$) | 0.2 | FM 1 | (KBr): C=O 1641.3; 1691.5 |
| 322 | N63 | AS1 | A0 | C8 | DMF/THF als Lösemittel | 19 | EI: $M^+$ = 729/31/33 ($Br_2$) | 0.3 | FM 1 | (KBr): C=O 1629.8; 1695.3 |
|  | N11 | AS1 | A3 | C1 |  | 46 | ESI: $(M+H)^+$ = 873/5/7 ($Br_2$) |  |  | (KBr): C=O 1625.9; 1645.2 |
|  | N18 | AS1 | A3 | C1 | THF/DMF als Lösemittel | 77 | ESI: $(M+H)^+$ = 875/7/9 ($Br_2$) | 0.78 | FM7 | (KBr): C=O 1641.3 |
|  | N20 | AS1 | A3 | C1 | THF/DMF als Lösemittel | 88 | ESI: $(M+H)^+$ = 863/5/7 ($Br_2$) | 0.67 | FM7 | (KBr): C=O 1643.3 |
|  | N21 | AS1 | A3 | C1 | THF/DMF als Lösemittel | 78 | ESI: $(M+H)^+$ = 917/6/8 ($Br_2$) | 0.47 | FM7 | (KBr): C=O 1643.3 |
|  | N46 | AS1 | A3 | C1 | THF/DMF als Lösemittel | 80 | ESI: $(M+H)^+$ = 905/7/9 ($Br_2$) | 0.65 | FM7 | (KBr): C=O 1643.3 |
|  | N43 | AS1 | A3 | C1 | THF/DMF als Lösemittel | 75 |  | 0.75 | FM7 | (KBr): C=O 1645.2 |
|  | N44 | AS1 | A3 | C1 | THF/DMF als Lösemittel | 79 | ESI: $(M+H)^+$ = 896/98/900 ($Br_2$) | 0.65 | FM7 | (KBr): C=O 1629.8 |

Referenzbeispiel 9

Herstellung von Verbindungen der allgemeinen Formel:

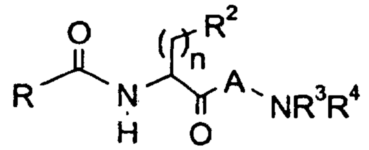

1-[$N^2$-[N-[[[(3-Methoxyphenyl)ethyl]amino]carbonyl]-3,5-dichlor-D-tyrosyl]-L-lysyl]-4-(4-pyridinyl)-piperazin-bis-(trifluoracetat (Lfd. Nr. 20)

Zu einer auf -10 °C gekühlten Suspension von 0.33 g (2 mmol) CDT und 1 ml Triethylamin in ca. 30 ml Tetrahydrofuran wurde unter Rühren die Lösung von 1.0 g (1.6 mmol) 1-[$N^2$(-3.5-Dichlor-D-tyrosyl]-$N^6$-[(1.1-dimethylethoxy)carbonyl]-L-lysyl]-4-(4-pyridinyl)-piperazin in 50 ml Tetrahydrofuran innerhalb von 60 Minuten zugetropft. Das Reaktionsgemisch wurde 1 Stunde bei 0 °C, anschließend 2 Stunden bei Raumtemperatur gerührt, mit einer Tetrahydrofuran-Lösung von 0.24 g (1.6 mmol) (3-Methoxyphenyl)-ethanamin versetzt, 3 Stunden unter Rückfluß gekocht und über Nacht bei Raumtemperatur gerührt. Nach Entfernen des Lösemittels im Vakuum wurde der Rückstand säulenchromatographisch (MN-Kieselgel 60, Macherey-Nagel, 70-230 mesh ASTM, Fließmittel: FM1) gereinigt. Die so erhaltene Zwischenverbindung wurde in einer Mischung aus 5 ml Trifluoressigsäure und 80 ml Dichlormethan über Nacht gerührt, das Lösemittel im Vakuum entfernt und der Rückstand mit Äther verrieben. Man erhielt 709 mg (43% der Theorie) der gewünschten Verbindung als amorphen Feststoff.
IR (KBr): 1643, 1676 $cm^{-1}$ (C=O)
$R_f$: 0.41(FM2)
ESI-MS: $(M+H)^+$ = 700/702/704 ($Br_2$)
$(M+2H)^{++}$ = 350.7/351.7/352.7 ($Br_2$)

Entsprechend wurden hergestellt (jeweils n = 1):

| Lfd. Nr. | RCO | $R^2$ | A | $NR^3R^4$ | Anmerkungen | % Ausbeute | MS | $R_1$ | Fließmittel | IR [$cm^{-1}$] |
|---|---|---|---|---|---|---|---|---|---|---|
| 23 | N17 | AS1 | A1 | C1 | $NEt_3$ als Base | 55 | ESI: $(M+H)^+$ = 798/800/802 ($Br_2$) | 0.2 | FM1 | (KBr): C=O 1643.3; 1676 |
| 47 | N39 | AS1 | A1 | C1 | $NEt_3$ als Base | 69.4 | ESI: $(M+H)^+$ = 676/78/80 ($Br_2$) | 0.1 | FM1 | (KBr): C=O 1645.2; 1676 |
| 50 | N64 | AS1 | A1 | C1 | $NEt_3$ als Base | 76 | ESI: $(M+H)^+$ = 828/830/832 ($Br_2$) | 0.2 | FM1 | (KBr): C=O 1643.3; 1678 |
| 51 | N40 | AS1 | A1 | C1 | $NEt_3$ als Base: Dehydratisierung | 79.7 | ESI: $(M+H)^+$ = 826/828/30 ($Br_2$) | 0.2 | FM1 | (KBr): C=O 1643.3; 1678 |
| 52 | N41 | AS1 | A1 | C1 | $NEt_3$ als Base; Dehydratisierung | 21.8 | ESI: $(M+H)^+$ = 826/828/30 ($Br_2$) | 0.2 | FM1 | (KBr): C=O 1645.2; 1679.9 |
| 56 | N16 | AS1 | A1 | C4 | $NEt_3$ als Base | 5 | ESI: $(M+H)^+$ = 873/75/77 ($Br_2$) | 0.3 | FM1 | (KBr): C=O 1637.5: 1676 |
| 57 | N45 | AS1 | A1 | C4 | $NEt_3$ als Base | 32 | ESI: $(M+H)^+$ = 867/9/71 ($Br_2$) | 0.2 | FM1 | (KBr): C=O 1635.5; 1678 |
| 66 | N47 | AS1 | A1 | C4 | $NEt_3$ als Base | 28.4 | ESI: $(M+H)^+$ = 764/6/8 ($Br_2$) | 0.1 | FM1 | (KBr): C=O 1635.5; 1676 |
| 46 | N38 | AS1 | A1 | C1 | $NEt_3$ als Base | 86 | ESI: $(M+H)^+$ = 826/28/30 ($Br_2$) | 0.35 | FM1 | (KBr): C=O 1645.2; 1684 |
| 232 | N66 | AS4 | A1 | C8 | | 69 | ESI: $(M+H)^+$ = 872/4/6 ($Br_2$) | 0.33 | FM1 | (KBr): C=O 1645 |
| 233 | N66 | AS4 | A1 | C1 | THF / DMF als Lösemittel | 16 | ESI: $(M+H)^+$ = 867/69/71 ($Br_2$) | 0.32 | FM1 | (KBr): C=O 1653 |
| 234 | N66 | AS4 | A1 | C4 | | 68 | ESI: $(M+H)^+$ = 867/69/71 ($Br_2$) | 0.42 | FM1 | (KBr): C=O 1645 |
| 235 | N66 | AS1 | A1 | C8 | | 26 | ESI: $(M+H)^+$ = 873/5/7 ($Br_2$) | 0.27 | FM1 | (KBr): C=O 1645 |
| 236 | N71 | AS1 | A1 | C1 | | 30 | ESI: $(M+H)^+$ = 880/2/4 ($Br_2$) | 0.22 | FM1 | (KBr): C=O 1636; 1678 |
| 237 | N71 | AS4 | A1 | C8 | | 28 | ESI: $(M+H)^+$ = 884/6/8 (Br2) | 0.25 | FM1 | |

(fortgesetzt)

| Lfd. Nr. | RCO | $R^2$ | A | $NR^3R^4$ | Anmerkungen | % Ausbeute | MS | $R_1$ | Fließmittel | IR [$cm^{-1}$] |
|---|---|---|---|---|---|---|---|---|---|---|
| 238 | N71 | AS4 | A1 | C1 | | 20 | ESI: $(M+H)^+$ = 879/81/83 ($Br_2$) | 0.3 | FM1 | (KBr): C=O 1641; 1682 |
| 18 | N14 | AS1 | A1 | C1 | Abspaltung der Boc-Schutzgruppe mit methanolischer HCl-Lösung | 26.3 | ESI: $(M+H)^+$ = 813/5/7 ($Br_2$) | 0.55 | FM2 | (KBr): C=O 1641.3; 1716.5 |
| 17 | N13 | AS1 | A1 | C1 | Abspaltung der Boc-Schutzgruppe mit methanolischer HCl-Lösung | 55.2 | ESI: $(M+H)^+$ = 799/801/803 ($Br_2$) | 0.55 | FM2 | (KBr): C=O 1639.4 |
| 9 | N6 | AS1 | A1 | C1 | Abspaltung der Boc-Schutzgruppe mit methanolischer HCl-Lösung | 41.3 | ESI: $(M+H)^+$ = 829/31/33 ($Br_2$) | 0.44 | FM2 | (KBr): C=O 1639.4 |
| 10 | N7 | AS1 | A1 | C1 | Abspaltung der Boc-Schutzgruppe mit methanolischer HCl-Lösung | 57.6 | ESI: $(M+H)^+$ = 829/31/33 ($Br_2$) | 0.32 | FM2 | (KBr): C=O 1639.4 |
| 20 | N2 | AS3 | A1 | C1 | | 43 | ESI: $(M+H)^+$ = 700/2/4 ($Br_2$) | 0.41 | FM2 | (KBr): C=O 1643.3: 1676.0 |
| 283 | N102 | AS4 | A3 | C4 | $NEt_3$ als Base | 65 | ESI: $(M+H)^+$ = 864/6/8 ($Br_2$) | 0.24 | FM1 | (KBr): C=O 1637.5; 1676 |

Referenzbeispiel 10

Herstellung von Verbindungen der allgemeinen Formel:

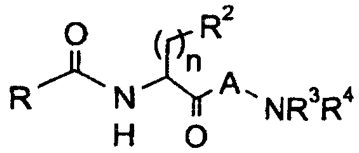

1-[$N^2$-[N-[4-(2,3-Dichlorphenyl)-1-piperazinyl]carbonyl]-3,5-dibrom-D-tyrosyl]-L-lysyl]-4-(4-pyridinyl)-piperazin-tris-(trifluoracetat) (Lfd. Nr. 74)

Zu der Lösung von 0.35 g (2.1 mmol) CDT in 50 ml Tetrahydrofuran gab man unter Kühlung (0 °C) und Rühren 1.0 g (1.4 mmol) 1-[$N^2$-(3,5-Dibrom-D-tyrosyl)-$N^6$-[(1,1-dimethylethoxy)carbonyl]-L-lysyl]-4-(4-pyridinyl)-piperazin und rührte 30 Minuten bei 0°C und weitere 30 Minuten bei Raumtemperatur. Nach Zugabe von 0.47 g (1.75 mmol) 1-(2,3-Dichlorphenyl)piperazin-hydrochlorid und 0.25 ml Triethylamin wurde das Reaktionsgemisch 5 Stunden unter Rückfluß gekocht und nach dem Erkalten mit 70 ml gesättigter wässeriger Natriumhydrogencarbonat-Lösung versetzt. Die organische Phase wurde abgetrennt, die wässerige Phase zweimal mit je 50 ml Tetrahydrofuran ausgeschüttelt. Die vereinigten organischen Phasen wurde mit gesättigter wässeriger Kochsalz-Lösung gewaschen, über Magnesiumsulfat getrocknet, filtriert und im Vakuum eingeengt. Der Rückstand wurde mit Äther verrieben, abgenutscht und anschließend 2 Stunden mit einer Mischung aus 50ml Dichlormethan und 5 ml Trifluoressigsäure gerührt. Nach Einengen des Reaktionsgemisches im Vakuum und Verreiben des Rückstandes mit Äther verblieben 0.8 g (47% der Theorie) eines amorphen Feststoffes.
IR (KBr): 1643.3, 1676 $cm^{-1}$ (C=O)
$R_f$: 0.78 (FM7)
ESI-MS: $(M+H)^+$ = 867/869/871/873/875 ($Br_2$, $Cl_2$)
$(M+2H)^{++}$ = 434/435/436/437 ($Br_2$, $Cl_2$)

Entsprechend wurden hergestellt (jeweils n = 1):

| Lfd. Nr. | RCO | $R^2$ | A | $NR^3R^4$ | % Ausbeute | MS | $R_f$ | Fließmittel | IR [$cm^{-1}$] |
|---|---|---|---|---|---|---|---|---|---|
| 36 | N29 | AS1 | A1 | C1 | 17.3 | ESI: $(M+H)^+$ = 889/91/93 ($Br_2$) | 0.35 | MeOH/NH4 OH = 9/1 (v/ v) | (KBr): C=O 1643.3; 1674.1 |
| 208 | N15 | AS1 | A1 | C1 | 53.5 | ESI: $(M+H)^+$ = 854/6/8 ($Br_2$) | 0.43 | FM2 | (KBr): C=O 1691.5; 1635.5 |
| 209 | N15 | AS1 | A1 | C1 | 47.7 | ESI: $(M+H)^+$ = 854/6/8 ($Br_2$) | 0.55 | FM2 | (KBr): C=O 1695.3; 1637.5 |
| 210 | N15 | AS1 | A1 | C1 | 28 | ESI: $(M+H)^+$ = 854/6/8 ($Br_2$) | 0.48 | FM2 | (KBr): C=O 1689.5, 1639.4 |
| 75 | N50 | AS1 | A1 | C1 | 16.5 | ESI: $(M+H)^+$ = 867/69/71/73/75 ($Br_2$,$Cl_2$) | 0.63 | FM2 | (KBr): C=O 1643.3; 1676.0; |
| 74 | N49 | AS1 | A1 | C1 | 47 | ESI: $(M+H)^+$ = 867/69/71/73/75 ($Br_2$,$Cl_2$) | 0.65 | FM2 | (KBr): C=O 1643.3; 1676.0 |
| 76 | N51 | AS1 | A1 | C1 | 13.4 | ESI: $(M+H)^+$ = 824/6/8 ($Br_2$) | 0.58 | FM2 | (KBr): C=O 1643.3; 1676.0; CN 2219.9 |

(fortgesetzt)

| Lfd. Nr. | RCO | $R^2$ | A | $NR^3R^4$ | % Ausbeute | MS | $R_f$ | Fließmittel | IR [$cm^{-1}$] |
|---|---|---|---|---|---|---|---|---|---|
| 79 | N52 | AS1 | A1 | C1 | 11.4 | ESI: $(M+H)^+$ = 901/3/5/7 ($Br_2$, Cl) | 0.59 | FM2 | (KBr): C=O 1645.2; 1676 3 |
| 45 | N37 | AS1 | A1 | C1 | 43 | ESI: $(M+H)^+$ = 784/6/8 ($Br_2$) | 0.6 | FM2 | (KBr): C=O 1643.3; 1678.0 |
| 39 | N32 | AS1 | A1 | C1 | 48.3 | ESI: $(M+H)^+$ = 795/7/9 ($Br_2$) | 0.57 | FM2 | (KBr): C=O 1643.3; 1678.0 |
| 38 | N31 | AS1 | A1 | C1 | 54.1 | ESI: $(M+H)^+$ = 844/6/8 ($Br_2$) | 0.6 | FM2 | (KBr): C=O 1643.3; 1678.0: NO2 1543.0 |
| 37 | N30 | AS1 | A1 | C1 | 61.6 | ESI: $(M+H)^+$ = 813/5/7 ($Br_2$) | 0.6 | FM2 | (KBr): C=O 1643.3; 1676.0 |
| 35 | N28 | AS1 | A1 | C1 | 74.8 | ESI: $(M+H)^+$ = 800/2/4 ($Br_2$) | 0.55 | FM2 | (KBr): C=O 1639.4; |
| 34 | N27 | AS1 | A1 | C1 | 36.8 | ESI: $(M+H)^+$ = 800/2/4 ($Br_2$) | 0.43 | FM2 | (KBr): C=O 1641.3; 1714.6; NH+ 3409.9 |
| 32 | N25 | AS1 | A1 | C1 | 50,0 | ESI: $(M+H)^+$ = 737/39/41 ($Br_2$) | 0.44 | FM2 | (KBr): C=O 1645.2; 1645.2; |
| 33 | N26 | AS1 | A1 | C1 | 42 | ESI: $(M+H)^+$ = 767/69/71 ($Br_2$) | 0.33 | FM2 | (KBr): C=O 1676.0 |
| 40 | N33 | AS1 | A1 | C1 | 14.5 | ESI: $(M+H)^+$ = 802/4/6 ($Br_2$) | 0.58 | FM2 | (KBr): C=O 1643.3; 1676.0 |
| 28 | N6 | AS3 | A1 | C1 | 67.2 | ESI: $(M+H)^+$ = 741/3/5 ($Cl_2$) | 0.43 | FM2 | (KBr): C=O 1641.3; 1716.5 |
| 64 | N23 | AS1 | A1 | C4 | 39 | ESI: $(M+H)^+$ = 832/4/6/8 ($Br_2$, Cl) | 0.68 | FM2 | (KBr): C=O 1627.8; 1678.0 |
| 65 | N15 | AS1 | A1 | C4 | 41 | ESI: $(M+H)^+$ = 853/5/7 ($Br_2$) | 0.61 | FM2 | (KBr): C=O 1631.7; 1695.3 |
| 365 | N111 | AS1 | A1 | C1 | 36.9 | ESI: $(M+H)^+$ = 839/41/43 (Br2) | 0.09 | FM1 | (KBr): C=O 1626/1676 |

Referenzbeispiel 11

1-[N2-[N-[[[2-(2,5-Dimethoxyphenyl)ethyl]amino]carbonyl]-3,5-dibrom-D,L-tyrosyl]-L-lysyl]-4-(4-pyridinyl)-piperazin (Lfd. Nr. 3)

Die Mischung aus 0.8 g (0.84 mmol) 1-[$N^2$-[N-[[[2-(2,5-Dimethoxyphenyl)ethyl]amino]carbonyl]-3,5-dibrom-D-tyrosyl]-$N^6$-[(phenylmethoxy)carbonyl]-L-lysyl]-4-(4-pyridinyl)-piperazin, 50 ml Eisessig, 25 ml einer 33proz. Lösung von Bromwasserstoff in Eisessig und 2 ml Anisol wurde 12 Stunden bei Raumtemperatur gerührt. Das Reaktionsgemisch wurde in Diethylether eingerührt und der entstandene Niederschlag abgenutscht. Der feste Rückstand wurde säulenchromatographisch (MN-Kieselgel 60, Macherey-Nagel, 70-230 mesh ASTM, Fließmittel: Essigester/Methanol/konz. wässeriges Ammoniak = 8/2/0.2 (v/v/v)) gereinigt. Man erhielt 0.3 g (44 % der Theorie) des gesuchten Produkts als amorphe Festsubstanz.
IR (KBr): 1643.3 $cm^{-1}$ (C=O)
$R_f$: 0.17 (Essigester/Methanol/konz. wässeriges Ammoniak = 6/4/1)

ESI-MS: $(M+H)^+$ = 818/820/822 ($Br_2$)
$(M+2H)^{++}$ = 409.5/410.5/411.5 ($Br_2$)

Referenzbeispiel 12

1-[$N^2$-[3,5-Dibrom-N-[[[2-(3-methoxyphenyl)ethyl]amino]carbonyl]-D-tyrosyl]-L-arginyl]-4-(4-pyridinyl)-piperazin-bis-(trifluoracetat)(Lfd. Nr. 4)

Die gerührte Mischung aus 20 ml Trifluoressigsäure, 1.3 ml Anisol und 0.9 ml Ethandithiol wurde unter Eiskühlung mit 2.1 g (1.9 mmol) festem 1-[$N^2$-[3,5-Dibrom-N-[[[2-(3-methoxyphenyl)-ethyl]amino]carbonyl]-D-tyrosyl]-$N^G$-(2,2,5,7,8-pentamethylchroman-6-sulfonyl)-L-arginyl]-4-(4-pyridinyl)-piperazin versetzt und weitere 45 Minuten unter Eiskühlung, danach 3 Stunden bei Raumtemperatur gerührt. Der entstandene Niederschlag wurde abgenutscht und verworfen, das Filtrat im Vakuum eingeengt, der verbliebene Rückstand mit Toluol versetzt und nochmals im Vakuum eingedampft. Der so erhaltene feste Rückstand wurde mit einer Mischung aus Diethylether und Aceton verrieben und der entstehende weiße Feststoff abgenutscht und getrocknet. Man erhielt 1.7 g (65% der Theorie) der gesuchten Titelverbindung.
IR (KBr): 1674, 1645 $cm^{-1}$ (C=O)
$R_f$: 0.15 (FM: BuOH/AcOH/$H_2O$ 4/1/1 (v/v/v))
ESI-MS: $(M+H)^+$ = 816/818/820 ($Br_2$)
$(M+2H)^{++}$ = 408.6/409.6/410.6 ($Br_2$)

Referenzbeispiel 13

Herstellung von Verbindungen der allgemeinen Formel:

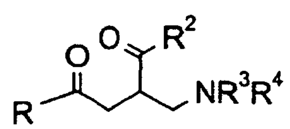

(R,S)-1-[2-(4-Amino-3,5-dibrombenzoyl)-4-[4-(3,4-dihydro-2(1H)-oxochinazolin-3-yl)-1-piperidinyl]-4-oxobutyl]-4-(4-pyridinyl)-piperidin (Lfd. Nr. 291)

Die Mischung aus 0.97 g (1.8 mmol) (R,S)-4-Amino-3,5-dibrom-γ-oxo-β-[[4-(4-pyridinyl)-1-piperidinyl]methyl]-benzenbutansäure, 0.48g (1.8 mmol) 4-(3,4-Dihydro-2(1H)-oxochinazolin-3-yl)-piperidin, 2 ml Triethylamin, 0.58 g (1.8 mmol) TBTU, 0.24 g (1.8 mmol) HOBt, 25 ml THF und 25 ml DMF wurde 4 Stunden bei Raumtemperatur gerührt. Das Reaktionsgemisch wurde im Vakuum eingeengt, der Rückstand in einer Mischung aus Essigsäureethylester und Methanol (95/5 (v/v)) aufgenommen und mit gesättigter wäßriger Natriumhydrogencarbonat-Lösung gewaschen. Die organische Phase wurde getrocknet und im Vakuum eingeengt. Der Rückstand wurde säulenchromatographisch (MN-Kieselgel 60, Macherey-Nagel, 70-230 mesh ASTM, Fließmittel: Essigester/Methanol = 9/1 (v/v); anschließend MN-Kieselgel 60, Macherey-Nagel, 70-230 mesh ASTM, Fließmittel: Methylenchlorid/Ethanol = 9/1 (v/v)) gereinigt. Man erhielt 0.2 g (15% der Theorie) des gesuchten Produkts als weiße amorphe Festsubstanz.
IR (KBr): 1668.3 $cm^{-1}$ (C=O)
$R_f$: 0.5 (FM2)
ESI-MS: $(M+H)^+$ = 737/739/741 ($Br_2$)
$(M+Na)^+$ = 759/761/763 ($Br_2$)

Analog wurden hergestellt:

| Lfd, Nr. | RCO | $R^2$ | NR3R4 | % Ausbeute | MS | $R_f$ | Fließmittel | IR [cm-1] |
|---|---|---|---|---|---|---|---|---|
| 291 | N66 | AS4 | C4 | 15 | ESI: $(M+H)^+$ = 737/39/41 ($Br_2$) | 0.36 | $CH_2Cl_2$/ EtOH | (KBr): C=O 1668 |

(fortgesetzt)

| Lfd, Nr. | RCO | $R^2$ | NR3R4 | % Ausbeute | MS | $R_f$ | Fließmittel | IR [cm-1] |
|---|---|---|---|---|---|---|---|---|
| 296 | N66 | AS4 | C8 | 14 | ESI: $(M+H)^+$ = 743/5/7 ($Br_2$) | 0.66 | FM1 | (KBr): C=O 1668 |
| 302 | N71 | AS4 | C8 | 19 | ESI: $(M+H)^+$ = 755/7/9 ($Br_2$) | 0.54 | FM1 | (KBr): C=O 1682 |

Referenzbeispiel 14

1-[4-Amino-N-[[4-[7-(aminocarbonyl)-3,4-dihydro-2(1H)-oxochinazolin-3-yl]-1-piperidinyl]carbonyl]-3,5-dibrom-D-phenylalanyl]-4-(4-pyridinyl)-piperidin (Lfd. Nr. 312)

a) 1-[4-Amino-3,5-dibrom-N-[[4-[3,4-dihydro-7-(methoxycarbonyl)-2(1H)-oxochinazolin-3-yl]-1-piperidinyl]carbonyl]-D-phenylalanyl]-4-(4-pyridinyl)-piperidin (Lfd. Nr. 307)

Hergestellt analog Beispiel 3 aus 3,4-Dihydro-3-(4-piperidinyl)-2(1H)-oxochinazolin-7-carbonsäuremethylester, 1-[4-Amino-3,5-dibrom-D-phenylalanyl]- 4-(4-pyridinyl)-piperidin und CDT in einer Ausbeute von 27.2 % der Theorie. Farblose, amorphe Substanz vom $R_f$ 0.5 (Fließmittel: Dichlormethan/ Cyclohexan/Methanol/ konz. Ammoniak = 7/1.5/1.5/0.2 (v/v/v/v)).
IR(KBr): 1718.5, 1670.3, 1618.2 $cm^{-1}$ (C=O)
ESI-MS: $(M+H)^+$ = 796/798/800 ($Br_2$)
$(M+Na)^+$ = 818/820/822 ($Br_2$)

Entsprechend wurden erhalten:

Aus 3,4-Dihydro-3-(4-piperidinyl)-2(1H)-oxochinazolin-7-carbonsäuremethylester, 1-[4-Amino-3,5-dibrom-D-phenylalanyl]- 4-(1-piperidinyl)-piperidin und CDT in einer Ausbeute von 30.3 % der Theorie das 1-[4-Amino-3,5-dibrom-N-[[4-[3,4-dihydro-7-(methoxycarbonyl)-2(1H)-oxochinazolin-3-yl]-1-piperidinyl]carbonyl]-D-phenylalanyl]-4-(1-piperidinyl)-piperidin (Lfd. Nr. 304) vom
$R_f$ = 0.75 (FM1).
IR(KBr): 1720.4, 1668.3, 1620.1 $cm^{-1}$ (C=O)
ESI-MS: $(M+H)^+$ = 802/804/806 ($Br_2$)
$(M+Na)^+$ = 824/826/828 ($Br_2$)

Aus 3,4-Dihydro-3-(4-piperidinyl)-2(1H)-oxochinazolin-7-carbonsäuremethylester, 1-(3,5-Dibrom-D-tyrosyl)-4-(1-piperidinyl)-piperidin und CDT in einer Ausbeute von 35 % der Theorie das 1-[3,5-Dibrom-N-[[4-[7-(methoxycarbonyl)-3,4-dihydro-2(1H)-oxochinazolin-3-yl]-1-piperidinyl]carbonyl]-D-tyrosyl]-4-(1-piperidinyl)-piperidin (Lfd. Nr. 422) vom $R_f$ 0.54 (Fließmittel: Dichlormethan/ Cyclohexan/ Methanol/ konz. Ammoniak = 7/1.5/1.5/0.2 (v/v/v/v)).
IR(KBr): 1720.4, 1668.3, 1627.8 $cm^{-1}$ (C=O)
ESI-MS: $(M+H)^+$ = 803/805/807 ($Br_2$)
$(M+Na)^+$ = 825/827/829 ($Br_2$)

Aus 3,4-Dihydro-3-(4-piperidinyl)-2(1H)-oxochinazolin-7-carbonsäuremethylester, 1-(3,5-Dibrom-D-tyrosyl)-4-(4-pyridinyl)-piperidin und CDT in einer Ausbeute von 45 % der Theorie das 1-[3,5-Dibrom-N-[[4-[7-(methoxycarbonyl)-3,4-dihydro-2(1H)-oxochinazolin-3-yl]-1-piperidinyl]carbonyl]-D-tyrosyl]-4-(4-pyridinyl)-piperidin (Lfd. Nr. 420) vom $R_f$ 0.56 (FM1).
IR(KBr): 1718.5, 1664.5, 1624.0 $cm^{-1}$ (C=O)
ESI-MS: $(M+H)^+$ = 797/799/801 ($Br_2$)
$(M+Na)^+$ = 819/821/823 ($Br_2$)

b) 1-[4-Amino-3,5-dibrom-N-[[4-[3,4-dihydro-7-(hydroxycarbonyl)-2(1H)-oxochinazolin-3-yl)-1-piperidinyl]carbonyl]-D-phenylalanyl]-4-(4-pyridinyl)-piperidin (Lfd. Nr. 309)

Hergestellt analog Beispiel A37) aus 1-[4-Amino-3,5-dibrom-N-[[4-[3,4-dihydro-7-(methoxycarbonyl)-2(1H)-oxochinazolin-3-yl]-1-piperidinyl]carbonyl]-D-phenylalanyl]-4-(4-pyridinyl)-piperidin durch Verseifung mit Lithiumhydroxid in einer Ausbeute von 95 % der Theorie. Farblose, amorphe Substanz vom $R_f$ 0.25 (Fließmittel: Dichlormethan/ Methanol/ konz: Ammoniak = 7.5/2.5/0.5 (v/v/v)).
IR(KBr): 1666.4, 1614.3 $cm^{-1}$ (C=O)
ESI-MS: $(M+H)^+$ = 780/782/784 ($Br_2$)

Entsprechend wurden erhalten:

Aus 1-[4-Amino-3,5-dibrom-N-[[4-[3,4-dihydro-7-(methoxycarbonyl)-2(1H)-oxochinazolin-3-yl]-1-piperidinyl]carbonyl]-D-phenylalanyl]-4-(1-piperidinyl)-piperidin in einer Ausbeute von 60.2 % der Theorie das 1-[4-Amino-3,5-dibrom-N-[[4-[3,4-dihydro-7-(hydroxycarbonyl)-2(1H)-oxochinazolin-3-yl]-1-piperidinyl]carbonyl]-D-phenylalanyl]-4-(1-piperidinyl)-piperidin (Lfd. Nr. 306) vom $R_f$ 0.15 (FM1).
IR(KBr): 1635.5 $cm^{-1}$, breit (C=O)
ESI-MS: $(M+H)^+$ = 788/790/792 ($Br_2$)
$(M+Na)^+$ = 810/812/814 ($Br_2$)

Aus 1-[3,5-Dibrom-N-[[4-[7-(methoxycarbonyl)-3,4-dihydro-2(1H)-oxochinazolin-3-yl]-1-piperidinyl]carbonyl]-D-tyrosyl]-4-(1-piperidinyl)-piperidin in einer Ausbeute von 62 % der Theorie das 1-[3,5-Dibrom-N-[[4-[7-(hydroxycarbonyl)-3,4-dihydro-2(1H)-oxochinazolin-3-yl]-1-piperidinyl]carbonyl]-D-tyrosyl]-4-(1-piperidinyl)-piperidin (Lfd. Nr. 423) vom $R_f$ 0.03 (Fließmittel: Dichlormethan/ Cyclohexan/ Methanol/ konz. Ammoniak = 7/1.5/1.5/0.2 (v/v/v/v)).
IR(KBr): 1635.5 $cm^{-1}$, breit (C=O)
ESI-MS: $(M+H)^+$ = 789/791/793 ($Br_2$)

Aus 1-[3,5-Dibrom-N-[[4-(1,3-dihydro-5-(methoxycarbonyl)-2(2H)-oxobenzimidazol-1-yl)-1-piperidinyl]carbonyl]-D-tyrosyl]-4-(4-pyridinyl)-piperidin in einer Ausbeute von 80 % der Theorie das 1-[3,5-Dibrom-N-[[4-(1,3-dihydro-5-(hydroxycarbonyl)-2-(2H)-oxobenzimidazol-1-yl)-1-piperidinyl]carbonyl]-D-tyrosyl]-4-(4-pyridinyl)-piperidin (Lfd. Nr. 151). Farblose, amorphe Substanz.
IR(KBr): 1701.1, 1625.9 $cm^{-1}$ (C=O)
ESI-MS: $(M+H)^+$ = 767/769/771 ($Br_2$)
$(M+2H)^{++}$ = 383/384/385 ($Br_2$)

Aus 1-[3,5-Dibrom-N-[[4-[7-(methoxycarbonyl)-3,4-dihydro-2(1H)-oxochinazolin-3-yl]-1-piperidinyl]carbonyl]-D-tyrosyl]-4-(4-pyridinyl)-piperidin in einer Ausbeute von 82 % der Theorie das 1-[3,5-Dibrom-N-[[4-[7-(hydroxycarbonyl)-3,4-dihydro-2(1H)-oxochinazolin-3-yl]-1-piperidinyl]carbonyl]-D-tyrosyl]-4-(4-pyridinyl)-piperidin (Lfd. Nr. 421) vom $R_f$ 0.03 (FM1). Farblose, amorphe Substanz.
IR(KBr): 1625 breit $cm^{-1}$ (C=O)
ESI-MS: $(M+H)^+$ = 783/785/787 ($Br_2$)
$(M+Na)^+$ = 805/807/809 ($Br_2$)

c) 1-[4-Amino-N-[[4-[7-(aminocarbonyl)-3,4-dihydro-2(1H)-oxochinazolin-3-yl]-1-piperidinyl]carbonyl]-3,5-dibrom-D-phenylalanyl]-4-(4-pyridinyl)-piperidin (Lfd. Nr. 312)

Hergestellt analog Beispiel 1 aus 1-[4-Amino-3,5-dibrom-N-[[4-[3,4-dihydro-7-(hydroxycarbonyl)-2(1H)-oxochinazolin-3-yl]-1-piperidinyl]carbonyl]-D-phenylalanyl]-4-(4-pyridinyl)-piperidin und Ammoniumcarbonat in Gegenwart von TBTU in einer Ausbeute von 40.6 % der Theorie. Farblose, amorphe Substanz vom $R_f$ 0.8 (Fließmittel: Dichlormethan/ Methanol/ konz. Ammoniak = 7.5/2.5/0.5 (v/v/v)).
IR(KBr): 1670.3, 1616.3 $cm^{-1}$ (C=O)
ESI-MS: $(M+H)^+$ = 781/783/785 ($Br_2$)
$(M+Na)^+$ = 803/805/807 ($Br_2$)

Entsprechend wurden erhalten:

Aus 1-[4-Amino-3,5-dibrom-N-[[4-[3,4-dihydro-7-(hydroxycarbonyl)-2(1H)-oxochinazolin-3-yl]-1-piperidinyl]carbonyl]-D-phenylalanyl]-4-(4-pyridinyl)-piperidin und Ethanolamin in einer Ausbeute von 34.6 % der Theorie das 1-[4-Amino-3,5-dibrom-N- [[4-[7-(2-hydroxyethylaminocarbonyl)-3,4-dihydro-2(1H)-oxochinazolin-3-yl]-1-piperidinyl]carbonyl]-D-phenylalanyl]-4-(4-pyridinyl)-piperidin (Lfd. Nr. 313) vom $R_f$ = 0.7 (Fließmittel: Dichlormethan/Methanol/konz. Ammoniak = 7.5/2.5/0.5 v/v/v).
IR(KBr): 1662.5, 1618.2 $cm^{-1}$ (C=O)
ESI-MS: $(M+H)^+$ = 825/827/829 ($Br_2$)
$(M+Na)^+$ = 847/849/851 ($Br_2$)
$(M+2H)^{++}$ = 413/414/415 ($Br_2$)

$(M+H+Na)^{++}$ = 424/425/426 ($Br_2$)

Aus 1-[4-Amino-3,5-dibrom-N-[[4-[3,4-dihydro-7-(hydroxycarbonyl)-2(1H)-oxochinazolin-3-yl]-1-piperidinyl)carbonyl]-D-phenylalanyl]-4-(4-pyridinyl)-piperidin und 1-Methylpiperazin in einer Ausbeute von 44.9 % der Theorie das 1-[4-Amino-3,5-dibrom-N-[[4-[7-[(4-methyl-1-piperazinyl)carbonyl]-3,4-dihydro-2 (1H)-oxochinazolin-3-yl]-1-piperidinyl]carbonyl]-D-phenylalanyl]-4-(4-pyridinyl)-piperidin (Lfd. Nr. 430) vom $R_f$ = 0.28 (Fließmittel: Essigsäureethylester/Methanol/konz. Ammoniak = 8/1.5/0.3 v/v/v).
IR(KBr): 1618.2 $cm^{-1}$ (C=O)
ESI-MS: $(M+H)^+$ = 864/866/868 ($Br_2$)
$(M+Na)^+$ + = 886/888/890 ($Br_2$)
$(M+2H)^{++}$ = 432/433/434.7 ($Br_2$)

Aus 1-[4-Amino-3,5-dibrom-N-[[4-[3,4-dihydro-7-(hydroxycarbonyl)-2(1H)-oxochinazolin-3-yl]-1-piperidinyl]carbonyl]-D-phenylalanyl]-4-(4-pyridinyl)-piperidin und Methylammoniumchlorid in einer Ausbeute von 37 % der Theorie das 1-[4-Amino-3,5-dibrom-N-[[4-[7-(methylaminocarbonyl)-3,4-dihydro-2(1H)-oxochinazolin-3-yl]-1-piperidinyl] carbonyl]-D-phenylalanyl]-4-(4-pyridinyl)-piperidin (Lfd. Nr. 424) vom $R_f$ = 0.49 (FM1). IR(KBr): 1662.5, 1622 $cm^{-1}$ (C=O)
ESI-MS: $(M+H)^+$ = 795/797/799 ($Br_2$)
$(M+Na)^+$ = 817/819/821 ($Br_2$)

Aus 1-[4-Amino-3,5-dibrom-N-[[4-[3,4-dihydro-7-(hydroxycarbonyl)-2(1H)-oxochinazolin-3-yl]-1-piperidinyl]carbonyl]-D-phenylalanyl]-4-(1-piperidinyl)-piperidin und Ammoniumcarbonat in einer Ausbeute von 12 % der Theorie das 1-[4-Amino-N-[[4-[7-(aminocarbonyl)-3,4-dihydro-2(1H)-oxochinazolin-3-yl]-1-piperidinyl]carbonyl]-3,5-dibrom-D-phenylalanyl]-4-(1-piperidinyl)-piperidin (Lfd. Nr. 310) vom $R_f$ = 0.7 (Fließmittel: Dichlormethan/Methanol/konz. Ammoniak = 7.5/2.5/0.5 (v/v/v)). IR(KBr): 1670.3, 1618.2 $cm^{-1}$ (C=O)
ESI-MS: $(M+H)^+$ = 787/789/791 ($Br_2$)

Aus 1-[4-Amino-3,5-dibrom-N-[[4-[3,4-dihydro-7-(hydroxycarbonyl)-2(1H)-oxochinazolin-3-yl]-1-piperidinyl]carbonyl]-D-phenylalanyl]-4-(1-piperidinyl)-piperidin und Ethanolamin in einer Ausbeute von 11.4 % der Theorie das 1-[4-Amino-3,5-dibrom-N-[[4-[7-(2-hydroxyethylaminocarbonyl]-3,4-dihydro-2(1H)-oxochinazolin-3-yl]-1-piperidinyl]carbonyl]-D-phenylalanyl]-4-(1-piperidinyl)-piperidin (Lfd. Nr. 311) vom $R_f$ = 0.65 (Fließmittel: Dichlormethan/Methanol/konz. Ammoniak = 7.5/2.5/0.5 (v/v/v)).
IR(KBr): 1660.6, 1620.1 $cm^{-1}$ (C=O)
ESI-MS: $(M+H)^+$ = 831/833/835 ($Br_2$)
$(M+2H)^{++}$ = 416/417/418 ($Br_2$)
$(M+H+Na)^{++}$ = 427/428/429 ($Br_2$)

Referenzbeispiel 15

4-(1-Acetyl-4-piperidinyl)-1-[4-amino-3,5-dibrom-N-[[4-(3,4-dihydro-2(1H)-oxochinazolin-3-yl)-1-piperidinyl]carbonyl] -Dphenylalanyl]-piperidin (Lfd. Nr. 372)

a) 1-[4-Amino-3,5-dibrom-D-phenylalanyl)-4-[1-(1,1-dimethylethoxycarbonyl)-4-piperidinyl]-piperidin

Das Gemisch aus 5.60 g (0.01 mol) 4-Amino-3,5-dibrom-$N^2$-(9-fluorenylmethoxycarbonyl)-D-phenylalanin, 1.35 g (0.01 mol) HOBt, 3.21 g (0.01 mol) TBTU, 1.29 g (0.01 mol) DIEA, 2.68 g (0.01 mol) 4-[1-(1,1-Dimethylethoxycarbonyl)-4-piperidinyl]-piperidin und 150 ml Tetrahydrofuran wurde 2 Stunden bei Zimmertemperatur gerührt. Nach vollständiger Umsetzung gab man 20 ml Diethylamin zu und rührte weitere 18 Stunden bei Zimmertemperatur. Die Reaktionsmischung wurde im Vakuum eingedampft, der Rückstand in 200 ml Dichlormethan aufgenommen und nacheinander mit je 100 ml gesättigter Natriumchlorid-Lösung und gesättigter Natriumhydrogencarbonat-Lösung gewaschen und über Magnesiumsulfat getrocknet. Das nach Entfernen des Lösemittels verbleibende rötliche Öl wurde an Kieselgel (30 - 60 μm) unter Verwendung von anfangs Dichlormethan, dann FM4 zum Eluieren säulenchromatographisch gereinigt. Man erhielt die Titelverbindung in Form einer farblosen, amorphen Substanz und in einer Ausbeute von 4.31 g (73.3 % der Theorie).
IR(KBr): 1687.6 $cm^{-1}$ (C=O)
MS: $M^+$ = 586/588/590 ($Br_2$)

b) 1-[4-Amino-3,5-dibrom-N-[[4-(3,4-dihydro-2(1H)-oxochinazolin-3-yl)-1-piperidinyl]carbonyl]-D-phenylalanyl]-4-[1-(1,1-dimethylethoxycarbonyl)-4-piperidinyl]-piperidin

Hergestellt analog Beispiel 4 aus 1-[4-Amino-3,5-dibrom-D-phenylalanyl]-4-[1-(1,1-dimethylethoxycarbonyl)-4-piperidinyl]-piperidin, CDT und 3,4-Dihydro-3-(4-piperidinyl)-2(1H)-chinazolinon in quantitativer Ausbeute. Farblose, amorphe Substanz.
IR (KBr) : 1676 $cm^{-1}$ (C=O)
MS: $(M+H)^+$ = 844/846/848 ($Br_2$)
$(M+Na)^+$ = 866/868/870 ($Br_2$)

c) 1-[4-Amino-3,5-dibrom-N-[[4-(3,4-dihydro-2(1H)-oxochinazolin-3-yl)-1-piperidinyl]carbonyl]-D-phenylalanyl]-4-(4-piperidinyl)-piperidin (Lfd. Nr. 521)

Hergestellt analog Beispiel A1b), jedoch unter Verwendung von Natronlauge an Stelle von Ammoniak, aus 1-[4-Amino-3,5-dibrom-N-[[4-(3,4-dihydro-2(1H)-oxochinazolin-3-yl)-1-piperidinyl]-carbonyl)-D-phenylalanyl]-4-[1-(1,1-dimethylethoxycarbonyl)-4-piperidinyl]-piperidin durch Behandlung mit Trifluoressigsäure in einer Ausbeute von 75 % der Theorie. Farblose, amorphe Substanz.
IR(KBr): 1666.4, 1620.1 $cm^{-1}$ (C=O)
MS: $(M+H)^+$ + = 744/746/748 ($Br_2$)
$(M+2H)^{++}$ = 372/373/374.5 ($Br_2$)

d) 4-(1-Acetyl-4-piperidinyl)-1-[4-amino-3,5-dibrom-N-[[4-(3,4-dihydro-2(1H)-oxochinazolin-3-yl)-1-piperidinyl]carbonyl]-D-phenylalanyl]-piperidin (Lfd. Nr. 372)

Die Lösung von 0.372 g (0.499 mmol) 1-[4-Amino-3,5-dibrom-N-[[4-(3,4-dihydro-2(1H)-oxochinazolin-3-yl)-1-piperidinyl]carbonyl]-D-phenylalanyl]-4-(4-piperidinyl)-piperidin und 0.07 g (5.5 mmol) DIEA in 50 ml Dichlormethan wurde unter äußerer Kühlung mit Eiswasser tropfenweise mit 0.043 g (5.48 mmol) Acetylchlorid versetzt und anschließend 1 Stunde bei Zimmertemperatur gerührt. Das Lösemittel wurde im Vakuum entfernt, der Rückstand mit Wasser verrührt und filtriert. Der Filterrückstand wurde im Vakuum getrocknet und an Kieselgel (30-60 µm) unter Verwendung von FM4 zum Eluieren säulenchromatographisch gereinigt. Die geeigneten Eluate wurden eingedampft, der Rückstand mit Diethylether verrieben und abgenutscht. Man erhielt 230 mg (58.5 % der Theorie) an farblosen Kristallen.
IR(KBr): 1622 $cm^{-1}$ (C=O)
MS: $(M+H)^+$ = 786/788/790 ($Br_2$)
$(M+Na)^+$ = 808/810/812 ($Br_2$)

Entsprechend wurde erhalten:

1-[4-Amino-3,5-dibrom-N-[[4-[3,4-dihydro-2(1H)-oxochinazolin-3-yl]-1-piperidinyl]carbonyl]-D-phenylalanyl]-4-(1-benzoyl-4-piperidinyl)-piperidin (Lfd. Nr. (485)).
Farblose Kristalle
$R_f$ 0.74 (FM1)
IR(KBr): 1626, 1668 $cm^{-1}$ (C=O)
ESI-MS: $(M+H)^+$ = 848/850/852 ($Br_2$)

Referenzbeispiel 16

1-[4-Amino-3,5-dibrom-N-[[4-[3,4-dihydro-2 (1H)-oxochinazolin-3-yl]-1-piperidinyl]carbonyl]-D-phenylalanyl]-4-(1-methylsulfonyl-4-piperidinyl)-piperidin (Lfd. Nr. 486)

Die Lösung von 0.372 g (0.499 mmol) 1-[4-Amino-3,5-dibrom-N-[[4-(3,4-dihydro-2(1H)-oxochinazolin-3-yl)-1-piperidinyl]carbonyl]-D-phenylalanyl]-4-(4-piperidinyl)-piperidin und 0.07 g (5.5 mmol) DIEA in 50 ml Dichlormethan wurde unter äußerer Kühlung mit Eiswasser tropfenweise mit 0.063 g (5.5 mmol) Methansulfonylchlorid versetzt und anschließend 1 Stunde bei Zimmertemperatur gerührt. Das Lösemittel wurde im Vakuum entfernt, der Rückstand mit Wasser verrührt und filtriert. Der Filterrückstand wurde im Vakuum getrocknet und an Kieselgel (30-60 µm) unter Verwendung von anfangs Dichlormethan, dann FM4 zum Eluieren säulenchromatographisch gereinigt. Die geeigneten Eluate wurden eingedampft, der Rückstand mit Diethylether verrieben und abgenutscht. Man erhielt 220 mg (53.5 % der Theorie) an farblosen Kristallen.
IR(KBr): 1668, 1618 $cm^{-1}$ (C=O)
MS: $(M+H)^+$ = 822/824/826 ($Br_2$)

$(M+Na)^+$ = 844/846/848 ($Br_2$)
$(M+K)^+$ = 860/862/864 ($Br_2$)

Entsprechend wurden erhalten:

(1) 1-[3,5-Dibrom-N-[[4-(3,4-dihydro-2(1H)-oxochinazolin-3-yl)-1-piperidinyl]carbonyl]-4-(methylsulfonyloxy)-D-phenylalanyl]-4-[1-(methylsulfonyl)-4-piperidinyl]-piperidin (Lfd. Nr. (523)) in einer Ausbeute von 12 % der Theorie.
$R_f$ 0.54 (FM1)
IR(KBr): 1628, 1665 $cm^{-1}$ (C=O)
ESI-MS: $(M+H)^+$ = 901/903/905 ($Br_2$)

(2) 1-[3,5-Dibrom-N-[[4-(3,4-dihydro-2(1H)-oxochinazolin-3-yl)-1-piperidinyl]carbonyl]-D-tyrosyl]-4-[1-(methylsulfonyl)-4-piperidinyl]-piperidin (Lfd. Nr. (524)) in einer Ausbeute von 12 % der Theorie.
$R_f$ 0.50 (FM1)
ESI-MS: $(M+H)^+$ = 823/825/827 ($Br_2$)

(3) (R,S)-1-[4-[4-(3,4-Dihydro-2(1H)-oxochinazolin-3-yl)-1-piperidinyl]-2-[(1-naphthyl)methyl]-1,4-dioxobutyl]-4-(1-methylsulfonyl-4-piperidinyl)-piperidin (Lfd. Nr. (668)) in einer Ausbeute von 56 % der Theorie.
$R_f$ 0.70 (FM1)
IR(KBr): 1630, 1666 $cm^{-1}$ (C=O)
MS : $M^+$ = 699

Referenzbeispiel 17

1-[4-Amino-3,5-dibrom-N-[[4-[3,4-dihydro-2(1H)-oxochinazalin-3-yl]-1-piperidinyl]carbonyl]-D-phenylalanyl]-4-[1-(3-carboxy-1-oxopropyl)-4-piperidinyl]-piperidin (Lfd. Nr. 487)

Die Mischung aus 0.372 g (0.499 mmol) 1-[4-Amino-3,5-dibrom-N-[[4-(3,4-dihydro-2(1H)-oxochinazolin-3-yl)-1-piperidinyl]carbonyl]-D-phenylalanyl]-4-(4-piperidinyl)-piperidin, 0.11 g (1.1 mmol) Bernsteinsäureanhydrid und 150 ml Tetrahydrofuran wurde 1 Stunde unter Rückfluß gekocht. Das Reaktionsgemisch wurde im Vakuum vom Lösemittel befreit, der Rückstand an Kieselgel (30-60 μm) unter Verwendung von FM1 zum Eluieren säulenchromatographisch gereinigt. Die geeigneten Eluate wurden eingedampft, der Rückstand mit Diethylether verrieben und abgenutscht. Man erhielt 175 mg (41.5 % der Theorie) an farblosen Kristallen.
IR(KBr): 1668, 1608 $cm^{-1}$ (C=O)
MS: $(M+H)^+$ = 842/844/846 ($Br_2$)
$(M+Na)^+$ = 868/870/872 ($Br_2$)

Referenzbeispiel 18

1-[4-Amino-3,5-dibrom-N-[[4-[3,4-dihydro-2(1H)-oxochinazolin-3-yl]-1-piperidinyl]carbonyl]-D-phenylalanyl]-4-(1-hexyl-4-piperidinyl)-piperidin (Lfd. Nr. 488)

Das Gemisch aus 0.372 g (0.499 mmol) 1-[4-Amino-3,5-dibrom-N-[[4-(3,4-dihydro-2(1H)-oxochinazolin-3-yl)-1-piperidinyl]carbonyl]-D-phenylalanyl]-4-(4-piperidinyl)-piperidin, 0.05 g (0.499 mmol) Hexanal, 0.03 g (0.5 mmol) Eisessig und 150 ml Tetrahydrofuran wurde 1 Stunde bei Zimmertemperatur gerührt. Nach Zugabe von 0.116 g ( 0.52 mmol) 95proz. Natriumtriacetoxyborhydrid wurde weitere 2.5 Stunden bei Zimmertemperatur gehalten. Man befreite im Vakuum vom Lösemittel, verteilte den Rückstand zwischen 20proz. wässeriger Natriumcarbonat-Lösung und Dichlormethan, trocknete die organische Phase über Magnesiumsulfat und dampfte sie ein. Der Rückstand wurde an Kiesel-. gel (30 - 60 μm) unter Verwendung von FM4 zum Eluieren säulenchromatographisch gereinigt. Die geeigneten Eluate wurden eingedampft, der Rückstand mit Diethylether verrieben und abgenutscht. Man erhielt 100 mg (24.2 % der Theorie) an farblosen Kristallen.
IR(KBr): 1666, 1620 $cm^{-1}$ (C=O)
MS: $(M+H)^+$ = 828/830/832 ($Br_2$)
$(M+Na)^+$ = 850/852/854 ($Br_2$)

Entsprechend wurden erhalten:

(1) 1-[4-Amino-3,5-dibrom-N-[[4-[3,4-dihydro-2(1H)-oxochinazolin-3-yl]-1-piperidinyl]carbonyl]-D-phenylalanyl]-4-(1-cyclopropylmethyl-4-piperidinyl)-piperidin (Lfd. Nr. (489)) in einer Ausbeute von 23 % der Theorie.
$R_f$ 0.65 (FM1)

IR(KBr): 1622, 1666 $cm^{-1}$ (C=O)
ESI-MS: $(M+H)^+$ = 798/800/802 ($Br_2$)

(2) 1-[4-Amino-3,5-dibrom-N-[[4-[3,4-dihydro-2(1H)-oxochinazolin-3-yl]-1-piperidinyl]carbonyl]-D-phenylalanyl]-4-[1-(ethoxycarbonylmethyl)-4-piperidinyl]-piperidin (Lfd. Nr. (493)) in einer Ausbeute von 43 % der Theorie.
$R_f$ 0.72 (FM1)
IR(KBr): 1620, 1666 $cm^{-1}$ (C=O)
ESI-MS: $(M+H)^+$ = 730/732/734 ($Br_2$)

(3) 1-[3,5-Dibrom-N-[[4-(3,4-dihydro-2(1H)-oxochinazolin-3-yl)-1-piperidinyl]carbonyl]-D-tyrosyl]-4-[1-(cyclopropylmethyl)-4-piperidinyl]-piperidin (Lfd. Nr. (525)) in einer Ausbeute von 46.5 % der Theorie.
$R_f$ 0.50 (FM1)
IR(KBr): 1622, 1662 $cm^{-1}$ (C=O)
ESI-MS: $(M+H)^+$ = 799/801/803 ($Br_2$)

Referenzbeispiel 19

Herstellung von Verbindungen der allgemeinen Formel:

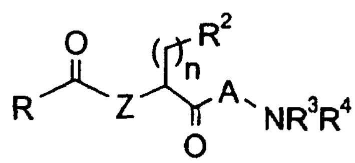

1-[N-[[4-[3,4-Dihydro-2(1H)-oxochinazolin-3-yl]-1-piperidinyl]-carbonyl]-3-ethenyl-D,L-phenylalanyl]-4-(hexahydro-1H-1-azepinyl)-piperidin (Lfd. Nr. 532)

Die Mischung aus 200 mg (3 mMol) 1-[3-Brom-N-[[4-[3,4-dihydro-2(1H)-oxochinazolin-3-yl]-1-piperidinyl]carbonyl]-D,L-phenylalanyl]-4-(hexahydro-1H-1-azepinyl)-piperidin, 108 mg (0.33 mMol) Vinyl-tributylzinn (ALDRICH No. 27143-8), 50 mg Tetrakis-(triphenylphosphin)-palladium (Merck No. 818193), einer Spur 2,6-Di-tert.-butyl-4-methylphenol und 10 ml wasserfreiem Toluol wurde 5 Stunden unter Rückfluß gekocht. Das erkaltete Reaktionsgemisch wurde über ein Aktivkohlefilter filtriert, das Filtrat im Vakuum eingedampft. Der verbliebene Rückstand wurde unter Verwendung von anfangs reinem Dichlormethan, dann von Methanol/konz. Ammoniak (9/1 v/v) zum Eluieren säulenchromatographisch an Kieselgel gereinigt. Die geeigneten Eluate wurden mit tert.-Butyl-methylether gründlich verrieben und abgenutscht. Man erhielt 60 mg (32.6 % der Theorie) an farblosen Kristallen vom $R_f$ 0.25 (FM1).
MS: $M^+$ = 612

Analog wurden hergestellt (jeweils n=1):

| Lfd. Nr. | RCO | Z | $R^2$ | A | $NR^3R^4$ | % Ausbeute | MS | Fließmittel | $R_f$ | IR [$cm^{-1}$] |
|---|---|---|---|---|---|---|---|---|---|---|
| 647 | N66 | $CH_2$ | AS40 | A0 | C8 | 75 | EI: $M^+$ = 611 | FM1 | 0.6 | (KBr): C=O 1639/1668 |
| 648 | N66 | $CH_2$ | AS41 | A0 | C8 | 56 | EI: $M^+$ = 647 | FM1 | 0.7 | (KBr): C=O 1639/1668 |
| 649 | N66 | $CH_2$ | AS42 | A0 | C8 | 8 | EI: $M^+$ = 648 | FM1 | 0.6 | (KBr): C=O 1635/1668 |
| 650 | N66 | $CH_2$ | AS43 | A0 | C8 | 11 | EI: $M^+$ = 654 | FM1 | 0.6 | (KBr): C=O 1635/1666 |
| 651 | N66 | $CH_2$ | AS44 | A0 | C8 | 84 | EI: $M^+$ = 637 | FM1 | 0.6 | (KBr): C=O 1633/1664 |

(fortgesetzt)

| Lfd. Nr. | RCO | Z | $R^2$ | A | $NR^3R^4$ | % Ausbeute | MS | Fließmittel | $R_f$ | IR [$cm^{-1}$] |
|---|---|---|---|---|---|---|---|---|---|---|
| 652 | N66 | $CH_2$ | AS45 | A0 | C8 | 83 | EI: $M^+$ = 613 | FM1 | 0.6 | (KBr): C=O 1637/1667 |

Referenzbeispiel 20

Herstellung von Verbindungen der allgemeinen Formel:

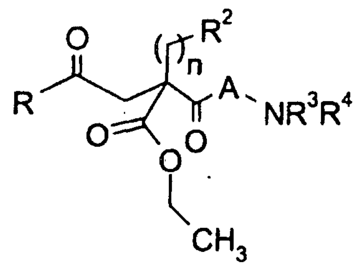

(R,S)-1-[4-[4-(3,4-Dihydro-2(1H)-oxochinazolin-3-yl)-1-piperidinyl]-2-(ethoxycarbonyl)-2-[[1-methyl-1H-indol-3-yl]methyl]-1,4-dioxobutyl]-4-(4-methyl-1-piperazinyl)-piperidin (Lfd. Nr. 599)

Hergestellt analog Beispiel 1 aus (R,S)-4-[4-(3,4-Dihydro-2(1H)-oxochinazolin-3-yl)-1-piperidinyl]-2-(ethoxycarbonyl)-2-[[1-methyl-1H-indol-3-yl]methyl]-4-oxobutansäure, 4-(4-Methyl-1-piperazinyl)piperidin und TBTU in einer Ausbeute von 10 % der Theorie. Farblose, amorphe Substanz vom $R_f$ = 0.2 (Dichlormethan/Methanol/konz. Ammoniak 90/10/1 v/v/v).
IR(KBr): 1722, 1662, 1637 $cm^{-1}$ (C=O)
MS: $M^+$ = 711

Entsprechend erhielt man aus (R,S)-4-[4-(1,3-Dihydro-4-phenyl-2(2H)-oxoimidazol-1-yl)-1-piperidinyl]-2-(ethoxycarbonyl)-2-[[1-methyl-1H-indol-3-yl]methyl]-4-oxobutansäure, 4-(4-Methyl-1-piperazinyl)piperidin und TBTU das (R,S)-1-[4-[4-(Aminocarbonylamino)-1-piperidinyl]-2-(ethoxycarbonyl)-2-[[1-methyl-1H-indol-3-yl]methyl]-1,4-dioxobutyl]-4-(4-methyl-1-piperazinyl)-piperidin (Lfd. Nr. 601) in einer Ausbeute von 20 % der Theorie. Farblose, amorphe Substanz vom $R_f$ = 0.25 (Dichlormethan/Methanol/konz. Ammoniak 90/10/1 v/v/v).
ESI-MS: $(M+H)^+$ = 624
$(M+Na)^+$ = 646
$(M+H+Na)^+$ = 323.8

Referenzbeispiel 21

1-[3,5-Dibrom-N-[[4-(1,3-dihydro-2(2H)-oxobenzimidazol-1-yl)-1-piperidinyl]carbonyl]-D-tyrosyl]-4-(hydroxycarbonyl)-piperidin (Lfd. Nr. 211)

Hergestellt analog Beispiel A38 aus 1-[3,5-Dibrom-N-[[4-(1,3-dihydro-2(2H)-oxobenzimidazol-1-yl)-1-piperidinyl]carbonyl]-Dtyrosyl]-4-(ethoxycarbonyl)-piperidin und wässeriger Lithiumhydroxid-Lösung in einer Ausbeute von 79 % der Theorie. Farblose, amorphe Substanz vom $R_f$ 0.54 (Essigsäureethylester/Methanol/Eisessig 9/1/0.3 v/v/v).
IR(KBr) : 1691.5, 1622.0 $cm^{-1}$ (C=O)
ESI-MS: $(M+H)^6$ = 690/2/4 ($Br_2$)

Referenzbeispiel 22

1-[3,5-Dibrom-N-[[4-(1,3-dihydro-2(2H)-oxobenzimidazol-1-yl)-1-piperidinyl]carbonyl]-D-tyrosyl]-4-(4-piperidinyl)-piperazin (Lfd. Nr. 214)

Hergestellt analog Beispiel A24 aus 3,5-Dibrom-N-[[4-(1,3-dihydro-2(2H)-oxobenzimidazol-1-yl)-1-piperidinyl]carbonyl]-Dtyrosin, 1-(1,1-Dimethylethoxycarbonyl)-4-(1-piperazinyl)piperidin und TBTU sowie anschließende Umsetzung des erhaltenen Zwischenprodukts mit Trifluoressigsäure entsprechend Beispiel A1b) in einer Ausbeute von 4.2 % der Theorie. Farblose, amorphe Substanz vom $R_f$ 0.25 (FM1).
IR(KBr): 1624.0 $cm^{-1}$ (C=O)
ESI-MS: $(M+H)^+$ = 732/4/6 ($Br_2$)

Referenzbeispiel 23

(R)-1-[2-[N-[[4-(1,3-Dihydro-2(2H)-oxobenzimidazol-1-yl)-1-piperidinyl]carbonyl]-N-methylamino]-3-(3,5-dibrom-4-hydroxyphenyl)-propyl]-4-(1-piperidinyl)-piperidin (Lfd. Nr. 219)

a) 1-(Chlorcarbonyl)-4-(1,3-dihydro-2(2H)-oxobenzimidazol-1-yl)-piperidin

Zur Lösung von 3.0 g (13.8 mMol) 4-(1,3-Dihydro-2(2H)-oxobenzimidazol-1-yl)piperidin und 2.7 ml (15 mMol) DIEA in 100 ml Toluol tropfte man unter äußerer Kühlung mit Eiswasser die Lösung von 1.8 ml (14.9 mMol) Diphosgen in 15 ml Toluol und hielt anschließend noch 17 Stunden bei Zimmertemperatur. Der Niederschlag wurde abgenutscht, mit Petrolether gründlich gewaschen und anschließend in 50 ml Dichlormethan gelöst. Die erhaltene Lösung wurde zweimal mit je 50 ml 7proz. wässeriger Natriumhydrogencarbonat-Lösung ausgeschüttelt, über Natriumsulfat getrocknet und im Vakuum eingedampft. Man erhielt 3.0 g (78 % der Theorie) einer farblosen Substanz vom $R_f$ 0.25 (Dichlormethan / Aceton 9/1 v/v), die ohne Reinigung weiterverarbeitet wurde.

b) (R)-1-[3-(3,5-Dibrom-4-hydroxyphenyl)-2-(N-methylamino)-propyl]-4-(1-piperidinyl)-piperidin

Zu der Suspension von 2.3 g (60 mMol) Lithiumaluminiumhydrid in 100 ml wasserfreiem Tetrahydrofuran tropfte man unter Rühren und bei Zimmertemperatur die Lösung von 11.0 g (18.66 mMol) 1-[3,5-Dibrom-N-(1,1-dimethylethoxycarbonyl)-D-tyrosyl]-4-(1-piperidinyl)piperidin in 100 ml wasserfreiem Tetrahydrofuran. Man rührte noch 15 Minuten bei Zimmertemperatur und kochte anschließend 3 Stunden unter Rückfluß. Die erkaltete Mischung wurde mit 3 ml 20proz. wässeriger Ammoniumchlorid-Lösung versetzt, dann mit Magnesiumsulfat getrocknet. Man filtrierte, wusch den Filterkuchen mit insgesamt 300 ml einer Essigsäureethylester-Methanol-Mischung (1/1 v/v) und dampfte die vereinigten Filtrate im Vakuum ein. Der Rückstand wurde an Kieselgel unter Verwendung von Essigsäureethylester / Methanol (8/2 v/v) zum Eluieren säulenchromatographisch gereinigt. Aus den geeigneten Fraktionen isolierte man
1. 2.9 g (31 % der Theorie) einer farblosen Substanz vom $R_f$ 0.13 (Fließmittel: Methanol), die als 1-(3,5-Dibrom-N-methyl-D-tyrosyl)-4-(1-piperidinyl)piperidin identifiziert wurde:
IR(KBr): 1668.3 $cm^{-1}$ (C=O)
MS: $M^+$ = 501/3/5 ($Br_2$)
und
2. 1.8 g (20 % der Theorie) einer farblosen Substanz vom $R_f$ 0.05 (Fließmittel: Methanol), die als die gesuchte Verbindung identifiziert wurde:
ESI-MS: $(M+H)^+$ = 488/490/492 ($Br_2$)
$(M+2H)^{++}$ = 244/245/246.5 ($Br_2$)

c) (R)-1-[2-[N-[[4-(1,3-Dihydro-2(2H)-oxobenzimidazol-1-yl)-1-piperidinyl]carbonyl]-N-methylamino]-3-(3,5-dibrom-4-hydroxyphenyl)-propyl]-4-(1-piperidinyl)-piperidin (Lfd. Nr. 219)

Zu der Mischung von 0.9 g (1.84 mMol) (R)-1-[3-(3,5-Dibrom-4-hydroxyphenyl)-2-(N-methylamino)-propyl]-4-(1-piperidinyl)-piperidin und 0.65 ml (3.7 mMol) DIEA in einem Gemisch aus 50 ml Tetrahydrofuran und 20 ml Dimethylformamid tropfte man die Lösung von 0.57 g (2.02 mMol) 1-(Chlorcarbonyl)-4-(1,3-dihydro-2(2H)-oxobenzimidazol-1-yl)-piperidin in 30 ml Dimethylformamid. Man ließ über Nacht bei Zimmertemperatur rühren und dampfte dann den Ansatz im Vakuum ein. Der Rückstand wurde mit 300 ml eines Tetrahydrofuran-Essigsaureethylester-Gemischs (1/1 v/v) behandelt und die entstandene Lösung mit zweimal je 100 mi einer gesättigten wässerigen Natriumhydrogencarbonat-Lösung ausgeschuttelt, über Natriumsulfat getrocknet und im Vakuum eingedampft. der Rückstand wurde an Kieselgel unter Verwendung von Dichlormethan/ Methanol (8.5/1.5 v/v) zum Eluieren saulenchromatographisch gereinigt. Aus den geeigneten Fraktionen isolierte man 390 mg (29 % der Theorie) einer farblosen Substanz vom $R_f$ 0.46 (Dichlormethan/Cyclohexan/Methanol/konz. Ammoniak 75/15/15/2 v/v/v/v).
IR(KBr): 1695.3, 1624.0 $cm^{-1}$ (C=O)
ESI-MS: $(M+H)^+$ = 731/3/5 ($Br_2$)

Referenzbeispiel 24

1-[3,5-Dibrom-N-[[4-[5-[(4-morpholinyl)carbonyl]-1,3-dihydro-2(2H)-oxobenzimidazol-1-yl]-1-piperidinyl]carbonyl]-D-tyrosyl]-4-(4-pyridinyl)-piperidin (Lfd. Nr. 223)

Zur Lösung von 400 mg (0.5 mMol) 1-[3,5-Dibrom-N-[[4-(1,3-dihydro-5-(hydroxycarbonyl)-2(2H)-oxobenzimidazol-1-yl)-1-piperidinyl]carbonyl]-D-tyrosyl]-4-(4-pyridinyl)-piperidin in 10 ml wasserfreiem Tetrahydrofuran gab man bei Zimmertemperatur 100 mg (0.6 mMol) N,N'-Carbonyldiimidazol, erwärmte anschließend 30 Minuten auf 50 °C und gab dann 90 mg (1 mMol) Morpholin zu. Nach zweistündigem Erwärmen auf 50 bis 60 °C wurde das Lösemittel im Vakuum entfernt und der Rückstand säulenchromatographisch an Kieselgel (30 - 60 µm) unter Verwendung von anfangs Dichlormethan, dann Dichlormethan/Methanol 9/1 (v/v), zuletzt von Dichlormethan / Methanol/konz. Amoniak 9/1/0.2 (v/v/v) als Eluentien gereinigt. Aus den geeigneten Eluaten erhielt man 250 mg (60 % der Theorie) einer amorphen, farblosen Substanz.
IR(KBr): 1712.7, 1625.9 $cm^{-1}$ (C=O)
ESI-MS: $(M+H)^+$ = 838/840/842 ($Br_2$)
$(M+2H)^{++}$ = 419/420/421.5 ($Br_2$)

Entsprechend erhielt man:

Aus 1-[3,5-Dibrom-N-[[4-(1,3-dihydro-5-(hydroxycarbonyl)-2(2H)-oxobenzimidazol-1-yl)-1-piperidinyl]carbonyl]-D-tyrosyl]-4-(4-pyridinyl)-piperidin, 1-Methylpiperazin und N,N'-Carbonyldiimidazol das 1-[3,5-Dibrom-N-[[4-[5-[(4-methyl-1-piperazinyl)-carbonyl]-1,3-dihydro-2(2H)-oxobenzimidazol-1-yl]-1-piperidinyl]carbonyl]-D-tyrosyl]-4-(4-pyridinyl)-piperidin (Lfd. Nr. 224) in einer Ausbeute von 52 % der Theorie. Farblose, amorphe Substanz.
IR(KBr): 1710.8, 1625.9 $cm^{-1}$ (C=O)
ESI-MS: $(M+H)^+$ = 851/853/855 ($Br_2$)
$(M+2H)^{++}$ = 426/427/428 ($Br_2$)

Referenzbeispiel 25

1-[4-Amino-3,5-dibrom-N-[[4-[3,4-dihydro-2(1H)-oxochinazolin-3-yl]-1-piperidinyl]carbonyl]-D-phenylalanyl]-4-[1-(carboxymethyl)-4-piperidinyl]-piperidin (Lfd. Nr. 494)

Hergestellt analog Beispiel A37, jedoch unter Verwendung von Tetrahydrofuran an Stelle von Methanol, aus 1-[4-Amino-3,5-dibrom-N-[[4-[3,4-dihydro-2(1H)-oxochinazolin-3-yl]-1-piperidinyl]carbonyl]-D-phenylalanyl]-4-[1-(ethoxycarbonylmethyl)-4-piperidinyl]-piperidin durch Einwirkung von wässeriger Lithiumhydroxid-Lösung in einer Ausbeute von 51 % der Theorie. Farblose, amorphe Substanz.
ESI-MS: $(M+H)^+$ = 800/802/804 ($Br_2$)
$(M+H)^+$ = 802/804/806 ($Br_2$)
$(M+Na)^+$ = 824/826/828 ($Br_2$)

Referenzbeispiel 26

1-[3,5-Dibrom-N-[[4-(3,4-dihydro-2(1H)-oxochinazolin-3-yl)-1-piperidinyl]carbonyl]-D-tyrosyl]-4-[1-(hydroxycarbonylmethyl)-4-piperidinyl]-piperidin (Lfd. Nr. 526)

Hergestellt analog Beispiel 18 aus 1-[3,5-Dibrom-N-[[4-(3,4-dihydro-2(1H)-oxochinazolin-3-yl)-1-piperidinyl]carbonyl]-Dtyrosyl]-4-(4-piperidinyl)-piperidin, Glyoxylsäureethylester und Natriumtriacetoxyborhydrid sowie anschließende Verseifung des als Zwischenprodukt erhaltenen, aber nicht charakterisierten 1-[3,5-Dibrom-N-[[4-(3,4-dihydro-2(1H)-oxochinazolin-3-yl)-1-piperidinyl]carbonyl]-D-tyrosyl]-4-[1-(ethoxycarbonylmethyl)-4-piperidinyl]-piperidins mit Natronlauge entsprechend Beispiel A55. Man erhielt die farblose, amorphe Substanz in einer Ausbeute von 35 % der Theorie.
IR(KBr): 1625.9 breit $cm^{-1}$ (C=O)
ESI-MS: $(M+H)^+$ = 803/805/807 ($Br_2$)

$(M+Na)^+$ = 825/827/829 ($Br_2$)

Referenzbeispiel 27

1-[4-Amino-N-[(4-amino-1-piperidinyl)carbonyl]-3,5-dibrom-Dphenylalanyl]-4-(1-methyl-4-piperidinyl)-piperidin (Lfd. Nr. 564)

Zu dem Gemisch aus 930 mg (1.48 mMol) 1-[4-Amino-3,5-dibrom-N-[(4-oxo-1-piperidinyl)carbonyl]-D-phenylalanyl]-4-(1-methyl-4-piperidinyl)-piperidin, 1143 mg (14.8 mMol) Ammoniumacetat (Merck No. 1115) und 30 ml wasserfreiem Methanol gab man unter Rühren und bei Zimmertemperatur 653 mg (10.4 mMol) 95proz. Natriumcyanoborhydrid (Aldrich 15.615-9) und rührte anschließend über Nacht. Der Ansatz wurde mit konz. Salzsäure auf einen pH ≤ 2 eingestellt und im Vakuum eingedampft. Der Rückstand wurde in Wasser aufgenommen und mit 40proz. Natronlauge alkalisch gestellt. Man extrahierte erschöpfend mit Dichlormethan, trocknete die vereinigten Extrakte über Natriumsulfat und dampfte sie im Vakuum ein. Der Rückstand wurde an 100 g Kieselgel (Amicon, 35 - 70 µm) unter Verwendung von Dichlormethan/Methanol/konz. Ammoniak (60/40/5 v/v/v) zum Eluieren säulenchromatographisch gereinigt. Aus den geeigneten Fraktionen isolierte man 250 mg (27 % der Theorie) der gesuchten Substanz als farbloses, amorphes Produkt vom $R_f$ 0.15 (Dichlormethan/Methanol/konz. Ammoniak 50/50/0.5 v/v/v).
IR(KBr): 1618 breit $cm^{-1}$ (C=O)
ESI-MS: $(M+H)^+$ = 627/629/631 ($Br_2$)
$(M+Na)^+$ = 649/651/653 ($Br_2$)
$(M+2H)^{++}$ = 314/315/316 ($Br_2$)

Referenzbeispiel 28

(R,S)-1-[4-[4-(3,4-Dihydro-2(1H)-oxochinazolin-3-yl)-1-piperidinyl]-2-[(3,4-dichlorphenyl)methyl]-1,4-dioxobutyl]-4-(hydroxycarbonylmethyl)-piperidin (Lfd. Nr. 596)

Hergestellt analog Beispiel A55 aus (R,S)-1-[4-[4-(3,4-Dihydro-2(1H)-oxochinazolin-3-yl)-1-piperidinyl]-2-[(3,4-dichlorphenyl)methyl]-1,4-dioxobutyl]-4-(ethoxycarbonylmethyl)-piperidin durch Verseifung mit Natronlauge in einer Ausbeute von 86 % der Theorie. Farblose, amorphe Substanz vom $R_f$ 0.76 (Essigsäureethylester/Methanol/Eisessig 70/30/1 v/v/v).
IR(KBr): 1716, 1635 $cm^{-1}$ (C=O)
ESI-MS: $(M+H)^+$ = 613/615/617 ($Cl_2$)
$(M+H)^+$ = 615/617/619 ($Cl_2$)
$(M+Na)^+$ = 637/639/641 ($Cl_2$)

Referenzbeispiel 29

1-[N-[[4-[3,4-Dihydro-2(1H)-oxochinazolin-3-yl]-1-piperidinyl]-carbonyl]-3-(1H-tetrazol-5-yl)-D,L-phenylalanyl]-4-(1-piperidinyl)-piperidin (Lfd. Nr. 632)

Zu der Lösung von 1.6 g (2.68 mMol) 1-[3-Cyan-N-[[4-[3,4-dihydro-2(1H)-oxochinazolin-3-yl]-1-piperidinyl] carbonyl]-D,L-phenylalanyl]-4-(1-piperidinyl)-piperidin in 400 ml Toluol gab man 8.5 g (35 mMol) Tributylzinn(IV)-azid (Synthesis 1976, 330) und kochte die Mischung 4 Tage lang unter Rückfluß. Der nach dem Vertreiben des Lösemittels verbleibende Rückstand wurde mit Essigsäureethylester verrührt, der entstandene Niederschlag abgenutscht und unter Verwendung von FM1 als Eluens an Kieselgel säulenchromatographisch gereinigt. Nach üblicher weiterer Aufarbeitung erhielt man 400 mg (24 % der Theorie) an farblosen Kristallen vom $R_f$ 0.2 (FM1).
IR(KBr): 1653 $cm^{-1}$ (C=O) ESI-MS: $(M+H)^+$ + = 641
$(M+Na)^+$ = 663

Die nachfolgenden Beispiele beschreiben die Herstellung pharmazeutischer Anwendungsformen, die als Wirkstoff eine erfindungsgemäße Verbindung enthalten:

Beispiel I

Kapseln zur Pulverinhalation mit 1 mg Wirkstoff

| Zusammensetzung: | |
|---|---|
| 1 Kapsel zur Pulverinhalation enthält: | |
| Wirkstoff | 1.0 mg |
| Milchzucker | 20.0 mg |
| Hartgelatinekapseln | 50.0 mg |
| | 71.0 mg |

Herstellungsverfahren:

Der Wirkstoff wird auf die für Inhalativa erforderliche Korngröße gemahlen. Der gemahlene Wirkstoff wird mit dem Milchzucker homogen gemischt. Die Mischung wird in Hartgelatinekapseln abgefüllt.

Beispiel II

Inhalationslösung für Respimat® mit 1 mg Wirkstoff

| Zusammensetzung: | |
|---|---|
| 1 Hub enthält: | |
| Wirkstoff | 1.0 mg |
| Benzalkoniumchlorid | 0.002 mg |
| Dinatriumedetat | 0.0075 mg |
| Wasser gereinigt ad | 15.0 µl |

Herstellungsverfahren:

Der Wirkstoff und Benzalkoniumchlorid werden in Wasser gelöst und in Respimat®-Kartuschen abgefüllt.

Beispiel III

Inhalationslösung für Vernebler mit 1 mg Wirkstoff

| Zusammensetzung: | |
|---|---|
| 1 Fläschchen enthält: | |
| Wirkstoff | 0.1 g |
| Natriumchlorid | 0.18 g |
| Benzalkoniumchlorid | 0.002 g |
| Wasser gereinigt ad | 20.0 ml |

Herstellungsverfahren:

Wirkstoff. Natriumchlorid und Benzalkoniumchlorid werden in Wasser gelöst.

Beispiel IV

Treibgas-Dosieraerosol mit 1 mg Wirkstoff

| Zusammensetzung: | |
|---|---|
| 1 Hub enthält: | |
| Wirkstoff | 1.0 mg |
| Lecithin | 0.1 % |
| Treibgas ad | 50.0 µl |

Herstellungsverfahren:

Der mikronisierte Wirkstoff wird in dem Gemisch aus Lecithin und Treibgas homogen suspendiert. Die Suspension wird in einen Druckbehälter mit Dosierventil abgefüllt.

Beispiel V

Nasalspray mit 1 mg Wirkstoff

| Zusammensetzung: | |
|---|---|
| Wirkstoff | 1.0 mg |
| Natriumchlorid | 0.9 mg |
| Benzalkoniumchlorid | 0.025 mg |
| Dinatriumedetat | 0.05 mg |
| Wasser gereinigt ad | 0.1 ml |

Herstellungsverfahren:

Der Wirkstoff und die Hilfsstoffe werden in Wasser gelöst und in ein entsprechendes Behältnis abgefüllt.

Beispiel VI

Injektionslösung mit 5 mg Wirksubstanz pro 5 ml

| Zusammensetzung: | |
|---|---|
| Wirksubstanz | 5 mg |
| Glucose | 250 mg |
| Human-Serum-Albumin | 10 mg |
| Glykofurol | 250 mg |
| Wasser für Injektionszwecke ad | 5 ml |

Herstellung:

Glykofurol und Glucose in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

Beispiel VII

Injektionslösung mit 100 mg Wirksubstanz pro 20 ml

| Zusammensetzung: | |
|---|---|
| Wirksubstanz | 100 mg |
| Monokaliumdihydrogenphosphat = $KH_2PO_4$ | 12 mg |
| Dinatriumhydrogenphosphat = $Na_2HPO_4 \cdot 2H_2O$ | 2 mg |
| Natriumchlorid | 180 mg |
| Human-Serum-Albumin | 50 mg |
| Polysorbat 80 | 20 mg |
| Wasser für Injektionszwecke ad | 20 ml |

Herstellung:

Polysorbat 80, Natriumchlorid, Monokaliumdihydrogenphosphat und Dinatriumhydrogenphosphat in Wasser für Injektionszwecke (WfI) auflösen; Human-Serum-Albumin zugeben; Wirkstoff unter Erwärmen auflösen; mit WfI auf Ansatzvolumen auffüllen; in Ampullen abfüllen.

Beispiel VIII

Lyophilisat mit 10 mg Wirksubstanz

| Zusammensetzung: | |
|---|---|
| Wirksubstanz | 10 mg |
| Mannit | 300 mg |
| Human-Serum-Albumin | 20 mg |

Herstellung:

Mannit in Wasser für Injektionszwecke (WfI) auflösen; Human-Serum-Albumin zugeben; Wirkstoff unter Erwärmen auflösen; mit WfI auf Ansatzvolumen auffüllen; in Vials abfüllen; gefriertrocknen.

| Lösungsmittel für Lyophilisat: | |
|---|---|
| Polysorbat 80 = Tween 80 | 20 mg |
| Mannit | 200 mg |
| Wasser für Injektionszwecke ad | 10 ml |

Herstellung:

Polysorbat 80 und Mannit in Wasser für Injektionszwecke (WfI) auflösen; in Ampullen abfüllen.

Beispiel IX

Tabletten mit 20 mg Wirksubstanz

| Zusammensetzung: | |
|---|---|
| Wirksubstanz | 20 mg |
| Lactose | 120 mg |
| Maisstärke | 40 mg |
| Magnesiumstearat | 2 mg |
| Povidon K 25 | 18 mg |

Herstellung:

Wirksubstanz, Lactose und Maisstärke homogen mischen; mit einer wässerigen Lösung von Povidon granulieren; mit Magnesiumstearat mischen; auf einer Tablettenpresse abpressen; Tablettengewicht 200 mg.

Beispiel X

Kapseln mit 20 mg Wirksubstanz

| Zusammensetzung: | |
|---|---|
| Wirksubstanz | 20 mg |
| Maisstärke | 80 mg |
| Kieselsäure. hochdispers | 5 mg |
| Magnesiumstearat | 2.5 mg |

Herstellung:

Wirksubstanz, Maisstärke und Kieselsäure homogen mischen; mit Magnesiumstearat mischen; Mischung auf einer Kapselfüllmaschine in Hartgelatine-Kapseln Grösse 3 abfüllen.

Beispiel XI

Zäpfchen mit 50 mg Wirksubstanz

| Zusammensetzung: | |
|---|---|
| Wirksubstanz | 50 mg |
| Hartfett (Adeps solidus) q.s. ad | 1700 mg |

Herstellung:

Hartfett bei ca. 38°C aufschmelzen; gemahlene Wirksubstanz im geschmolzenen Hartfett homogen dispergieren; nach Abkühlen auf ca. 35°C in vorgekühlte Formen ausgiessen.

Beispiel XII

Wäßrige Lösung für die nasale Applikation mit 10 mg Wirksubstanz

| Zusammensetzung: | |
|---|---|
| Wirksubstanz | 10.0 mg |

(fortgesetzt)

| Zusammensetzung: | |
|---|---|
| Salzsäure in der zur Bildung eines neutralen Salzes erforderlichen Menge<br>Parahydroxybenzoesäuremethylester (PHB) | 0.01 mg |
| Parahydroxybenzoesäurepropylester (PHB) | 0.005 mg |
| Wasser gereinigt ad | 1.0 ml |

Herstellung:

Der Wirkstoff wird in gereinigtem Wasser aufgelöst; Salsäure wird zugegeben, bis die Lösung klar wird; PHB-Methyl- und Propylester werden zugegeben; die Lösung wird mit gereinigtem Wasser auf Ansatzvolumen aufgefüllt; die Lösung wird sterilfiltriert und in ein entsprechendes Behältnis abgefüllt.

Beispiel XIII

Wäßrige Lösung für die nasale Applikation mit 5 mg Wirksubstanz

| Zusammensetzung: | |
|---|---|
| Wirksubstanz | 5 mg |
| 1,2-Propandiol | 300 mg |
| Hydroxyethylcellulose | 5 mg |
| Sorbinsäure | 1 mg |
| Wasser gereinigt ad | 1 ml |

Herstellung:

Der Wirkstoff wird in 1,2-Propandiol gelöst; eine Hydroxyethylcellulose-Lösung in gereinigtem Wasser enthaltend Sorbinsäure wird hergestellt und zur Wirkstoff-Lösung gegeben; die Lösung wird sterilfiltriert und in ein entsprechendes Behältnis abgefüllt.

Beispiel XIV

Wäßrige Lösung für die intravenöse Applikation mit 5 mg Wirksubstanz

| Zusammensetzung: | |
|---|---|
| Wirksubstanz | 5 mg |
| 1,2-Propandiol | 300 mg |
| Mannit | 50 mg |
| Wasser für Injektionszwecke (Wfl) ad | 1 ml |

Herstellung:

Der Wirkstoff wird in 1,2-Propandiol gelöst; die Lösung wird mit Wfl auf annähernd Ansatzvolumen aufgefüllt; das Mannit wird zugegeben und mit Wfl auf Ansatzvolumen aufgefüllt; die Lösung wird sterilfiltriert, in Einzelbehältnisse abgefüllt und autoklaviert.

Beispiel XV

Liposomale Formulierung für die intravenöse Injektion mit 7.5 mg Wirksubstanz

| Zusammensetzung: | |
|---|---|
| Wirksubstanz | 7.5 mg |
| Ei-lecithin, z.B. Lipoid E 80 | 100.0 mg |
| Cholesterol | 50.0 mg |
| Glycerin | 50.0 mg |
| Wasser für Injektionszwecke ad | 1.0 ml |

Herstellung:

Der Wirkstoff wird in einer Mischung aus Lecithin und Cholesterol gelöst; die Lösung wird zu einer Mischung aus Glycerin und Wfl gegeben und mittels Hochdruck-Homogenisation oder Microfluidizer-Technik homogenisiert; die so erhaltene liposomale Formulierung wird uncer aseptischen Bedingungen in ein entsprechendes Behältnis abgefüllt.

Beispiel XVI

Suspension für die nasale Applikation mit 20 mg Wirksubstanz

| Zusammensetzung: | |
|---|---|
| Wirksubstanz | 20.0 mg |
| Carboxymethylcellulose (CMC) | 20.0 mg |
| Natriummonohydrogenphosphat/Natriumdihydrogenphosphat-Puffer pH 6.8 | q.s. |
| Natriumchlorid | 8.0 mg |
| Parahydroxybenzoesäuremethylester | 0.01 mg |
| Parahydroxybenzoesäurepropylester | 0.003 mg |
| Wasser gereinigt ad | 1.0 ml |

Herstellung :

Der Wirkstoff wird in einer wässrigen CMC-Lösung suspendiert; die anderen Bestandteile werden nacheinander zur Suspension gegeben und die Suspension mit gereinigtem Wasser auf Ansatzvolumen aufgefüllt.

Beispiel XVII

Wässrige Lösung für die subcutane Applikation mit 10 mg Wirksubstanz

| Zusammensetzung: | |
|---|---|
| Wirksubstanz | 10.0 mg |
| Natriummonohydrogenphosphat/Natriumdihydrogenphosphat-Puffer q.s. ad pH | 7.0 |
| Natriumchlorid | 4.0 mg |
| Wasser für Injektionszwecke ad | 0.5 ml |

Herstellung:

Der Wirkstoff wird in der Phosphatpufferlösung gelöst, nach Zugabe des Kochsalz wird mit Wasser auf Ansatzvolumen aufgefüllt. Die Lösung wird sterilfiltriert und nach Abfüllung in ein entsprechendes Behältnis autoklaviert.

Beispiel XVIII

Wässrige Suspension für die subcutane Applikation mit 5 mg Wirksubstanz

| Zusammensetzung: | |
|---|---|
| Wirksubstanz | 5.0 mg |
| Polysorbat 80 | 0.5 mg |
| Wasser für Injektionszwecke | 0.5 ml |

Herstellung:

Der Wirkstoff wird in der Polysorbat 80-Lösung suspendiert und mittels geeigneter Dispiergiertechnik (z.B. Naßmahlung, Hochdruckhomogenisation, Mikrofluidisierung etc.) auf eine Teilchengröße von ca. 1 µm zerkleinert. Die Suspension wird unter aseptischen Bedingungen in ein entsprechendes Behältnis abgefüllt.

**Patentansprüche**

1. Die folgenden Verbindungen:

   (1) 1-[$N^2$-[3,5-Dibrom-N-[[4-(3,4-dihydro-2(1H)-oxochinazolin-3-yl)-1-piperidinyl]carbonyl]-D-tyrosyl]-L-lysyl]-4-(4-pyridinyl)-piperazin und

   (2) 1-[4-Amino-3,5-dibrom-N-[[4-(2,3,4,5-tetrahydro-2(1H)-oxo-1,3-benzodiazepin-3-yl)-1-piperidinyl]carbonyl]-D-phenylalanyl]-4-(1-piperidinyl)-piperidin,

   deren Tautomere, deren Diastereomere, deren Enantiomere und deren Salze.

2. Physiologisch verträgliche Salze der Verbindungen nach Anspruch 1 mit anorganischen oder organischen Säuren oder Basen.

3. Arzneimittel, enthaltend eine Verbindung gemäß Anspruch 1 oder ein physiologisch verträgliches Salz gemäß Anspruch 2 neben gegebenenfalls einem oder mehreren inerten Trägerstoffen und/oder Verdünnungsmitteln.

4. Verwendung einer Verbindung nach einem der Ansprüche 1 oder 2 zur Herstellung eines Arzneimittels, das zur akuten und prophylaktischen Behandlung von Kopfschmerzen, zur Behandlung des nicht-insulinabhängigen Diabetes mellitus, von cardiovaskulären Erkrankungen, Erkrankungen der Haut, von entzündlichen Erkrankungen, der allergischen Rhinitis, von Asthma, von Erkrankungen, die mit einer überschießenden Gefäßerweiterung und dadurch bedingter verringerter Gewebedurchblutung einhergehen, z.B. Schock und Sepsis, und der Morphintoleranz geeignet ist.

5. Verfahren zur Herstellung eines Arzneimittels gemäß Anspruch 3, **dadurch gekennzeichnet, daß** auf nichtchemischem Wege eine Verbindung nach einem der Ansprüche 1 oder 2 in einen oder mehrere inerte Trägerstoffe und/oder Verdünnungsmittel eingearbeitet wird.

6. Verwendung der Verbindungen gemäß den Ansprüchen 1 oder 2 zur Erzeugung und Reinigung von Antikörpern.

7. Verwendung der markierten Verbindungen gemäß den Ansprüchen 1 oder 2 in RIA- und ELISA-Assays und als diagnostische oder analytische Hilfsmittel in der Neurotransmitter-Forschung.

## Claims

1. The following compounds:

 (1) 1-[$N^2$-[3,5-dibromo-N-[[4-(3,4-dihydro-2(1H)-oxoquinazolin-3-yl)-1-piperidinyl]carbonyl]-D-tyrosyl]-L-lysyl]-4-(4-pyridinyl)-piperazine and

 (2) 1-[4-amino-3,5-dibromo-N-[[4-(2,3,4,5-tetrahydro-2(1H)-oxo-1,3-benzodiazepin-3-yl)-1-piperidinyl]carbonyl]-D-phenylalanyl]-4-(1-piperidinyl)-piperidine,

 the tautomers, the diastereomers, the enantiomers and the salts thereof.

2. Physiologically acceptable salts of the compounds according to claim 1 with inorganic or organic acids or bases.

3. Pharmaceutical compositions containing a compound according to claim 1 or a physiologically acceptable salt according to claim 2 optionally together with one or more inert carriers and/or diluents.

4. Use of a compound according to one of claims 1 or 2 for the preparation of a pharmaceutical composition which is suitable for the acute and prophylactic treatment of headaches, for treating non-insulin-dependent diabetes mellitus, cardiovascular disorders, skin diseases, inflammatory diseases, allergic rhinitis, asthma, diseases accompanied by excessive vasodilatation and consequently reduced blood circulation, e.g. shock and sepsis, and morphine tolerance.

5. Process for preparing a pharmaceutical composition according to claim 3, **characterised in that** a compound according to one of claims 1 or 2 is incorporated in one or more inert carriers and/or diluents by a non-chemical method.

6. Use of the compounds according to claims 1 or 2 for the production and purification of antibodies.

7. Use of the labelled compounds according to claims 1 or 2 in RIA and ELISA assays and as diagnostic or analytical aids in neurotransmitter research.

## Revendications

1. Composés suivants :

 (1) 1-[$N^2$-[3,5-dibromo-N-[[4-(3,4-dihydro-2(1H)-oxoquinazolin-3-yl)-1-pipéridinyl]carbonyl]-D-tyrosyl]-L-lysyl]-4-(4-pyridinyl)-pipérazine et
 (2) 1-[4-amino-3,5-dibromo-N-[[4-(2,3,4,5-tétrahydro-2(1H)-oxo-1,3-benzodiazépin-3-yl)-1-pipéridinyl]-carbonyl]-D-phénylalanyl]-4-(1-pipéridinyl)-pipéridine,

 leurs tautomères, leurs diastéréoisomères, leurs énantiomères et leurs sels.

2. Sels physiologiquement acceptables des composés selon la revendication 1 avec des acides ou des bases inorganiques ou organiques.

3. Médicament contenant un composé selon la revendication 1 ou un sel physiologiquement acceptable selon la revendication 2, outre éventuellement un ou plusieurs supports et/ou diluants inertes.

4. Utilisation d'un composé selon l'une des revendications 1 et 2 pour la production d'un médicament qui convient pour le traitement aigu et prophylactique des maux de tête, pour le traitement du diabète sucré non insuline-dépendant, des maladies cardiovasculaires, des maladies de la peau, des maladies inflammatoires, de la rhinite allergique, de l'asthme, des maladies qui s'accompagnent d'un élargissement excessif des vaisseaux et d'une irrigation sanguine réduite des tissus qui en résulte, par exemple le choc et la septicémie, et de la tolérance à la morphine.

5. Procédé de production d'un médicament selon la revendication 3 **caractérisé en ce que**, par voie non chimique,

un composé selon l'une des revendications 1 et 2 est incorporé dans un ou plusieurs supports et/ou diluants inertes.

**6.** Utilisation des composés selon les revendications 1 et 2 pour la production et la purification d'anticorps.

**7.** Utilisation des composés marqués selon les revendications 1 et 2 dans des analyses RIA et ELISA et comme auxiliaires diagnostiques ou analytiques dans la recherche de neurotransmitteurs.